(19) Europäisches Patentamt
European Patent Office
Office européen des brevets

(11) EP 3 915 986 A1

(12) EUROPEAN PATENT APPLICATION
published in accordance with Art. 153(4) EPC

(43) Date of publication:
**01.12.2021 Bulletin 2021/48**

(21) Application number: **20768933.2**

(22) Date of filing: **13.03.2020**

(51) Int Cl.:
*C07D 471/04* (2006.01) *A61K 31/437* (2006.01)
*A61K 31/519* (2006.01) *C07D 487/04* (2006.01)
*A61P 35/00* (2006.01) *A23L 29/00* (2016.01)
*A23L 33/10* (2016.01)

(86) International application number:
**PCT/KR2020/003558**

(87) International publication number:
**WO 2020/185044 (17.09.2020 Gazette 2020/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.03.2019 KR 20190028917**
**27.03.2019 KR 20190035391**

(71) Applicants:
- **Voronoi Inc.**
  **Incheon 21984 (KR)**
- **Voronoibio Inc.**
  **Incheon 21984 (KR)**

(72) Inventors:
- **KIM, U Bin**
  **Incheon 21982 (KR)**
- **LEE, Youn Ho**
  **Incheon 21996 (KR)**
- **KANG, Se In**
  **Incheon 21966 (KR)**
- **KANG, Ju Hee**
  **Uiwang-si Gyeonggi-do 16103 (KR)**
- **HWANG, Seon Ah**
  **Incheon 22008 (KR)**
- **KIM, Da Mi**
  **Seoul 05555 (KR)**
- **KIM, Seung Su**
  **Incheon 21667 (KR)**
- **JUNG, Myung Ho**
  **Hwaseong-si, Gyeonggi-do 18238 (KR)**
- **KIM, Hyun Kyung**
  **Incheon 21982 (KR)**
- **JUNG, Hong Ryul**
  **Incheon 21986 (KR)**
- **KIM, Yeon Sil**
  **Incheon 21510 (KR)**
- **JANG, Hye Jin**
  **Hwaseong-si, Gyeonggi-do 18238 (KR)**
- **CHOI, Ji Eun**
  **Seoul 07651 (KR)**
- **LEE, Sun Hwa**
  **Incheon 21982 (KR)**
- **SON, Jung Beom**
  **Incheon 21986 (KR)**
- **KIM, Nam Doo**
  **Incheon 22008 (KR)**

(74) Representative: **Plasseraud IP**
**66, rue de la Chaussée d'Antin**
**75440 Paris Cedex 09 (FR)**

# (54) HETEROARYL DERIVATIVES AND PHARMACEUTICAL COMPOSITION COMPRISING SAME AS ACTIVE INGREDIENT

(57) The present invention relates to a heteroaryl derivative and a pharmaceutical composition for the prevention or treatment of cancer comprising the same as an active ingredient, and a compound according to an aspect of the present invention, a stereoisomer thereof, a hydrate thereof, or a pharmaceutically acceptable salt thereof suppresses kinases, particularly, a TTK kinase, and thus can remarkably suppress the proliferation of cancer cells, and accordingly, can be effectively used as a pharmaceutical composition for the prevention or treatment of cancer.



Printed by Jouve, 75001 PARIS (FR)

## Description

[Technical Field]

The present invention relates to a heteroaryl derivative and a pharmaceutical composition for preventing or treating cancer including the same as an active ingredient.

[Background Art]

Among therapeutic agents used for cancer treatment, there are taxanes and vinca alkaloids that act on microtubules to stabilize or destabilize the microtubules' role. They disturb the action of normal mitotic spindles to prevent the correct attachment of a chromosome and induce mitotic arrest. This arrest is forced by a spindle assembly checkpoint, and prevents the separation of sister chromatids to form two daughter cells. Prolonged mitotic arrest forces cells into mitotic exit without any cytokinesis, or drives the cells into mitotic catastrophe, which leads to apoptosis. Although mitosis-inducing agents have been widely used to treat solid tumors, there is a need for development of a new pharmaceutical composition for treatment of cancer due to the side effects associated with such preparations and the resistance of many types of tumors to the current therapeutic methods. The role of genes involved in the spindle assembly checkpoint during normal development, and the potential role of the genes in diseases such as cancer have been extensively studied. Many components are phosphorylated during mitosis. Among these components, there are some kinases. In this case, one of the kinases is a dual-specificity kinase (i.e., a tyrosine threonine kinase (TTK)).

TTK expression is associated with highly proliferating cells and tissues because TTK is overexpressed as observed in many cancer cell lines and tumor types. In some species, the silencing of TTK prevents the cells from undergoing mitotic arrest in response to spindle toxins, which points out the essential function of TTK in spindle assembly signaling.

Meanwhile, Mps-1 (TTK) is a dual-specificity Ser/Thr kinase that plays an important role in the activation of a mitotic checkpoint (also known as a "spindle checkpoint" or "spindle assembly checkpoint"), thereby ensuring proper chromosome segregation during mitosis (Abrieu A et al., Cell, 2001, 106, 83-93). Every dividing cell has to ensure equal separation of the replicated chromosomes into two daughter cells. Upon entry into mitosis, chromosomes are attached at their kinetochores to the microtubules of the spindle apparatus. The mitotic checkpoint is a surveillance mechanism that is active as long as unattached kinetochores are present and prevents mitotic cells from entering anaphase and thereby completing cell division with unattached chromosomes. Once all the kinetochores are attached in a correct amphitelic, i.e., bipolar, fashion with the mitotic spindle, the checkpoint is satisfied and the cell enters anaphase and mitosis proceeds. The mitotic checkpoint consists of a complex network of a number of essential proteins, including members of the MAD (mitotic arrest deficient, MAD 1-3) and Bub (Budding uninhibited by benzimidazole, Bub 1-3) families, the motor protein CENP-E, the Mps-1 kinase as well as other components, many of these being over-expressed in proliferating cells (e.g., cancer cells) and tissues

Therefore, mitotic checkpoint abrogation through pharmacological inhibition of the Mps-1 kinase or other components of the mitotic checkpoint suggests a new approach for treatment of proliferative disorders including solid tumors such as carcinomas and sarcomas and leukaemia and lymphoid malignancies, or other disorders associated with uncontrolled cell proliferation.

[Disclosure]

[Technical Problem]

According to one aspect of the present invention, the present invention is directed to providing a compound, which exhibits a kinase inhibitor effect, particularly a TTK kinase inhibitor effect, and thus can be used to prevent or treat kinase-related diseases or cancer, or a stereoisomer, a hydrate thereof, or a pharmaceutically acceptable salt thereof.

According to another aspect of the present invention, the present invention is directed to providing a pharmaceutical composition preventing or treating a kinase-related disease, which contains the compound, or the stereoisomer thereof, the hydrate thereof, or the pharmaceutically acceptable salt thereof as an active ingredient.

According to still another aspect of the present invention, the present invention is directed to providing a pharmaceutical composition for preventing or treating cancer, which contains the compound, or the stereoisomer thereof, the hydrate thereof, or the pharmaceutically acceptable salt thereof as an active ingredient.

According to yet another aspect of the present invention, the present invention is directed to providing a method of treating cancer, which includes administering the compound, or the stereoisomer thereof, the hydrate thereof, or the pharmaceutically acceptable salt thereof to an individual or a subject in need thereof.

According to yet another aspect of the present invention, the present invention is directed to providing the

compound, or the stereoisomer thereof, the hydrate thereof, or the pharmaceutically acceptable salt thereof for use in the treatment of cancer.

According to yet another aspect of the present invention, the present invention is directed to providing a use of the compound, or the stereoisomer thereof, the hydrate thereof, or the pharmaceutically acceptable salt thereof for use in the preparation of drugs for treating cancer.

[Technical Solution]

According to one aspect of the present invention, there is provided a compound represented by the following Formula 1, or a stereoisomer thereof, a hydrate thereof, or a pharmaceutically acceptable salt thereof:

[Formula 1]

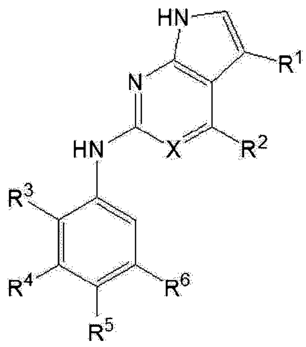

(wherein,

X is CH or N;
$R^1$ is -H, a halogen, a cyano, or a haloalkyl;
$R^2$ is a $C_{3-10}$ cycloalkyl, a $C_{3-10}$ cycloalkenyl, -$NHA^1$, or -$OA^2$, wheren $A^1$ is a $C_{1-10}$ linear or branched alkyl, a $C_{3-10}$ cycloalkyl, or a 3- to 9-membered heterocycloalkyl including one or more heteroatoms selected from the group consisting of N, O, and S, wherein the alkyl, the cycloalkyl, and the heterocycloalkyl is each independently unsubstituted or substituted with one or more non-hydrogen substituents selected from the group consisting of a halogen, a $C_{1-5}$ linear or branched alkyl, a $C_{3-10}$ cycloalkyl, a $C_{1-4}$ linear or branched alkylsulfonyl, a $C_{1-4}$ alkylaminosulfonyl, and a $C_{1-5}$ linear or branched alkoxy, and $A^2$ is a $C_{3-10}$ cycloalkyl, wherein the cycloalkyl is unsubstituted or substituted with one or more non-hydrogen substituents selected from a $C_{1-3}$ linear or branched alkyl and hydroxy,
$R^3$ is -H, a $C_{1-6}$ linear or branched alkoxy, or acrylamide, wherein the $C_{1-6}$ linear or branched alkoxy is unsubstituted or substituted with a haloalkyl, and $R^4$ is -H or a $C_{1-6}$ linear or branched alkoxy, or
$R^3$ and $R^4$, together with a benzene ring containing carbon atoms to which they are bonded, form a 9- to 10-membered bicyclic ring including one or more heteroatoms selected from the group consisting of N, O, and S,
$R^5$ is -H, a $C_{1-6}$ alkylaminocarbonyl, unsubstituted or substituted phenyl, oxooxazolidinonyl, dioxidothiazolidinyl, oxopyrrolidinyl, dioxidothiazinanyl, oxomorpholinyl, or a heteroaryl selected from the group consisting of pyrazolyl, triazolyl, thiazolyl, oxazolyl, pyridinyl, and imidazolyl, wherein the heteroaryl may be unsubstituted or substituted with one or more non-hydrogen substituents selected from the group consisting of a $C_{1-5}$ alkyl, a halogen, and a 3- to 7-membered heterocycloalkyl including one or more heteroatoms selected from the group consisting of N, O, and S, or may be fused with a $C_{3-10}$ cycloalkyl to form a bicyclic ring, and the substituted phenyl is substituted with hydroxy, or sequentially substituted with a $C_{1-5}$ alkyl, a 3- to 7-membered heterocycloalkyl including one or more heteroatoms selected from the group consisting of N, O, and S, and a $C_{3-10}$ cycloalkyl or alkylcarbonyl,
$R^6$ is -H, a halogen, or a $C_{1-10}$ linear or branched alkyl).

According to another aspect of the present invention, there is provided a pharmaceutical composition for preventing or treating diseases associated with one or more protein kinases selected from the group consisting of TTK, JAK2, SNARK, YSK4, PRKCE, CAMKK1, JNK3, TYK2, RSK2, CAMKK2, ULK3, ULK1, RSK4, TRKB, AAK1, GAK, SBK1, TYK2, CAMK2D, MAP3K2, KIT, FLT3, LRRK2, CSNK1D, CSNK1E, MEK4, RIOK1, DYRK1B, PKN2, SRPK3, JNK1, LRRK2, JNK3, LRRK2, FLT3, JNK2, RIPK5, MEK3, ABL1, MAPKAPK2, GRK4, and SRPK3, which contains the compound, or the stereoisomer thereof, the hydrate thereof, or the pharmaceutically acceptable salt thereof as an active ingredient.

According to still another aspect of the present invention, there is provided a pharmaceutical composition for preventing or treating cancer, which contains the compound, or the stereoisomer thereof, the hydrate thereof, or the pharmaceutically acceptable salt thereof as an active ingredient.

According to yet another aspect of the present invention, there is provided a method of treating cancer, which includes administering the compound, or the stereoisomer thereof, the hydrate thereof, or the pharmaceutically acceptable salt thereof to an individual or a subject in need thereof.

According to yet another aspect of the present invention, there is provided the compound, or the stereoisomer thereof, the hydrate thereof, or the pharmaceutically acceptable salt thereof for use in the treatment of cancer.

According to yet another aspect of the present invention, there is provided a use of the compound, or the stereoisomer thereof, the hydrate thereof, or the pharmaceutically acceptable salt thereof for use in the preparation of drugs for treating cancer.

[Advantageous Effects]

A compound provided according to an aspect of the present invention, or a stereoisomer thereof, a hydrate thereof, or a pharmaceutically acceptable salt thereof can inhibit kinases (particularly TTK kinases) to remarkably inhibit the proliferation of cancer cells, and thus can be effectively used as a pharmaceutical composition for preventing or treating cancer.

[Best Mode]

Hereinafter, the present invention will be described in detail.

Meanwhile, it should be understood that exemplary embodiments of the present invention may be implemented in various forms, and are not intended to limit the scope of the present invention. Also, the exemplary embodiments of the present invention are provided to more completely describe the present invention to those having ordinary skill in the art. Throughout the specification of the present invention, when an element is said to "include" another element, this also means that it may further include other elements, not to exclude other elements unless specifically stated otherwise.

In this specification, the term "halogen" may be fluoro, chloro, bromo, or iodo.

In this specification, the term "haloalkyl" may refer to a linear or branched alkyl (a hydrocarbon) having carbon atoms substituted with one or more halogen atoms. Examples of the haloalkyl include methyl, ethyl, propyl, isopropyl, isobutyl, and *N*-butyl, which are independently substituted with one or more halogen atoms, for example F, Cl, Br, and I, but the present invention is not limited thereto.

In this specification, the term "alkyl" may refer to a linear or branched acyclic saturated hydrocarbon consisting of carbon atoms. A representative -($C_{1-8}$ alkyl) includes - methyl, -ethyl, -*N*-propyl, -*N*-butyl, -*N*-pentyl, -N-hexyl, -*N*-heptyl, and -N-octyl; a branched chain saturated alkyl may include -isopropyl, -secondary (sec)-butyl, -isobutyl, -tertiary (tert)-butyl, -isopentyl, 2-methylpentyl, 3-methylpentyl, 4-methylpentyl, 2,3-dimethylbutyl, and the like. The -($C_{1-8}$ alkyl) may also be substituted or unsubstituted. For example, a $C_{1-8}$ alkyl group may be substituted with phenyl to form a benzyl group.

In this specification, the term "cycloalkyl" may refer to a non-aromatic, saturated or unsaturated carbocycle. A representative cycloalkyl includes cyclopropyl, cyclobutyl, cyclopentyl, cyclopentadienyl, cyclohexyl, cyclohexenyl, 1,3-cyclohexadienyl, 1,4-cyclohexadienyl, cycloheptyl, 1,3-cycloheptadienyl, 1,3,5-cycloheptatrienyl, cyclooctyl, and cyclooctadienyl, but the present invention is not limited thereto. The cycloalkyl group may be substituted or unsubstituted. According to one embodiment, this cycloalkyl group may be a $C_{3-8}$ cycloalkyl group. A $C_7$ or more cycloalkyl group may have two or more cyclic structures, and one specific example of the $C_7$ or more cycloalkyl group may be bicycloalkyl group. More specifically, bicycloheptane may be used in the present invention.

In this specification, the term "aryl" may refer to any functional group or substituent derived by removing one hydrogen atom from an aromatic hydrocarbon ring. The aryl group may be a monocyclic aryl group or a polycyclic aryl group. The aryl group may have 5 or more and 30 or less, 5 or more and 20 or less, or 5 or more and 15 or less ring-forming carbon atoms. Examples of the aryl group may include a phenyl group, a naphthyl group, a fluorenyl group, an anthracenyl group, a phenanthryl group, a biphenyl group, a terphenyl group, a quaterphenyl group, a quinquephenyl group, a sexiphenyl group, a triphenylene group, a pyrenyl group, a benzofluoranthenyl group, a chrycenyl group, and the like, but the present invention is not limited thereto.

In this specification, the term "heteroaryl" may refer to an aryl cyclic group including one or more selected from O, N, P, Si, and S as a heteroatom. The heteroaryl group may have 2 or more and 30 or less, or 2 or more and 20 or less ring-forming carbon atoms. The heteroaryl may be a monocyclic heteroaryl or a polycyclic heteroaryl. The polycyclic heteroaryl may, for example, have a bicyclic or tricyclic structure. Examples of the heteroaryl may include thienyl, thiophene, furyl, pyrrolyl, pyrazolyl, imidazolyl, thiazolyl, oxazolyl, isothiazolyl, oxadiazolyl, triazolyl, pyridinyl, bipyridyl, pyrimidyl, triazinyl, triazolyl, an acrydyl group, a pyridazinyl group, pyrazinyl, quinolinyl, quinazoline, quinoxalinyl, phenoxazyl, phthalazinyl, pyrimidinyl, pyrido-pyrimidinyl, pyrido-pyrazinyl, pyrazino-pyrazinyl, isoquinoline, indole, carbazole, imidazopyridazinyl, imidazopyridinyl, imidazopyrimidinyl, pyrazolopyrimidinyl, imidazopyrazinyl or pyrazolopyridinyl, N-arylcarbazole, *N*-heteroarylcarbazole, an *N*-alkylcarbazole group, benzoxazole, benzoimidazole, benzothiazole, benzocarbazole, benzothiophene, dibenzothiophenyl, thienothiophene, benzofuranyl, phenanthroline, isooxazolyl, oxadiazolyl, thiadiazolyl, benzothiazolyl, tetrazolyl, phenothiazinyl, dibenzosilole, dibenzofuranyl, and the like, but the present invention is not limited thereto. According to one embodiment of the present invention, the heteroaryl may also include a bicyclic heterocycloaryl including an aryl ring fused to a heterocycloalkyl ring or a heteroaryl fused to a cycloalkyl ring.

One or more of the above-described homogeneous or heterogeneous substituents may be substituted at the same or different positions, and may also be substituted sequentially. The expression "sequentially" means that a residue is substituted with one substituent in the formula, and the substituent is then continuously substituted with another substituent. For example, when a residue is substituted with an alkyl group, the alkyl group is substituted with a cycloalkyl group, and the cycloalkyl group is sequentially substituted with a carbonyl group, this may be named "carbonyl cycloalkyl alkyl," which indicates that the residue is sequentially substituted with the substituents.

One aspect of the present invention provides a compound represented by the following Formula 1, or a stereoisomer thereof, a hydrate thereof, or a pharmaceutically acceptable salt thereof:

[Formula 1]

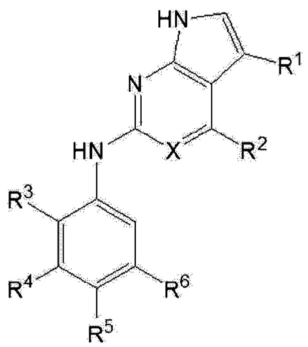

(wherein,

X is CH or N;

$R^1$ is -H, a halogen, a cyano, or a haloalkyl;

$R^2$ is a $C_{3-10}$ cycloalkyl, a $C_{3-10}$ cycloalkenyl, $-NHA^1$, or $-OA^2$,

wherein $A^1$ is a $C_{1-10}$ linear or branched alkyl, a $C_{3-10}$ cycloalkyl, or a 3- to 9-membered heterocycloalkyl including one or more heteroatoms selected from the group consisting of N, O, and S, wherein the alkyl, the cycloalkyl, and the heterocycloalkyl are each independently unsubstituted or substituted with one or more non-hydrogen substituents selected from the group consisting of a halogen, a $C_{1-5}$ linear or branched alkyl, a $C_{3-10}$ cycloalkyl, a $C_{1-4}$ linear or branched alkylsulfonyl, a $C_{1-4}$ alkylaminosulfonyl, and a $C_{1-5}$ linear or branched alkoxy,

$A^2$ is a $C_{3-10}$ cycloalkyl, wherein the cycloalkyl is unsubstituted or substituted with one or more non-hydrogen substituents selected from a $C_{1-3}$ linear or branched alkyl and hydroxyl;

$R^3$ is -H, a $C_{1-6}$ linear or branched alkoxy, or acrylamide, wherein the $C_{1-6}$ linear or branched alkoxy is unsubstituted or substituted with a haloalkyl, and $R^4$ is -H or a $C_{1-6}$ linear or branched alkoxy, or

$R^3$ and $R^4$, together with a benzene ring containing carbon atoms to which they are bonded, form a 9- to 10-membered bicyclic ring including one or more heteroatoms selected from the group consisting of N, O and S;

$R^5$ is -H, a $C_{1-6}$ alkylaminocarbonyl, unsubstituted or substituted phenyl, oxooxazolidinonyl, dioxidothiazolidinyl, oxopyrrolidinyl, dioxidothiazinanyl, oxomorpholinyl, or a heteroaryl selected from the group consisting of pyrazolyl, triazolyl, thiazolyl, oxazolyl, pyridinyl, and imidazolyl, wherein the heteroaryl may be unsubstituted or substituted with one or more non-hydrogen substituents selected from the group consisting of a $C_{1-5}$ alkyl, a halogen, and a 3- to 7-membered heterocycloalkyl including one or more heteroatoms selected from the group consisting of N, O, and S, or may be fused with a $C_{3-10}$ cycloalkyl to form a bicyclic ring, and the substituted phenyl is substituted with hydroxyl, or sequentially substituted with a $C_{1-5}$ alkyl, a 3- to 7-membered heterocycloalkyl including one or more heteroatoms selected from the group consisting of N, O, and S, and a $C_{3-10}$ cycloalkyl or alkylcarbonyl; and

$R^6$ is -H, a halogen, or a $C_{1-10}$ linear or branched alkyl).

According to one specific embodiment, $R^2$ is a $C_{3-8}$ cycloalkyl, a $C_{3-6}$ cycloalkenyl,-$NHA^1$, or -$OA^2$, wherein $A^1$ is a $C_{1-6}$ linear or branched alkyl, a $C_{3-7}$ cycloalkyl, or a 3- to 6-membered heterocycloalkyl including one or more O atoms;

wherein, when $A^1$ is a $C_{1-6}$ linear or branched alkyl, the alkyl is unsubstituted or substituted with one or more non-hydrogen substituents selected from the group consisting of a $C_{3-6}$ cycloalkyl, a $C_{1-3}$ linear or branched alkylsulfonyl, a $C_{1-3}$ alkylaminosulfonyl, and a $C_{1-3}$ linear or branched alkoxy,
when $A^1$ is a $C_{3-7}$ cycloalkyl, the cycloalkyl is unsubstituted or substituted with one or more fluoro moieties,
when $A^1$ is a 3- to 6-membered heterocycloalkyl including one or more O atoms, the heterocycloalkyl is unsubstituted or substituted with one or more $C_{1-3}$ linear alkyl moieties; and
$A^2$ is a $C_{3-6}$ cycloalkyl, wherein the cycloalkyl is unsubstituted or substituted with one or more non-hydrogen substituents selected from a $C_{1-3}$ linear or branched alkyl and a hydroxyl group.

According to one more specific embodiment,
$R^2$ may be

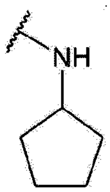 , 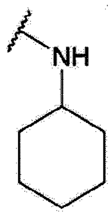 , 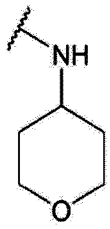 , 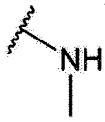 , 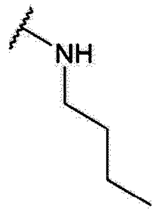 , 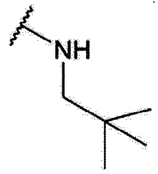 ,

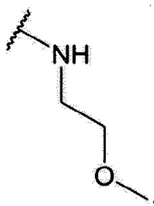 , 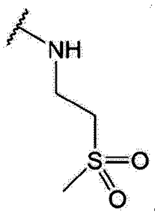 , 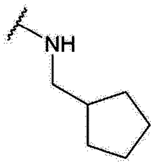 , 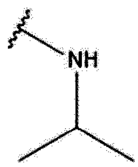 , 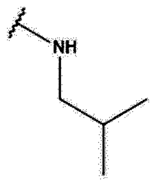 , 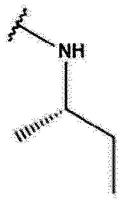 , 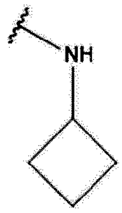 ,

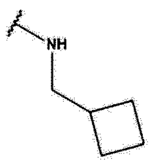 , 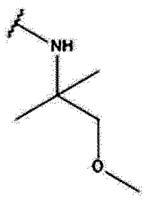 , 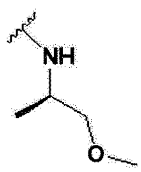 , 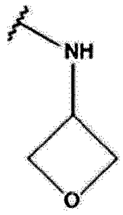 , 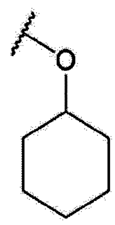 , 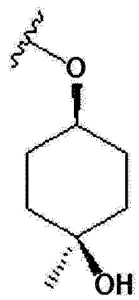 , 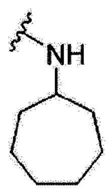 ,

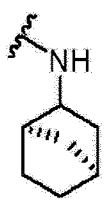 , 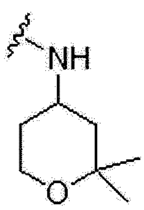 , 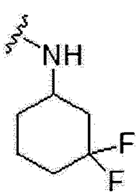 , 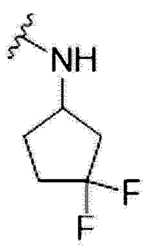 , 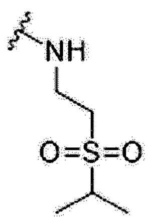 , 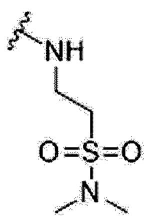 , 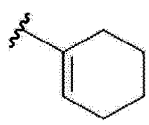 ,

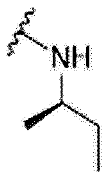, 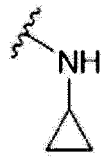, or 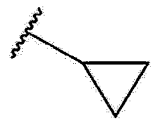.

According to one specific embodiment, $R^3$ is -H, a $C_{1-4}$ linear or branched alkoxy, or acrylamide, wherein the $C_{1-4}$ linear alkoxy is unsubstituted or substituted with trifluoromethyl, and $R^4$ is -H or a $C_{1-3}$ linear alkoxy, or

$R^3$ and $R^4$, together with a benzene ring containing carbon atoms to which they are bonded, may form a 9- to 10-membered bicyclic ring. More specifically, the 9- to 10-membered bicyclic ring may be dihydrobenzodioxin or dihydrobenzofuranyl.

According to one specific embodiment, $R^5$ is -H, a $C_{1-3}$ alkylaminocarbonyl, unsubstituted or substituted phenyl, oxooxazolidinonyl, dioxidothiazolidinyl, oxopyrrolidinyl, dioxidothiazinanyl, oxomorpholinyl, or a heteroaryl selected from the group consisting of pyrazolyl, triazolyl, thiazolyl, oxazolyl, pyridinyl, and imidazolyl, wherein the heteroaryl may be unsubstituted or substituted with one or more non-hydrogen substituents selected from the group consisting of a $C_{1-3}$ alkyl, fluoro, and a 4- to 6-membered heterocycloalkyl including one or more O atoms, or may be fused with a $C_{3-5}$ cycloalkyl to form a bicyclic ring, and the substituted phenyl is substituted with hydroxy or a $C_{1-3}$ alkyl, wherein the $C_{1-3}$ alkyl may be substituted with a $C_{1-3}$ alkylcarbonylpiperazine or a $C_{3-6}$ cycloalkylpiperazine.

According to one more specific embodiment,

$R^5$ may be -H,

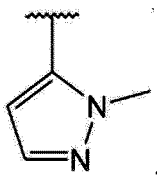, 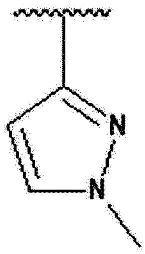, 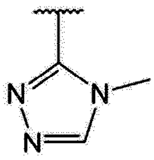, 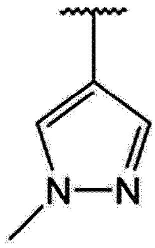, 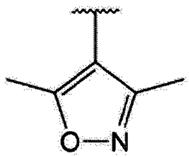,

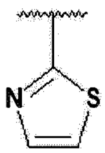, 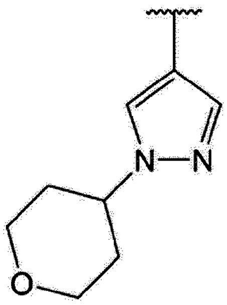, 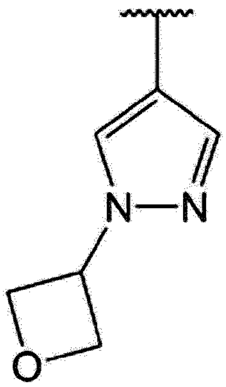, 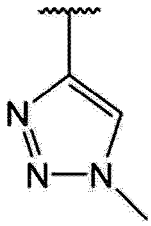, 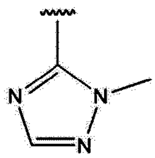, 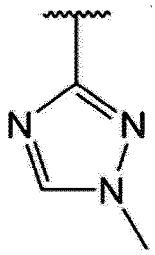,

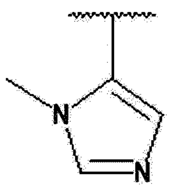, 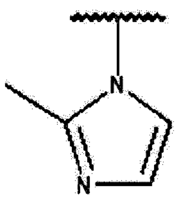, 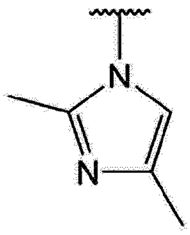, 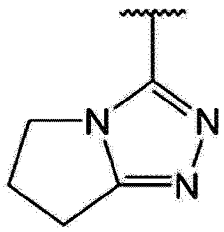, 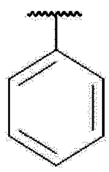, 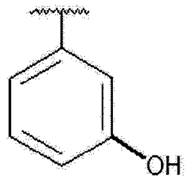,

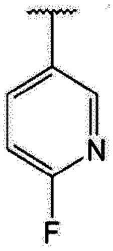, 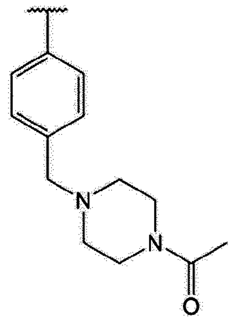, 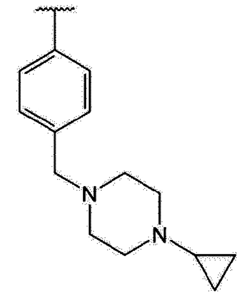, 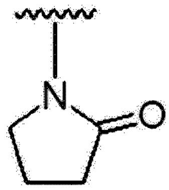, 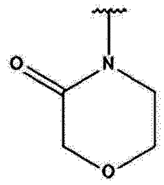,

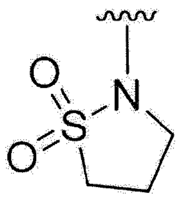, 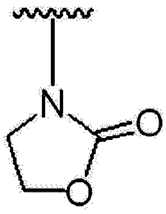, 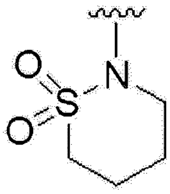, or 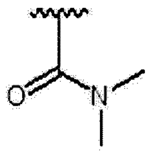.

According to another one aspect, in the compound represented by Formula 1, when X is N,

$R^1$ is -H, chloro, fluoro, bromo, iodo, cyano, or trifluoromethyl,
$R^2$ is a $C_{3-7}$ cycloalkyl, cyclohexenyl, $-NHA^1$, or $-OA^2$,
$A^1$ is a $C_{1-6}$ linear or branched alkyl, a $C_{3-7}$ cycloalkyl, or a 3- to 6-membered heterocycloalkyl including one or more O atoms,
wherein when $A^1$ is a $C_{1-6}$ linear or branched alkyl, the alkyl is unsubstituted or substituted with one or more non-hydrogen substituents selected from the group consisting of a $C_{3-6}$ cycloalkyl, a $C_{1-3}$ linear or branched alkylsulfonyl, a $C_{1-3}$ alkylaminosulfonyl, and a $C_{1-3}$ linear or branched alkoxy,
when $A^1$ is a $C_{3-7}$ cycloalkyl, the cycloalkyl is unsubstituted or substituted with one or more fluoro moieties, and when $A^1$ is a 3- to 6-membered heterocycloalkyl including one or more O atoms, the heterocycloalkyl is unsubstituted or substituted with one or more $C_{1-3}$ linear alkyl moieties,
$A^2$ is a $C_{3-6}$ cycloalkyl, wherein the cycloalkyl is unsubstituted or substituted with one or more non-hydrogen substituents selected from a $C_{1-3}$ linear or branched alkyl and a hydroxyl group,
$R^3$ is -H, a $C_{1-4}$ linear or branched alkoxy, or acrylamide, wherein the $C_{1-4}$ linear alkoxy is unsubstituted or substituted with trifluoromethyl, and $R^4$ is -H or a $C_{1-3}$ linear alkoxy, or
$R^3$ and $R^4$, together with a benzene ring containing carbon atoms to which they are bonded, form a 9- to 10-membered bicyclic ring including one or more O atoms,
$R^5$ is -H, a $C_{1-3}$ alkylaminocarbonyl, unsubstituted or substituted phenyl, oxooxazolidinonyl, dioxidothiazolidinyl, oxopyrrolidinyl, dioxidothiazinanyl, oxomorpholinyl, or a heteroaryl selected from the group consisting of pyrazolyl, triazolyl, thiazolyl, oxazolyl, pyridinyl, and imidazolyl, wherein the heteroaryl may be unsubstituted or substituted with one or more non-hydrogen substituents selected from the group consisting of a $C_{1-3}$ alkyl, fluoro, and a 4- to 6-membered heterocycloalkyl including one or more O atoms, or may be fused with a $C_{3-5}$ cycloalkyl to form a bicyclic ring, and the substituted phenyl is substituted with hydroxy or a $C_{1-3}$ alkyl, wherein the $C_{1-3}$ alkyl is substituted with $C_{1-3}$ alkylcarbonylpiperazine or $C_{3-6}$ cycloalkylpiperazine, and
$R^6$ may be -H, a halogen, or a $C_{1-3}$ linear alkyl.

According to one specific embodiment, in the compound represented by Formula 1, when X is N,

$R^1$ is -H, chloro, cyano, or trifluoromethyl,
$R^2$ is

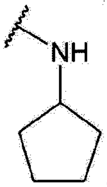, 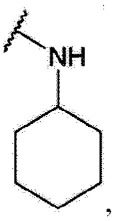, 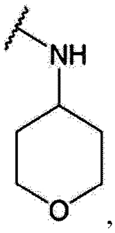, 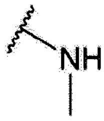, 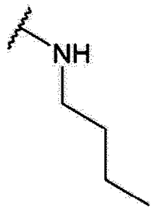, 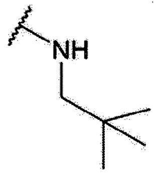, 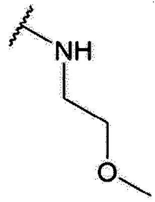,

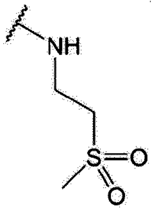, 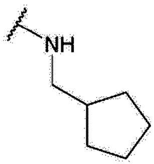, 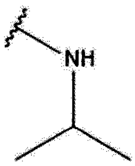, 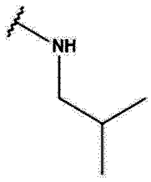, 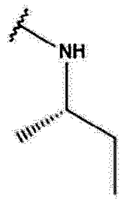, 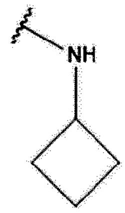, 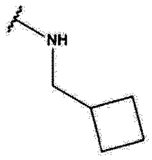,

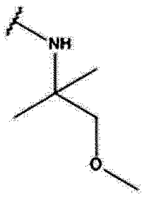, 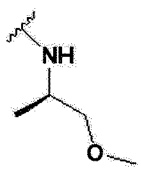, 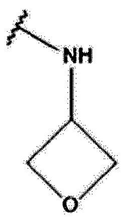, 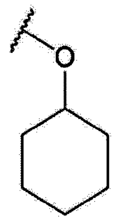, 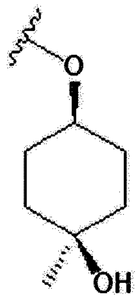, 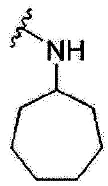, 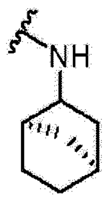,

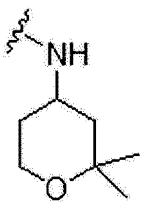, 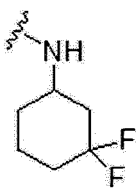, 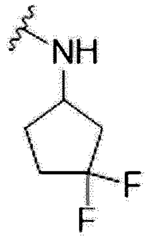, 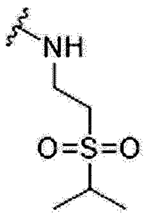, 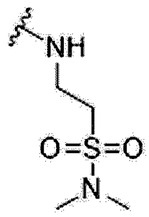, 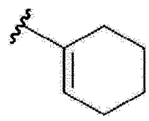, 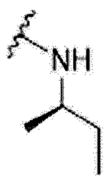,

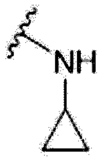, or 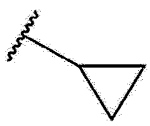,

$R^3$ is -H, a $C_{1-4}$ linear or branched alkoxy, or acrylamide, wherein the $C_{1-4}$ linear alkoxy is unsubstituted or substituted with trifluoromethyl, and $R^4$ is -H or a $C_{1-3}$ linear alkoxy, or

$R^3$ and $R^4$, together with a benzene ring containing carbon atoms to which they are bonded, form a 9- to 10-membered bicyclic ring including one or more O atoms, wherein the 9- to 10-membered bicyclic ring is dihydrobenzodioxin or dihydrobenzofuran,

$R^5$ is -H,

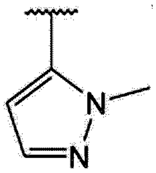, 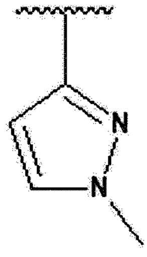, 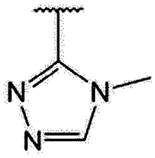, 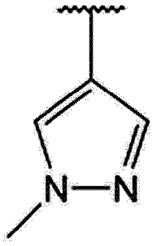, 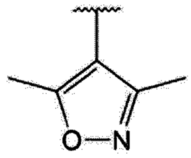,

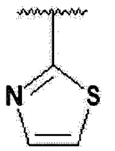, 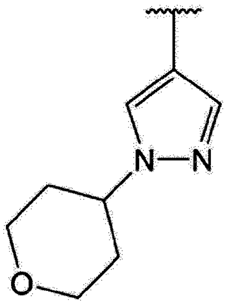, 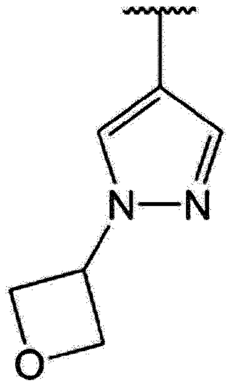, 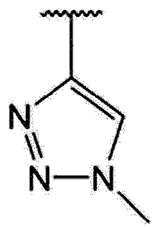, 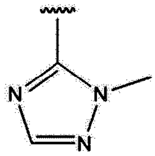, 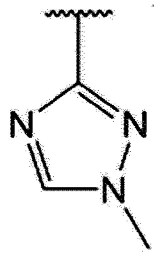,

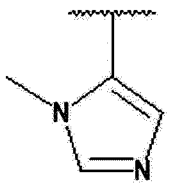, 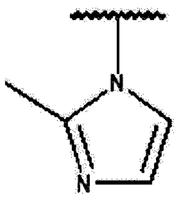, 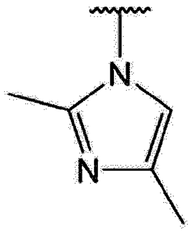, 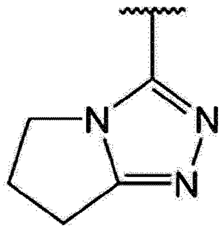, 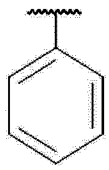, 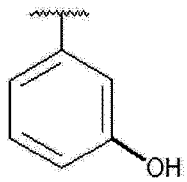,

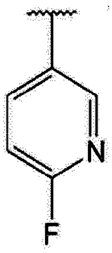, 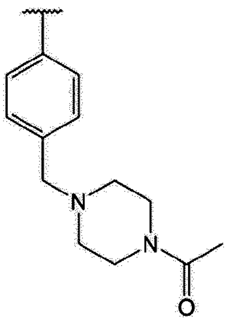, 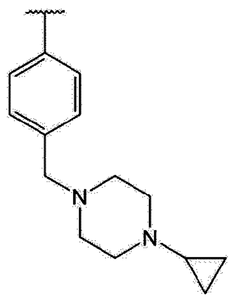, 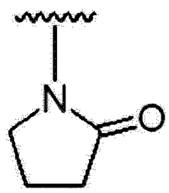, 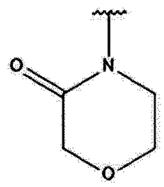,

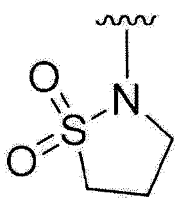, 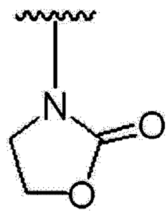, 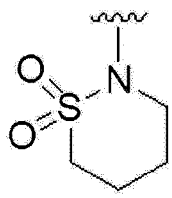, or 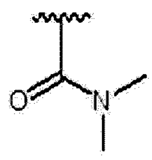,

and
$R^6$ may be -H, a halogen, or a $C_{1-3}$ linear alkyl.

According to still another one aspect, in the compound represented by Formula 1,

when X is N, and the $R^3$ and $R^4$, together with a benzene ring containing carbon atoms to which they are bonded, form a bicyclic ring of dihydrobenzodioxin or dihydrobenzofuran,

$R^1$ is -H, chloro, cyano, or trifluoromethyl,

$R^2$ is

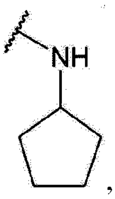, 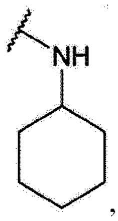, 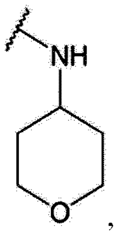, 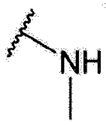, 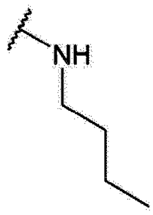, 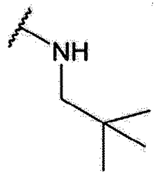, 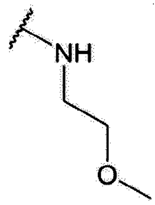,

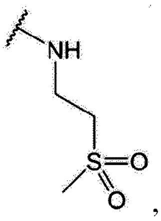, 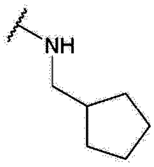, 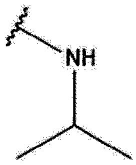, 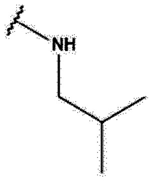, 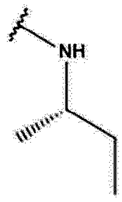, 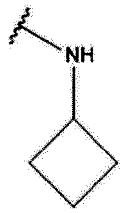, 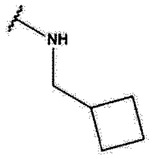,

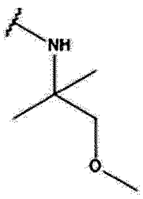, 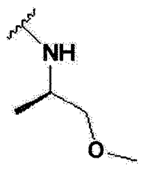, 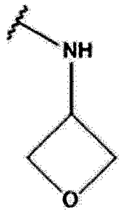, 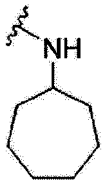, 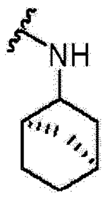, 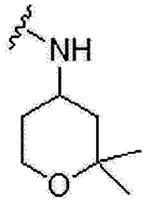, 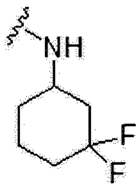,

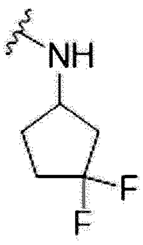, 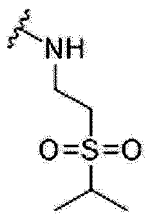, 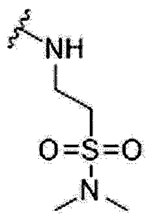, 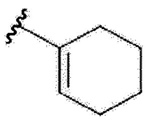, 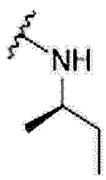, 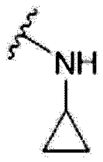, or 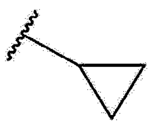,

$R^5$ is -H,

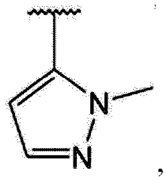, 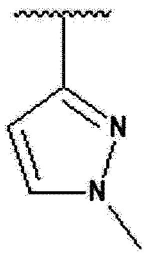, 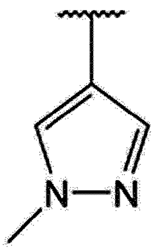, or 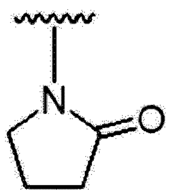,

and

$R^6$ may be -H.

According to yet another one aspect, in the compound represented by Formula 1,

when X is N, and $R^3$ is -H, a $C_{1-4}$ linear or branched alkoxy, or acrylamide, wherein the $C_{1-4}$ linear alkoxy is unsubstituted or substituted with trifluoromethyl, and
when $R^4$ is -H or a $C_{1-3}$ linear alkoxy,
$R^1$ is -H, chloro, cyano, or trifluoromethyl,
$R^2$ is

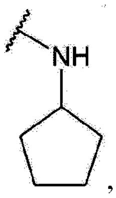, 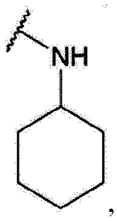, 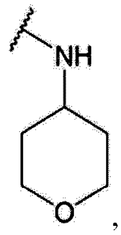, 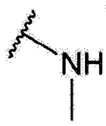, 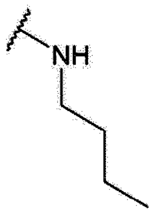, 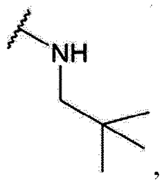, 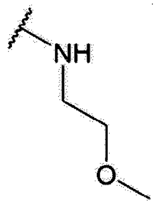,

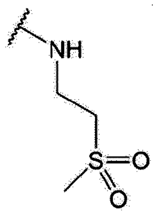, 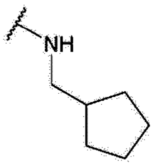, 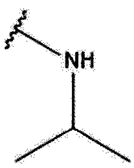, 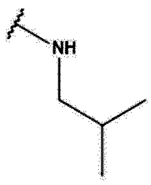, 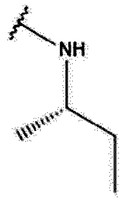, 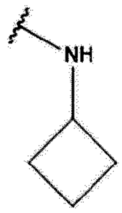, 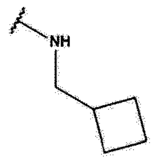,

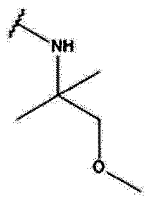, 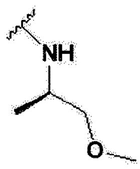, 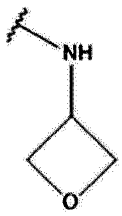, 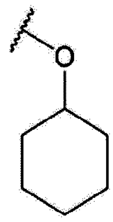, 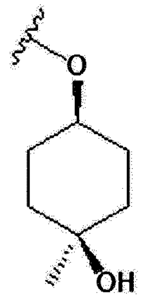, 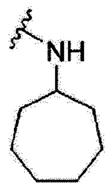, 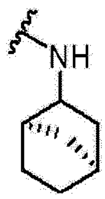,

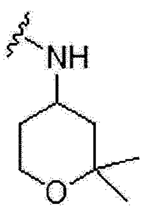, 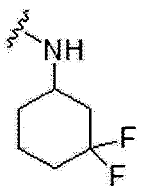, 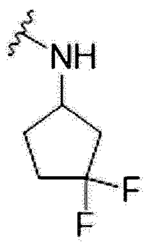, 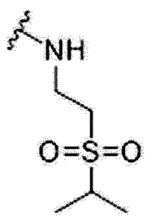, 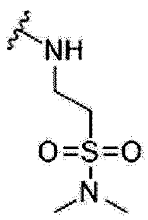, 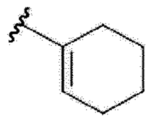,

or

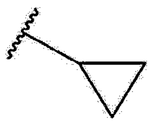,

$R^5$ is -H,

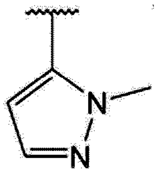, 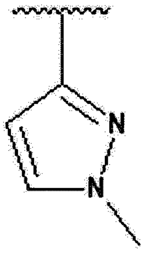, 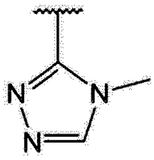, 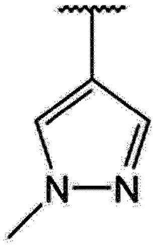, 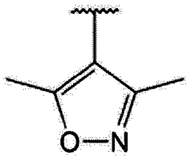,

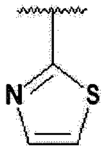, 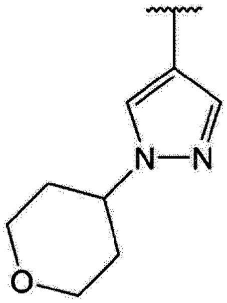, 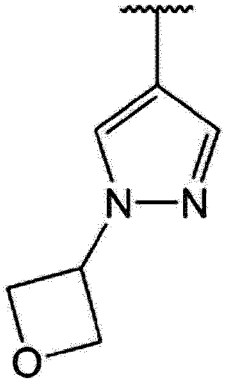, 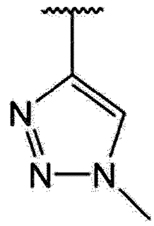, 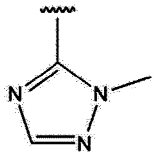, 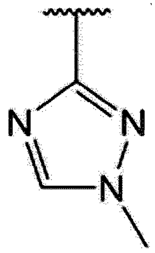,

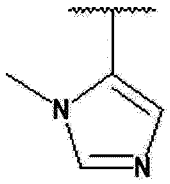, 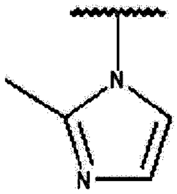, 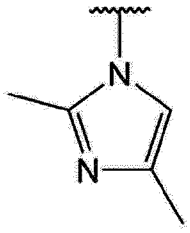, 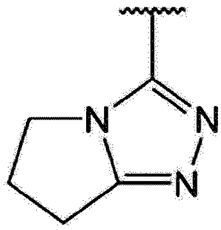, 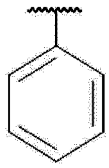, 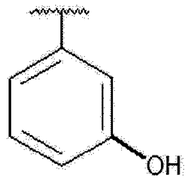,

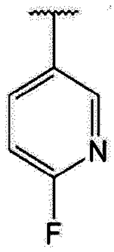, 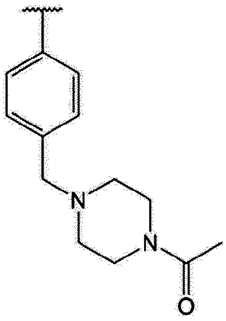, 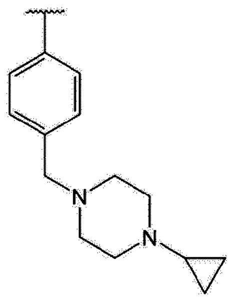, 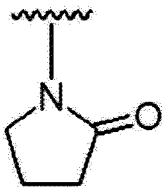, 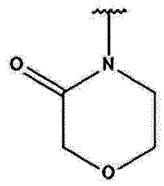,

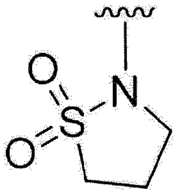, 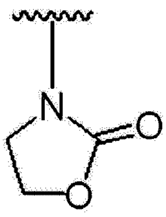, 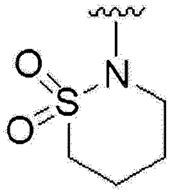, or 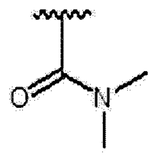,

and

$R^6$ may be -H, a halogen, or a $C_{1-3}$ linear alkyl.

According to yet another one aspect,

when X is CH,
$R^1$ is cyano or trifluoromethyl;
$R^2$ is $-NHA^1$, wherein $A^1$ is a $C_{3-7}$ cycloalkyl,
$R^3$ is a $C_{1-6}$ linear or branched alkoxy, $R^4$ is -H, or $R^3$ and $R^4$, together with a benzene ring containing carbon atoms to which they are bonded, form a 9- and 10-membered bicyclic ring including one or more O atoms,
$R^5$ is oxopyrrolidinyl, and
$R^6$ may be -H.

According to yet another one aspect,

Examples of the compound represented by Formula 1 according to the present invention, or the pharmaceutically acceptable salt thereof may include compounds of Examples 1 to 177 listed in Table 1 below, or pharmaceutically acceptable salts thereof.

The compound represented by Formula 1 according to the present invention may be used in the form of a pharmaceutically acceptable salt, and an acid addition salt formed by a pharmaceutically acceptable free acid is usefully used as the salt. The acid addition salt is obtained from inorganic acids such as hydrochloric acid, nitric acid, phosphoric acid, sulfuric acid, hydrobromic acid, hydriodic acid, nitrous acid, and phosphorous acid; nontoxic organic acids such as aliphatic mono- and dicarboxylates, phenyl-substituted alkanoates, hydroxy alkanoates and alkandioates, aromatic acids, and aliphatic and aromatic sulfonic acids; or organic acids such as trifluoroacetic acid, acetate, benzoic acid, citric acid, lactic acid, maleic acid, gluconic acid, methanesulfonic acid, 4-toluenesulfonic acid, tartaric acid, and fumaric acid. Types of such a pharmaceutically non-toxic salt include sulfates, pyrosulfates, bisulfates, sulfites, bisulfites, nitrates, phosphates, monohydrogen phosphates, dihydrogen phosphates, methaphosphates, pyrophosphate chlorides, bromides, iodides, fluorides, acetates, propionates, decanoates, caprylates, acrylates, formates, isobutyrates, caprates, heptanoates, propiolates, oxalates, malonates, succinates, suberates, sebacates, fumarates, maleates, butyne-1,4-dioates, hexane-1,6-dioates, benzoates, chlorobenzoates, methylbenzoates, dinitro benzoates, hydroxybenzoates, methoxybenzoates, phthalates, terephthalates, benzenesulfonates, toluenesulfonates, chlorobenzene sulfonates, xylenesulfonates, phenylacetates, phenylpropionates, phenylbutyrates, citrates, lactates, β-hydroxybutyrates, glycolates, maleates, tartrates, methanesulfonates, propanesulfonates, naphthalene-1-sulfonates, naphthalene-2-sulfonates, and mandelates, and the like.

The acid addition salt according to the present invention may be prepared using conventional methods, and, for example, by dissolving a derivative of Formula 1 in an organic solvent such as methanol, ethanol, acetone, methylenechloride, or acetonitrile and filtering and drying a precipitate generated by adding an organic acid or an inorganic acid thereto, or may be prepared by distilling a solvent and excess acid under reduced pressure, followed by drying and crystallization under an organic solvent.

Also, a pharmaceutically acceptable metal salt may be prepared using a base. An alkali metal or an alkaline earth metal salt is, for example, obtained by dissolving a compound in an excessive amount of an alkali metal hydroxide or alkaline earth metal hydroxide solution, filtering the non-dissolved compound salt, and evaporating and drying the filtrate. In this case, the metal salt is pharmaceutically suitable for preparing a sodium, potassium or calcium salt. Also, a salt corresponding to the metal salt is obtained by reacting an alkali metal or alkaline earth metal salt with a suitable silver salt (e.g., silver nitrate).

Also, the present invention includes all types of the compound represented by Formula 1 or the pharmaceutically acceptable salts thereof, as well as solvates, optical isomers, hydrates, and the like, which may be prepared therefrom.

In the present specification, "hydrate" may refer to a compound of the present disclosure or salt thereof containing a stoichiometric or non-stoichiometric amount of water bonded by a non-covalent intermolecular force. A hydrate of the compound represented by Formula 1 may contain a stoichiometric or non-stoichiometric amount of water bonded by a non-covalent intermolecular force. The hydrate may contain 1 equivalent or more, preferably, about 1 equivalent to about 5 equivalents of water. Such a hydrate may be prepared by crystallizing the compound represented by Formula 1 of the present disclosure, an isomer thereof, or a pharmaceutically acceptable salt thereof from water or a solvent containing water.

In the present specification, "solvate" may refer to a compound of the present disclosure or salt thereof containing a stoichiometric or non-stoichiometric amount of a solvent bonded by a non-covalent intermolecular force. Suitable solvents therefor include volatile solvents, non-toxic solvents, and/or solvents suitable for administration to humans.

In the present specification, "isomer" may refer to a compound of the present disclosure or salt thereof having the same chemical formula or molecular formula, but being structurally or sterically different. Such isomers include structural isomers such as a tautomer, R or S isomers having an asymmetric carbon center, stereoisomers such as a geometric isomer (trans or cis), and optical isomers (enantiomers). All these isomers and mixtures thereof also fall within the scope of the present disclosure.

As shown in the following Scheme A, another aspect of the present invention provides a method of preparing the compound represented by Formula 1, which includes:

introducing $R^1$ into a compound represented by Formula 00 to prepare a compound represented by Formula AA (STEP 1);
attaching an SEM protective group to the compound represented by Formula AA to prepare a compound represented by Formula BB (STEP 2);
reacting the compound represented by Formula BB with the compound represented by Formula CC to prepare a compound represented by Formula 2 (STEP 3);
reacting the compound represented by Formula 2 with the compound represented by Formula 3 to prepare a compound represented by Formula 4 (STEP 4); and
removing the SEM protective group from the compound represented by Formula 4 prepared in the previous step to prepare the compound Formula 1 (STEP 5).

[Scheme A]

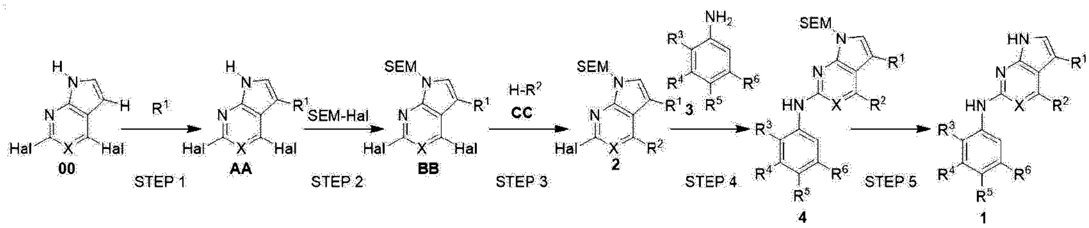


(wherein,

X, and $R^1$ to $R^6$ are as defined in Formula 1;
Hal is a halogen; and
SEM is a protective group).

Hereinafter, the method of preparing the compound represented by Formula 1 according to the present invention will be described.
In the method of preparing the compound represented by Formula 1,
**[0052]** STEP 1 is a step of introducing $R^1$ into a compound represented by Formula 00 to prepare a compound represented by Formula AA. In this step, the reaction may be performed in a DMF solvent. In this case, the reaction may be performed at a reaction temperature of approximately 0 to 25°C for a reaction time of approximately 30 minutes to 4 hours, but the reaction may be performed without any limitation on reaction conditions as long as the reaction may proceed smoothly.
STEP 2 is a step of introducing an SEM protective group into a compound represented by Formula AA to prepare a compound represented by Formula BB. In this step, the reaction may be performed in a DMF solvent. In this case, the reaction may be performed at a reaction temperature of approximately 0 to 25°C for a reaction time of approximately 30 minutes to 4 hours, but the reaction may be performed without any limitation on reaction conditions as long as the reaction may proceed smoothly.
STEP 3 is a step of introducing $R^2$ into a compound represented by Formula BB to prepare a compound represented by Formula 2. In this step, the reaction may be performed in an alcohol solvent. In this case, the reaction may be performed at a reaction temperature of approximately 80°C for a reaction time of approximately 12 to 24 hours, but the reaction may be performed without any limitation on reaction conditions as long as the reaction may proceed smoothly.
STEP 4 is a step of reacting the compound represented by Formula 2 with the compound represented by Formula 3 to prepare a compound represented by Formula 4. In this step, the reaction may be performed in an alcohol solvent. In this case, the reaction may be performed at a reaction temperature of approximately 60 to 120°C for a reaction time of approximately 30 minutes to 90 minutes, but the reaction may be performed without any limitation on reaction conditions as long as the reaction may proceed smoothly.
STEP 5 is a step of removing a protective group (-SEM) from the compound represented by Formula 4 prepared in STEP 4 to prepare the compound represented by Formula 1. Because this step is for removing the protective group (-SEM), this step may be performed using a known method of removing a protective group, depending on the types of protective group. Examples of the protective group may include a 2-(trimethylsilyl)ethoxymethyl group, a trimethylsilyl(TMS) group, a benzyl group, an acetyl group, or the like.
Another aspect of the present invention provides a pharmaceutical composition for preventing or treating diseases associated with one or more protein kinases selected from the group consisting of JAK2, SNARK, TTK, YSK4,

JNK1, FLT3, PRKCE, CAMKK1, JNK3, TYK2, RSK2, CAMKK2, ULK3, ULK1, RSK4, TRKB, LRRK2, JNK3, AAK1, GAK, SBK1, TYK2, CAMK2D, MAP3K2, KIT, CSNK1D, CSNK1E, MEK4, RIOK1, DYRK1B, PKN2, FLT3, JNK2, RIPK5, MEK3, ABL1, MAPKAPK2, GRK4, and SRPK3, which contains the compound, or a stereoisomer thereof, a hydrate thereof, or a pharmaceutically acceptable salt thereof as an active ingredient. The kinases may be in a wild-type or mutant form.

Still another aspect of the present invention provides a pharmaceutical composition for preventing or treating cancer, which includes the compound represented by Formula 1, or the stereoisomer thereof, the hydrate thereof, or the pharmaceutically acceptable salt thereof as an active ingredient. In this case, the compound may exhibit an inhibitory activity against a TTK kinase to prevent or ameliorate cancer.

Yet another aspect of the present invention provides a method of preventing or treating the protein kinase-related disease, particularly, cancer, which includes administering the compound represented by Formula 1, or the stereoisomer thereof, the hydrate thereof, or the pharmaceutically acceptable salt thereof, or a pharmaceutical composition including the same as active ingredient into a subject in need thereof.

The cancer treatment may be applied without any limitations as long as it is a cancer known in the art, but some specific examples of the cancer may include one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphocytic leukemia, basal cell carcinoma, ovarian epithelial cancer, ovarian germ cell cancer, male breast cancer, brain cancer, pituitary adenomas, multiple myeloma, gallbladder cancer, biliary tract cancer, colorectal cancer, chronic myelogenous leukemia, chronic lymphocytic leukemia, retinoblastoma, choroidal melanoma, ampulla of vater cancer, bladder cancer, peritoneal cancer, parathyroid cancer, adrenal cancer, nasal and paranasal sinus cancer, non-small cell lung cancer, tongue cancer, astrocytoma, small cell lung cancer, childhood brain cancer, pediatric lymphoma, pediatric leukemia, small intestine cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureter cancer, urethral cancer, cancer of unknown primary site, gastric lymphoma, gastric cancer, gastric carcinoid tumors, gastrointestinal stromal tumors, Wilms cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid tumors, vaginal cancer, spinal carcinoma, acoustic neuroma, pancreatic cancer, salivary gland cancer, Kaposi's sarcoma, Paget's disease, tonsillar cancer, squamous cell carcinoma, lung adenocarcinoma, lung cancer, lung squamous cell carcinoma, skin cancer, anal cancer, rhabdomyosarcoma, laryngeal cancer, pleural cancer, blood cancer, and thymic carcinoma.

The compound represented by Formula 1 or the pharmaceutically acceptable salt thereof may be administered in various oral and parenteral formulations upon clinical administration. When a composition is formulated, the composition may be prepared using a commonly used diluent or excipient such as a filler, an extending agent, a binding agent, a wetting agent, a disintegrating agent, a surfactant, and the like. Examples of solid preparations for oral administration include tablets, pills, powder, granules, capsules, and the like, and these solid preparations may be formulated by mixing one or more compounds with one or more excipients, for example, starch, calcium carbonate, sucrose, lactose, or gelatin. In addition to simple excipients, formulation may be performing using lubricants such as magnesium stearate and talc. Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a flavoring agent, a preservative, and the like. Preparations for parenteral administration include an aqueous sterile solution, a non-aqueous solvent, a suspension, an emulsion, and the like. Non-limiting examples of the non-aqueous solvent and the suspension include propylene glycol, polyethylene glycol, a vegetable oil such as olive oil, and an injectable ester such as ethyl oleate.

A pharmaceutical composition including the compound represented by Formula 1 or the pharmaceutically acceptable salt thereof as an active ingredient may be parenterally administered, and the parenteral administration is performed by a method of injecting a subcutaneous injection, an intravenous injection, an intramuscular injection, or an intrathoracic injection. In this regard, to formulate preparations for parenteral administration, the pharmaceutical composition may be prepared by mixing the compound represented by Formula 1 or a pharmaceutically acceptable salt thereof with a stabilizer or a buffer in water to prepare a solution or a suspension, followed by preparation into an ampoule or vial unit dosage form. The composition may be sterilized and/or include an adjuvant such as a preservative, a stabilizer, a wetting agent or an emulsion accelerator, a salt for the control of osmotic pressure, and/or a buffer, and other therapeutically effective materials, and may be formulated using a general method, such as mixing, granulation, or coating.

For example, the formulations for oral administration include a tablet, a pill, a hard/soft capsule, a liquid, a suspending agent, an emulsifying agent, a syrup, a granule, an elixir, a troche, and the like. In this case, these formulations contain diluents (e.g., lactose, dextrose, sucrose, mannitol, sorbitol, cellulose, and/or glycine), and lubricants (e.g., silica, talc, stearic acid and a magnesium or calcium salt thereof, and/or polyethylene glycol) in addition to the active ingredient. The tablet may contain a binding agent such as magnesium aluminum silicate, starch paste, gelatin, methyl cellulose,

sodium carboxymethyl cellulose, and/or polyvinyl pyrrolidone, and may contain a disintegrating agent (starch, agar, alginic acid or a sodium salt thereof, or the like) or a boiling mixture and/or an absorbing agent, a coloring agent, a flavoring agent, and a sweetening agent, when, necessary.

The pharmaceutical composition for preventing or treating cancer, which contains the compound represented by Formula 1, or the optical isomer thereof or the pharmaceutically acceptable salt thereof as an active ingredient, may be administered as a separate therapeutic agent, or may be used in combination with other anticancer drugs in use.

Yet another aspect of the present invention provides the compound, or the stereoisomer thereof, the hydrate thereof, or the pharmaceutically acceptable salt thereof for use in the treatment of cancer.

Yet another aspect of the present invention provides a use of the compound, or the stereoisomer thereof, the hydrate thereof, or the pharmaceutically acceptable salt thereof for use in the preparation of drugs for treating cancer.

The protein kinases and diseases associated with the protein kinases are as described above, and thus a specific description thereof will be omitted to avoid redundancy.

Hereinafter, the present invention will be described in detail with reference to Examples and Experimental Examples as will be described below.

However, it should be understood that the Examples and Experimental Examples described below are given for purpose of illustrating the present invention and not intended to limit the scope of the present invention.

<Analysis and Purification Conditions>

Compounds synthesized in Examples of the present invention were purified and structurally analyzed using the following methods.

1. Preparative Medium-Pressure Liquid Chromatography (MPLC)

CombiFlash Rf +UV (TELEEDYNE ISCO Inc.) was used for medium-pressure liquid chromatography.

2. Analytical HPLC Conditions (ACQUITY UPLC H-Class System)

An UPLC system manufactured by Waters (ACQUITY UPLC PDA Detector) equipped with a mass QDA detector manufactured by Waters was used. The column used was ACQUITY UPLC®BEH C18 (1.7 μm, 2.1 x 50 mm) available from Waters, and the column temperature was 30 °C.

Water containing 0.1% formic acid was used as mobile phase A, and acetonitrile containing 0.1% formic acid was used as mobile phase B.

Gradient conditions (10-100% B for 3 minutes, flow rate = 0.6 ml/min)

3. Preparative-Liquid Chromatography Mass Spectrometry (Prep-LCMS) for Purification

An autopurification HPLC system manufactured by Waters (2767 sample manager, 2545 binary gradient module, 2998 Photodiode Array Detector) equipped with a mass QDA detector manufactured by Waters was used. The column used was SunFire®Prep C18 OBDTM available from Waters (5 μm, 19 x 50 mm), and the column temperature was room temperature.

Water containing 0.035% trifluoroacetic acid was used as mobile phase A, and methanol containing 0.035% trifluoroacetic acid was used as mobile phase B.

Gradient conditions (15-100% B for 10 minutes, flow rate= 25 ml/min)

4. Preparative-Liquid Chromatography UV Spectrometry (Prep-150 LC System) for Purification

A Prep 150 LC system manufactured by Waters (2545 Quaternary gradient module, 2998 Photodiode Array Detector, Fraction collector III) was used. The column used was XTERRA®Prep RP18 OBDTM available from Waters (10 μm, 30 x 300 mm), and the column temperature was room temperature.

5. NMR Analysis

NMR analysis was performed using AVANCE III 400 or AVANCE III 400 HD manufactured by Bruker, and data was expressed in parts per million (ppm) (δ).

Commercially available reagents were used without further purification. In the present invention, the room

temperature refers to a temperature of approximately 20 to 25°C. Concentration under reduced pressure or removal of solvents by distillation was performed using a rotary evaporator.

**<Preparation Example 1-1> Preparation of 2-chloro-*N*-cyclohexyl-7-((2-(trimethylsilyl)ethoxy)methyl)-7*H*-pyrrolo[2,3-d]pyrimidine-4-amine**

The title compound was prepared in a manner as depicted in the following Scheme 1.

[Scheme 1]

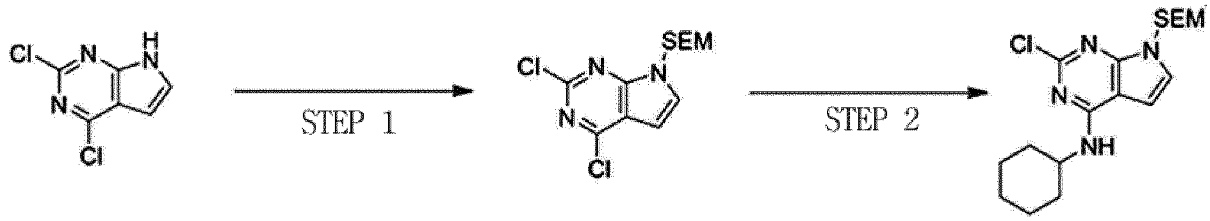


STEP 1: 2,4-dichloro-7*H*-pyrrolo[2,3-d]pyrimidine (1.0 equivalent) was dissolved in DMF (0.62 M) under nitrogen, and NaH (1.2 equivalents) was then slowly added thereto at 0°C. The reaction mixture was reacted at 15°C for an hour, and (2-(chloromethoxy)ethyl)trimethylsilane (1.3 equivalents) was further added thereto at 0°C, and then stirred at the same temperature for 1.5 hours. Distilled water was added to the resulting reaction product, and the organic matter was then extracted with EtOAc (x 2). After the collected organic layer was washed with brine, the remaining water was removed using $Na_2SO_4$, and the organic layer was concentrated under reduced pressure. The concentrated mixture was purified by column chromatography ($SiO_2$; PE:EA), and the target compound was obtained as a yellow liquid (yield: 84 %).

STEP 2: 2,4-dichloro-7-((2-(trimethylsilyl)ethoxy)methyl)-7*H*-pyrrolo[2,3-d]pyrimidine (1.0 equivalent), DIPEA (2.9 equivalents), and cyclohexylamine (1.5 equivalents) were dissolved in EtOH (0.32 M), and the reaction mixture was then stirred at 80°C for 16 hours. After the reaction was terminated, the organic solvent was removed by concentration under reduced pressure. An aqueous 1 N HCl solution (12.5 equivalents) was added to the resulting reaction product, and the organic matter was then extracted with EtOAc (x3). The collected organic layer was washed with an aqueous saturated $NaHCO_3$ solution and brine, and the remaining water was then removed using $Na_2SO_4$. Then, the organic layer was concentrated under reduced pressure to obtain the target compound as a white solid (yield: 95 %).

**<Preparation Example 2-1> Preparation of 3,6-dichloro-*N*-cyclohexyl-7-((2-(trimethylsilyl)ethoxy)methyl)-7*H*-pyrrolo[2,3-d]pyrimidine-4-amine**

The title compound was prepared in a manner as depicted in the following Scheme 2.

[Scheme 2]

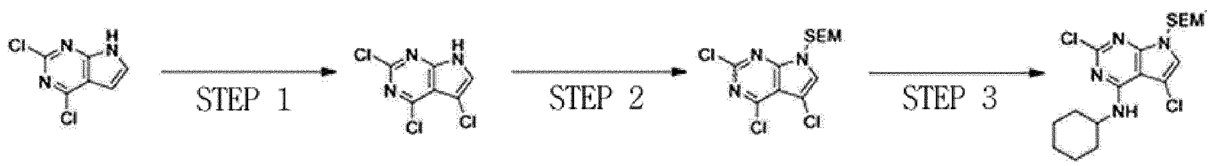


STEP 1: 2,4-dichloro-7*H*-pyrrolo[2,3-d]pyrimidine (1.0 equivalent) was dissolved in DMF (0.5 M) under nitrogen, and NCS (1.1 equivalents) was then slowly added thereto at 0°C. The reaction mixture was reacted at 0°C for 10 minutes, and stirred at room temperature for 4 hours. Ice water was added to the resulting reaction product, and the formed solid target compound was filtered (yield: 65 %).

STEP 2: 2,4,5-trichloro-7*H*-pyrrolo[2,3-d]pyrimidine (1.0 equivalent) was dissolved in DMF (1.1 M) under nitrogen, and NaH (1.5 equivalents) was then slowly added thereto at 0°C. The reaction mixture was reacted at 0°C for 30 minutes, and (2-(chloromethoxy)ethyl)trimethylsilane (1.2 equivalents) was further added, and then stirred at 20 °C for 4 hours. Distilled water was added to the resulting reaction product, and the organic matter was then extracted with ethyl acetate (x3). After the collected organic layer was washed with brine, the remaining water was removed using $Na_2SO_4$,

and the organic layer was concentrated under reduced pressure. The concentrated mixture was purified by MPLC (EtOAc:Hex), and the target compound was obtained as a white solid (yield: 59 %). STEP 3: 2,4,5-trichloro-7-((2-(trimethylsilyl)ethoxy)methyl)-7*H*-pyrrolo[2,3-d]pyrimidine (1.0 equivalent) was dissolved in NMP (1.0 M) under nitrogen, and cyclohexylamine (1.2 equivalents) was slowly added thereto. The reaction mixture was stirred at 80°C for 16 hours. Distilled water was added to the resulting reaction product, and the organic matter was then extracted with EtOAc (x3). After the collected organic layer was washed with brine, the remaining water was removed using $Na_2SO_4$, and the organic layer was concentrated under reduced pressure. The concentrated mixture was purified by MPLC (EtOAc:Hex), and the target compound was obtained as a white solid (yield: .98 %)

**<Preparation Example 3-1> Preparation of 2-chloro-*N*-cyclohexyl-5-(trifluoromethyl)-7-((2-(trimethylsilyl)ethoxy)methyl)-7*H*-pyrrolo[2,3-d]pyrimidine-4-amine**

The title compound was prepared in a manner as depicted in the following Scheme 3.

[Scheme 3]

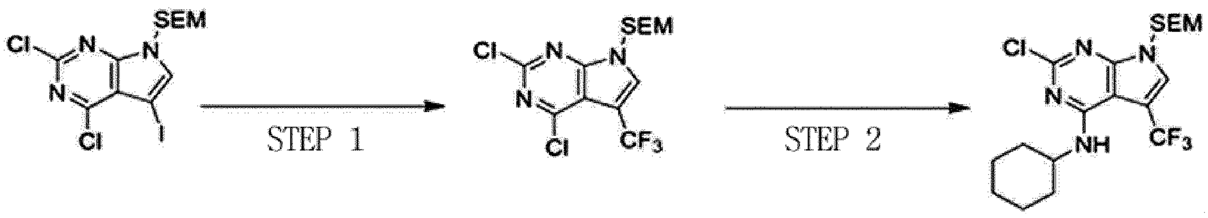


STEP 1: Moisture was removed while maintaining CuI (5.0 equivalents) and KF (5.0 equivalents) at a temperature of 150°C for 2 hours under reduced pressure close to a vacuum. The resulting reaction product was cooled to room temperature, and TMS-CF3 (5.0 equivalents) was then dissolved in NMP (1.12 M) under nitrogen, and slowly added to the reaction product through a syringe. The reaction mixture was reacted at room temperature for an hour, and 2,4-dichloro-5-iodo-7-((2-(trimethylsilyl)ethoxy)methyl)-7*H*-pyrrolo[2,3-d]pyrimidine (1.0 equivalent) was further dissolved in NMP (0.45 M) under nitrogen, and then slowly added through a syringe. The resulting reaction mixture was stirred at 50°C for 12 hours. After the reaction was terminated, the reaction product was cooled to room temperature. Then, distilled water was then added to the reaction product, and the organic matter was than extracted with EtOAc (x3). After the collected organic layer was washed with brine, the remaining water was removed using $Na_2SO_4$, and the organic layer was concentrated under reduced pressure. The concentrated mixture was purified by column chromatography ($SiO_2$; PE:EA), and the target compound was obtained as a yellow liquid (yield: .62 %)

STEP 2: 2,4-dichloro-5-(trifluoromethyl)-7-((2-(trimethylsilyl)ethoxy)methyl)-7*H*-pyrrolo[2,3-d]pyrimidine (1.0 equivalent), DIPEA (2.9 equivalents), and cyclohexylamine (1.5 equivalents) were dissolved in EtOH (0.25 M), and the reaction mixture was then stirred at 80°C for 16 hours. After the reaction was terminated, the organic solvent was removed by concentration under reduced pressure. The resulting reaction product was dissolved in EtOAc, and then washed with an aqueous 1 N HCl solution and brine. Then, the remaining water was removed using $Na_2SO_4$, and the reaction product was concentrated under reduced pressure to obtain the target compound as a brown solid (yield: 71 %).

**<Preparation Example 4-1> Preparation of 2-chloro-4-(methylamino)-7-((2-(trimethylsilyl)ethoxy)methyl)-7*H*-pyrrolo[2,3-d]pyrimidine-5-carbonitrile**

The title compound was prepared in a manner as depicted in the following Scheme 4.

[Scheme 4]

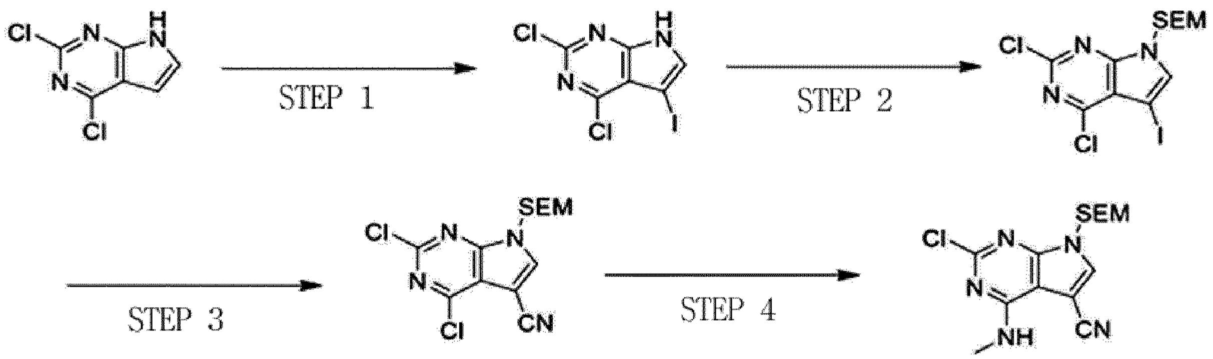


STEP 1: 2,4-dichloro-7*H*-pyrrolo[2,3-d]pyrimidine (1.0 equivalent) was dissolved in DCM (0.5 M) under nitrogen, and NIS (1.6 equivalents) was then slowly added thereto at 0°C. The reaction mixture was stirred at room temperature for 12 hours. After the reaction was terminated, the formed solid target compound was filtered. The filtered target compound was washed with distilled water to obtain the target compound as a yellow solid.

STEP 2: 2,4-dichloro-5-iodo-7*H*-pyrrolo[2,3-d]pyrimidine (1.0 equivalent) was dissolved in DMF (0.5 M) under nitrogen, and NaH (1.3 equivalents) was then slowly added thereto at 0°C. The reaction mixture was reacted at 0°C for 30 minutes, and (2-(chloromethoxy)ethyl)trimethylsilane (1.1 equivalents) was further added thereto, and then stirred at 20°C for an hour. Distilled water was added to the resulting reaction product, and the organic matter was than extracted with EA (x3). After the collected organic layer was washed with brine, the remaining water was removed using $Na_2SO_4$, and the organic layer was concentrated under reduced pressure. The concentrated mixture was purified by column chromatography ($SiO_2$; PE:EA), and the target compound was obtained as a white solid (yield: 94 %).

STEP 3: 2,4-dichloro-5-iodo-7-((2-(trimethylsilyl)ethoxy)methyl)-7*H*-pyrrolo[2,3-d]pyrimidine (1.0 equivalent) was dissolved in NMP (0.2 M), and CuCN (2.0 equivalents) was then slowly added thereto at 0°C. The reaction mixture was stirred at 120°C for 6 hours. Cold distilled water and EA were added to the resulting reaction product, and the resulting mixture was then filtered through a Celite filter. The resulting filtrate was separated into an organic layer and an aqueous layer, and the aqueous layer was then extracted with EtOAc (x2). After the collected organic layer was washed with brine, the remaining water was removed using $Na_2SO_4$, and the organic layer was concentrated under reduced pressure. The concentrated mixture was purified by column chromatography ($SiO_2$; PE:EA), and the target compound was obtained as a yellow solid (yield: 94 %).

STEP 4: 2,4-dichloro-7-((2-(trimethylsilyl)ethoxy)methyl)-7*H*-pyrrolo[2,3-d]pyrimidine-5-carbonitrile (1.0 equivalent), DIPEA (2.9 equivalents), and methyl amine (1.5 equivalents) were dissolved in EtOH (0.25 M), and the reaction mixture was then stirred at 80°C for 16 hours. After the reaction was terminated, the organic solvent was removed by concentration under reduced pressure. The resulting reaction product was dissolved in EtOAc, and then washed with an aqueous 1 N HCl solution and brine. Then, the remaining water was removed using $Na_2SO_4$, and the reaction product was concentrated under reduced pressure to obtain the target compound as a yellow solid (yield: 90 %).

### <Preparation Example 4-2> Preparation of 4-(butylamino)-2-chloro-7-((2-(trimethylsilyl)ethoxy)methyl)-7*H*-pyrrolo[2,3-d]pyrimidine-5-carbonitrile

The title compound was prepared in the same manner as in Preparation Example 4-1 (yield: 51 %).

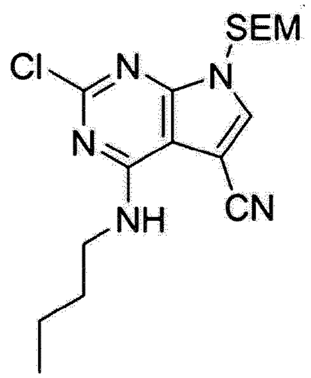

**<Preparation** Example 4-3> Preparation of (S)-4-(sec-butyl)-2-chloro-7-((2-**(trimethylsilyl)ethoxy)methyl)-7*H*-pyrrolo[2,3-d]pyrimidine-5-carbonitrile**

The title compound was prepared in the same manner as in Preparation Example 4-1 (yield: 85 %).

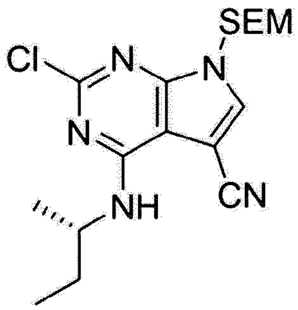

**<Preparation** Example 4-4> Preparation of 2-chloro-4-**((cyclopentylmethyl)amino)-7-((2-(trimethylsilyl)ethoxy)methyl)-7*H*-pyrrolo[2,3-d]pyrimidine-5-carbonitrile**

The title compound was prepared in the same manner as in Preparation Example 4-1 (yield: 78 %).

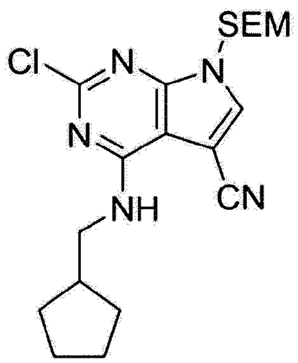

**<Preparation** Example 4-5> Preparation of 2-chloro-4-((2-**methoxyethyl)amino)-7-((2-(trimethylsilyl)ethoxy)methyl)-7*H*-pyrrolo[2,3-d]pyrimidine-5-carbonitrile**

The title compound was prepared in the same manner as in Preparation Example 4-1 (yield: 97 %).

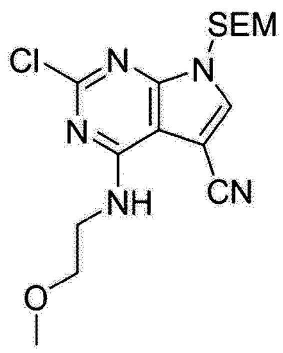

**<Preparation** Example 4-6> Preparation of 2-chloro-4-((2-**(methylsulfonyl)ethyl)amino)-7-((2-(trimethylsilyl)ethoxy)methyl)-7*H*-pyrrolo[2,3-d]pyrimidine-5-carbonitrile**

The title compound was prepared in the same manner as in Preparation Example 4-1 (yield: 53 %).

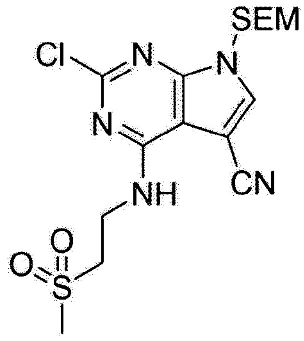

**<Preparation Example 4-7> Preparation of 2-chloro-4-((tetrahydro-2*H*-pyran-4-yl)amino)-7-((2-(trimethylsilyl)ethoxy)methyl)-7*H*-pyrrolo[2,3-d]pyrimidine-5-**carbonitrile

The title compound was prepared in the same manner as in Preparation Example 4-1 (yield: 86 %).

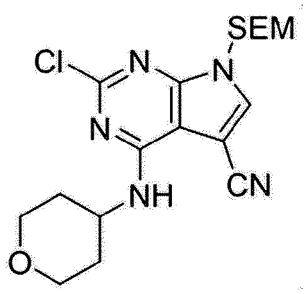

**<Preparation** Example 4-8> Preparation of 2-chloro-4-(cyclopentylamino)-7-((2-**(trimethylsilyl)ethoxy)methyl)-7*H*-pyrrolo[2,3-d]pyrimidine-5-carbonitrile**

The title compound was prepared in the same manner as in Preparation Example 4-1 (yield: 87 %).

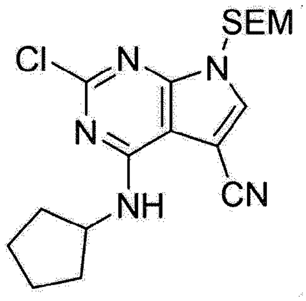

**<Preparation Example 4-9> Preparation of 2-chloro-4-(cyclohexylamino)-7-((2-(trimethylsilyl)ethoxy)methyl)-7*H*-pyrrolo[2,3-d]pyrimidine-5-carbonitrile**

The title compound was prepared in the same manner as in Preparation Example 4-1 (yield: 90 %).

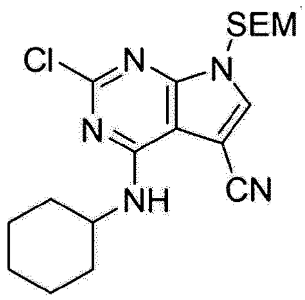

**<Preparation** Example 4-10> Preparation of 2-chloro-4-(cyclohexyloxy)-7-((2-**(trimethylsilyl)ethoxy)methyl)-7*H*-pyrrolo[2,3-d]pyrimidine-5-carbonitrile**

The title compound was prepared in the same manner as in Preparation Example 4-1 (yield: 62 %).

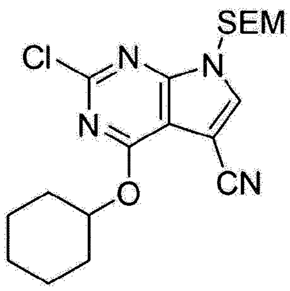

**<Preparation Example 4-11> Preparation of 2-chloro-4-(**

네오**pentylamino)-7-((2-(trimethylsilyl)ethoxy)methyl)-7*H*-pyrrolo[2,3-d]pyrimidine-5-carbonitrile**

The title compound was prepared in the same manner as in Preparation Example 2-1 (yield: 70 %).

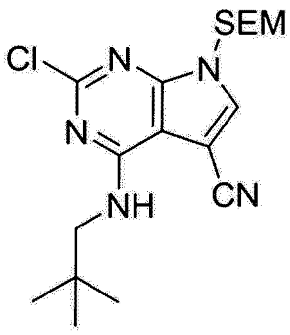

**<Preparation Example 5-1> Preparation of 2-chloro-4-cyclopropyl-7-((2-(trimethylsilyl)ethoxy)methyl)-7*H*-pyrrolo[2,3-b]pyrimidine-5-carbonitrile**

The title compound was prepared in a manner as depicted in the following Scheme 5.

[Scheme 5]

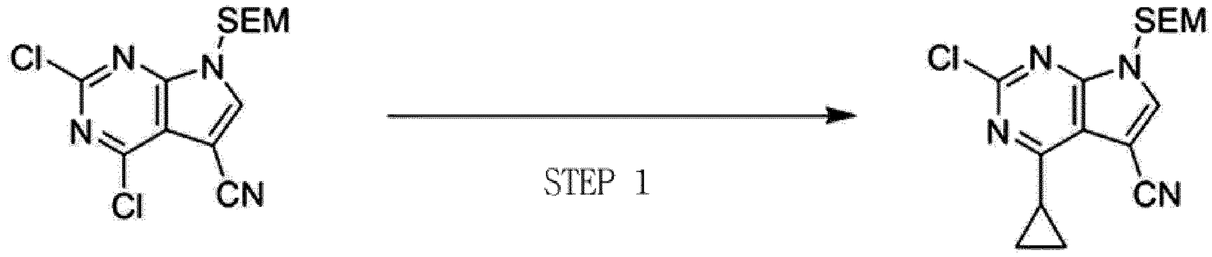


STEP 1: 2,4-dichloro-7-((2-(trimethylsilyl)ethoxy)methyl)-7*H*-pyrrolo[2,3-b]pyrimidine-5-carbonitrile (1.0 equivalent), cyclopropylboronic acid (1.5 equivalents), and K3PO4 (3.0 equivalents) were dissolved in 1,4-dioxane (0.20 M), and the resulting mixture was degassed by sonication for one minute. Pd(dppf)$Cl_2$ (0.1 equivalents) and $Ag_2O$ (0.5 equivalents) were added thereto under nitrogen, and the mixture was reacted at 90°C for 16 hours. The reaction mixture was filtered through a Celite filter, and washed several times with DCM. The resulting filtrate was concentrated, and then purified by MPLC (EtOAc:Hex), and the target compound was obtained (yield: 65 %).

**<Preparation Example 6-1> Preparation of 6-chloro-*N*-cyclohexyl-1-((2-(trimethylsilyl)ethoxy)methyl)-1*H*-pyrrolo[2,3-b]pyridine-4-amine**

The title compound was prepared in a manner as depicted in the following Scheme 6.

[Scheme 6]

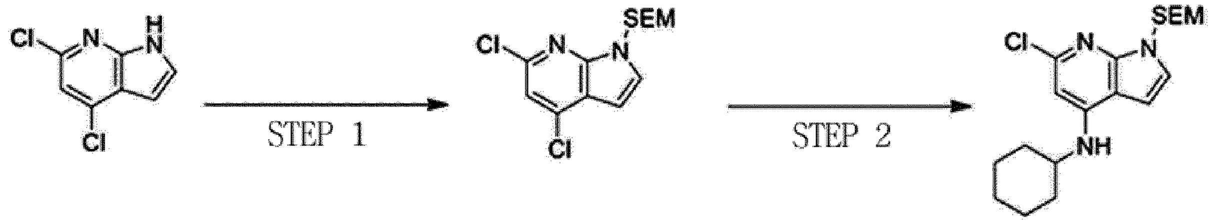


STEP 1: 4,6-dichloro-1*H*-pyrrolo[2,3-b]pyridine (1.0 equivalent) was dissolved in DMF (0.7 M) under nitrogen, and NaH (1.5 equivalents) was then slowly added thereto at 0°C. The reaction mixture was reacted at 0°C for 30 minutes, and (2-(chloromethoxy)ethyl)trimethylsilane (1.2 equivalents) was further added thereto, and then stirred at 25°C for 2 hours. Distilled water was added to the resulting reaction product, and the organic matter was then extracted with ethyl acetate (x3). After the collected organic layer was washed with brine, the remaining water was removed using $Na_2SO_4$, and the organic layer was concentrated under reduced pressure. The concentrated mixture was purified by MPLC (EtOAc:Hex), and the target compound was obtained as a white solid (yield: 88 %).

STEP 2: 4,6-dichloro- 1-((2-(trimethylsilyl)ethoxy)methyl)-1*H*-pyrrolo[2,3-b]pyridine (1.0 equivalent) was dissolved in NMP (1.0 M) under nitrogen, and cyclohexylamine (2.0 equivalents) was then slowly added thereto. The reaction mixture was stirred at 100°C for 16 hours. Distilled water was added to the resulting reaction product, and the organic matter was then extracted with EtOAc (x3). After the collected organic layer was washed with brine, the remaining water was removed using $Na_2SO_4$, and the organic layer was concentrated under reduced pressure. The concentrated mixture was purified by MPLC (EtOAc:Hex), and the target compound was obtained as white solid (yield: 58.4 %).

**<Preparation Example 7-1> Preparation of 6-chloro-*N*-cyclohexyl-1-((2-(trimethylsilyl)ethoxy)methyl)-1*H*-pyrrolo[2,3-b]pyridine-4-amine**

The title compound was prepared in a manner as depicted in the following Scheme 7.

[Scheme 7]

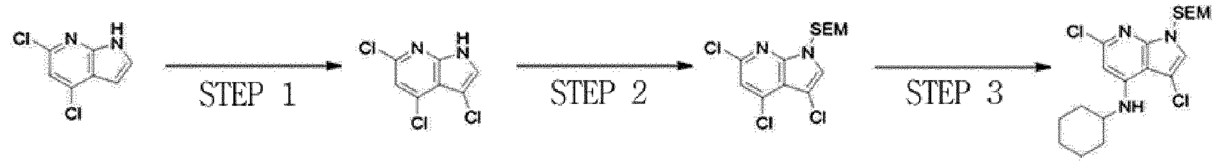


STEP 1: 4,6-dichloro-1*H*-pyrrolo[2,3-b]pyridine (1.0 equivalent) was dissolved in DMF (0.5 M) under nitrogen, and NCS (1.1 equivalents) was then slowly added thereto at 0°C. The reaction mixture was reacted at 0°C for 10 minutes, and stirred at room temperature for 4 hours. Ice water was added to the resulting reaction product, and the formed solid target compound was filtered (yield: 63 %).

STEP 2: 3,4,6-trichloro-1*H*-pyrrolo[2,3-b]pyridine (1.0 equivalent) was dissolved in DMF (1.1 M) under nitrogen, and NaH (1.5 equivalents) was then slowly added thereto at 0°C. The reaction mixture was reacted at 0°C for 30 minutes, and (2-(chloromethoxy)ethyl)trimethylsilane (1.2 equivalents) was further added thereto, and then stirred at 20°C for 4 hours. Distilled water was added to the resulting reaction product, and the organic matter was then extracted with ethyl acetate (x3). After the collected organic layer was washed with brine, the remaining water was removed using $Na_2SO_4$, and the organic layer was concentrated under reduced pressure. The concentrated mixture was purified by MPLC (EtOAc:Hex), and the target compound was obtained as a white solid (yield: 59 %).

STEP 3: 3,4,6-trichloro-1-((2-(trimethylsilyl)ethoxy)methyl)-1*H*-pyrrolo[2,3-b]pyridine (1.0 equivalent) was dissolved in NMP (1.0 M) under nitrogen, and cyclohexylamine (2.0 equivalents) was then slowly added thereto. The reaction mixture was stirred at 100°C for 16 hours. Distilled water was added to the resulting reaction product, and the organic matter was then extracted with EtOAc (x3). After the collected organic layer was washed with brine, the remaining water was removed using $Na_2SO_4$, and the organic layer was concentrated under reduced pressure. The concentrated mixture was purified by MPLC (EtOAc:Hex), and the target compound was obtained as a white solid (yield: 53 %).

**<Preparation Example 8-1> Preparation of 6-chloro-*N*-cyclohexyl-3-(trifluoromethyl)-1-((2-(trimethylsilyl)ethoxy)methyl)-1*H*-pyrrolo[2,3-b]pyridine-4-amine**

The title compound was prepared in a manner as depicted in the following Scheme 8.

[Scheme 8]

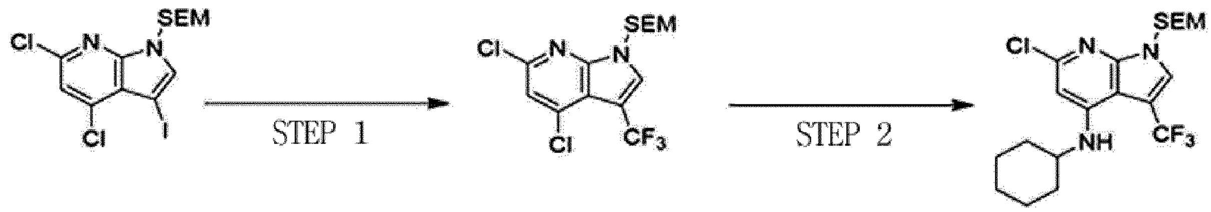


STEP 1: Moisture was removed while maintaining CuI (5.0 equivalents) and KF (5.0 equivalents) at a temperature of 200°C for 2 hours under reduced pressure close to a vacuum. The resulting reaction product was cooled to room temperature, and TMS-CF3 (5.0 equivalents) was then dissolved in DMF and NMP (1:1 ratio; a total of 0.2 M) under nitrogen, and slowly added to the reaction product through a syringe. The reaction mixture was reacted a room temperature for an hour, and 4,6-dichloro-3-iodo-1-((2-(trimethylsilyl)ethoxy)methyl)-1*H*-pyrrolo[2,3-b]pyridine (1.0 equivalent) was further dissolved in DMF and NMP (1:1 ratio; a total of 0.2 M) under nitrogen, and then slowly added through a syringe. The reaction mixture was stirred at 50°C for 16 hours. Distilled water was added to the resulting reaction product, and the organic matter was then extracted with ether (x3). After the collected organic layer was washed with brine, the remaining water was removed using $Na_2SO_4$, and the organic layer was concentrated under reduced pressure. The concentrated mixture was purified by MPLC (EtOAc:Hex), and target compound was obtained as a white solid (yield: 74 %).

STEP 2: 4,6-dichloro-3-(trifluoromethyl)-1-((2-(trimethylsilyl)ethoxy)methyl)-1*H*-pyrrolo[2,3-b]pyridine (1.0 equivalent) was dissolved in DMSO (1.0 M) under nitrogen, and $K_2CO_3$ (3.0 equivalents) and cyclohexylamine (2.0 equivalents) were then slowly added thereto. The reaction mixture was stirred at 100°C for 16 hours. Distilled water was added to the resulting reaction product, and the organic matter was then extracted with EtOAc (x3). After the collected organic layer was washed with brine, the remaining water was removed using $Na_2SO_4$, and the organic layer was

concentrated under reduced pressure. The concentrated mixture was purified by MPLC (EtOAc:Hex), and the target compound was obtained as a white solid (yield: 58 %).

**<Preparation Example 9-1> Preparation of 6-chloro-4-(cyclohexylamino)-1-((2-(trimethylsilyl)ethoxy)methyl)-1*H*-pyrrolo[2,3-b]pyridine-3-carbonitrile**

The title compound was prepared in a manner as depicted in the following Scheme 9.

[Scheme 9]

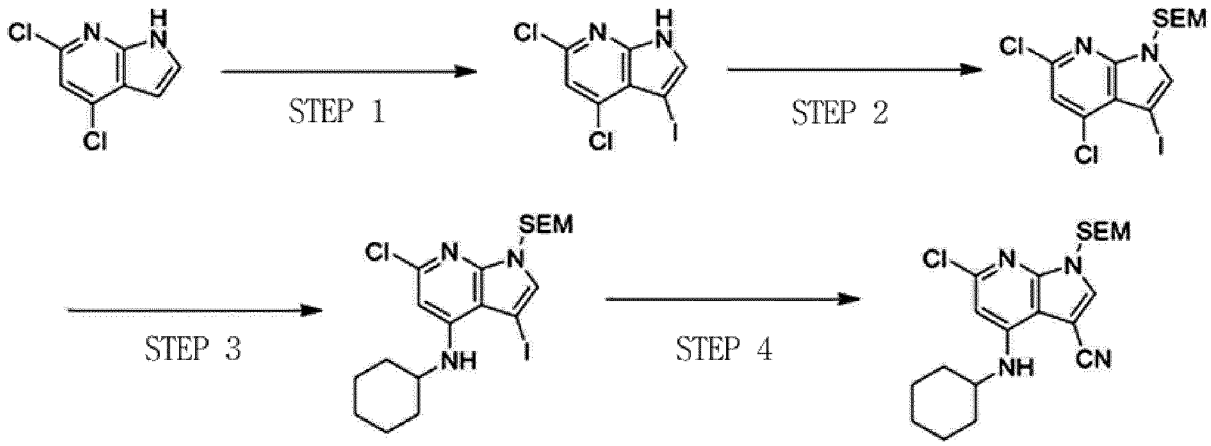


STEP 1: 4,6-dichloro-1*H*-pyrrolo[2,3-b]pyridine (1.0 equivalent) was dissolved in DCM (0.3 M) under nitrogen, and NIS (1.5 equivalents) was then slowly added thereto at 0°C. The reaction mixture was reacted at 0°C for 10 minutes, and stirred at room temperature for 4 hours. Ice water was added to the resulting reaction product, and the formed solid target compound was then filtered. The filtered target compound was washed with *n*-hexane to obtain the target compound as a light brown solid (yield: 91 %).

STEP 2: 4,6-dichloro-3-iodo-1*H*-pyrrolo[2,3-b]pyridine (1.0 equivalent) was dissolved in DMF (0.5 M) under nitrogen, and NaH (1.5 equivalents) was then slowly added thereto at 0°C. The reaction mixture was reacted at 0°C for 30 minutes, and (2-(2-(chloromethoxy)ethyl)trimethylsilane (1.2 equivalents) was further added, and then stirred at 20°C for 4 hours. Distilled water was added to the resulting reaction product, and the organic matter was than extracted with ether (x3). After the collected organic layer was washed with brine, the remaining water was removed using $Na_2SO_4$, and the organic layer was concentrated under reduced pressure. The concentrated mixture was purified by MPLC (EtOAc:Hex), and the target compound was obtained as a white solid (yield: 65 %).

STEP 3: 4,6-dichloro-3-iodo-1-((2-(trimethylsilyl)ethoxy)methyl)-1*H*-pyrrolo[2,3-b]pyridine (1.0 equivalent) was dissolved in EtOH (2.0 M), and cyclohexylamine (2.0 equivalents) was then slowly added thereto. The reaction mixture was stirred at 100°C for 16 hours. Distilled water was added to the resulting reaction product, and the organic matter was then extracted with ether (x3). After the collected organic layer was washed with brine, the remaining water was removed using $Na_2SO_4$, and the organic layer was concentrated under reduced pressure. The concentrated mixture was purified by MPLC (EtOAc:Hex), and the target compound was obtained as a white solid (yield: 44 %)

STEP 4: 6-chloro-*N*-cyclohexyl-3-iodo-1-((2-(trimethylsilyl)ethoxy)methyl)-1*H*-pyrrolo[2,3-b]pyridine-4-amine (1.0 equivalent) was dissolved in DMF (0.25 M), CuI (0.7 equivalents) and CuCN (2.0 equivalents) were then slowly added thereto. Pd(PPh3)4 (0.5 equivalents) was added at 50°C under nitrogen, and stirred at 80°C for 16 hours. Distilled water was added to the resulting reaction product, and the organic matter was then extracted with EtOAc (x3). After the collected organic layer was washed with brine, the remaining water was removed using $Na_2SO_4$, and the organic layer was concentrated under reduced pressure. The concentrated mixture was purified by MPLC (EtOAc:Hex), and the target

compound was obtained as a white solid (yield: 74 %).

**<Preparation Example 10-1> Preparation of 2-methoxy-4-(1-methyl-1*H*-pyrazol-3-yl)aniline**

The title compound was prepared in a manner as depicted in the following Scheme 10.

[Scheme 10]

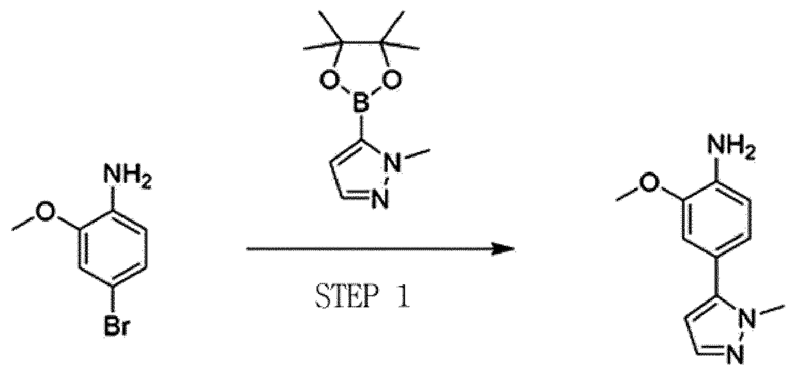


STEP 1: 1 M $Na_2CO_3$ (0.1 M) was added to a mixed solution of 1,4-dioxane (0.3 M) in which 4-bromo-2-methoxyaniline (1 equivalent) and 1-methyl-5-(4,4,5,5-tetramethyl-1,3,2-dioxaborolan-2-yl)-1*H*-pyrazole (1 equivalent) were dissolved, and the resulting mixture was sonicated while allowing nitrogen to flow for 10 minutes. Pd(PPh3)4 (0.1 equivalents) was added to the reaction mixture, and the reaction mixture was stirred at 95°C for 3 hours. When the reaction was completely terminated, the reaction mixture was extracted with ethyl acetate and water. The collected organic layer was washed with saline, dried over anhydrous sodium sulfate, concentrated under reduced pressure, and then purified by MPLC (EtOAc/Hexane) to obtain the target compound (yield 73%).

MS (m/z): 204.2 [M+H]+, UPLC r.t. (min): 0.94

$^1$H NMR (400 MHz, DMSO) δ 7.38 (d, J = 1.8 Hz, 1H), 6.88 (d, J = 1.8 Hz, 1H), 6.82 (dd, J = 8.0, 1.8 Hz, 1H), 6.70 (d, J = 8.0 Hz, 1H), 6.24 (d, J = 1.8 Hz, 1H), 4.99 (s, 2H), 3.81 (d, J = 1.4 Hz, 6H).

**<Preparation Example 10-2> Preparation of 4-(1-methyl-1*H*-pyrazol-3-yl)aniline**

The title compound was prepared in a similar manner as in Preparation Example 10-1.

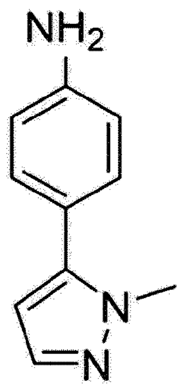

**<Preparation Example 10-3> Preparation of 5-(1-methyl-1*H*-pyrazol-3-yl)pyridine-2-amine**

The title compound was prepared in a similar manner as in Preparation Example 10-1.

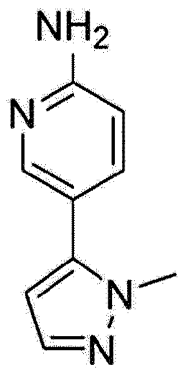

**<Preparation Example 10-4> Preparation of 2-ethoxy-4-(1-methyl-1*H*-pyrazol-3-yl)aniline**

The title compound was prepared in a similar manner as in Preparation Example 10-1.

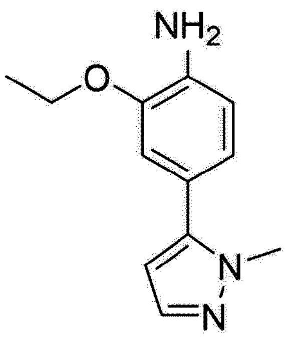

**<Preparation Example 10-5> Preparation of 4-(3,5-dimethylisooxazol -4-yl)-2-methoxyaniline**

The title compound was prepared in a similar manner as in the following Scheme 10-1.

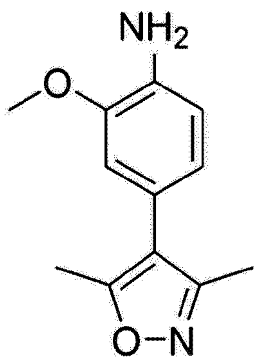

**<Preparation** Example 10-6> Preparation of 4-(6-fluoropyridin-3-yl)-2-methoxyaniline

The title compound was prepared in a similar manner as in Preparation Example 10-1.

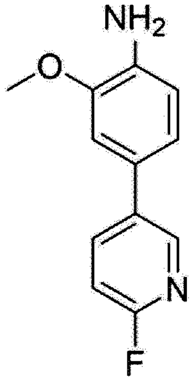

**<Preparation Example 10-7> Preparation of 1-(4-((4'-amino-3'-methoxy-[1,1'-biphenyl]-4-yl)methyl)piperazin-1-yl)ethan-1-one**

The title compound was prepared in a similar manner as in Preparation Example 10-1.

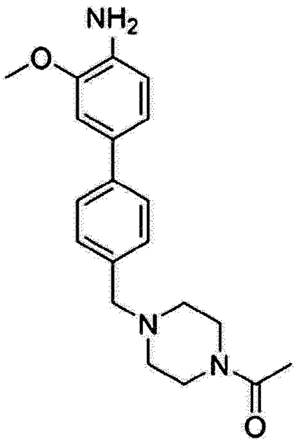

**<Preparation Example 10-8> Preparation of 2-methoxy-4-(1-methyl-1*H*-pyrazol-4-yl)aniline**

The title compound was prepared in the same manner as in the following Scheme 10-8.

[Scheme 10-8]

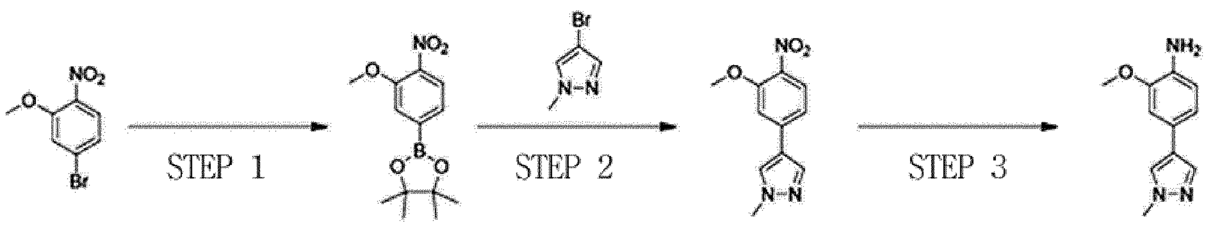


STEP 1: 4-bromo-2-methoxy-1-nitro-benzene (1 equivalent) and 4,4,5,5-tetramethyl-2-(4,4,5,5-tetramethyl-1,3,2-dioxaborolan-2-yl)-1,3,2-dioxaborolane (1.1 equivalents) were dissolved in 1,4-dioxane, and AcOK (2.5 equivalents) and Pd(dppf)$Cl_2$·$CH_2Cl_2$ (0.06 equivalents) were then added thereto at room temperature under nitrogen. The reaction solution was reacted at 90°C for 12 hours. After the reaction, the reaction solution was filtered through a Celite filter, and washed with EA. The washed organic solvent was concentrated under reduced pressure, and then subjected to silica gel chromatography (Petroleum ether/ethyl acetate=10/1 to 5/1) to obtain a white target compound (yield: 87.29%).

MS: m/z 198.0 $[M-82]^+$

$^1H$ NMR (400 MHz, CHLOROFORM-*d*) δ = 7.81 - 7.76 (m, 1H), 7.51 - 7.48 (m, 1H), 7.47 - 7.43 (m, 1H), 4.02 - 3.99 (m, 3H), 1.39 - 1.36 (m, 12H)

STEP 2: The 2-(3-methoxy-4-nitro-phenyl)-4,4,5,5-tetramethyl-1,3,2-dioxaborolane (1 equivalent) obtained in STEP 1 of Preparation Example 10-8, and 4-iodo-1-methylpyrazole (1.2 equivalents) were dissolved in 1,4-dioxane (0.3 M) and $H_2O$ (0.1 M) solvents under nitrogen, and $K_2CO_3$ (2.0 equivalents) and $Pd(dppf)Cl_2 \cdot CH_2Cl_2$ (0.01 equivalents) were further added thereto. Then, the reaction solvent was reacted at 90°C for 12 hours. After the reaction, the reaction solvent was filtered through a Celite filter, and washed with EA. The washed organic solvent was concentrated under reduced pressure, and then purified by silica gel chromatography a yellow target compound (yield 34.40%).

MS: m/z 234.2 $[M+H]^+$

$^1H$ NMR (400 MHz, CHLOROFORM-*d*) δ = 8.17 - 8.13 (m, 1H), 7.99 - 7.96 (m, 1H), 7.88 - 7.84 (m, 1H), 7.42 - 7.39 (m, 1H), 7.29 - 7.23 (m, 1H), 4.03 - 3.99 (m, 3H), 3.97 - 3.93 (m, 3H)

STEP 3: The 4-(3-methoxy-4-nitro-phenyl)-1-methyl-pyrazole (1 equivalent), obtained in STEP 2 of Preparation Example 10-8, and Fe (5 equivalents) were dissolved in EtOH (0.3 M) and $H_2O$ (0.03 M), and $NH_4Cl$ (5 equivalents) was added thereto. The reaction solvent was reacted at 80°C for 12 hours. After the reaction, the reaction solvent was filtered through a Celite filter, and washed with EA. The washed organic solvent was concentrated under reduced pressure, washed with water, and then filtered to obtain a dark brown target compound (yield: 91.80%).

MS: m/z 204.2 $[M+H]^+$

$^1H$ NMR (400 MHz, METHANOL-$d_4$) δ = 7.86 - 7.80 (m, 1H), 7.75 - 7.66 (m, 1H), 7.07 - 6.91 (m, 2H), 6.79 (br d, J = 7.7 Hz, 1H), 3.90 (s, 6H)

**<Preparation Example 10-9> Preparation of 2-methoxy-4-(1-methyl-1*H*-1,2,3-triazol-4-yl)aniline**

The title compound was prepared in a similar manner as in Preparation Example 10-8.

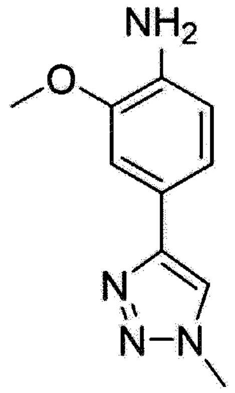

**<Preparation Example 10-10> Preparation of 2-methoxy-4-(1-methyl-1*H*-1,2,4-triazol-3-yl)aniline**

The title compound was prepared in a similar manner as in Preparation Example 10-8.

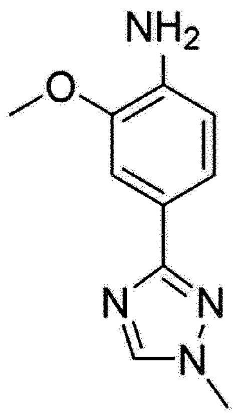

**<Preparation Example 10-11> Preparation of 2-methoxy-4-(1-methyl-1*H*-1,2,4-triazol-5-yl)aniline**

The title compound was prepared in a similar manner as in Preparation Example 10-8.

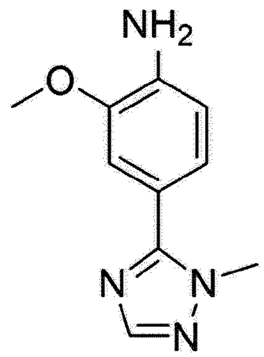

**<Preparation Example 10-12> Preparation of 2-ethoxy-4-(1-methyl-1*H*-1,2,4-triazol-3-yl)aniline**

The title compound was prepared in a similar manner as in Preparation Example 10-8.

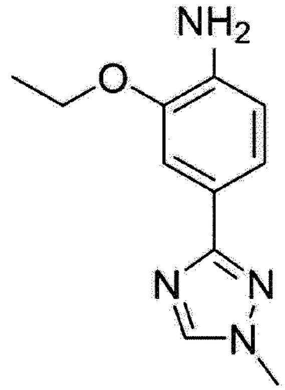

**<Preparation Example 10-13> Preparation of 1-(4-((4'-amino-[1,1'-biphenyl]-4-yl)methyl)piperazin-1-yl)ethan-1-one**

The title compound was prepared in a similar manner as in Preparation Example 10-8.

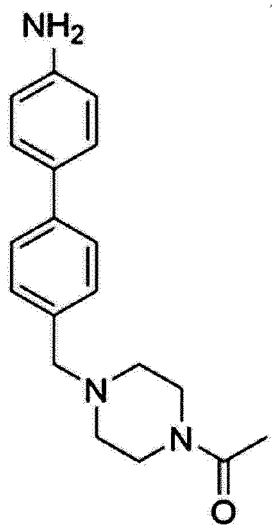

**<Preparation Example 10-14> Preparation of 4'-((4-cyclopropylpiperazin-1-yl)methyl)-[1,1'-biphenyl]-4-amine**

The title compound was prepared in a similar manner as in Preparation Example 10-8.

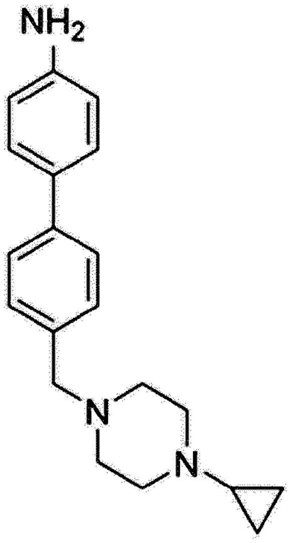

**<Preparation Example 10-15> Preparation of 2-methoxy-4-(1-(oxetan-3-yl)-1*H*-pyrazol-4-yl)aniline**

The title compound was prepared in a similar manner as in Preparation Example 10-8.

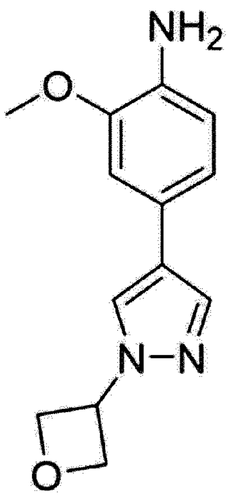

MS: m/z 246.1 $[M+H]^+$

$^1H$ NMR (400 MHz, METHANOL-$d_4$) $\delta$ = 8.02 (s, 1H), 7.87 - 7.84 (m, 1H), 7.07 - 7.03 (m, 1H), 7.00 - 6.95 (m, 1H), 6.82 - 6.75 (m, 1H), 5.61 - 5.50 (m, 1H), 5.09 - 5.03 (m, 4H), 3.94 - 3.87 (m, 3H)

**<Preparation Example 10-16> Preparation of 2-methoxy-4-(1-(tetrahydro-2*H*-pyran-4-yl)-1*H*-pyrazol-4-yl)aniline**

The title compound was prepared in a similar manner as in Preparation Example 10-8.

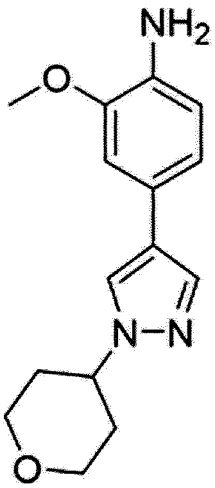

MS: m/z 274.1 $[M+H]^+$

$^1$H NMR (400 MHz, DMSO-$d_6$) δ = 8.09 - 8.03 (m, 1H), 7.75 - 7.68 (m, 1H), 7.02 - 6.97 (m, 1H), 6.93 - 6.87 (m, 1H), 6.63 - 6.56 (m, 1H), 4.67 - 4.61 (m, 1H), 4.01 - 3.91 (m, 2H), 3.84 - 3.77 (m, 3H), 3.52 - 3.42 (m, 2H), 2.03 - 1.88 (m, 4H)

**<Preparation Example 10-17> Preparation of tert-butyl 4-(4-(4-amino-3-methoxyphenyl)-1*H*-pyrazol-1-yl)piperidine-1-carboxylate**

The title compound was prepared in a similar manner as in Preparation Example 10-8.

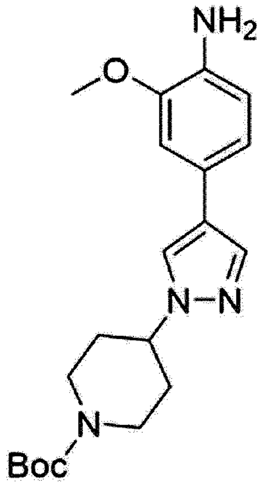

MS: m/z 373.2 $[M+H]^+$

$^1$H NMR (400 MHz, METHANOL-$d_4$) δ = 7.95 - 7.89 (m, 1H), 7.76 - 7.72 (m, 1H), 7.05 - 7.01 (m, 1H), 6.99 - 6.92 (m, 1H), 6.79 - 6.71 (m, 1H), 4.40 - 4.29 (m, 1H), 4.26 - 4.18 (m, 2H), 3.93 - 3.85 (m, 3H), 3.04 - 2.87 (m, 2H), 2.15 - 2.05 (m, 2H), 2.01 - 1.88 (m, 2H), 1.53 - 1.46 (m, 9H)

**<Preparation Example 10-18> Preparation of 2-methoxy-4-(4-methyl-4*H*-1,2,4-triazol-3-yl)aniline**

The title compound was prepared in the same manner as in the following Scheme 10-18.

[Scheme 10-18]

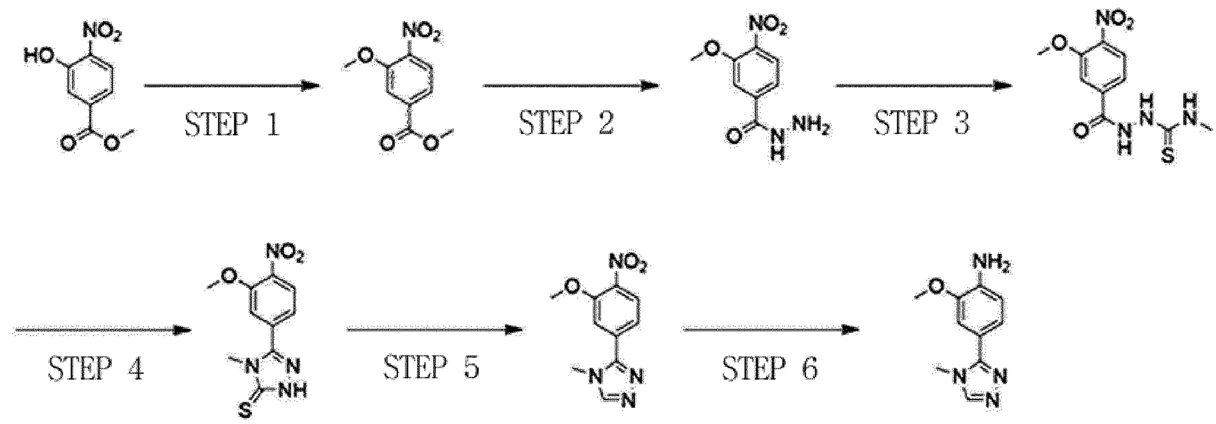


STEP 1: 3-hydroxy-4-nitro-benzoate (1 equivalent) was put into a round flask, and dissolved in DMF (0.3 M), and $K_2CO_3$ (3 equivalents) was further added thereto. Thereafter, iodomethane (2 equivalents) was added dropwise to the reaction solvent, and the reaction solvent was reacted at room temperature for 15 hours. After the reaction, water was added to the reaction solvent, and the reaction solvent was then precipitated, and filtered to obtain the target compound as a light yellow (yield: 87.76%).

MS: m/z 212.1 $[M+H]^+$

$^1$H NMR (400 MHz, CHLOROFORM-*d*) $\delta$ = 7.83 (d, J = 8.4Hz, 1H), 7.76 (s, 1H), 7.69 (d, J = 6.8Hz, 1H), 4.02 (s, 3H), 3.97 (s, 3H)

STEP 2: Methyl 3-methoxy-4-nitro-benzoate (1 equivalent) was put into a round flask, and dissolved in MeOH (0.3 M), and an $NH_2NH_2 \cdot H_2O$ solution (3 equivalents) was then added thereto. The reaction solution was reacted at 80°C for 12 hours. After the reaction, the reaction solution was cooled to room temperature, and the precipitated solid was filtered to obtain a light yellow target compound (yield: 95.74%).

MS: m/z 212.2 $[M+H]^+$

1H NMR (400 MHz, CHLOROFORM-*d*) $\delta$ = 10.07 (s, 1H), 7.95 (d, J = 8.4Hz, 1H), 7.71 (s, 1H), 7.52 (d, J = 6.8Hz, 1H), 4.62 (br, 2H), 3.98 (s, 3H)

STEP 3: 3-methoxy-4-nitro-benzohydrazide (1 equivalent) was put into a round flask, and dissolved in a THF (0.3 M) solvent, and methylimino(thio)methane (1 equivalent) was then added to the reaction solvent. The reaction solution was reacted at 70°C for 2 hours, cooled to room temperature, and then reacted at room temperature for 10 hours. The reaction solution was filtered, and washed with petroleum ether. Then, the solid was dried to obtain a yellow target compound (yield: 83.19%).

MS: m/z 285.2 $[M+H]^+$

1H NMR (400MHz, DMSO-$d_6$) $\delta$ = 9.43 (br, 1H), 8.00 (d, J = 8.4Hz, 1H), 7.79 (s, 1H), 7.62 (d, J = 6.8Hz, 1H), 3.99 (s, 3H), 2.90 (s, 3H)

STEP 4: 1-[(3-methoxy-4-nitro-benzoyl)amino]-3-methyl-thiourea (1 equivalent) was added to 1 M $NaHCO_3$ (2.54 equivalents) in a round flask, and the reaction solution was then reacted at 100°C for 4 hours. After the reaction, the reaction solution was cooled to room temperature, and the solid was filtered, and washed with water. The filtered solid was dried under reduced pressure to obtain a light yellow target compound (yield: 87.7%).

MS: m/z 267.2 $[M+H]^+$

$^1$H NMR (400MHz, DMSO-$d_6$) $\delta$ = 14.1 (s, 1H), 8.05 (d, J = 8.4, 1H), 7.67 (s, 1H), 7.46 (d, J = 8.4, 1H), 3.98 (s, 3H), 3.34 (s, 3H)

STEP 5: 3-(3-methoxy-4-nitro-phenyl)-4-methyl-1H-1,2,4-triazole-5-thione was dissolved in DCM (0.3 M), and a hydrogen peroxide-AcOH solution (3 equivalents) was then added dropwise at 0°C. After the addition, the resulting mixture was reacted at room temperature for 12 hours. After the reaction was completed, the reaction was stopped using an aqueous $Na_2SO_3$ solution, and a NaOH-saturated solution was added to the reaction solution so that the pH of the mixture was adjusted to pH 10. Then, the reaction solution was diluted with water, extracted with EtOAc, and then concentrated under reduced pressure to obtain the target compound as a brown solid (yield: 81.56%).

STEP 6: The 3-(3-methoxy-4-nitro-phenyl)-4-methyl-1,2,4-triazole (1 equivalent) obtained in STEP 5 of Preparation Example 10-18, and Fe (5 equivalents) were dissolved in EtOH (0.3 M) and $H_2O$ (0.03 M), and $NH_4Cl$ (5 equivalents) was further added thereto. The reaction solvent was reacted at 80°C for 12 hours. After the reaction, the reaction solvent was filtered through a Celite filter, and washed with EA. The washed organic solvent was concentrated under reduced pressure, washed with water, and then filtered to obtain a light gray target compound (yield: 94.3%).

MS: m/z 205.2 $[M+H]^+$

$^1H$ NMR (400MHz, MeOD) $\delta$ = 8.52 (s, 1H), 7.13 (s, 1H), 7.06 (d, J = 9.6Hz, 1H), 6.83 (d, J = 8.0Hz, 1H), 3.89 (s, 3H), 3.84 (s, 3H)

**<Preparation Example 10-19> Preparation of 2-ethoxy-4-(4-methyl-4*H*-1,2,4-triazol-3-yl)aniline**

The title compound was prepared in a similar manner as in Preparation Example 10-18.

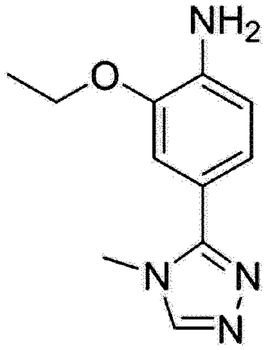

MS: m/z 219.3 $[M+H]^+$

$^1H$ NMR (400 MHz, METHANOL-$d_4$) $\delta$ = 8.46 (s, 1H), 7.16 - 7.10 (m, 1H), 7.09 - 7.04 (m, 1H), 6.88 - 6.83 (m, 1H), 4.18 - 4.09 (m, 2H), 3.78 (s, 3H), 1.50 - 1.41 (m, 3H)

**<Preparation Example 10-20> Preparation of 4-(6,7-dihydro-5*H*-pyrrolo[2,1-c][1,2,4]triazol-3-yl)-2-methoxyaniline**

The title compound was prepared in the same manner as in the following Scheme 10-20.

[Scheme 10-20]

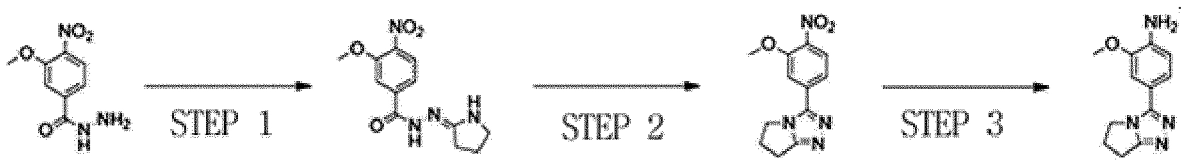


STEP 1: The 3-methoxy-4-nitro-benzohydrazide (1 equivalent) obtained in STEP 2 of Preparation Example 10-18 was dissolved in MeOH (0.3 M), and 5-methoxy-3,4-dihydro-2*H*-pyrrole (1 equivalent) was further added thereto. The reaction solution was reacted at room temperature for 48 hours. The resulting solid was filtered, and then dried under reduced pressure to obtain a yellow target compound (yield: 66.4%).

MS: m/z 279.2 $[M+H]^+$

$^1H$ NMR (400MHz, DMSO-$d_6$) $\delta$ = 7.85-7.67 (m, 3H), 3.97 (s, 3H), 3.70-3.59 (m, 2H), 2.67-2.64 (m, 2H), 2.33-2.04 (m, 2H)

STEP 2: (E)-3-methoxy-4-nitro-*N'*-(pyrrolidin-2-ylidene)benzohydrazide (1 equivalent) was added to 1 M $NaHCO_3$ (2.54 equivalents) in a round flask, and the reaction solution was then reacted at 100°C for 12 hours. After the reaction, the reaction solution was cooled to 0°C, and the solid was then filtered, and washed with water. The filtered solid was dried under reduced pressure to obtain a light yellow target compound (yield: .33.6%)

MS: m/z 261.2 $[M+H]^+$

1H NMR (400MHz, $CDCl_3$) $\delta$ = 7.95 (d, J = 8.4Hz, 1H), 7.85 (s, 1H), 7.27 (d, J = 8.4Hz, 1H), 4.30-4.26 (m, 2H), 4.05 (s, 3H), 3.09-3.06 (m, 2H), 2.90-2.88 (m, 2H)

STEP 3: The 3-(3-methoxy-4-nitrophenyl)-6,7-dihydro-5*H*-pyrrolo[2,1-c][1,2,4]triazole (1 equivalent) obtained in STEP 2 of Preparation Example 10-20, and Fe (5 equivalents) were dissolved in EtOH (0.3 M) and $H_2O$ (0.03 M), and $NH_4Cl$ (5 equivalents) was further added thereto. The reaction solvent was reacted at 80°C for 12 hours. After the reaction, the reaction solvent was filtered through a Celite filter, and washed with EA. The washed organic solvent was concentrated under reduced pressure, washed with water, and then filtered to obtain a light gray target compound (yield:

82.2%).

MS: m/z 231.2 $[M+H]^+$

$^1H$ NMR (400MHz, MeOD) δ = 7.30 (s, 1H), 7.15 (d, J = 7.6Hz, 1H), 6.78 (d, J = 8.4Hz, 1H), 4.24-4.20 (m, 2H), 3.91(s, 3H), 2.96 - 2.93 (m, 2H), 2.83 - 2.78 (m, 2H)

**<Preparation Example 10-21> Preparation of 4-(6,7-dihydro-5*H*-pyrrolo[2,1-c][1,2,4]triazol-3-yl)-2-ethoxyaniline**

The title compound was prepared in a similar manner as in Preparation Example 10-20.

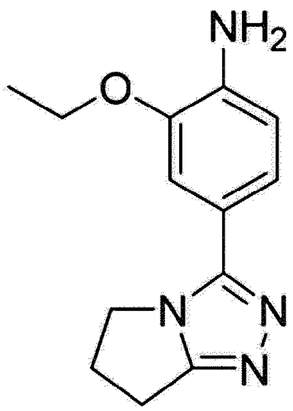

MS: m/z 245.3 $[M+H]^+$

$^1H$ NMR (400 MHz, DMSO-$d_6$) δ = 7.23 (s, 1H), 7.15 - 7.07 (m, 1H), 6.73 - 6.68 (m, 1H), 5.10 (br d, 2H), 4.20 - 4.12 (m, 2H), 4.10 - 4.02 (m, 2H), 2.85 - 2.78 (m, 2H), 2.72 - 2.61 (m, 2H), 1.37 (m, 3H)

**<Preparation Example 10-22> Preparation of 4-(2,4-dimethyl-1*H*-imidazol-1-yl)-2-methoxyaniline**

The title compound was prepared in the same manner as in the following Scheme 10-22.

[Scheme 10-22]

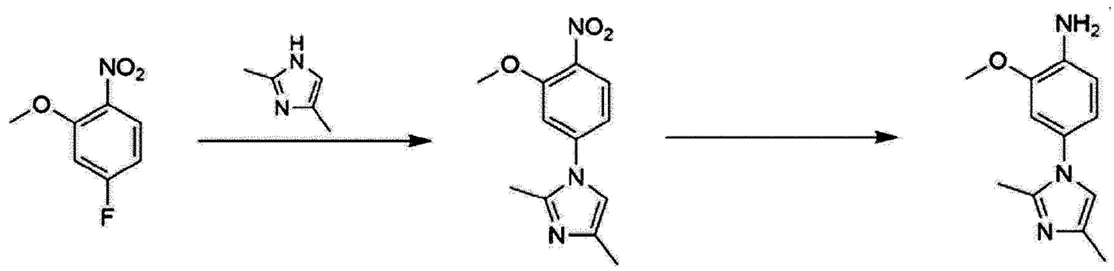

STEP 1: 4-fluoro-2-methoxy-1-nitro-benzene (1 equivalent) and 2,4-dimethyl-1*H*-imidazole (1 equivalent) were dissolved in DMF (0.1 M), and $K_2CO_3$ (2 equivalents) was added thereto. The reaction solution was reacted at 70°C for 52 hours. When the reaction was completed, the reaction solution was diluted with water, and the organic matter was then extracted with EtOAc (x2). After the collected organic layer was washed with brine, the remaining water was removed using $Na_2SO_4$, and concentrated under reduced pressure. The concentrated solid mixture was dried to obtain 1-(3-methoxy-4-nitro-phenyl)-2,4-dimethylimidazole as a white solid (yield: 57.68%).

$^1H$ NMR (400 MHz, DMSO-$d_6$) δ 8.02 (d, *J* = 8.4 Hz, 1H), 7.38 (d, *J* = 2.4 Hz, 1H), 7.17 (dd, *J* = 8.4, 2.4 Hz, 1H), 7.13 (d, *J* = 0.8 Hz, 1H), 3.98 (s, 3H), 2.34 (s, 3H), 2.10 (d, *J* = 0.8 Hz, 3H).

STEP 2: The 1-(3-methoxy-4-nitro-phenyl)-2,4-dimethyl-imidazole (1 equivalent) obtained in STEP 1 of Preparation Example 10-20 was dissolved in EtOH (0.1 M), and Pd/C (10% purity) was further added thereto. After a reaction flask was depressurized, the resulting mixture was reacted at room temperature for 10 hours under hydrogen. After the reaction, the reaction solution was filtered through a Celite filter to remove the Pd/C, and then washed with EtOAc. The filtered organic layer was concentrated under reduced pressure to obtain 4-(2,4-dimethyl-1H-imidazol-1-yl)-2-methoxyaniline as a white solid (yield: 89.74%).

MS: m/z: 218.2 $[M+H^+]$;

$^1H$ NMR (DMSO-$d_6$, 400 MHz) δ 6.81 (d, 1H, $J$ = 0.8 Hz), 6.77 (d, 1H, $J$ = 1.6 Hz), 6.6-6.7 (m, 2H), 4.93 (s, 2H), 3.78 (s, 3H), 2.16 (s, 3H), 2.06 (d, 3H, $J$ = 0.8 Hz)

**<Example 1> Preparation of compound according to the present invention**

The pyrrolopyridine and pyrrolopyrimidine derivative compounds according to the present invention were prepared in a manner as depicted in the following Scheme 11.

[Scheme 11]

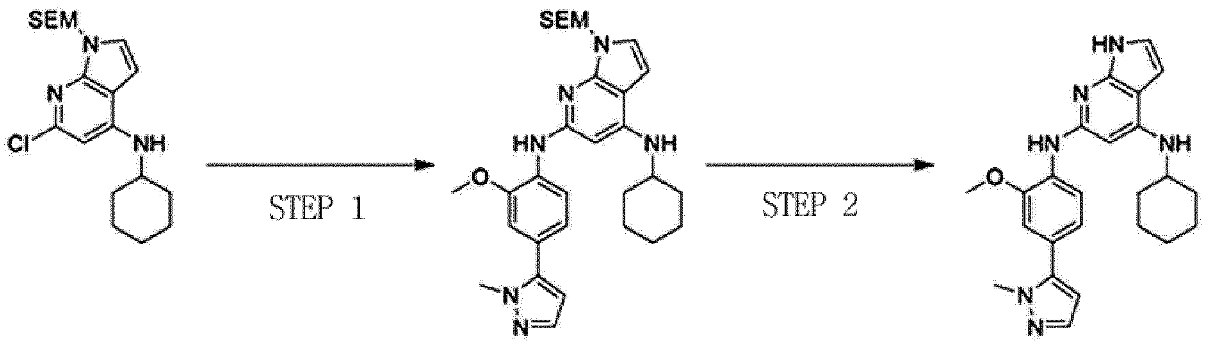


STEP 1: The compound 6-chloro-*N*-cyclohexyl-1-((2-(trimethylsilyl)ethoxy)methyl)-1*H*-pyrrolo[2,3-b]pyridine-4-amine (1.0 equivalent) prepared in Preparation Example 6-1, the 2-methoxy-4-(1-methyl-1*H*-pyrazol-3-yl)aniline (1.2 equivalents) prepared in Preparation Example 10-1, and $K_2CO_3$(5.0 equivalents) were added and dissolved in sec-BuOH (0.1 M), and then degassed by sonication for one minute. Pd2(dba)3 (0.1 equivalents) and Xphos (0.1 equivalents) were added to the reaction mixture at 80°C under nitrogen, and then stirred at 100°C for 2 hours. The reaction mixture was filtered through a Celite filter, and washed with ethyl acetate. The resulting filtrate was concentrated, and the resulting liquid mixture was used in the next step without any further purification (yield: 100%).

STEP 2: $N^4$-cyclohexyl-$N^6$-(2-methoxy-4-(1-methyl-1*H*-pyrazol-3-yl)phenyl)-1-((2-(trimethylsilyl)ethoxy)methyl)-1*H*-pyrrolo[2,3-b]pyridine-4,6-diamine (1.0 equivalent) was dissolved in $CH_2Cl_2$ (0.1 M), and TFA (70 equivalents) was then added thereto at room temperature. After reacting for 2 hours, the solvent was removed. The concentrated mixture was again dissolved in 1,4-dioxane (0.1 M), and $NH_4OH$ (0.1 M) was then added thereto. Then, the mixture was reacted at room temperature for 2 hours. After the reaction, the solvent was removed by concentration under reduced pressure. The concentrated mixture was purified by Pre-HPLC, and a solid target compound was obtained (yield: 51 %).

Compounds of Examples 2 to 177 were prepared in a similar manner as in Example 1. The chemical structures, the compound names, and NMR and mass analysis results of the compounds of Examples 1 to 177 are summarized and listed in Table 1 below.

[Table 1]

| 실시예 화합물 | 구조 | 화합물명 | $^1$H NMR, MS | UPLC r.t. (min) |
|---|---|---|---|---|
| 1 | 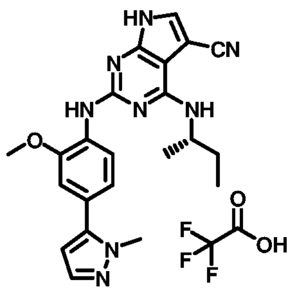 | (*S*)-4-(*sec*-butylamino)-2-((2-methoxy-4-(1-methyl-1*H*-pyrazol-5-yl)phenyl)amino)-7*H*-pyrrolo[2,3-*d*]pyrimidine-5-carbonitrile 2,2,2-trifluoro acetate | 1H NMR (400 MHz, DMSO-$d_6$) δ 12.40 (s, 1H), 8.55 (d, *J* = 8.3 Hz, 1H), 7.91 (d, *J* = 1.8 Hz, 1H), 7.78 (s, 1H), 7.46 (d, *J* = 1.9 Hz, 1H), 7.15 (d, *J* = 1.9 Hz, 1H), 7.09 (dd, *J* = 8.3, 1.9 Hz, 1H), 6.40 (d, *J* = 1.9 Hz, 1H), 5.97 (s, 1H), 4.28-4.14 (m, 1H), 3.96 (s, 3H), 3.88 (s, 3H), 1.65 (ddd, *J* = 26.8, 13.9, 6.9 Hz, 2H), 1.27 (d, *J* = 6.5 Hz, 3H), 0.95 (t, *J* = 7.4 Hz, 3H). MS: m/z 417.4 $[M+H]^+$ | 1.77 |
| 2 | 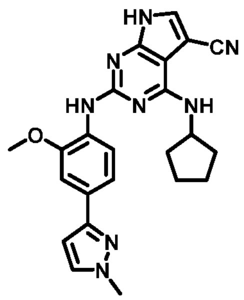 | 4-(cyclopentylamino)-2-((2-methoxy-4-(1-methyl-1*H*-pyrazol-3-yl)phenyl)amino)-7*H*-pyrrolo[2,3-*d*]pyrimidine-5-carbonitrile | 1H NMR (400 MHz, DMSO-$d_6$) δ 12.76 (s, 1H), 8.31 (d, *J* = 8.5 Hz, 1H), 7.97 (s, 1H), 7.72 (d, *J* = 2.2 Hz, 1H), 7.44 (s, 1H), 7.37 (d, *J* = 8.5 Hz, 1H), 7.21 (s, 1H), 7.09 (s, 1H), 6.71 (d, *J* = 2.2 Hz, 1H), 4.54 - 4.39 (m, 1H), 3.95 (s, 3H), 3.88 (s, 3H), 2.09 (s, 2H), 1.74 (d, *J* = 12.0 Hz, 2H), 1.70 - 1.58 (m, 4H). MS: m/z 429.4 $[M+H]^+$ | 1.85 |
| 3 | 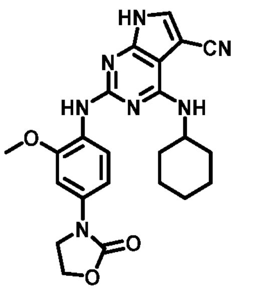 | 4-(cyclohexylamino)-2-((2-methoxy-4-(2-oxooxazolidin-3-yl)phenyl)amino)-7*H*-pyrrolo [2,3-*d*]pyrimidine-5-carbonitrile | 1H NMR (400 MHz, DMSO-*d*6) δ 12.13 (s, 1H), 8.32 (d, *J* = 8.8 Hz, 1H), 7.83 (s, 1H), 7.46 - 7.32 (m, 2H), 6.98 (dd, *J* = 8.8, 2.4 Hz, 1H), 5.69 (d, *J* = 7.7 Hz, 1H), 4.43 (dd, *J* = 9.1, 6.9 Hz, 2H), 4.08 (dd, *J* = 9.1, 7.0 Hz, 3H), 3.87 (s, 3H), 1.99 (dd, *J* = 8.6, 4.4 Hz, 2H), 1.74 (d, *J* = 10.9 Hz, 2H), 1.61 (d, *J* = 12.8 Hz, 1H), 1.47 - 1.34 (m, 4H), 1.25 (d, *J* = 11.3 Hz, 1H). MS: m/z 448.4 $[M+H]^+$ | 1.75 |

(continued)

| 실시 예 화합 물 | 구조 | 화합물명 | $^1$H NMR, MS | UPLC r.t. (min) |
|---|---|---|---|---|
| 4 | 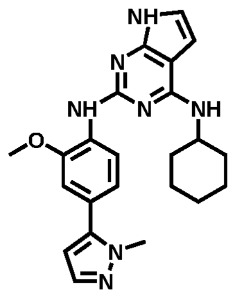 | *N*4-cyclohexyl-*N*2-(2-methoxy-4-(1-methyl-1*H*-pyrazol-5-yl) phenyl)-7*H*-pyrrolo[2,3-*d*] pyrimidine-2,4-diamine | 1H NMR (400 MHz, Methanol-$d_4$) δ 7.54 (d, *J* = 2.0 Hz, 1H), 7.22 - 7.17 (m, 1H), 7.14 (dd, *J* = 8.3, 1.8 Hz, 1H), 7.01 (s, 1H), 6.75 - 6.70 (m, 1H), 6.42 (d, *J* = 2.0 Hz, 1H), 4.05 (s, 3H), 3.93 (s, 3H), 2.13 (s, 2H), 1.93 (s, 2H), 1.79 (d, *J* = 13.2 Hz, 1H), 1.52 (d, *J* = 10.5 Hz, 4H), 1.31 (d, *J* = 3.1 Hz, 2H) MS: m/z 418.2 $[M+H]^+$ | 1.45 |
| 5 | 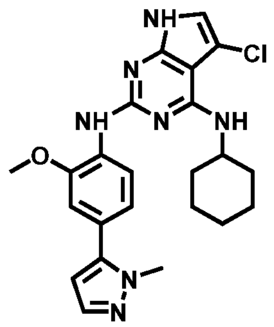 | 5-chloro-*N*4-cyclohexyl-*N*2-(2-methoxy-4-(1-methyl-1*H*-pyrazol-5-yl)phenyl)-7*H*-pyrrolo[2,3-d]pyrimidine-2,4-diamine | 1H NMR (400 MHz, Methanol-$d_4$) δ 8.44 (d, *J* = 8.3 Hz, 1H), 7.54 (d, *J* = 2.0 Hz, 1H), 7.21 (d, *J* = 1.8 Hz, 1H), 7.14 (dd, *J* = 8.3, 1.8 Hz, 1H), 7.06 (s, 1H), 6.42 (d, *J* = 2.0 Hz, 1H), 4.04 (s, 3H), 3.93 (s, 3H), 2.13 (d, *J* = 11.4 Hz, 2H), 1.89 (d, *J* = 12.1 Hz, 2H), 1.75 (d, *J* = 12.7 Hz, 1H), 1.61 - 1.46 (m, 4H), 1.37 (s, 1H), 1.32 (s, 1H). MS: m/z 451.2 $[M+H]^+$ | 1.88 |
| 6 | 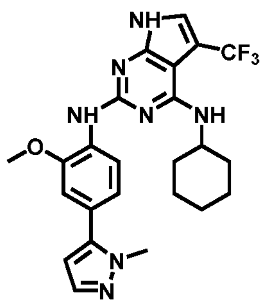 | *N*4-cyclohexyl-*N*2-(2-methoxy-4-(1-methyl-1*H*-pyrazol-5-yl) phenyl)-5-(trifluoromethyl)-7*H*-pyrrolo[2,3-*d*]pyrimidine-2,4-diamine | 1H NMR (400 MHz, Methanol-$d_4$) δ 8.42 (d, *J* = 8.3 Hz, 1H), 7.44 (t, *J* = 1.7 Hz, 2H), 7.09 (d, *J* = 1.8 Hz, 1H), 7.03 (dd, *J* = 8.3, 1.8 Hz, 1H), 6.32 (d, *J* = 2.0 Hz, 1H), 3.94 (s, 3H), 3.84 (s, 3H), 2.05 (d, *J* = 11.2 Hz, 2H), 1.74 (s, 2H), 1.63 (d, *J* = 12.2 Hz, 1H), 1.41 (d, *J* = 11.2 Hz, 4H), 1.22 (d, *J* = 2.6 Hz, 2H); MS: m/z 486.3 $[M+H]^+$ | 2.07 |

(continued)

| 실시예 화합물 | 구조 | 화합물명 | $^1$H NMR, MS | UPLC r.t. (min) |
|---|---|---|---|---|
| 7 | 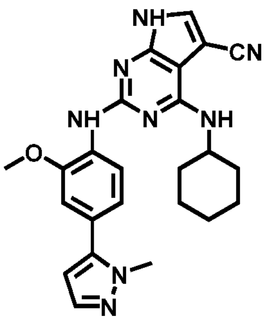 | 4-(cyclohexylamino)-2-((2-methoxy-4-(1-methyl-1*H*-pyrazol-5-yl)phenyl)amino)-7*H*-pyrrolo[2,3-*d*]pyrimidine-5-carbonitrile | 1H NMR (400 MHz, DMSO-$d_6$) δ 12.28 (s, 1H), 8.59 (d, *J* = 8.3 Hz, 1H), 7.88 (s, 1H), 7.55 (s, 1H), 7.45 (d, *J* = 1.8 Hz, 1H), 7.13 (d, *J* = 1.8 Hz, 1H), 7.07 (dd, *J* = 8.3, 1.8 Hz, 1H), 6.39 (d, *J* = 1.9 Hz, 1H), 5.83 - 5.72 (m, 1H), 4.05 (dd, *J* = 15.4, 8.3 Hz, 1H), 3.95 (s, 3H), 3.88 (s, 3H), 2.07 - 1.95 (m, 2H), 1.74 (d, *J* = 9.4 Hz, 2H), 1.61 (d, *J* = 11.7 Hz, 1H), 1.44 (t, *J* = 12.1 Hz, 4H), 1.25 (d, *J* = 11.6 Hz, 1H); MS: m/z 443.3 $[M+H]^+$ | 1.84 |
| 8 | 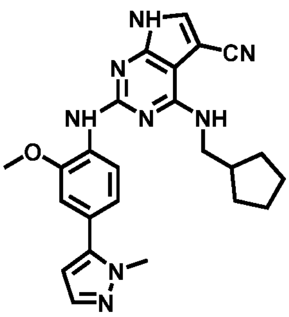 | 4-((cyclopentylmethyl) amino)-2-((2-methoxy-4-(1-methyl-1*H*-pyrazo-5-yl)phenyl)amino)-7*H*-pyrrolo[2,3-*d*]pyrimidine-5-carbonitrile | 1H NMR (400 MHz, DMSO-$d_6$) δ 12.35 - 12.27 (m, 1H), 8.59 (d, *J* = 8.3 Hz, 1H), 7.89 (d, *J* = 2.4 Hz, 1H), 7.61 (s, 1H), 7.45 (d, *J* = 1.9 Hz, 1H), 7.13 (d, *J* = 1.9 Hz, 1H), 7.07 (dd, *J* = 8.3, 1.9 Hz, 1H), 6.39 (d, *J* = 1.9 Hz, 1H), 6.33 (s, 1H), 3.95 (s, 3H), 3.88 (s, 3H), 3.48 (dd, *J* = 7.3, 5.6 Hz, 2H), 2.29 (dt, *J* = 14.9, 7.4 Hz, 1H), 1.74 (dq, *J* = 12.0, 6.9, 5.9 Hz, 2H), 1.67 - 1.46 (m, 4H), 1.37 - 1.27 (m, 2H). MS: m/z 443.4$[M+H]^+$ | 1.92 |
| 9 | 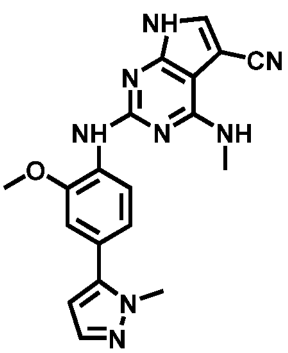 | 2-((2-methoxy-4-(1-methyl-1*H*-pyrazol-5 - yl)phenyl)amino)-4-(methylamino)-7*H*-pyrrolo[2,3-*d*]pyrimidine-5-carbonitrile | 1H NMR (400 MHz, DMSO-$d_6$) δ 12.38 (s, 1H), 8.62 (d, *J* = 8.3 Hz, 1H), 7.91 (d, *J* = 2.2 Hz, 1H), 7.76 (s, 1H), 7.45 (d, *J* = 1.9 Hz, 1H), 7.14 (d, *J* = 1.9 Hz, 1H), 7.13 - 7.07 (m, 1H), 6.72 (s, 1H), 6.39 (d, *J* = 1.9 Hz, 1H), 3.96 (s, 3H), 3.88 (s, 3H), 3.03 (d, *J* = 3.7 Hz, 3H).; MS: m/z 375.4$[M+H]^+$ | 1.45 |

(continued)

| 실시예 화합물 | 구조 | 화합물명 | 1H NMR, MS | UPLC r.t. (min) |
|---|---|---|---|---|
| 10 | 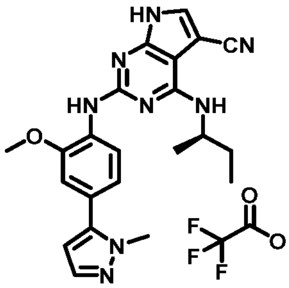 | (*R*)-4-(*sec*-butylamino)-2-((2-methoxy-4-(1-methyl-1*H*-pyrazol-5-yl)phenyl)amino)-7*H*-pyrrolo[2,3-*d*]pyrimidine-5-carbonitrile 2,2,2-trifluoro acetate | 1H NMR (400 MHz, DMSO-$d_6$) δ 12.45 - 12.36 (m, 1H), 8.56 (d, *J* = 8.3 Hz, 1H), 7.91 (s, 1H), 7.78 (s, 1H), 7.46 (d, *J* = 1.9 Hz, 1H), 7.15 (d, *J* = 1.9 Hz, 1H), 7.09 (dd, *J* = 8.4, 1.9 Hz, 1H), 6.40 (d, *J* = 1.9 Hz, 1H), 5.96 (s, 1H), 4.20 (q, *J* = 6.8 Hz, 1H), 3.96 (s, 3H), 3.88 (s, 3H), 1.65 (dh, *J* = 27.5, 6.9 Hz, 2H), 1.27 (d, *J* = 6.5 Hz, 3H), 0.95 (t, *J* = 7.4 Hz, 3H). ;<br>MS: m/z 417.4 $[M+H]^+$ | 1.78 |
| 11 | 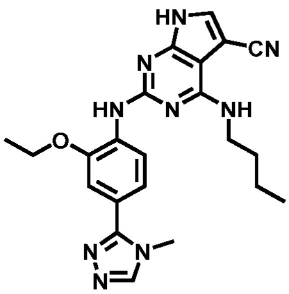 | 4-(butylamino)-2-((2-ethoxy-4-(4-methyl-4H-1,2,4-triazol-3-yl)phenyl)amino)-7*H*-pyrrolo [2,3-*d*]pyrimidine-5-carbonitrile | 1H NMR (400 MHz, Methanol-$d_4$) δ8.86 (d, *J* = 8.4 Hz, 1H), 8.51 (s, 1H), 7.61 (s, 1H), 7.31 - 7.23 (m, 2H), 4.26 (q, *J* = 6.9 Hz, 2H), 3.84 (s, 3H), 3.66 (t, *J* = 7.2 Hz, 2H), 1.74 (q, *J* = 7.5 Hz, 2H), 1.57 - 1.50 (m, 4H), 1.02 (t, *J* = 7.4 Hz, 3H);<br>MS: m/z 432.2 $[M+H]^+$ | 1.48 |
| 12 | 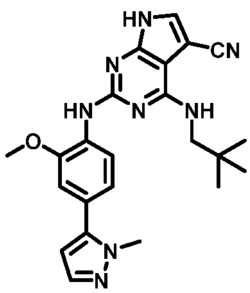 | 2-((2-methoxy-4-(1-methyl-1*H*-pyrazol-5-yl)phenyl)amino)-4-(neopentylamino)-7*H*-pyrrolo [2,3-*d*]pyrimidine-5-carbonitrile | 1H NMR (400 MHz, DMSO-$d_6$) δ 12.31 (d, *J* = 2.8 Hz, 1H), 8.59 (d, *J* = 8.3 Hz, 1H), 7.89 (d, *J* = 2.7 Hz, 1H), 7.57 (s, 1H), 7.45 (d, *J* = 1.8 Hz, 1H), 7.13 (d, *J* = 1.9 Hz, 1H), 7.09 (dd, *J* = 8.3, 1.9 Hz, 1H), 6.39 (d, *J* = 1.8 Hz, 1H), 5.98 (t, *J* = 6.1 Hz, 1H), 3.95 (s, 3H), 3.88 (s, 3H), 3.42 (d, *J* = 6.1 Hz, 2H), 0.98 (s, 9H).<br>MS: m/z 431.4 $[M+H]^+$ | 1.88 |
| 13 | 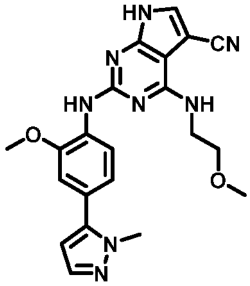 | 2-((2-methoxy-4-(1-methyl-1*H*-pyrazol-5-yl)phenyl)amino)-4-((2-methoxyethyl)amino) -7 H-pyrrolo[2,3-*d*]pyrimidine-5-carbonitrile | 1H NMR (400 MHz, DMSO-$d_6$) δ 12.37 - 12.29 (m, 1H), 8.58 (d, *J* = 8.3 Hz, 1H), 7.90 (d, *J* = 2.4 Hz, 1H), 7.60 (s, 1H), 7.45 (d, *J* = 1.8 Hz, 1H), 7.13 (d, *J* = 1.9 Hz, 1H), 7.08 (dd, *J* = 8.3, 1.9 Hz, 1H), 6.39 (d, *J* = 1.9 Hz, 1H), 6.31 (t, *J* = 5.6 Hz, 1H), 3.96 (s, 3H), 3.88 (s, 3H), 3.72 (q, *J* = 5.7 Hz, 2H), 3.59 (t, *J* = 5.8 Hz, 2H), 3.32 (s, 3H).<br>MS: m/z 419.3$[M+H]^+$ | 1.57 |

(continued)

| 실시 예 화합 물 | 구조 | 화합물명 | $^1$H NMR, MS | UPLC r.t. (min) |
|---|---|---|---|---|
| 14 | 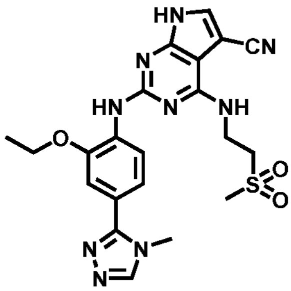 | 2-((2-ethoxy-4-(4-methyl-4H-1,2,4-triazol-3-yl)phenyl)amino)-4-((2-(methylsulfonyl)ethyl ) amino)-7*H*-pyrrolo[2,3-*d*] pyrimidine-5-carbonitrile | $^1$H NMR (400 MHz, DMSO-*d*$_6$) 612.38 (s, 1H), 8.74 (s, 1H), 8.69 (d, *J* = 8.4 Hz, 1H), 7.95 (d, *J* = 2.7 Hz, 1H), 7.73 (s, 1H), 7.40 - 7.30 (m, 2H), 6.84 (t, *J* = 5.9 Hz, 1H), 4.23 (q, *J* = 7.0 Hz, 2zH), 3.98 (q, *J* = 6.6 Hz, 2H), 3.80 (s, 3H), 3.52 (t, *J* = 7.0 Hz, 2H), 3.08 (s, 3H), 1.44 (t, *J* = 6.9 Hz, 3H).<br>MS: m/z 482.2 [M+H]$^+$ | 1.16 |
| 15 | 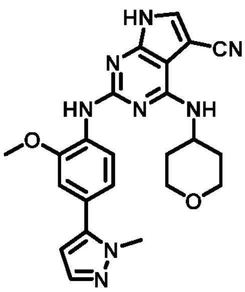 | 2-((2-methoxy-4-(1-methyl-1*H*-pyrazol-5-yl)phenyl)amino)-4-((tetrahydro-2*H*-pyran-4-yl) amino)-7*H*-pyrrolo[2,3-*d*] pyrimidine-5-carbonitrile | 1H NMR (400 MHz, DMSO-*d*$_6$) δ 12.33 (s, 1H), 8.55 (d, *J* = 8.3 Hz, 1H), 7.91 (d, *J* = 1.9 Hz, 1H), 7.66 (s, 1H), 7.45 (d, *J* = 1.9 Hz, 1H), 7.14 (d, *J* = 1.9 Hz, 1H), 7.09 (dd, *J* = 8.3, 1.9 Hz, 1H), 6.39 (d, *J* = 1.9 Hz, 1H), 6.11 (d, *J* = 7.5 Hz, 1H), 4.33—4.21 (m, 1H), 3.95 (s, 3H), 3.92 (t, *J* = 3.6 Hz, 2H), 3.88 (s, 3H), 3.49 (td, *J* = 11.5, 2.2 Hz, 2H), 2.05 - 1.95 (m, 2H), 1.72 - 1.60 (m, 2H).<br>MS: m/z 445.4[M+H]$^+$ | 1.55 |
| 16 | 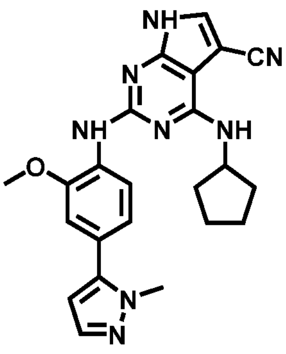 | 4-(cyclopentylamino)-2-((2-methoxy-4-(1-methyl-1*H*-pyrazol-5 - yl)phenyl)amino)-7*H*-pyrrolo[2,3-*d*]pyrimidine-5-carbonitrile | 1H NMR (400 MHz, DMSO-*d*$_6$) δ 12.29 (s, 1H), 8.62 (d, *J* = 8.3 Hz, 1H), 7.88 (s, 1H), 7.55 (s, 1H), 7.45 (d, *J* = 1.8 Hz, 1H), 7.13 (d, *J* = 1.8 Hz, 1H), 7.08 (dd, *J* = 8.3, 1.8 Hz, 1H), 6.39 (d, *J* = 1.8 Hz, 1H), 5.93 (d, *J* = 6.9 Hz, 1H), 4.47 (dd, *J* = 13.4, 6.8 Hz, 1H), 3.95 (s, 3H), 3.88 (s, 3H), 2.11 - 2.02 (m, 2H), 1.72 (d, *J* = 5.9 Hz, 2H), 1.60 (ddd, *J* = 18.9, 13.1, 7.1 Hz, 4H);<br>MS: m/z 429.3 [M+H]$^+$ | 1.77 |

(continued)

| 실시예 화합물 | 구조 | 화합물명 | $^1$H NMR, MS | UPLC r.t. (min) |
|---|---|---|---|---|
| 17 | 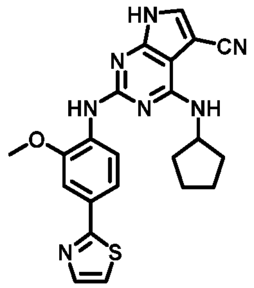 | 4-(cyclopentylamino)-2-((2-methoxy-4-(thiazol-2-yl)phenyl) amino)-7*H*-pyrrolo[2,3-*d*] pyrimidine-5-carbonitrile | 1H NMR (400 MHz, DMSO-$d_6$) δ 12.31 (s, 1H), 8.65 (d, *J* = 8.4 Hz, 1H), 7.90 (s, 1H), 7.86 (d, *J* = 3.3 Hz, 1H), 7.70 (d, *J* = 3.3 Hz, 1H), 7.63 (s, 1H), 7.56 (d, *J* = 1.9 Hz, 1H), 7.52 (dd, *J* = 8.4, 1.9 Hz, 1H), 5.97 (d, *J* = 7.0 Hz, 1H), 4.49 (h, *J* = 6.7 Hz, 1H), 4.00 (s, 3H), 2.08 (dt, *J* = 11.8, 6.5 Hz, 2H), 1.80 - 1.68 (m, 2H), 1.61 (ddt, *J* = 19.5, 13.4, 6.7 Hz, 4H). ; MS: m/z 432.4 $[M+H]^+$ | 1.97 |
| 18 | 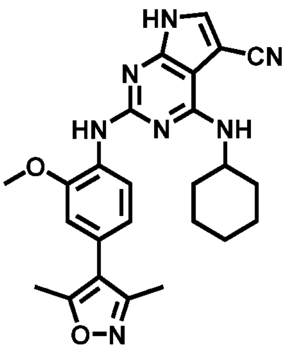 | 4-(cyclohexylamino)-2-((4-(3,5-dimethylisoxazol-4-yl)-2-methoxyphenyl)amin o)-7*H*-pyrrolo[2,3-*d*]pyrimidine-5-carbonitrile | 1H NMR (400 MHz, DMSO-$d_6$) δ 12.39 (s, 1H), 8.58 (d, *J* = 8.3 Hz, 1H), 7.96 (d, *J* = 2.3 Hz, 1H), 7.73 (s, 1H), 7.08 (d, *J* = 1.9 Hz, 1H), 7.00 (dd, *J* = 8.3, 1.8 Hz, 1H), 5.98 (s, 1H), 4.00 (s, 3H), 2.50 (s, 3H), 2.33 (s, 3H), 2.13 - 2.03 (m, 2H), 1.82 (d, *J* = 10.3 Hz, 2H), 1.69 (d, *J* = 12.8 Hz, 1H), 1.49 (t, *J* = 9.3 Hz, 4H), 1.35 (d, *J* = 6.4 Hz, 1H), 1.32 (d, *J* = 5.7 Hz, 1H). ; m/z = 458.3 $[M+H]^+$ | 1.92 |
| 19 | 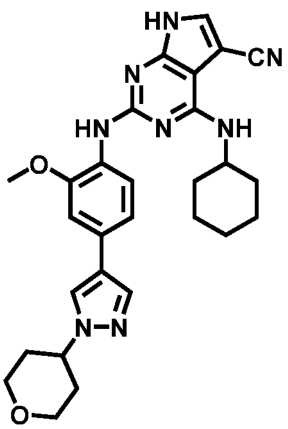 | 4-(cyclohexylamino)-2-((2-methoxy-4-(1-(tetrahydro-2*H*-pyran-4-yl)-1*H*-pyrazol-4-yl) phenyl)amino)-7*H*-pyrrolo[2,3-*d*] pyrimidine-5-carbonitrile | 1H NMR (400 MHz, DMSO-$d_6$) δ 12.25 (s, 1H), 8.37 (d, *J* = 8.3 Hz, 1H), 8.25 (s, 1H), 7.88 (s, 1H), 7.86 (s, 1H), 7.53 (s, 1H), 7.23 (d, *J* = 1.8 Hz, 1H), 7.13 (dd, *J* = 8.3, 1.8 Hz, 1H), 5.83 (d, *J* = 7.5 Hz, 1H), 4.40 (dt, *J* = 10.7, 5.4 Hz, 1H), 3.94 (s, 3H), 3.49 (td, *J* = 11.4, 3.1 Hz, 4H), 2.07 - 1.93 (m, 6H), 1.75 (dd, *J* = 9.0, 4.7 Hz, 2H), 1.63 (d, *J* = 12.2 Hz, 2H), 1.51 - 1.33 (m, 4H), 1.26 (dd, *J* = 6.1, 3.0 Hz, 1H).; m/z = 513.4 $[M+H]^+$ | 1.75 |

(continued)

| 실시예 화합물 | 구조 | 화합물명 | $^1$H NMR, MS | UPLC r.t. (min) |
|---|---|---|---|---|
| 20 | 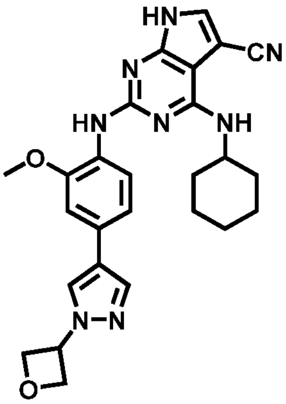 | 4-(cyclohexylamino)-2-((2-methoxy-4-(1-(oxetan-3-yl)-1*H*-pyrazol-4-yl)phenyl)amino)-7*H*-pyrrolo[2,3-*d*]pyrimidine-5-carbonitrile | 1H NMR (400 MHz, DMSO-$d_6$) δ 12.34 (s, 1H), 8.38 (s, 1H), 8.03 (s, 1H), 7.88 (s, 2H), 7.75 - 7.69 (m, 1H), 7.25 (dd, *J* = 14.9, 1.8 Hz, 1H), 7.15 (ddd, *J* = 10.3, 8.2, 1.8 Hz, 1H), 6.01 (s, 1H), 5.58 (t, *J* = 7.0 Hz, 1H), 4.99 - 4.90 (m, 4H), 3.95 (s, 3H), 3.79 - 3.76 (m, 1H), 2.01 (dt, *J* = 8.9, 4.9 Hz, 2H), 1.75 (td, *J* = 9.6, 5.1 Hz, 2H), 1.63 (d, *J* = 12.9 Hz, 2H), 1.43 (t, *J* = 9.3 Hz, 4H). ; m/z = 485.4 $[M+H]^+$ | 1.66 |
| 21 | 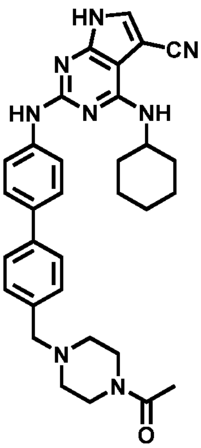 | 2-((4'-((4-acetylpiperazin-1-yl) methyl)-[1,1'-biphenyl]-4-yl) amino)-4-(cyclohexylamino)-7*H*-pyrrolo[2,3-*d*]pyrimidine-5-carbonitrile | 1H NMR (400 MHz, Methanol-$d_4$) δ7.81 (d, *J* = 8.5 Hz, 2H), 7.60 - 7.50 (m, 5H), 7.39 (d, *J* = 8.0 Hz, 2H), 4.24 - 4.14 (m, 1H), 3.60 (d, *J* = 4.3 Hz, 4H), 3.56 (t, *J* = 5.1 Hz, 2H), 2.50 (dt, *J* = 5.1, 18.1 Hz, 4H), 2.12 (d, *J* = 7.7 Hz, 1H), 2.09 (s, 3H), 1.83 (dq, *J* = 4.7, 5.2, 13.5 Hz, 2H), 1.75 - 1.64 (m, 1H), 1.42 (ddt, *J* = 12.6, 25.0, 48.3 Hz, 6H);<br>MS: m/z 549.3 $[M+H]^+$ | 1.42 |
| 22 | 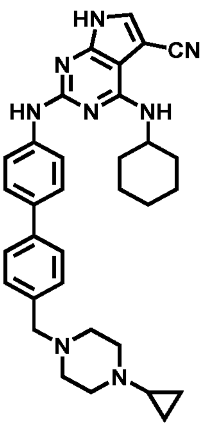 | 4-(cyclohexylamino)-2-((4'-((4-cyclopropylpiperazin-1-yl) methyl)-[1,1'-biphenyl]-4-yl) amino)-7*H*-pyrrolo[2,3-*d*] pyrimidine-5-carbonitrile | 1H NMR (400 MHz, Methanol-$d_4$) δ7.82 (d, *J* = 8.5 Hz, 2H), 7.63 (d, *J* = 7.9 Hz, 2H), 7.54 (d, *J* = 8.0 Hz, 3H), 7.44 (d, *J* = 8.0 Hz, 2H), 4.22 - 4.15 (m, 1H), 3.84 (s, 2H), 2.80 (s, 8H), 2.13 (d, *J* = 4.1 Hz, 1H), 1.82 (dtd, *J* = 3.7, 6.6, 7.7, 13.6 Hz, 3H), 1.75 - 1.65 (m, 1H), 1.43 (ddd, *J* = 11.8, 24.3, 45.8 Hz, 6H), 0.56 - 0.49 (m, 2H), 0.49 - 0.41 (m, 2H);<br>MS: m/z 547.3 $[M+H]^+$ | 1.51 |

(continued)

| 실시 예 화합 물 | 구조 | 화합물명 | 1H NMR, MS | UPLC r.t. (min) |
|---|---|---|---|---|
| 23 | 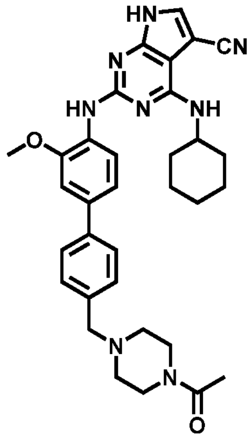 | 2-((4'-((4-acetylpiperazin-1-yl) methyl)-3-methoxy-[1,1'-biphenyl]-4-yl)amino)-4-(cyclohexylamino)-7*H*-pyrrolo [2,3-*d*]pyrimidine-5-carbonitrile | 1H NMR (400 MHz, DMSO-$d_6$) δ 12.27 (s, 1H), 9.85 (s, 1H), 8.57 (d, *J* = 8.4 Hz, 1H), 7.89 (d, *J* = 2.7 Hz, 1H), 7.83 (d, *J* = 7.9 Hz, 2H), 7.61 - 7.50 (m, 3H), 7.35 - 7.25 (m, 2H), 5.85 (d, *J* = 7.6 Hz, 1H), 4.50 - 4.33 (m, 4H), 4.13 - 4.03 (m, 2H), 4.00 (s, 3H), 3.43 - 3.30 (m, 4H), 2.05 (s, 3H), 1.80 - 1.69 (m, 2H), 1.66 - 1.57 (m, 1H), 1.48 - 1.38 (m, 6H), 1.35 - 1.19 (m, 2H); MS: m/z 579.3 $[M+H]^+$ | 1.45 |
| 24 | 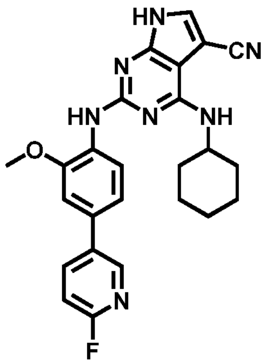 | 4-(cyclohexylamino)-2-((4-(6-fluoropyridin-3 -yl)-2-methoxyphenyl)amin o)-7*H*-pyrrolo[2,3-*d*]pyrimidine-5-carbonitrile | 1H NMR (400 MHz, DMSO-$d_6$) δ 12.28 (s, 1H), 8.64 - 8.54 (m, 2H), 8.32 (td, *J* = 2.5, 8.2 Hz, 1H), 7.88 (d, *J* = 2.7 Hz, 1H), 7.55 (s, 1H), 7.36 (d, *J* = 2.0 Hz, 1H), 7.28 (ddd, *J* = 2.4, 8.5, 11.3 Hz, 2H), 5.81 (d, *J* = 7.7 Hz, 1H), 4.15 - 4.03 (m, 1H), 4.00 (s, 3H), 2.05 - 1.97 (m, 2H), 1.79 - 1.70 (m, 2H), 1.66 - 1.57 (m, 1H), 1.52 - 1.34 (m, 5H), 1.27 (ddt, *J* = 5.3, 12.5, 18.1 Hz, 2H). MS: m/z 458.2 $[M+H]^+$ | 1.97 |
| 25 | 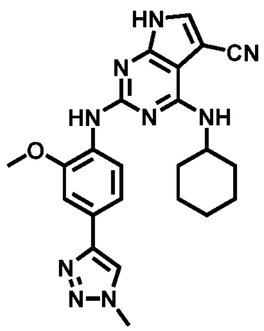 | 4-(cyclohexylamino)-2-((2-methoxy-4-(1-methyl-1*H*-1,2,3-triazol-4-yl)phenyl)amino)-7*H*-pyrrolo[2,3-*d*]pyrimidine-5-carbonitrile | 1H NMR (400 MHz, DMSO-$d_6$) δ 12.36 (s, 1H), 8.50 (s, 1H), 7.90 (s, 1H), 7.74 (d, *J* = 14.1 Hz, 1H), 7.49 (d, *J* = 1.8 Hz, 1H), 7.43—7.37 (m, 1H), 7.27 (d, *J* = 6.5 Hz, 1H), 6.01 (s, 1H), 4.09 (s, 3H), 3.97 (s, 3H), 3.10 (qd, *J* = 7.3, 4.8 Hz, 3H), 2.02 (p, *J* = 4.7 Hz, 2H), 1.82 - 1.70 (m, 2H), 1.43 (t, *J* = 9.3 Hz, 4H). ; m/z = 444.4 $[M+H]^+$ | 1.60 |

(continued)

| 실시 예 화합 물 | 구조 | 화합물명 | $^1$H NMR, MS | UPLC r.t. (min) |
|---|---|---|---|---|
| 26 | 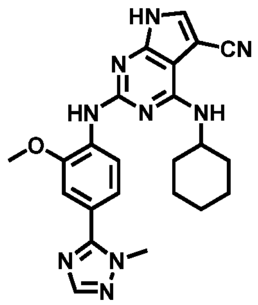 | 4-(cyclohexylamino)-2-((2-methoxy-4-(1-methyl-1*H*-1,2,4-triazol-5-yl)phenyl)amino)-7*H*-pyrrolo[2,3-*d*]pyrimidine-5-carbonitrile | 1H NMR (400 MHz, DMSO-$d_6$) δ 12.36 (s, 1H), 8.55 (d, *J* = 8.8 Hz, 1H), 8.50 (s, 1H), 7.90 (d, *J* = 1.7 Hz, 1H), 7.73 (s, 1H), 7.60 — 7.56 (m, 2H), 6.02 (s, 1H), 3.97 (s, 3H), 3.92 (s, 3H), 2.53 (d, *J* = 6.6 Hz, 1H), 2.03 (dd, *J* = 8.9, 4.8 Hz, 2H), 1.81 - 1.71 (m, 2H), 1.64 (d, *J* = 12.6 Hz, 1H), 1.43 (t, *J* = 9.3 Hz, 5H). ; m/z = 444.4[M+H]$^+$ | 1.60 |
| 27 | 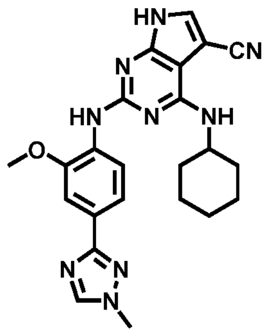 | 4-(cyclohexylamino)-2-((2-methoxy-4-(1-methyl-1*H*-1,2,4-triazol-3-yl)phenyl)amino)-7*H*-pyrrolo[2,3-*d*]pyrimidine-5-carbonitrile | 1H NMR (400 MHz, DMSO-$d_6$) δ 12.37 (s, 1H), 8.67 (d, *J* = 8.3 Hz, 1H), 8.00 (d, *J* = 2.0 Hz, 1H), 7.92 (d, *J* = 2.1 Hz, 1H), 7.38 (d, *J* = 1.8 Hz, 1H), 7.35 (dd, *J* = 8.4, 1.9 Hz, 1H), 7.27 (d, *J* = 6.6 Hz, 1H), 5.98 (s, 1H), 4.00 (s, 3H), 3.98 (s, 3H), 2.53 (d, *J* = 6.6 Hz, 1H), 2.01 (dd, *J* = 9.3, 5.1 Hz, 2H), 1.80 - 1.68 (m, 2H), 1.62 (d, *J* = 13.1 Hz, 2H), 1.43 (t, *J* = 9.2 Hz, 4H). m/z = 444.4[M+H]$^+$ | 1.60 |
| 28 | 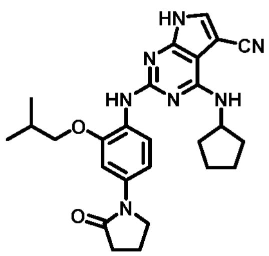 | 4-(cyclopentylamino)-2-((2-isobutoxy-4-(2-oxopyrrolidin-1-yl)phenyl)amino)-7*H*-pyrrolo [2,3-*d*]pyrimidine-5-carbonitrile | 1H NMR (400 MHz, DMSO-$d_6$) δ 12.21 (s, 1H), 8.35 (d, *J* = 8.8 Hz, 1H), 7.84 (s, 1H), 7.50 (d, *J* = 2.3 Hz, 1H), 7.43 (s, 1H), 7.04 (dd, *J* = 8.9, 2.3 Hz, 1H), 5.91 (d, *J* = 6.8 Hz, 1H), 4.41 (q, *J* = 6.7 Hz, 1H), 3.88 - 3.79 (m, 4H), 2.47 (d, *J* = 8.1 Hz, 1H), 2.07 (tt, *J* = 12.4, 5.9 Hz, 6H), 1.77 - 1.67 (m, 2H), 1.67 - 1.53 (m, 4H), 1.01 (d, *J* = 6.7 Hz, 6H). ; MS: m/z 474.5 [M+H]$^+$ | 1.98 |

(continued)

| 실시예 화합물 | 구조 | 화합물명 | $^1$H NMR, MS | UPLC r.t. (min) |
|---|---|---|---|---|
| 29 | 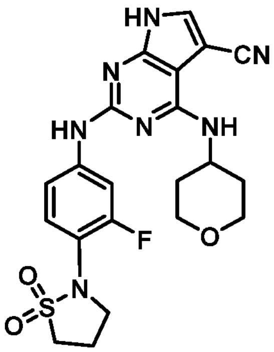 | 2-((4-(1,1-dioxidoisothiazolidin - 2-yl)-3-fluorophenyl)amino)-4-((tetrahydro-2*H*-pyran-4-yl) amino)-7*H*-pyrrolo[2,3-*d*] pyrimidine-5-carbonitrile | 1H NMR (400 MHz, DMSO-$d_6$) δ 12.26 (s, 1H), 9.40 (s, 1H), 8.07 (dd, *J* = 14.4, 2.4 Hz, 1H), 7.90 (d, *J* = 2.7 Hz, 1H), 7.47 - 7.36 (m, 1H), 7.29 (t, *J* = 8.9 Hz, 1H), 6.03 (d, *J* = 7.5 Hz, 1H), 4.26 (d, *J* = 10.0 Hz, 1H), 3.93 (d, *J* = 11.4 Hz, 2H), 3.67 (t, *J* = 6.7 Hz, 2H), 3.53 - 3.43 (m, 2H), 3.38 (t, *J* = 7.5 Hz, 2H), 2.41 (p, *J* = 7.1 Hz, 2H), 2.00 (d, *J* = 12.2 Hz, 2H), 1.66 (qd, *J* = 11.8, 4.4 Hz, 2H)<br>MS: m/z 472.3 [M+H]$^+$ | 1.39 |
| 30 | 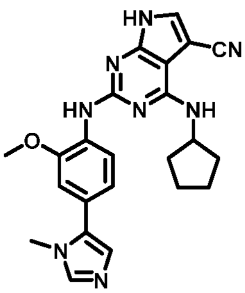 | 4-(cyclopentylamino)-2-((2-methoxy-4-(1-methyl-1*H*-imidazol-5-yl)phenyl)amino)-7*H*-pyrrolo[2,3-*d*]pyrimidine-5-carbonitrile | 1H NMR (400 MHz, DMSO-$d_6$) δ 12.28 (s, 1H), 8.62 (d, *J* = 8.3 Hz, 1H), 8.33 (s, 1H), 7.88 (d, *J* = 2.0 Hz, 1H), 7.57 (s, 1H), 7.40 (d, *J* = 12.3 Hz, 1H), 7.15 (d, *J* = 1.9 Hz, 1H), 7.10 (dd, *J* = 8.3, 1.9 Hz, 1H), 5.95 (d, *J* = 7.1 Hz, 1H), 4.47 (q, *J* = 6.7 Hz, 1H), 3.95 (s, 3H), 3.78 (s, 3H), 2.07 (td, *J* = 12.6, 11.5, 7.0 Hz, 2H), 1.79 - 1.68 (m, 2H), 1.60 (ddt, *J* = 19.5, 13.9, 6.7 Hz, 4H).<br>MS: m/z 429.4 [M+H]$^+$ | 1.53 |
| 31 | 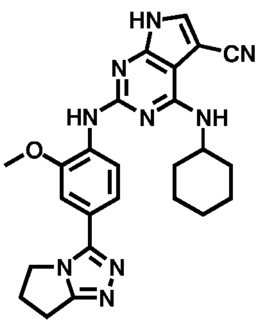 | 4-(cyclohexylamino)-2-((4-(6,7-dihydro-5*H*-pyrrolo[2,1-*c*][1,2,4] triazol-3-yl)-2-methoxyphenyl) amin o)-7*H*-pyrrolo[2,3-*d*] pyrimidine-5-carbonitrile | 1H NMR (400 MHz, DMSO-$d_6$) δ 12.36 (s, 1H), 8.76 (d, *J* = 9.0 Hz, 1H), 7.94 (d, *J* = 1.9 Hz, 1H), 7.79 (s, 1H), 7.53 - 7.44 (m, 2H), 5.94 (d, *J* = 7.7 Hz, 1H), 4.48 (t, *J* = 7.2 Hz, 2H), 4.01 (s, 3H), 3.08 (t, *J* = 7.7 Hz, 2H), 2.79 (p, *J* = 7.7 Hz, 2H), 2.54 (s, 1H), 2.09 - 1.97 (m, 2H), 1.82 - 1.70 (m, 2H), 1.63 (d, *J* = 13.0 Hz, 2H), 1.44 (t, *J* = 9.3 Hz, 4H). ;MS: m/z 470.4 [M+H]$^+$ | 1.44 |

(continued)

| 실시 예 화합 물 | 구조 | 화합물명 | $^1$H NMR, MS | UPLC r.t. (min) |
|---|---|---|---|---|
| 32 | 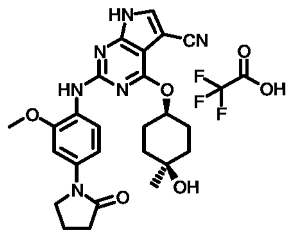 | 4-(((1s,4s)-4-hydroxy-4-methylcyclohexyl)ox y)-2-((2-methoxy-4-(2-oxopyrrolidin-1-yl) phenyl)amino)-7*H*-pyrrolo[2,3-*d*] pyrimidine-5-carbonitrile 2,2,2-trifluoro acetate | 1H NMR (400 MHz, DMSO-$d_6$) δ 12.40 (d, *J* = 2.7 Hz, 1H), 8.10 (d, *J* = 8.7 Hz, 1H), 7.93 (d, *J* = 2.8 Hz, 1H), 7.83 (s, 1H), 7.51 (d, *J* = 2.4 Hz, 1H), 7.07 (dd, *J* = 8.8, 2.4 Hz, 1H), 5.16 (d, *J* = 7.2 Hz, 1H), 3.85 (d, *J* = 4.0 Hz, 5H), 3.17 (s, 1H), 2.07 (p, *J* = 7.6 Hz, 2H), 1.85 (t, *J* = 6.0 Hz, 4H), 1.67 (d, *J* = 13.1 Hz, 2H), 1.46 (d, *J* = 14.5 Hz, 2H), 1.15 (d, *J* = 5.2 Hz, 5H). MS: m/z 477.4 $[M+H]^+$ | 1.49 |
| 33 | 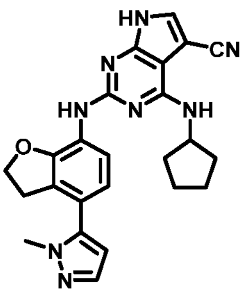 | 4-(cyclopentylamino)-2-((4-(1-methyl-1*H*-pyrazol-5-yl)-2,3-dihydrobenzofuran-7-yl)amino)-7*H*-pyrrolo[2,3-*d*]pyrimidine-5-carbonitrile | 1H NMR (400 MHz,DMSO-$d_6$) δ 12.28 (s, 1H), 8.14 (d, *J* = 8.3 Hz, 1H), 7.85 (d, *J* = 2.3 Hz, 1H), 7.65 - 7.57 (m, 1H), 7.48 (d, *J* = 1.8 Hz, 1H), 6.90 (d, *J* = 8.4 Hz, 1H), 6.36 (d, *J* = 1.9 Hz, 1H), 5.96 (s, 1H), 4.62 (t, *J* = 8.7 Hz, 2H), 4.43 (q, *J* = 6.7 Hz, 1H), 3.79 (s, 3H), 3.20 (t, *J* = 8.7 Hz, 2H), 2.05 (dt, *J* = 11.8, 6.2 Hz, 2H), 1.77 — 1.65 (m, 2H), 1.57 (ddq, *J* = 20.1, 13.3, 6.4 Hz, 4H). ; MS: m/z 441.4 $[M+H]^+$ | 1.93 |
| 34 | 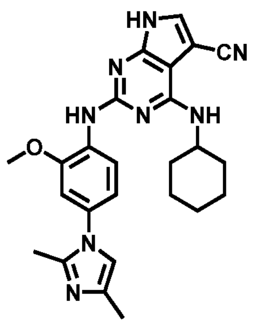 | 4-(cyclohexylamino)-2-((4-(2,4-dimethyl-1*H*-imidazol-1-yl)-2-methoxyphenyl)amin o)-7*H*-pyrrolo[2,3-*d*]pyrimidine-5-carbonitrile | 1H NMR (400 MHz, Methanol-$d_4$) δ 8.83 (d, *J* = 8.7 Hz, 1H), 7.66 (s, 1H), 7.37 (q, *J* = 1.1 Hz, 1H), 7.20 (d, *J* = 2.3 Hz, 1H), 7.11 (dd, *J* = 8.7, 2.4 Hz, 1H), 4.15 (d, *J* = 3.9 Hz, 1H), 4.03 (s, 3H), 2.56 (s, 3H), 2.39 (d, *J* = 1.2 Hz, 3H), 2.18 - 2.11 (m, 2H), 1.92 - 1.80 (m, 2H), 1.73 (s, 1H), 1.50 (d, *J* = 10.4 Hz, 5H); MS: m/z 457.3 $[M+H]^+$ | 1.44 |

(continued)

| 실시예 화합물 | 구조 | 화합물명 | $^1$H NMR, MS | UPLC r.t. (min) |
|---|---|---|---|---|
| 35 | 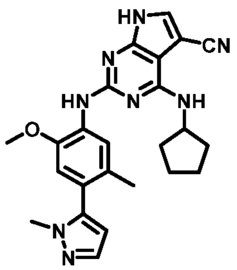 | 4-(cyclopentylamino)-2-((2-methoxy-5-methyl-4-(1-methyl-1*H*-pyrazol-5-yl)phenyl)amino)-7*H*-pyrrolo[2,3-*d*]pyrimidine-5-carbonitrile | 1H NMR (400 MHz, DMSO-$d_6$) δ 12.23 (s, 1H), 8.52 (s, 1H), 7.88 (s, 1H), 7.52 — 7.45 (m, 2H), 6.87 (s, 1H), 6.25 (d, *J* = 1.8 Hz, 1H), 5.97 (d, *J* = 7.1 Hz, 1H), 4.49 (q, *J* = 6.9 Hz, 1H), 3.88 (s, 3H), 3.63 (s, 3H), 2.09 (s, 5H), 1.74 (d, *J* = 5.3 Hz, 2H), 1.61 (tt, *J* = 14.1, 6.6 Hz, 4H).; MS: m/z 443.4 $[M+H]^+$ | 1.98 |
| 36 | 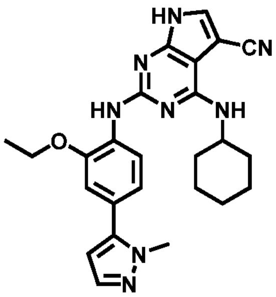 | 4-(cyclohexylamino)-2-((2-ethoxy-4-(1-methyl-1*H*-pyrazol-5-yl)phenyl)amino)-7*H*-pyrrolo [2,3-*d*]pyrimidine-5-carbonitrile | 1H NMR (400 MHz, DMSO-$d_6$) δ 12.29 (s, 1H), 8.58 (d, *J* = 8.4 Hz, 1H), 7.90 (d, *J* = 2.6 Hz, 1H), 7.60 (s, 1H), 7.44 (d, *J* = 1.8 Hz, 1H), 7.13 (d, *J* = 1.9 Hz, 1H), 7.06 (dd, *J* = 8.4, 1.8 Hz, 1H), 6.38 (d, *J* = 1.9 Hz, 1H), 5.92 (s, 1H), 4.24 (d, *J* = 6.9 Hz, 1H), 4.21 (d, *J* = 6.9 Hz, 1H), 3.87 (s, 3H), 2.01 (d, *J* = 14.3 Hz, 2H), 1.74 (s, 2H), 1.62 (d, *J* = 12.9 Hz, 1H), 1.44 (s, 2H), 1.42 (s, 3H), 1.40 (s, 2H), 1.23 (s, 2H). MS: m/z 457.2 $[M+H]^+$ | 1.93 |
| 37 | 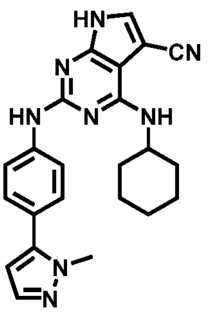 | 4-(cyclohexylamino)-2-((4-(1-methyl-1*H*-pyrazol-5-yl)phenyl) amino)-7*H*-pyrrolo[2,3-*d*] pyrimidine-5-carbonitrile | 1H NMR (400 MHz, Methanol-$d_4$) δ 7.88 - 7.82 (m, 2H), 7.68 (s, 1H), 7.54 (d, *J* = 2.1 Hz, 1H), 7.51 - 7.46 (m, 2H), 6.39 (d, *J* = 2.1 Hz, 1H), 4.19 (s, 1H), 3.92 (s, 3H), 2.15 (s, 2H), 1.86 (s, 2H), 1.73 (d, *J* = 12.6 Hz, 1H), 1.49 (d, *J* = 9.8 Hz, 4H), 1.35 (d, *J* = 24.3 Hz, 2H). MS: m/z 413.2 $[M+H]^+$ | 1.77 |
| 38 | 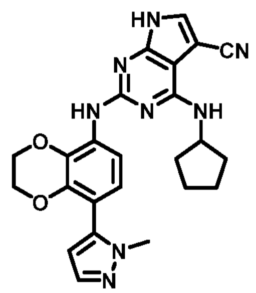 | 4-(cyclopentylamino)-2-((8-(1-methyl-1H-pyrazol-5-yl)-2,3-dihydrobenzo [b] [1,4] dioxin-5-yl) amino)-7*H*-pyrrolo[2,3-*d*] pyrimidine-5-carbonitrile | 1H NMR (400 MHz, DMSO-$d_6$) δ 12.78 (s, 1H), 7.98 (s, 2H), 7.45 (t, *J* = 1.9 Hz, 1H), 6.85 (d, *J* = 8.6 Hz, 2H), 6.31 - 6.18 (m, 2H), 4.47 (s, 1H), 4.42—4.27 (m, 4H), 3.69 (s, 3H), 2.08 (s, 2H), 1.73 (d, *J* = 11.8 Hz, 2H), 1.69 - 1.55 (m, 4H). ; MS: m/z 457.4 $[M+H]^+$ | 1.80 |

(continued)

| 실시 예 화합 물 | 구조 | 화합물명 | $^1$H NMR, MS | UPLC r.t. (min) |
|---|---|---|---|---|
| 39 | 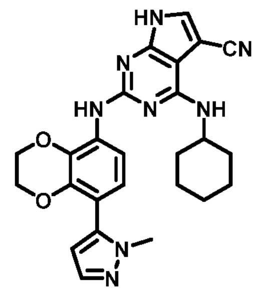 | 4-(cyclohexylamino)-2-((8-(1-methyl-1*H*-pyrazol-5-yl)-2,3-dihydrobenzo[b][1,4]dioxin-5-yl) amino)-7*H*-pyrrolo[2,3-*d*] pyrimidine-5-carbonitrile | 1H NMR (400 MHz, DMSO-$d_6$) δ 12.31 (s, 1H), 8.12 (d, *J* = 8.5 Hz, 1H), 7.89 (d, *J* = 2.5 Hz, 1H), 7.50 (s, 1H), 7.42 (d, *J* = 1.9 Hz, 1H), 6.80 (d, *J* = 8.5 Hz, 1H), 6.23 (d, *J* = 1.9 Hz, 1H), 5.86 (s, 1H), 4.40 (dd, *J* = 5.5, 2.6 Hz, 2H), 4.32 (dd, *J* = 5.6, 2.6 Hz, 2H), 4.06 (s, 2H), 3.68 (s, 3H), 2.00 (d, *J* = 7.6 Hz, 2H), 1.73 (s, 2H), 1.61 (d, *J* = 12.9 Hz, 1H), 1.41 (t, *J* = 9.3 Hz, 4H). MS: m/z 471.4 [M+H]$^+$ | 1.84 |
| 40 | 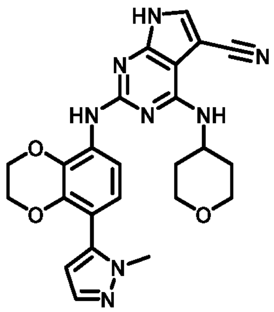 | 2-((8-(1-methyl-1*H*-pyrazol-5-yl)-2,3-dihydrobenzo [b] [1,4] dioxin-5-yl)amino) -4-((tetrahydro-2*H*-pyran-4-yl) amino)-7*H*-pyrrolo[2,3-*d*] pyrimidine-5-carbonitrile | 1H NMR (400 MHz, MeOD) δ 8.05 - 7.91 (m, 2H), 7.85 (s, 1H), 7.05 (d, *J* = 8.5 Hz, 1H), 6.63 (d, *J* = 2.4 Hz, 1H), 4.49 (p, *J* = 2.8 Hz, 2H), 4.46 — 4.31 (m, 3H), 4.06 (dt, *J* = 9.5, 2.3 Hz, 2H), 3.93 (s, 3H), 3.60 (td, *J* = 11.6, 1.9 Hz, 2H), 2.21 - 2.02 (m, 2H), 1.97 - 1.77 (m, 2H). MS: m/z 473.35 [M+H]$^+$ | 1.49 |
| 41 | 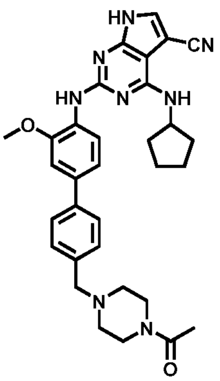 | 2-((4'-((4-acetylpiperazin-1-yl) methyl)-3-methoxy-[1,1'-biphenyl]-4-yl)amino)-4-(cyclopentylamino)-7*H*-pyrrolo [2,3-*d*]pyrimidine-5-carbonitrile | 1H NMR (400 MHz, Methanol-$d_4$) δ8.65 (d, *J* = 8.4 Hz, 1H), 7.61 (d, *J* = 7.9 Hz, 2H), 7.56 (s, 1H), 7.41 (d, *J* = 8.0 Hz, 2H), 7.24 - 7.14 (m, 2H), 4.02 (s, 3H), 3.62 (d, *J* = 6.0 Hz, 4H), 3.57 (t, *J* = 5.1 Hz, 2H), 2.56 (t, *J* = 5.0 Hz, 2H), 2.51 (t, *J* = 5.2 Hz, 2H), 2.18 (dt, *J* = 6.0, 13.1 Hz, 2H), 2.09 (s, 3H), 1.79 (ddt, *J* = 5.1, 8.2, 24.7 Hz, 4H), 1.65 (dd, *J* = 6.3, 12.6 Hz, 2H); MS: m/z 565.3 [M+H]$^+$ | 1.33 |

(continued)

| 실시예 화합물 | 구조 | 화합물명 | $^1$H NMR, MS | UPLC r.t. (min) |
|---|---|---|---|---|
| 42 | 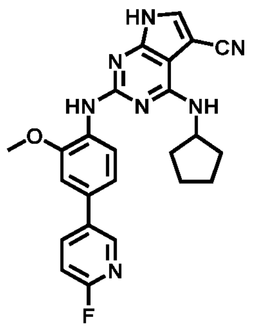 | 4-(cyclopentylamino)-2-((4-(6-fluoropyridin-3-yl)-2-methoxyphenyl)amin o)-7*H*-pyrrolo[2,3-*d*]pyrimidine-5-carbonitrile | 1H NMR (400 MHz, DMSO-$d_6$) δ 12.30 (d, *J* = 2.9 Hz, 1H), 8.67 - 8.55 (m, 2H), 8.32 (td, *J* = 2.6, 8.3 Hz, 1H), 7.88 (d, *J* = 2.7 Hz, 1H), 7.55 (s, 1H), 7.37 (d, *J* = 2.0 Hz, 1H), 7.28 (ddd, *J* = 2.4, 8.5, 18.1 Hz, 2H), 5.94 (d, *J* = 7.1 Hz, 1H), 4.48 (q, *J* = 6.8 Hz, 1H), 4.00 (s, 3H), 2.07 (dt, *J* = 6.6, 12.2 Hz, 2H), 1.79 - 1.52 (m, 6H);<br>MS: m/z 444.2 $[M+H]^+$ | 1.85 |
| 43 | 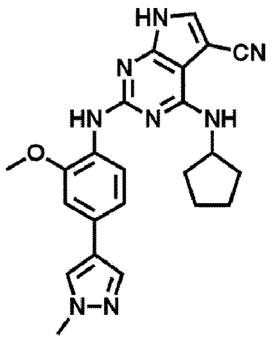 | 4-(cyclopentylamino)-2-((2-methoxy-4-(1-methyl-1*H*-pyrazol-4-yl)phenyl)amino)-7*H*-pyrrolo[2,3-*d*]pyrimidine-5-carbonitrile | 1H NMR (400 MHz, DMSO-$d_6$) δ 12.34 (s, 1H), 8.35 (d, *J* = 8.3 Hz, 1 H), 8.11 (s, 1H), 7.88 (s, 1H), 7.86 (s, 1H), 7.75 (s, 1H), 7.21 (d, *J* = 1.8 Hz, 1H), 7.13 (dd, *J* = 8.3, 1.9 Hz, 1H), 6.19 (s, 1H), 4.44 (p, *J* = 6.6 Hz, 1H), 3.94 (s, 3H), 3.86 (s, 3H), 2.06 (p, *J* = 6.1 Hz, 2H), 1.73 (d, *J* = 6.0 Hz, 2H), 1.60 (tq, *J* = 16.1, 6.4, 5.7 Hz, 4H). ;<br>m/z = 429.4 $[M+H]^+$ | 1.64 |
| 44 | 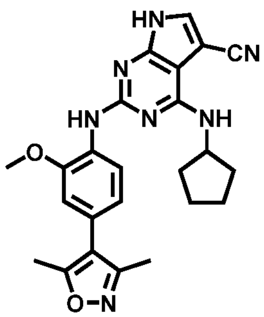 | 4-(cyclopentylamino)-2-((4-(3,5-dimethylisoxazol-4-yl)-2-methoxyphenyl)amin o)-7*H*-pyrrolo[2,3-*d*]pyrimidine-5-carbonitrile | 1H NMR (400 MHz, DMSO-$d_6$) δ 12.37 (s, 1H), 8.52 (d, *J* = 8.3 Hz, 1H), 7.89 (s, 1H), 7.75 (s, 1H), 7.01 (d, *J* = 1.9 Hz, 1H), 6.94 (dd, *J* = 8.3, 1.9 Hz, 1H), 6.16 (s, 1H), 4.45 (q, *J* = 6.7 Hz, 1H), 3.93 (s, 3H), 2.43 (s, 3H), 2.26 (s, 3H), 2.06 (td, *J* = 11.1, 9.2, 5.6 Hz, 2H), 1.72 (q, *J* = 12.8, 9.2 Hz, 2H), 1.61 (td, *J* = 11.5, 10.7, 6.7 Hz, 4H).<br>m/z = 444.3 [M+H]+ | 1.78 |

(continued)

| 실시예 화합물 | 구조 | 화합물명 | $^1$H NMR, MS | UPLC r.t. (min) |
|---|---|---|---|---|
| 45 | 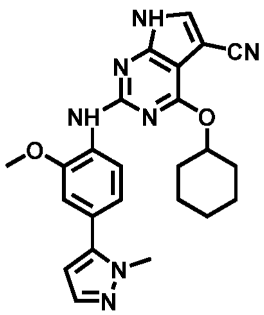 | 4-(cyclohexyloxy)-2-((2-methoxy-4-(1-methyl-1*H*-pyrazol-5-yl)phenyl)amino)-7*H*-pyrrolo[2,3-*d*]pyrimidine-5-carbonitrile | 1H NMR (400 MHz, DMSO-$d_6$) δ 12.52 (d, *J* = 3.2 Hz, 1H), 8.43 (d, *J* = 8.3 Hz, 1H), 7.99 (d, *J* = 2.8 Hz, 1H), 7.92 (s, 1H), 7.46 (d, *J* = 2.0 Hz, 1H), 7.18 - 7.07 (m, 2H), 6.41 (d, *J* = 2.0 Hz, 1H), 5.35 - 5.30 (m, 1H), 3.95 (s, 3H), 3.89 (s, 3H), 1.94 (d, *J* = 11.1 Hz, 2H), 1.79 (d, *J* = 8.5 Hz, 2H), 1.68 (d, *J* = 10.0 Hz, 2H), 1.45 (s, 4H), 1.23 (s, 2H).<br>MS: m/z 444.2 $[M+H]^+$ | 1.89 |
| 46 | 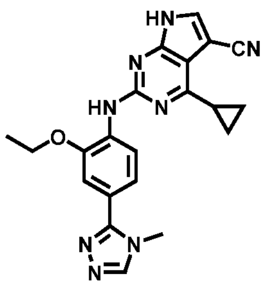 | 4-cyclopropyl-2-((2-ethoxy-4-(4-methyl-4*H*-1,2,4-triazol-3-yl) phenyl)amino)-7*H*-pyrrolo[2,3-*d*] pyrimidine-5-carbonitrile | 1H NMR (400 MHz, DMSO-$d_6$) δ12.67 (d, *J* = 2.8 Hz, 1H), 8.81 (s, 1 H), 8.57 (d, *J* = 8.3 Hz, 1H), 8.24 (d, *J* = 2.7 Hz, 1H), 7.93 (s, 1H), 7.42 - 7.31 (m, 2H), 4.24 (q, *J* = 7.0 Hz, 2H), 3.83 (s, 3H), 2.65 - 2.57 (m, 1H), 1.44 (t, *J* = 6.9 Hz, 3H), 1.34 - 1.19 (m, 4H).<br>MS: m/z 401.2 $[M+H]^+$ | 1.44 |
| 47 | 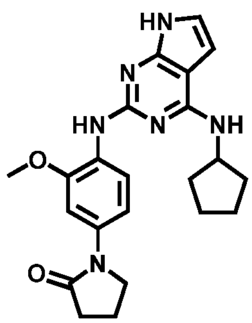 | 1-(4-((4-(cyclopentylamino)-7*H*-pyrrolo[2,3-*d*]pyrimidin-2-yl) amino)-3-methoxyphenyl)pyrro lidin-2-one | $^1$HNMR (400 MHz, DMSO-$d_6$): δ = 10.98 (s, 1H), 8.57 (d, *J* = 8.8 Hz, 1H), 7.47 (d, J = 2.4 Hz, 1H), 7.14 - 7.08 (m, 2H), 7.00 (m, 1H), 6.76 (m, 1H), 6.47 (m, 1H), 4.49 - 4.39 (m, 1H), 3.87 (s, 3H), 3.83 (t, J = 7.2 Hz, 2H), 3.29 - 3.26 (m, 1H), 2.48 - 2.43 (m, 2H), 2.10 - 2.02 (m, 2H), 2.02 - 1.94 (m, 2H), 1.77 - 1.67 (m, 2H), 1.63 - 1.51 (m, 4H)<br>MS: m/z 407.2 $[M+H]^+$ | 2.84 |

(continued)

| 실시예 화합물 | 구조 | 화합물명 | $^1$H NMR, MS | UPLC r.t. (min) |
|---|---|---|---|---|
| 48 | 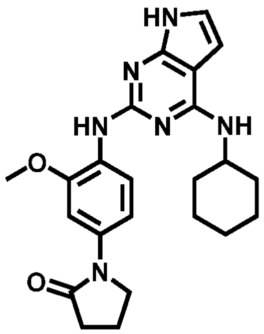 | 1-(4-((4-(cyclohexylamino)-7*H*-pyrrolo[2,3-*d*]pyrimidin-2-yl) amino)-3-methoxyphenyl)pyrrolidin-2-one | $^1$HNMR (400 MHz, DMSO-$d_6$): δ = 11.97 - 11.71 (m, 1H), 8.91 - 8.75 (m, 1H), 8.68 - 8.43 (m, 1H), 8.24 - 8.08 (m, 1H), 7.57 (d, J = 2.1 Hz, 1H), 7.14 (dd, J = 2.1, 8.9 Hz, 1H), 6.98 (br s, 1H), 6.69 (br s, 1H), 3.92 (br s, 1H), 3.89 (s, 3H), 3.87 - 3.83 (m, 2H), 2.53 (s, 2H), 2.08 (quin, J = 7.5 Hz, 2H), 1.99 (br s, 2H), 1.81 (br d, J = 4.8 Hz, 2H), 1.67 (br d, J = 12.0 Hz, 1H), 1.48 - 1.28 (m, 4H), 1.26 - 1.10 (m, 1H);<br>MS: m/z 421.2 $[M+H]^+$ | 1.75 |
| 49 | 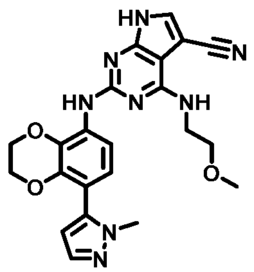 | 4-((2-methoxyethyl)amino)-2-((8-(1-methyl-1*H*-pyrazol-5-yl)-2,3-dihydrobenzo [b] [1,4] dioxin-5-yl)amino)-7*H*-pyrrolo [2,3-*d*]pyrimidine-5-carbonitrile | 1H NMR (400 MHz, MeOD) δ 7.88 (d, *J* = 8.6 Hz, 1H), 7.83 (d, *J* = 2.4 Hz, 1H), 7.74 (s, 1H), 6.91 (d, *J* = 8.5 Hz, 1H), 6.48 (d, *J* = 2.4 Hz, 1H), 4.34 (ddt, *J* = 26.9, 5.9, 3.0 Hz, 4H), 3.79 (d, *J* = 3.6 Hz, 5H), 3.62 (t, *J* = 5.3 Hz, 2H), 3.32 (s, 3H), 3.21 (p, *J* = 1.6 Hz, 4H).<br>MS: m/z 447.33 $[M+H]^+$ | 1.48 |
| 50 | 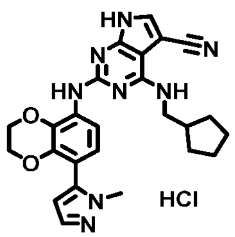 | 4-((cyclopentylmethyl) amino)-2-((8-(1-methyl-1*H*-pyrazol-5-yl)-2,3-dihydrobenzo [b] [1,4] dioxin-5-yl)amino)-7*H*-pyrrolo [2,3-*d*]pyrimidine-5-carbonitrile hydrochloride | 1H NMR (400 MHz, MeOD) δ 8.05 (d, *J* = 8.5 Hz, 1H), 7.71 (s, 1H), 7.52 (d, *J* = 2.0 Hz, 1H), 6.86 (d, *J* = 8.5 Hz, 1H), 6.29 (d, *J* = 2.0 Hz, 1H), 4.40 (ddd, *J* = 33.8, 6.1, 3.4 Hz, 4H), 3.77 (s, 3H), 3.60 (d, *J* = 7.4 Hz, 2H), 2.43 - 2.29 (m, 1H), 2.01 (s, 1H), 1.87 (dt, *J* = 12.0, 5.8 Hz, 2H), 1.80 - 1.55 (m, 4H), 1.50 - 1.22 (m, 3H).<br>MS: m/z 471.41 $[M+H]^+$ | 1.84 |

(continued)

| 실시 예 화합 물 | 구조 | 화합물명 | $^1$H NMR, MS | UPLC r.t. (min) |
|---|---|---|---|---|
| 51 | 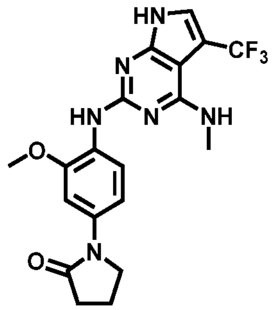 | 1-(3-methoxy-4-((4-(methylamino)-5-(trifluoromethyl)-7*H*-pyrrolo [2,3-*d*]pyrimidin-2-yl)amino) phenyl)pyrr olidin-2-one | $^1$HNMR (400 MHz, DMSO-$d_6$) δ = 12.03 (br s, 1H), 8.38 (d, *J* = 8.9 Hz, 1H), 7.76 - 7.60 (m, 1H), 7.57 - 7.50 (m, 2H), 7.06 (dd, *J* = 2.3, 8.8 Hz, 1H), 6.10 (br s, 1H), 3.90 - 3.81 (m, 5H), 3.44 - 3.35 (m, 2H), 3.04 (d, *J* = 4.5 Hz, 3H), 2.11 - 2.02 (m, 2H); MS: m/z 421.2 [M+H]$^+$ | 2.3 |
| 52 | 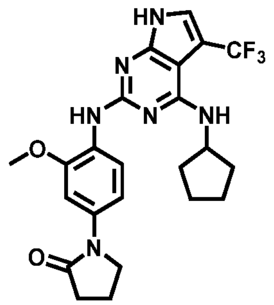 | 1-(4-((4-(cyclopentylamino)-5-(trifluoromethyl)-7*H*-pyrrolo [2,3-*d*]pyrimidin-2-yl)amino)-3-methoxyphenyl)pyrro lidin-2-one | $^1$HNMR (400 MHz, DMSO-$d_6$): δ = 11.96 (br s, 1H), 8.38 (d, *J* = 8.9 Hz, 1H), 7.55 (s, 1H), 7.51 (d, *J* = 2.4 Hz, 1H), 7.41 (s, 1H), 7.05 (dd, *J* = 2.3, 8.8 Hz, 1H), 5.16 (br dd, *J* = 1.6, 6.8 Hz, 1H), 4.59 - 4.38 (m, 1H), 3.89 - 3.82 (m, 5H), 2.46 (s, 2H), 2.11 - 2.00 (m, 4H), 1.74 - 1.59 (m, 4H), 1.55 - 1.45 (m, 2H); MS: m/z 475.2 [M+H]$^+$ | 3.28 |
| 53 | 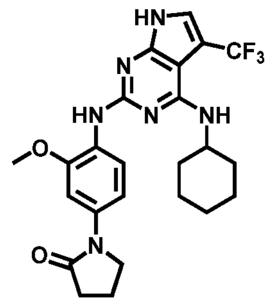 | 1-(4-((4-(cyclohexylamino)-5-(trifluoromethyl)-7*H*-pyrrolo [2,3-*d*]pyrimidin-2-yl)amino)-3-methoxyphenyl)pyrro lidin-2-one | $^1$HNMR (400 MHz, DMSO-$d_6$): δ = 11.92 (br d, *J* = 1.2 Hz, 1H), 8.35 (d, *J* = 8.8 Hz, 1H), 7.55 (s, 1H), 7.49 (d, *J* = 2.2 Hz, 1H), 7.39 (s, 1H), 7.04 (dd, *J* = 2.3, 8.8 Hz, 1H), 5.13 (br d, *J* = 6.5 Hz, 1H), 4.21 - 4.01 (m, 1H), 3.94 - 3.74 (m, 5H), 2.46 (br s, 2H), 2.12 - 1.94 (m, 4H), 1.69 (br dd, *J* = 3.8, 8.7 Hz, 2H), 1.59 (br dd, *J* = 3.7, 8.6 Hz, 1H), 1.48 - 1.25 (m, 5H); MS: m/z 489.2 [M+H]$^+$ | 3.4 |
| 54 | 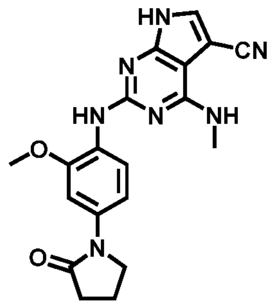 | 2-((2-methoxy-4-(2-oxopyrrolidin-1-yl)phenyl) amino)-4-(methylamino)-7*H*-pyrrolo[2,3-*d*]pyrimidine-5-carbonitrile | $^1$HNMR (400 MHz, DMSO-$d_6$): δ = 12.31 (br s, 1H), 8.34 (d, *J*=8.8 Hz, 1H), 7.87 (s, 1H), 7.72 (br s, 1H), 7.53 (d, *J*=2.2 Hz, 1H), 7.07 (dd, *J*=2.3, 8.9 Hz, 1H), 6.71 (br s, 1H), 3.93 - 3.81 (m, 5H), 3.02 (d, *J*=3.5 Hz, 3H), 2.07 (quin, *J*=7.5 Hz, 2H); MS: m/z 378.2 [M+H]$^+$ | 2.43 |

(continued)

| 실시 예 화합 물 | 구조 | 화합물명 | $^1$H NMR, MS | UPLC r.t. (min) |
|---|---|---|---|---|
| 55 | 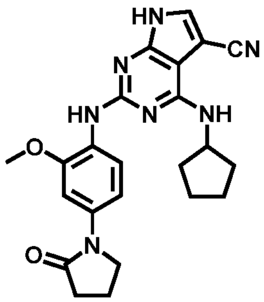 | 4-(cyclopentylamino)-2-((2-methoxy-4-(2-oxopyrrolidin-1-yl) phenyl)amino)-7*H*-pyrrolo[2,3-*d*] pyrimidine-5-carbonitrile | $^1$HNMR (400 MHz, DMSO-$d_6$): δ = 12.19 (br s, 1H), 8.35 (d, *J* = 8.8 Hz, 1H), 7.84 (s, 1H), 7.51 (d, *J* = 2.2 Hz, 1H), 7.44 (s, 1H), 7.05 (dd, *J* = 2.3, 8.8 Hz, 1H), 5.86 (d, *J* = 7.1 Hz, 1H), 4.51 - 4.38 (m, 1H), 3.87 (s, 3H), 3.86 - 3.82 (m, 2H), 2.49 - 2.44 (m, 2H), 2.10 - 2.01 (m, 4H), 1.80 - 1.69 (m, 2H), 1.67 - 1.49 (m, 4H); MS: m/z 432.2 $[M+H]^+$ | 2.3 |
| 56 | 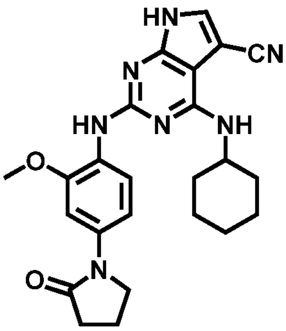 | 4-(cyclohexylamino)-2-((2-methoxy-4-(2-oxopyrrolidin-1-yl) phenyl)amino)-7*H*-pyrrolo[2,3-*d*] pyrimidine-5-carbonitrile | $^1$HNMR (400 MHz, DMSO-$d_6$): δ = 12.18 (br s, 1H), 8.32 (d, *J* = 8.8 Hz, 1H), 7.84 (s, 1H), 7.49 (d, *J* = 2.3 Hz, 1H), 7.43 (s, 1H), 7.05 (dd, *J* = 2.3, 8.9 Hz, 1H), 5.71 (d, *J* = 7.7 Hz, 1H), 4.11 - 4.01 (m, 1H), 3.87 (s, 3H), 3.86 - 3.82 (m, 2H), 2.47 (s, 2H), 2.11 - 2.04 (m, 2H), 2.01 (br dd, *J* = 5.2, 7.8 Hz, 2H), 1.80 - 1.69 (m, 2H), 1.62 (br d, *J* = 11.7 Hz, 1H), 1.48 - 1.36 (m, 4H), 1.32 - 1.22 (m, 1H); MS: m/z 446.2 $[M+H]^+$ | 3.1 |
| 57 | 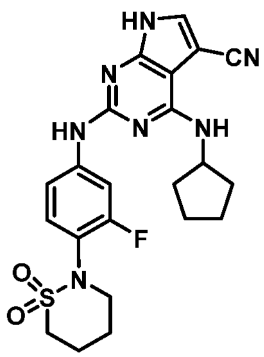 | 4-(cyclopentylamino)-2-((4-(1,1-dioxido-1,2-thiazinan-2-yl)-3-fluorophenyl)amino)-7*H*-pyrrolo [2,3-*d*]pyrimidine-5-carbonitrile | 1H NMR (400 MHz, DMSO-$d_6$) δ 12.25 (s, 1H), 9.38 (s, 1H), 8.08 (dd, *J* = 13.9, 2.4 Hz, 1H), 7.88 (s, 1H), 7.42 (dd, *J* = 9.0, 2.4 Hz, 1H), 7.30 (t, *J* = 8.9 Hz, 1H), 5.92 (d, *J* = 7.1 Hz, 1H), 4.49 (q, *J* = 6.8 Hz, 1H), 3.59 (t, *J* = 5.5 Hz, 2H), 3.27 (t, *J* = 6.1 Hz, 2H), 2.18 (d, *J* = 8.2 Hz, 2H), 2.09 (s, 2H), 1.75 (d, *J* = 12.3 Hz, 4H), 1.60 (dt, *J* = 13.2, 9.3 Hz, 4H) ; MS: m/z 470.3 $[M+H]^+$ | 1.71 |

(continued)

| 실시예 화합물 | 구조 | 화합물명 | $^1$H NMR, MS | UPLC r.t. (min) |
|---|---|---|---|---|
| 58 | 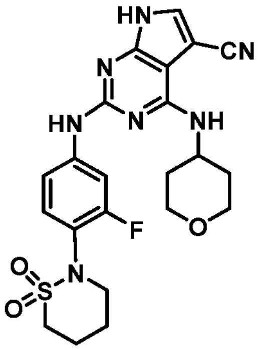 | 2-((4-(1,1-dioxido-1,2-thiazinan-2-yl)-3-fluorophenyl)amino)-4-((tetrahydro-2*H*-pyran-4-yl) amino)-7*H*-pyrrolo[2,3-*d*] pyrimidine-5-carbonitrile | 1H NMR (400 MHz, DMSO-$d_6$) δ 12.26 (s, 1H), 9.39 (s, 1H), 8.05 (dd, *J* = 13.7, 2.4 Hz, 1H), 7.90 (s, 1H), 7.40 - 7.36 (m, 1H), 7.30 (t, *J* = 8.8 Hz, 1H), 6.02 (d, *J* = 7.5 Hz, 1H), 4.32 - 4.21 (m, 1H), 3.93 (d, *J* = 11.8 Hz, 2H), 3.59 (t, *J* = 5.7 Hz, 2H), 3.47 (t, *J* = 11.2 Hz, 2H), 3.27 (t, *J* = 6.1 Hz, 2H), 2.17 (s, 2H), 2.03 - 1.97 (m, 2H), 1.78 (s, 2H), 1.66 (qd, *J* = 11.6, 4.4 Hz, 2H)<br>MS: m/z 486.3 [M+H]$^+$ | 1.45 |
| 59 | 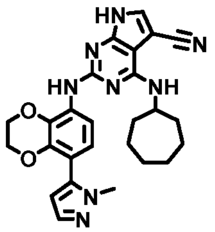 | 4-(cycloheptylamino)-2-((8-(1-methyl-1H-pyrazol-5-yl)-2,3-dihydrobenzo[b][1,4] dioxin-5-yl) amino)-7*H*-pyrrolo[2,3-*d*] pyrimidine-5-carbonitrile | 1H NMR (400 MHz, DMSO-$d_6$) δ 12.40 - 12.13 (m, 1H), 8.12 (d, *J* = 8.5 Hz, 1H), 7.88 (d, *J* = 2.5 Hz, 1H), 7.60 - 7.28 (m, 2H), 6.79 (d, *J* = 8.5 Hz, 1H), 6.23 (d, *J* = 1.9 Hz, 1H), 5.81 (d, *J* = 7.7 Hz, 1H), 4.32 (tdd, *J* = 32.4, 7.6, 3.9 Hz, 5H), 2.00 (td, *J* = 10.4, 8.5, 4.3 Hz, 2H), 1.78 - 1.30 (m, 12H), 1.27 - 1.12 (m, 1H).<br>MS: m/z 485.40 [M+H]$^+$ | 1.92 |
| 60 | 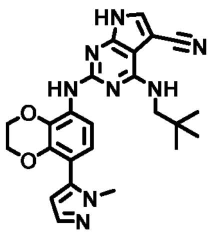 | 2-((8-(1-methyl-1*H*-pyrazol-5-yl)-2,3-dihydrobenzo [b] [1,4] dioxin-5-yl)amino) -4-(neopentylamino)-7*H*-pyrrolo [2,3-*d*]pyrimidine-5-carbonitrile | 1H NMR (400 MHz, DMSO-$d_6$) δ 12.35 (d, *J* = 2.7 Hz, 1H), 8.12 (d, *J* = 8.5 Hz, 1H), 7.89 (d, *J* = 2.3 Hz, 1H), 7.53 (s, 1H), 7.43 (d, *J* = 1.8 Hz, 1H), 6.81 (d, *J* = 8.5 Hz, 1H), 6.23 (d, *J* = 1.8 Hz, 1H), 6.05 (s, 1H), 4.36 (ddd, *J* = 35.4, 6.2, 3.1 Hz, 4H), 3.68 (s, 3H), 3.41 (d, *J* = 6.1 Hz, 2H), 0.97 (s, 9H).<br>MS: m/z 459.36 [M+H]$^+$ | 1.84 |

(continued)

| 실시 예 화합 물 | 구조 | 화합물명 | 1H NMR, MS | UPLC r.t. (min) |
|---|---|---|---|---|
| 61 | 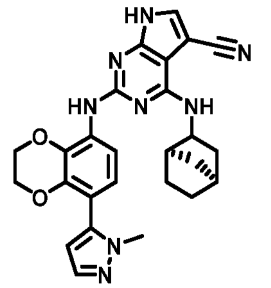 | 4-(((1R,4S)-bicyclo[2.2.1] heptan-2-yl)amino)-2-((8-(1-methyl-1*H*-pyrazol-5-yl)-2,3-dihydrobenzo [b] [1,4] dioxin-5-yl) amino)-7*H*-pyrrolo[2,3-*d*] pyrimidine-5-carbonitrile | 1H NMR (400 MHz, DMSO-$d_6$) δ 12.30 (d, *J* = 2.9 Hz, 1H), 8.18 (d, *J* = 8.5 Hz, 1H), 7.88 (d, *J* = 2.5 Hz, 1H), 7.63 — 7.31 (m, 2H), 6.79 (d, *J* = 8.5 Hz, 1H), 6.23 (d, *J* = 1.8 Hz, 1H), 5.68 (d, *J* = 6.4 Hz, 1H), 4.36 (ddd, *J* = 35.8, 6.0, 3.1 Hz, 4H), 3.96 - 3.90 (m, 1H), 3.68 (s, 3H), 2.35 (dd, *J* = 27.2, 4.5 Hz, 2H), 1.95 - 1.78 (m, 1H), 1.64 - 1.39 (m, 3H), 1.39 - 1.25 (m, 2H), 1.25 - 1.11 (m, 2H).<br>MS: m/z 483.37 $[M+H]^+$ | 1.89 |
| 62 | 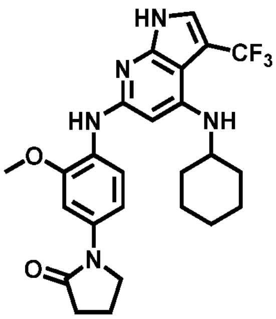 | 1-(4-((4-(cyclohexylamino)-3-(trifluoromethyl)-1*H*-pyrrolo [2,3-*b*]pyridin-6-yl)amino)-3-methoxyphenyl)pyrro lidin-2-one | 1H NMR (400 MHz, MeOD) δ 7.69 (d, J = 2.3 Hz, 1H), 7.47 (d, J = 1.4 Hz, 1H), 7.41 (d, J = 8.5 Hz, 1H), 7.18 (dd, J = 8.6, 2.3 Hz, 1H), 5.92 (s, 1H), 4.00 (t, J = 7.1 Hz, 2H), 3.90 (s, 3H), 3.64 - 3.53 (m, 1H), 2.67 (t, J = 8.1 Hz, 2H), 2.30 - 2.18 (m, 2H), 2.15 - 2.04 (m, 2H), 1.87 - 1.75 (m, 2H), 1.74 - 1.64 (m, 1H), 1.58 - 1.39 (m, 5H).<br>MS: m/z 488.4 $[M+H]^+$ | 1.58 |
| 63 | 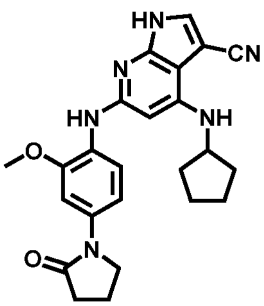 | 4-(cyclopentylamino)-6-((2-methoxy-4-(2-oxopyrrolidin-1-yl) phenyl)amino)-1*H*-pyrrolo[2,3-*b*] pyridine-3-carbonitrile | $^1$HNMR (400 MHz, DMSO-$d_6$): δ = 12.0 (br s, 1H), 8.35 (d, *J*=8.0 Hz, 1H), 7.87 (s, 1H), 7.77 (s, 1H), 7.48 (s, 1H), 6.98 (d, J=8 , 1H), 6.19 (s, 1H), 5.04 (s, 1H), 3.84 (s, 6H), 2.05 (s, 4H), 1.50-1.72 (m, 8H)<br>MS: m/z 431.3 $[M+H]^+$ | 1.38 |

(continued)

| 실시예 화합물 | 구조 | 화합물명 | $^1$H NMR, MS | UPLC r.t. (min) |
|---|---|---|---|---|
| 64 | 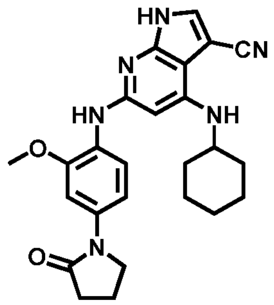 | 4-(cyclohexylamino)-6-((2-methoxy-4-(2-oxopyrrolidin-1-yl) phenyl)amino)-1*H*-pyrrolo[2,3-*b*] pyridine-3-carbonitrile | 1H NMR (400 MHz, DMSO-d6) δ 11.99 (s, 1H), 8.45 (s, 1H), 7.98 (d, *J* = 2.9 Hz, 1H), 7.82 (d, *J* = 2.3 Hz, 1H), 7.54 (d, *J* = 2.3 Hz, 1H), 7.07 (d, *J* = 8.8 Hz, 1H), 6.07 (s, 1H), 5.41 (s, 1H), 3.86 (d, *J* = 6.9 Hz, 2H), 3.83 (d, *J* = 2.7 Hz, 3H), 3.44 (s, 2H), 2.07 (p, *J* = 7.5 Hz, 2H), 2.03 - 1.95 (m, 2H), 1.77 - 1.68 (m, 2H), 1.64 - 1.54 (m, 1H), 1.47 - 1.25 (m, 6H). ;<br>MS: m/z 445.4 $[M+H]^+$ | 1.43 |
| 65 | 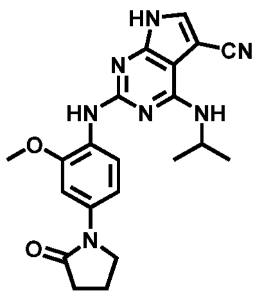 | 4-(isopropylamino)-2-((2-methoxy-4-(2-oxopyrrolidin-1-yl) phenyl)amino)-7*H*-pyrrolo[2,3-*d*] pyrimidine-5-carbonitrile | $^1$HNMR (400 MHz, DMSO-$d_6$): δ = 12.29 (br s, 1H), 8.29 (d, *J*=8.7 Hz, 1H), 7.87 (s, 1H), 7.68 (br s, 1H), 7.53 (d, *J*=2.3 Hz, 1H), 7.07 (dd, *J*=2.3, 8.8 Hz, 1H), 5.98 (br s, 1H), 4.37 - 4.30 (m, 1H), 3.93 - 3.80 (m, 5H), 2.48 (s, 2H), 2.07 (quin, *J*=7.5 Hz, 2H);<br>MS: m/z 406.1 $[M+H]^+$ | 2.33 |
| 66 | 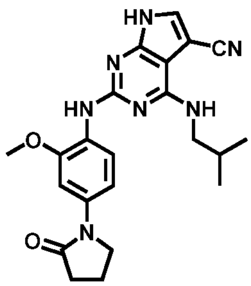 | 4-(isobutylamino)-2-((2-methoxy-4-(2-oxopyrrolidin-1-yl) phenyl)amino)-7*H*-pyrrolo[2,3-*d*] pyrimidine-5-carbonitrile | $^1$HNMR (400 MHz, DMSO-$d_6$): δ = 12.33 (br s, 1H), 8.27 (d, *J*=8.8 Hz, 1H), 7.88 (s, 1H), 7.78 (br s, 1H), 7.52 (d, *J*=2.3 Hz, 1H), 7.07 (dd, *J*=2.3, 8.8 Hz, 1H), 6.56 (br s, 1H), 3.88 - 3.83 (m, 4H), 3.37 (t, *J*=6.3 Hz, 2H), 2.48 (s, 2H), 2.11 - 1.95 (m, 3H), 0.96 (d, *J*=6.7 Hz, 6H);<br>MS: m/z 420.1 $[M+H]^+$ | 2.34 |
| 67 | 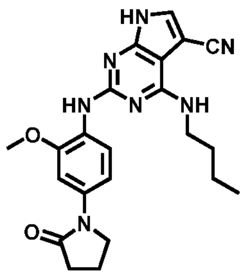 | 4-(butylamino)-2-((2-methoxy-4-(2-oxopyrrolidin-1-yl)phenyl) amino)-7*H*-pyrrolo[2,3-*d*] pyrimidine-5-carbonitrile | $^1$HNMR (400 MHz, DMSO-$d_6$): δ = 12.18 (br s, 1H), 8.34 (d, J = 8.8 Hz, 1H), 7.84 (s, 1H), 7.50 (d, J = 2.3 Hz, 1H), 7.41 (s, 1H), 7.04 (dd, J = 2.3, 8.8 Hz, 1H), 6.27 (t, J = 5.6 Hz, 1H), 3.92 - 3.77 (m, 5H), 3.51 (m, 2H), 2.49 - 2.45 (m, 2H), 2.07 (m, 2H), 1.63 (m, 2H), 1.47 - 1.29 (m, 2H), 0.94 (t, J = 7.4 Hz, 3H);<br>MS: m/z 420.2 $[M+H]^+$ | 3.0 |

(continued)

| 실시예 화합물 | 구조 | 화합물명 | $^1$H NMR, MS | UPLC r.t. (min) |
|---|---|---|---|---|
| 68 | 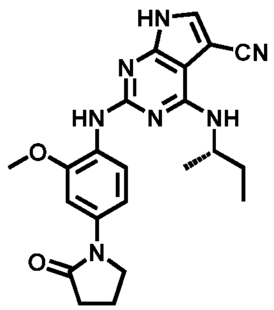 | (*S*)-4-(*sec*-butylamino)-2-((2-methoxy-4-(2-oxopyrrolidin-1-yl) phenyl)amino)-7*H*-pyrrolo[2,3-*d*] pyrimidine-5-carbonitrile | $^1$HNMR (400 MHz, DMSO-$d_6$): $\delta$ = 12.20 (br s, 1H), 8.32 (d, *J*=8.8 Hz, 1H), 7.84 (s, 1H), 7.51 (d, *J*=2.3 Hz, 1H), 7.43 (s, 1H), 7.05 (dd, *J*=2.3, 8.8 Hz, 1H), 5.64 (br d, *J*=8.0 Hz, 1H), 4.25 - 4.15 (m, 1H), 3.88 - 3.82 (m, 5H), 2.49 - 2.39 (m, 2H), 2.10 - 2.00 (m, 2H), 1.71 - 1.54 (m, 2H), 1.25 (d, *J*=6.5 Hz, 3H), 0.94 (t, *J*=7.4 Hz, 3H);<br>MS: m/z 420.2 $[M+H]^+$ | 2.45 |
| 69 | 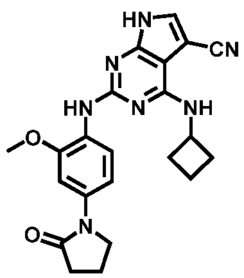 | 4-(cyclobutylamino)-2-((2-methoxy-4-(2-oxopyrrolidin-1-yl) phenyl)amino)-7*H*-pyrrolo[2,3-*d*] pyrimidine-5-carbonitrile | $^1$HNMR (400 MHz, DMSO-$d_6$): $\delta$ = 8.46 (d, *J* = 8.8 Hz, 1H), 7.75 (s, 1H), 7.50 (d, *J* = 2.3 Hz, 1H), 7.29 (s, 1H), 7.04 (dd, *J* = 2.3, 8.9 Hz, 1H), 6.02 (br d, *J* = 6.7 Hz, 1H), 4.67 - 4.57 (m, 1H), 3.89 - 3.83 (m, 5H), 2.50 - 2.46 (m, 3H), 2.39 - 2.32 (m, 2H), 2.10 - 2.01 (m, 4H), 1.78 - 1.70 (m, 2H);<br>MS: m/z 418.2 $[M+H]^+$ | 2.87 |
| 70 | 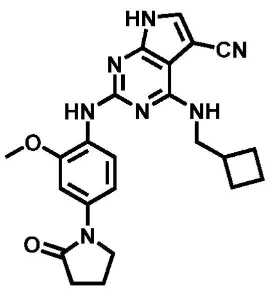 | 4-((cyclobutylmethyl)a mino)-2-((2-methoxy-4-(2-oxopyrrolidin-1-yl)phenyl) amino)-7*H*-pyrrolo[2,3-*d*] pyrimidine-5-carbonitrile | $^1$HNMR (400 MHz, DMSO-$d_6$): $\delta$ = 12.33 - 12.01 (m, 1H), 8.33 (d, *J* = 8.8 Hz, 1H), 7.84 (s, 1H), 7.50 (d, *J* = 2.3 Hz, 1H), 7.42 (s, 1H), 7.04 (dd, *J* = 2.3, 8.9 Hz, 1H), 6.19 (t, *J* = 5.6 Hz, 1H), 3.92 - 3.80 (m, 5H), 3.63 - 3.53 (m, 2H), 2.74 - 2.65 (m, 1H), 2.49 - 2.44 (m, 2H), 2.11 - 2.01 (m, 4H), 1.92 - 1.71 (m, 4H);<br>MS: m/z 432.2 $[M+H]^+$ | 3.02 |

(continued)

| 실시 예 화합물 | 구조 | 화합물명 | $^1$H NMR, MS | UPLC r.t. (min) |
|---|---|---|---|---|
| 71 | 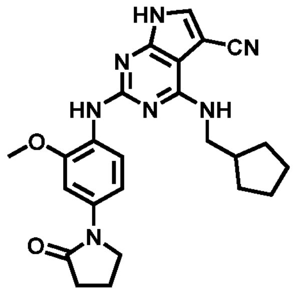 | 4-((cyclopentylmethyl) amino)-2-((2-methoxy-4-(2-oxopyrrolidin-1-yl)phenyl) amino)-7*H*-pyrrolo[2,3-*d*] pyrimidine-5-carbonitrile | $^1$HNMR (400 MHz, DMSO-*d*$_6$): δ = 12.20 (s, 1H), 8.33 (d, J = 8.8 Hz, 1H), 7.84 (s, 1H), 7.50 (d, J = 2.3 Hz, 1H), 7.42 (s, 1H), 7.04 (dd, J = 2.3, 8.8 Hz, 1H), 6.20 (t, J = 5.6 Hz, 1H), 3.91 - 3.79 (m, 5H), 3.46 (dd, J = 6.0, 7.0 Hz, 2H), 2.49 - 2.47 (m, 2H), 2.37 - 2.20 (m, 1H), 2.06 (m, 2H), 1.81 - 1.69 (m, 2H), 1.69 - 1.61 (m, 2H), 1.58 - 1.46 (m, 2H), 1.41 - 1.21 (m, 2H); MS: m/z 446.2 [M+H]$^+$ | 2.94 |
| 72 | 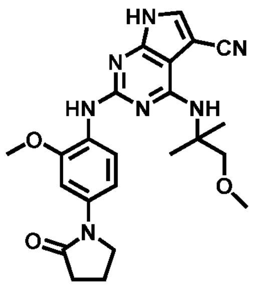 | 4-((1-methoxy-2-methylpropan-2-yl)amino)-2-((2-methoxy-4-(2-oxopyrrolidin-1-yl)phenyl) amino)-7*H*-pyrrolo[2,3-*d*] pyrimidine-5-carbonitrile | $^1$HNMR (400 MHz, DMSO-*d*$_6$): δ = 12.20 (br s, 1H), 8.13 (d, *J* = 8.8 Hz, 1H), 7.82 (s, 1H), 7.58 - 7.44 (m, 2H), 7.06 (dd, *J* = 2.3, 8.8 Hz, 1H), 5.73 (s, 1H), 3.89 - 3.81 (m, 5H), 3.48 (s, 2H), 3.34 (s, 3H), 2.11 - 2.00 (m, 2H), 1.47 (s, 6H); MS: m/z 450.2 [M+H]$^+$ | 2.9 |
| 73 | 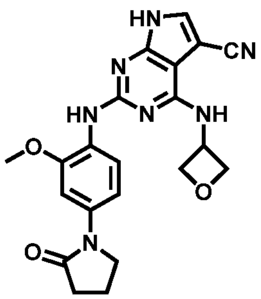 | 2-((2-methoxy-4-(2-oxopyrrolidin-1-yl)phenyl) amino)-4-(oxetan-3-ylamino)-7*H*-pyrrolo[2,3-*d*]pyrimidine-5-carbonitrile | $^1$HNMR (400 MHz, DMSO-*d*$_6$): δ = 12.00 (br s, 1H), 8.00 (s, 1H), 7.64 - 7.33 (m, 3H), 7.10 (dd, J = 2.3, 8.6 Hz, 1H), 4.86 (br s, 1H), 4.38 - 4.22 (m, 1H), 4.08 - 3.96 (m, 1H), 3.88 (t, J = 7.1 Hz, 3H), 3.80 (s, 3H), 3.62 (td, J = 4.4, 10.6 Hz, 1H), 3.45 (td, J = 5.4, 10.8 Hz, 1H), 2.54 (d, J = 2.0 Hz, 2H), 2.09 (m, 2H); MS: m/z 420.2 [M+H]$^+$ | 2.13 |

(continued)

| 실시예 화합물 | 구조 | 화합물명 | 1H NMR, MS | UPLC r.t. (min) |
|---|---|---|---|---|
| 74 | 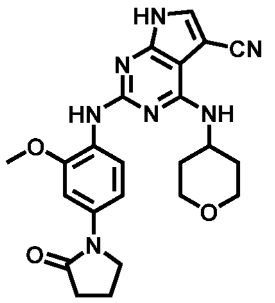 | 2-((2-methoxy-4-(2-oxopyrrolidin-1-yl)phenyl) amino)-4-((tetrahydro-2*H*-pyran-4-yl)amino)-7*H*-pyrrolo[2,3-*d*] pyrimidine-5-carbonitrile | $^1$HNMR (400 MHz, DMSO-$d_6$): δ = 12.13 (br s, 1H), 8.21 (d, J = 8.8 Hz, 1H), 7.78 (s, 1H), 7.43 (d, J = 2.4 Hz, 1H), 7.39 (s, 1H), 6.97 (dd, J = 2.3, 8.8 Hz, 1H), 5.88 (d, J = 7.4 Hz, 1H), 4.29 - 4.08 (m, 1H), 3.87 - 3.80 (m, 2H), 3.80 - 3.75 (m, 5H), 3.40 (dt, J = 1.9, 11.4 Hz, 2H), 2.46 (br d, J = 1.9 Hz, 2H), 2.06 - 1.94 (m, 2H), 1.90 (br dd, J = 2.2, 12.3 Hz, 2H), 1.61 - 1.47 (m, 2H);<br>MS: m/z 448.2 [M+H]$^+$ | 2.66 |
| 75 | 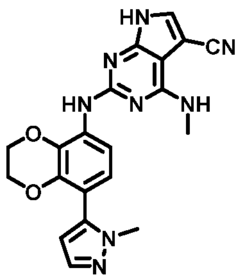 | 2-((8-(1-methyl-1*H*-pyrazol-5-yl)-2,3-dihydrobenzo [b] [1,4] dioxin-5-yl)amino)-4-(methylamino)-7*H*-pyrrolo[2,3-*d*]pyrimidine-5-carbonitrile | 1H NMR (400 MHz, DMSO-$d_6$) δ 12.35 (s, 1H), 8.17 (d, *J* = 8.5 Hz, 1H), 7.89 (d, *J* = 2.5 Hz, 1H), 7.56 (s, 1H), 7.42 (d, *J* = 1.8 Hz, 1H), 6.81 (d, *J* = 8.5 Hz, 1H), 6.64 (s, 1H), 6.23 (d, *J* = 1.8 Hz, 1H), 4.36 (ddd, *J* = 36.8, 5.9, 3.0 Hz, 4H), 3.68 (s, 3H), 3.01 (d, *J* = 4.1 Hz, 3H).<br>MS: m/z 403.3[M+H]$^+$ | 1.42 |
| 76 | 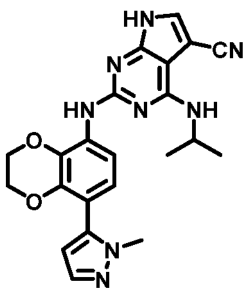 | 4-(isopropylamino)-2-((8-(1-methyl-1*H*-pyrazol-5-yl)-2,3-dihydrobenzo[b][1,4] dioxin-5-yl) amino)-7*H*-pyrrolo[2,3-*d*] pyrimidine-5-carbonitrile | 1H NMR (400 MHz, Chloroform-*d*) δ 7.99 (d, *J* = 8.5 Hz, 1H), 7.51 (d, *J* = 26.0 Hz, 2H), 7.21 (s, 1H), 6.81 (d, *J* = 8.5 Hz, 1H), 6.28 (s, 1H), 5.50 (d, *J* = 7.6 Hz, 1H), 4.50 (dp, *J* = 13.3, 6.6 Hz, 1H), 4.43 - 4.28 (m, 4H), 3.80 (s, 3H), 1.36 (d, *J* = 6.5 Hz, 6H), 1.30 - 1.23 (m, 1H).<br>MS: m/z 431.3 [M+H]$^+$ | 1.65 |
| 77 | 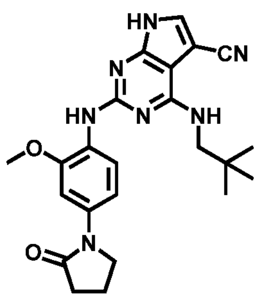 | 2-((2-methoxy-4-(2-oxopyrrolidin-1-yl)phenyl) amino)-4-(neopentylamino)-7*H*-pyrrolo[2,3-*d*]pyrimidine-5-carbonitrile | 1H NMR (400 MHz, Methanol-$d_4$) δ 8.56 (d, *J* = 8.8 Hz, 1H), 7.57 (s, 1H), 7.41 (d, *J* = 2.3 Hz, 1H), 6.99 (dd, *J* = 2.4, 8.9 Hz, 1H), 3.94 (d, *J* = 5.1 Hz, 5H), 3.49 (s, 2H), 2.60 (t, *J* = 8.1 Hz, 2H), 2.20 (td, *J* = 6.8, 8.0 Hz, 2H), 1.04 (s, 9H);<br>MS: m/z 434.2 [M+H]$^+$ | 1.68 |

(continued)

| 실시예 화합물 | 구조 | 화합물명 | $^1$H NMR, MS | UPLC r.t. (min) |
|---|---|---|---|---|
| 78 | 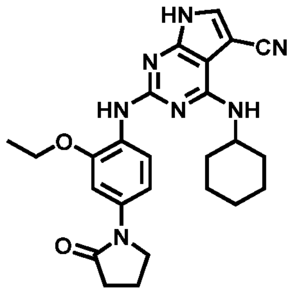 | 4-(cyclohexylamino)-2-((2-ethoxy-4-(2-oxopyrrolidin-1-yl) phenyl)amino)-7*H*-pyrrolo[2,3-*d*] pyrimidine-5-carbonitrile | 1H NMR (400 MHz, DMSO-$d_6$) δ 12.17 (s, 1H), 8.36 (d, *J* = 8.8 Hz, 1H), 7.84 (s, 1H), 7.49 (d, *J* = 2.3 Hz, 1H), 7.39 (s, 1H), 7.03 (dd, *J* = 8.9, 2.3 Hz, 1H), 5.78 - 5.73 (m, 1H), 4.12 (q, *J* = 6.9 Hz, 2H), 3.83 (t, *J* = 7.0 Hz, 2H), 2.47 (d, *J* = 8.0 Hz, 2H), 2.06 (d, *J* = 7.5 Hz, 2H), 2.03 (d, *J* = 7.9 Hz, 2H), 1.74 (s, 2H), 1.62 (d, *J* = 12.7 Hz, 1H), 1.41 (s, 2H), 1.40 (s, 3H), 1.38 (s, 2H), 1.23 (s, 2H);<br>MS: m/z 460.3 $[M+H]^+$ | 1.74 |
| 79 | 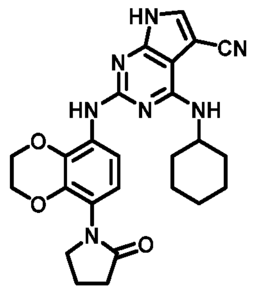 | 4-(cyclohexylamino)-2-((8-(2-oxopyrrolidin-1-yl)-2,3-dihydrobenzo[b][1,4] dioxin-5-yl) amino)-7*H*-pyrrolo[2,3-*d*] pyrimidine-5-carbonitrile | 1H NMR (400 MHz, DMSO-$d_6$) δ 12.28 (s, 1H), 7.90 - 7.83 (m, 2H), 7.48 (s, 1H), 6.73 (d, *J* = 8.8 Hz, 1H), 5.81 (d, *J* = 7.7 Hz, 1H), 4.34 (dd, *J* = 5.7, 2.7 Hz, 2H), 4.28 (dd, *J* = 5.7, 2.7 Hz, 2H), 4.05 (s, 2H), 3.64 (t, *J* = 7.0 Hz, 2H), 2.36 (q, *J* = 8.0 Hz, 2H), 2.13 - 2.06 (m, 2H), 2.06 - 1.95 (m, 2H), 1.73 (p, *J* = 5.2 Hz, 2H), 1.40 (t, *J* = 9.2 Hz, 5H). ;<br>MS: m/z 474.3 $[M+H]^+$ | 1.74 |
| 80 | 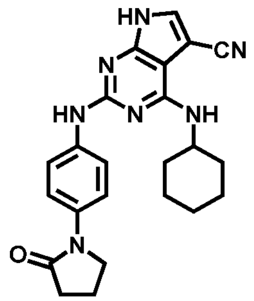 | 4-(cyclohexylamino)-2-((4-(2-oxopyrrolidin-1-yl)phenyl) amino)-7*H*-pyrrolo[2,3-*d*] pyrimidine-5-carbonitrile | 1H NMR (400 MHz, Methanol-$d_4$) δ 7.76 (d, *J* = 9.0 Hz, 2H), 7.53 (s, 1H), 7.45 (d, *J* = 9.0 Hz, 2H), 4.22 - 4.11 (m, 1H), 3.91 (t, *J* = 7.1 Hz, 2H), 2.59 (t, *J* = 8.1 Hz, 2H), 2.25 - 2.08 (m, 5H), 1.87 - 1.77 (m, 2H), 1.69 (d, *J* = 12.6 Hz, 1H), 1.54 - 1.32 (m, 4H);<br>MS: m/z 416.2 $[M+H]^+$ | 1.65 |

(continued)

| 실시예 화합물 | 구조 | 화합물명 | 1H NMR, MS | UPLC r.t. (min) |
|---|---|---|---|---|
| 81 | 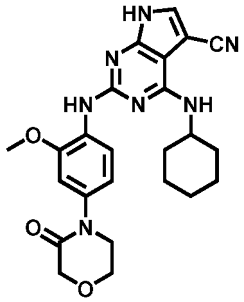 | 4-(cyclohexylamino)-2-((2-methoxy-4-(3-oxomorpholino) phen yl)amino)-7*H*-pyrrolo[2,3-*d*] pyrimidine-5-carbonitrile | 1H NMR (400 MHz, Methanol-$d_4$) δ 8.66 (d, *J* = 8.6 Hz, 1H), 7.57 (s, 1H), 7.00 (d, *J* = 2.3 Hz, 1H), 6.89 (dd, *J* = 2.3, 8.7 Hz, 1H), 4.28 (s, 2H), 4.21 - 4.09 (m, 1H), 4.05 (dd, *J* = 4.2, 5.9 Hz, 2H), 3.96 (s, 3H), 3.78 (dd, *J* = 4.2, 6.0 Hz, 2H), 2.19 - 2.08 (m, 2H), 1.90 - 1.78 (m, 3H), 1.76 - 1.64 (m, 1H), 1.59 - 1.35 (m, 4H);<br>MS: m/z 462.2 $[M+H]^+$ | 1.63 |
| 82 | 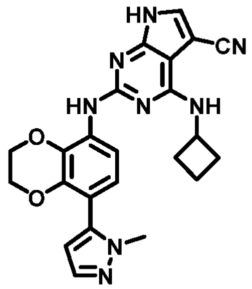 | 4-(cyclobutylamino)-2-((8-(1-methyl-1*H*-pyrazol-5-yl)-2,3-dihydrobenzo[b][1,4] dioxin-5-yl) amino)-7*H*-pyrrolo[2,3-*d*] pyrimidine-5-carbonitrile | 1H NMR (400 MHz, DMSO-$d_6$) δ 12.29 (s, 1H), 8.16 (d, *J* = 8.5 Hz, 1H), 7.88 (s, 1H), 7.42 (t, *J* = 1.5 Hz, 2H), 6.82 (d, *J* = 8.5 Hz, 1H), 6.38 (d, *J* = 7.4 Hz, 1H), 6.23 (d, *J* = 1.8 Hz, 1H), 4.62 (h, *J* = 8.0 Hz, 1H), 4.36 (ddd, *J* = 36.5, 6.1, 3.0 Hz, 4H), 3.68 (s, 3H), 3.17 (d, *J* = 4.4 Hz, 2H), 2.42 - 2.28 (m, 2H), 2.13 - 2.05 (m, 2H).<br>MS: m/z 443.3 $[M+H]^+$ | 1.70 |
| 83 | 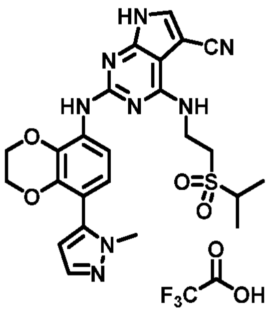 | 4-((2-(isopropylsulfonyl)et hyl) amino)-2-((8-(1-methyl-1*H*-pyrazol-5-yl)-2,3-dihydrobenzo[b][1,4] dioxin-5-yl) amino)-7*H*-pyrrolo[2,3-*d*] pyrimidine-5-carbonitrile 2,2,2-trifluoroacetate | 1H NMR (400 MHz, DMSO-$d_6$) δ 12.38 (d, *J* = 3.0 Hz, 1H), 8.12 (d, *J* = 8.5 Hz, 1H), 7.92 (d, *J* = 2.3 Hz, 1H), 7.57 (s, 1H), 7.43 (d, *J* = 1.8 Hz, 1H), 7.28 - 6.95 (m, 1H), 6.82 (d, *J* = 8.2 Hz, 2H), 6.22 (d, *J* = 1.8 Hz, 1H), 4.36 (ddd, *J* = 35.6, 6.0, 3.1 Hz, 4H), 3.94 (dd, *J* = 9.4, 4.6 Hz, 2H), 3.68 (s, 3H), 3.51 - 3.30 (m, 3H), 1.27 (d, *J* = 6.8 Hz, 6H).<br>MS: m/z 523.3 $[M+H]^+$ | 1.45 |

(continued)

| 실시예 화합물 | 구조 | 화합물명 | $^1$H NMR, MS | UPLC r.t. (min) |
|---|---|---|---|---|
| 84 | 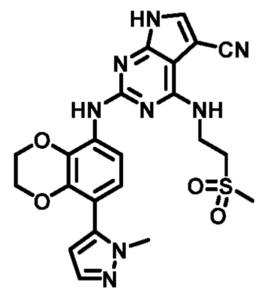 | 2-((8-(1-methyl-1*H*-pyrazol-5-yl)-2,3-dihydrobenzo[b][1,4] dioxin-5-yl)amino)-4-((2-(methylsulfonyl)ethyl ) amino)-7*H*-pyrrolo[2,3-*d*] pyrimidine-5-carbonitrile | 1H NMR (400 MHz, DMSO-$d_6$) δ 12.35 (d, *J* = 2.9 Hz, 1H), 8.10 (d, *J* = 8.5 Hz, 1H), 7.91 (d, *J* = 2.7 Hz, 1H), 7.58 (s, 1H), 7.42 (d, *J* = 1.9 Hz, 1H), 6.81 (d, *J* = 8.5 Hz, 1H), 6.22 (d, *J* = 1.8 Hz, 1H), 4.36 (ddd, *J* = 35.0, 5.9, 3.0 Hz, 4H), 3.91 (dq, *J* = 29.3, 6.5 Hz, 3H), 3.68 (s, 3H), 3.51 (t, *J* = 6.9 Hz, 2H), 3.06 (s, 3H).<br>MS: m/z 495.2 $[M+H]^+$ | 1.32 |
| 85 | 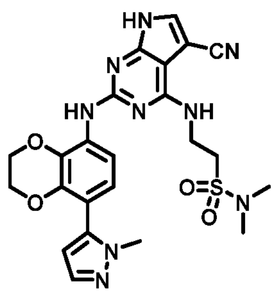 | 2-((5-cyano-2-((8-(1-methyl-1*H*-pyrazol-5-yl)-2,3-dihydrobenzo[b][1,4] dioxin-5-yl) amino)-7*H*-pyrrolo[2,3-d] pyrimidin-4-yl)amino)- N,N-dimethylethane-1-sulfonamide | 1H NMR (400 MHz, DMSO-$d_6$) δ 12.35 (s, 1H), 8.13 (d, *J* = 8.5 Hz, 1H), 7.92 (d, *J* = 1.7 Hz, 1H), 7.51 (s, 1H), 7.42 (d, *J* = 1.8 Hz, 1H), 6.82 (d, *J* = 8.5 Hz, 1H), 6.74 (t, *J* = 5.8 Hz, 1H), 6.22 (d, *J* = 1.9 Hz, 1H), 4.47 - 4.25 (m, 4H), 3.91 (q, *J* = 6.4 Hz, 2H), 3.68 (s, 3H), 3.48 - 3.40 (m, 2H), 2.81 (s, 6H).<br>MS: m/z 524.3 $[M+H]^+$ | 1.42 |
| 86 | 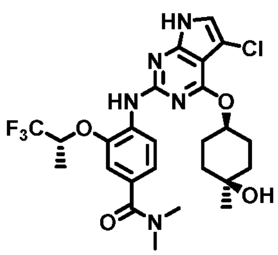 | 4-((5-chloro-4-(((1s,4*S*)-4-hydroxy-4-methylcyclohexyl)oxy)-7*H*-pyrrolo[2,3-d]pyrimidin-2-yl)amino)-*N,N*-dimethyl-3-(((*R*)-1,1,1-trifluoropropan-2-yl)oxy) benzamide | 1H NMR (400 MHz, DMSO-$d_6$) δ 11.75 (s, 1H), 8.43 (d, *J* = 8.4 Hz, 1H), 7.62 (s, 1H), 7.32 (d, *J* = 1.8 Hz, 1H), 7.16 (s, 1H), 7.12 (dd, *J* = 8.4, 1.7 Hz, 1H), 5.35 (p, *J* = 6.5 Hz, 1H), 5.17 - 5.09 (m, 1H), 4.24 (s, 1H), 2.98 (s, 6H), 1.92 - 1.83 (m, 4H), 1.68 (d, *J* = 13.0 Hz, 2H), 1.51 - 1.39 (m, 5H), 1.16 (s, 3H) ;<br>MS: m/z 556.4 $[M+H]^+$ | 2.59 |

(continued)

| 실시예 화합물 | 구조 | 화합물명 | $^1$H NMR, MS | UPLC r.t. (min) |
|---|---|---|---|---|
| 87 | 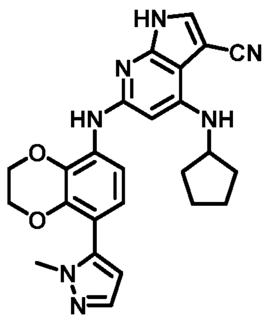 | 4-(cyclopentylamino)-6-((2-methoxy-4-(1-methyl-1*H*-pyrazol-5-yl)phenyl)amino)-1*H*-pyrrolo[2,3-*b*]pyridine-3-carbonitrile | 1H NMR (400 MHz, DMSO-$d_6$) δ 12.12 (s, 1H), 8.39 (s, 1H), 7.85 (d, *J* = 2.3 Hz, 1H), 7.46 (d, *J* = 1.8 Hz, 1H), 7.14 (d, *J* = 1.9 Hz, 1H), 7.07 (dd, *J* = 8.2, 1.9 Hz, 1H), 6.40 (d, *J* = 1.9 Hz, 1H), 6.24 (s, 1H), 5.42 (s, 1H), 3.93 (s, 3H), 3.91 (s, 1H), 3.89 (s, 3H), 2.53 (s, 1H), 2.10 (dq, *J* = 12.9, 6.5, 5.9 Hz, 2H), 1.78 - 1.71 (m, 2H), 1.71 — 1.61 (m, 2H), 1.55 (dq, *J* = 12.2, 6.1, 5.1 Hz, 2H).<br>MS: m/z 428.3 [M+H]$^+$ | 1.57 |
| 88 | 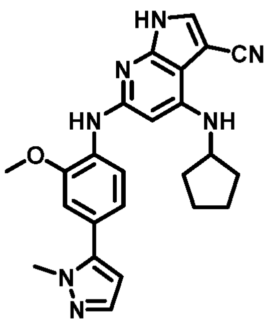 | 4-(cyclopentylamino)-6-((8-(1-methyl-1*H*-pyrazol-5-yl)-2,3-dihydrobenzo[b][1,4] dioxin-5-yl) amino)-1*H*-pyrrolo[2,3-*b*] pyridine-3-carbonitrile | 1H NMR (400 MHz, DMSO-$d_6$) δ 12.14 (s, 1H), 8.59 (s, 1H), 7.85 (d, *J* = 2.3 Hz, 1H), 7.44 (d, *J* = 1.9 Hz, 1H), 6.80 (d, *J* = 8.4 Hz, 1H), 6.23 (dd, *J* = 7.2, 2.0 Hz, 2H), 5.53 (s, 1H), 4.41 - 4.29 (m, 4H), 3.90 (p, *J* = 6.1 Hz, 1H), 3.69 (s, 3H), 2.53 (s, 1H), 2.10 (dq, *J* = 13.0, 6.4 Hz, 2H), 1.74 (tq, *J* = 9.5, 5.6, 4.4 Hz, 2H), 1.64 (tdd, *J* = 11.6, 6.3, 3.9 Hz, 2H), 1.55 (dq, *J* = 13.1, 6.4 Hz, 2H). ;<br>MS: m/z 456.3 [M+H]$^+$ | 1.52 |
| 89 | 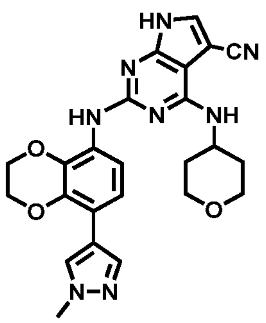 | 2-((8-(1-methyl-1*H*-pyrazol-4-yl)-2,3-dihydrobenzo[b][1,4] dioxin-5-yl)amino)-4-((tetrahydro-2*H*-pyran-4-yl) amino)-7*H*-pyrrolo[2,3-*d*] pyrimidine-5-carbonitrile | 1H NMR (400 MHz, DMSO-*d6*) δ 12.21 (s, 1H), 8.01 (s, 1H), 7.95 (d, *J* = 8.6 Hz, 1H), 7.84 (d, *J* = 14.6 Hz, 2H), 7.36 (s, 1H), 7.09 (d, *J* = 8.6 Hz, 1H), 5.96 (d, *J* = 7.5 Hz, 1H), 4.37 (s, 4H), 4.22 (d, *J* = 4.8 Hz, 1H), 3.94 - 3.87 (m, 2H), 3.86 (s, 3H), 3.53 - 3.44 (m, 2H), 1.98 (d, *J* = 12.6 Hz, 2H), 1.63 (qd, *J* = 11.6, 4.1 Hz, 2H) ;<br>MS: m/z 473.3 [M+H]$^+$ | 1.40 |

(continued)

| 실시예 화합물 | 구조 | 화합물명 | $^1$H NMR, MS | UPLC r.t. (min) |
|---|---|---|---|---|
| 90 | 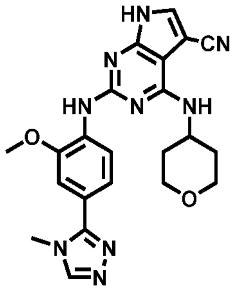 | 2-((2-methoxy-4-(4-methyl-4*H*-1,2,4-triazol-3-yl)phenyl)amino)-4-((tetrahydro-2*H*-pyran-4-yl) amino)-7*H*-pyrrolo[2,3-*d*] pyrimidine-5-carbonitrile | 1H NMR (400 MHz, DMSO-$d_6$) δ 12.32 (d, J = 2.7 Hz, 1H), 8.64 (d, J = 8.4 Hz, 1H), 8.54 (s, 1H), 7.91 (d, *J* = 2.6 Hz, 1H), 7.64 (s, 1H), 7.35 (d, J = 1.9 Hz, 1H), 7.30 (dd, J = 8.4, 1.9 Hz, 1H), 6.07 (d, J = 7.5 Hz, 1H), 4.34 - 4.20 (m, 1H), 3.96 (s, 3H), 3.91 (dt, J = 11.6, 3.4 Hz, 2H), 3.77 (s, 3H), 3.50 (td, J = 11.6, 2.2 Hz, 2H), 1.99 (dd, J = 9.4, 5.8 Hz, 2H), 1.72 - 1.58 (m, 2H) ; MS: m/z 446.3 $[M+H]^+$ | 1.22 |
| 91 | 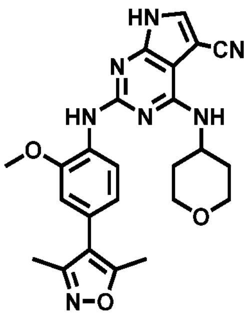 | 2-((4-(3,5-dimethylisoxazol-4-yl)-2-methoxyphenyl)amin o)-4-((tetrahydro-2*H*-pyran-4-yl) amino)-7*H*-pyrrolo[2,3-*d*] pyrimidine-5-carbonitrile | 1H NMR (400 MHz, DMSO-*d*6) δ 12.26 (s, 1H), 8.50 (d, *J* = 8.3 Hz, 1H), 7.88 (s, 1H), 7.53 (s, 1H), 6.99 (d, *J* = 1.8 Hz, 1H), 6.93 (dd, *J* = 8.3, 1.9 Hz, 1H), 6.01 (d, *J* = 7.5 Hz, 1H), 4.31 - 4.19 (m, 1H), 3.92 (s, 5H), 3.54 - 3.43 (m, 2H), 2.43 (s, 3H), 2.26 (s, 3H), 1.98 (d, *J* = 11.3 Hz, 2H), 1.64 (qd, *J* = 11.6, 4.3 Hz, 2H) ; MS: m/z 460.3 $[M+H]^+$ | 1.61 |
| 92 | 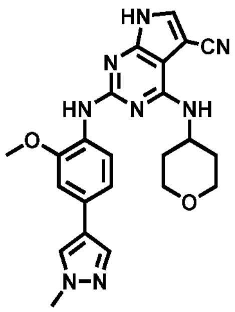 | 2-((2-methoxy-4-(1-methyl-1*H*-pyrazol-4-yl)phenyl)amino)-4-((tetrahydro-2*H*-pyran-4-yl) amino)-7*H*-pyrrolo[2,3-*d*] pyrimidine-5-carbonitrile | 1H NMR (400 MHz, DMSO-*d*6) δ 12.21 (s, 1H), 8.35 (d, *J* = 8.3 Hz, 1H), 8.11 (s, 1H), 7.88 - 7.81 (m, 2H), 7.45 (s, 1H), 7.19 (d, *J* = 1.8 Hz, 1H), 7.12 (dd, *J* = 8.3, 1.8 Hz, 1H), 5.97 (d, *J* = 7.5 Hz, 1H), 4.32 - 4.18 (m, 1H), 3.93 (s, 5H), 3.86 (s, 3H), 3.49 (td, *J* = 11.6, 2.2 Hz, 2H), 1.99 (d, *J* = 11.1 Hz, 2H), 1.69 - 1.57 (m, 2H) ; MS: m/z 445.3 $[M+H]^+$ | 1.41 |

(continued)

| 실시 예 화합 물 | 구조 | 화합물명 | $^1$H NMR, MS | UPLC r.t. (min) |
|---|---|---|---|---|
| 93 | 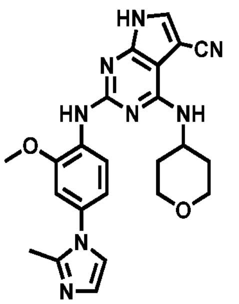 | 2-((2-methoxy-4-(2-methyl-1*H*-imidazol-1-yl)phenyl)amino)-4-((tetrahydro-2*H*-pyran-4-yl) amino)-7*H*-pyrrolo[2,3-*d*] pyrimidine-5-carbonitrile | 1H NMR (400 MHz, DMSO-*d*6) δ 12.28 (s, 1H), 8.53 (d, *J* = 8.6 Hz, 1H), 7.89 (s, 1H), 7.58 (s, 1H), 7.26 (d, *J* = 1.4 Hz, 1H), 7.08 (d, *J* = 2.3 Hz, 1H), 6.99 (dd, *J* = 8.6, 2.3 Hz, 1H), 6.89 (d, *J* = 1.3 Hz, 1H), 6.02 (d, *J* = 7.5 Hz, 1H), 4.31 - 4.20 (m, 1H), 3.96 - 3.85 (m, 5H), 3.54 - 3.44 (m, 2H), 2.30 (s, 3H), 2.03 - 1.92 (m, 2H), 1.64 (qd, *J* = 11.5, 4.3 Hz, 2H)<br>MS: m/z 445.3 $[M+H]^+$ | 1.15 |
| 94 | 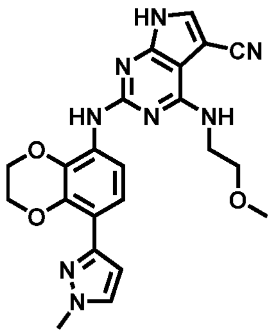 | 4-((2-methoxyethyl)amino) -2-((8-(1-methyl-1*H*-pyrazol-4-yl)-2,3-dihydrobenzo[b][1,4] dioxin-5-yl)amino)-7*H*-pyrrolo [2,3-*d*]pyrimidine-5-carbonitrile | 1H NMR (400 MHz, DMSO-$d_6$) δ 12.26 (s, 1H), 8.01 (s, 1H), 7.95 (d, *J* = 8.6 Hz, 1H), 7.86 (s, 1H), 7.82 (s, 1H), 7.36 (s, 1H), 7.09 (d, *J* = 8.6 Hz, 1H), 6.22 (t, *J* = 5.5 Hz, 1H), 4.38 (s, 4H), 3.86 (s, 3H), 3.69 (q, *J* = 5.6 Hz, 2H), 3.57 (t, *J* = 5.7 Hz, 2H), 3.31 (s, 3H).<br>MS: m/z 447.3 [M+H]+ | 1.38 |
| 95 | 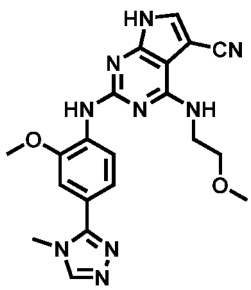 | 2-((2-methoxy-4-(4-methyl-4*H*-1,2,4-triazol-3-yl)phenyl)amino)-4-((2-methoxyethyl)amino) -7*H*-pyrrolo[2,3-*d*]pyrimidine-5-carbonitrile | 1H NMR (400 MHz, DMSO-$d_6$) δ 12.35 (d, *J* = 2.9 Hz, 1H), 8.67 (d, *J* = 8.4 Hz, 1H), 8.64 (d, *J* = 3.1 Hz, 1H), 7.92 (d, *J* = 2.6 Hz, 1H), 7.66 (s, 1H), 7.37 (d, *J* = 1.9 Hz, 1H), 7.31 (dd, *J* = 8.4, 1.9 Hz, 1H), 6.34 (t, *J* = 5.5 Hz, 1H), 3.97 (s, 3H), 3.79 (s, 3H), 3.72 (q, *J* = 5.7 Hz, 2H), 3.59 (t, *J* = 5.8 Hz, 2H), 3.32 (s, 3H).<br>MS: m/z 420.3 $[M+H]^+$ | 1.21 |
| 96 | 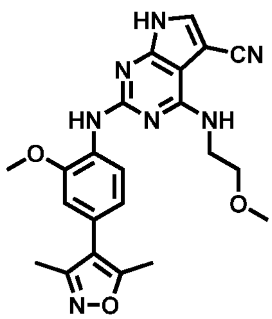 | 2-((4-(3,5-dimethylisoxazol-4-yl)-2-methoxyphenyl)amin o)-4-((2-methoxyethyl)amino) -7*H*-pyrrolo[2,3-*d*]pyrimidine-5-carbonitrile | 1H NMR (400 MHz, DMSO-$d_6$) δ 12.28 (s, 1H), 8.54 (d, *J* = 8.3 Hz, 1H), 7.88 (s, 1H), 7.53 (s, 1H), 6.99 (d, *J* = 1.9 Hz, 1H), 6.92 (dd, *J* = 8.3, 1.8 Hz, 1H), 6.26 (t, *J* = 5.5 Hz, 1H), 3.93 (s, 3H), 3.71 (q, *J* = 5.7 Hz, 2H), 3.58 (t, *J* = 5.7 Hz, 2H), 3.32 (s, 3H), 2.42 (s, 3H), 2.25 (s, 3H). ;<br>MS: m/z 434.3 $[M+H]^+$ | 1.62 |

(continued)

| 실시 예 화합 물 | 구조 | 화합물명 | 1H NMR, MS | UPLC r.t. (min) |
|---|---|---|---|---|
| 97 | 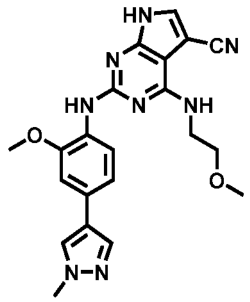 | 2-((2-methoxy-4-(1-methyl-1*H*-pyrazol-4-yl)phenyl)amino)-4-((2-methoxyethyl)amino) -7*H*-pyrrolo[2,3-*d*]pyrimidine-5-carbonitrile | 1H NMR (400 MHz, DMSO-$d_6$) δ 12.24 (s, 1H), 8.37 (d, *J* = 8.3 Hz, 1H), 8.10 (s, 1H), 7.85 (d, *J* = 6.7 Hz, 2H), 7.45 (s, 1H), 7.19 (d, *J* = 1.9 Hz, 1H), 7.11 (dd, *J* = 8.3, 1.8 Hz, 1H), 6.22 (t, *J* = 5.5 Hz, 1H), 3.93 (s, 3H), 3.86 (s, 3H), 3.70 (q, *J* = 5.6 Hz, 2H), 3.58 (t, *J* = 5.7 Hz, 2H), 3.32 (s, 3H). MS: m/z 419.3 $[M+H]^+$ | 1.40 |
| 98 | 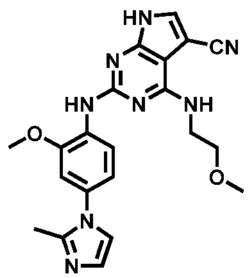 | 2-((2-methoxy-4-(2-methyl-1*H*-imidazol-1-yl)phenyl)amino)-4-((2-methoxyethyl)amino) -7*H*-pyrrolo[2,3-*d*]pyrimidine-5-carbonitrile | 1H NMR (400 MHz, DMSO-$d_6$) δ 12.31 (s, 1H), 8.56 (d, *J* = 8.6 Hz, 1H), 7.89 (s, 1H), 7.56 (s, 1H), 7.26 (s, 1H), 7.08 (d, *J* = 2.3 Hz, 1H), 6.98 (dd, *J* = 8.5, 2.2 Hz, 1H), 6.89 (s, 1H), 6.31 - 6.23 (m, 1H), 3.93 (s, 3H), 3.71 (q, *J* = 5.7 Hz, 2H), 3.58 (t, *J* = 5.7 Hz, 2H), 3.32 (s, 3H), 2.30 (s, 3H). ;<br>MS: m/z 419.2 $[M+H]^+$ | 1.11 |
| 99 | 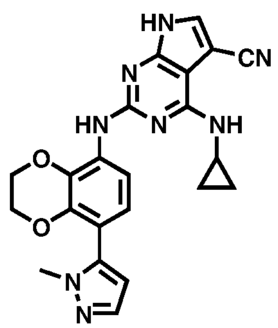 | 4-(cyclopropylamino)-2-((8-(1-methyl-1*H*-pyrazol-5-yl)-2,3-dihydrobenzo[b][1,4] dioxin-5-yl) amino)-7*H*-pyrrolo[2,3-*d*] pyrimidine-5-carbonitrile | 1H NMR (400 MHz, DMSO-$d_6$) δ 12.31 (s, 1H), 8.31 (d, *J* = 8.5 Hz, 1H), 7.90 (s, 1H), 7.42 (d, *J* = 1.9 Hz, 2H), 6.81 (d, *J* = 8.6 Hz, 1H), 6.59 (s, 1H), 6.22 (d, *J* = 1.8 Hz, 1H), 4.37 (ddd, *J* = 37.5, 6.1, 3.1 Hz, 4H), 3.68 (s, 3H), 2.93 (tq, *J* = 7.0, 3.6 Hz, 1H), 0.84 (td, *J* = 7.0, 4.8 Hz, 2H), 0.70 - 0.60 (m, 2H).<br>MS: m/z 429.3 $[M+H]^+$ | 1.49 |
| 100 | 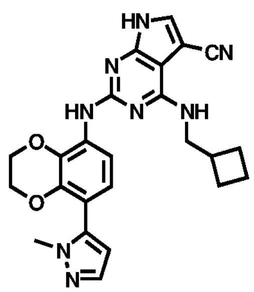 | 4-((cyclobutylmethyl)a mino)-2-((8-(1-methyl-1*H*-pyrazol-5-yl)-2,3-dihydrobenzo[b][1,4] dioxin-5-yl)amino)-7*H*-pyrrolo [2,3-*d*]pyrimidine-5-carbonitrile | 1H NMR (400 MHz, DMSO-$d_6$) δ 12.37 - 12.26 (m, 1H), 8.14 (d, *J* = 8.5 Hz, 1H), 7.88 (d, *J* = 2.4 Hz, 1H), 7.51 - 7.36 (m, 2H), 6.80 (d, *J* = 8.5 Hz, 1H), 6.30 (t, *J* = 5.6 Hz, 1H), 6.23 (d, *J* = 1.8 Hz, 1H), 4.36 (ddd, *J* = 36.0, 6.0, 3.1 Hz, 4H), 3.58 (d, *J* = 6.5 Hz, 2H), 2.68 (hept, *J* = 7.1 Hz, 1H), 2.10 - 1.96 (m, 2H), 1.94 - 1.70 (m, 4H).<br>MS: m/z 457.3 $[M+H]^+$ | 1.76 |

(continued)

| 실시예 화합물 | 구조 | 화합물명 | $^1H$ NMR, MS | UPLC r.t. (min) |
|---|---|---|---|---|
| 101 | | (*R*)-4-((1-methoxypropan-2-yl) amino)-2-((8-(1-methyl-1*H*-pyrazol-5-yl)-2,3-dihydrobenzo[b][1,4] dioxin-5-yl) amino)-7*H*-pyrrolo[2,3-*d*] pyrimidine-5-carbonitrile | 1H NMR (400 MHz, DMSO-$d_6$) δ 12.37 (s, 1H), 8.11 (d, *J* = 8.6 Hz, 1H), 7.94 - 7.85 (m, 1H), 7.57 (s, 1H), 7.43 (d, *J* = 1.9 Hz, 1H), 6.81 (d, *J* = 8.5 Hz, 1H), 6.24 (d, *J* = 1.8 Hz, 1H), 5.97 (d, *J* = 7.9 Hz, 1H), 4.53 - 4.27 (m, 5H), 3.69 (s, 3H), 3.57 - 3.43 (m, 2H), 1.28 (d, *J* = 6.7 Hz, 3H). MS: m/z 461.2 $[M+H]^+$ | 1.55 |
| 102 | 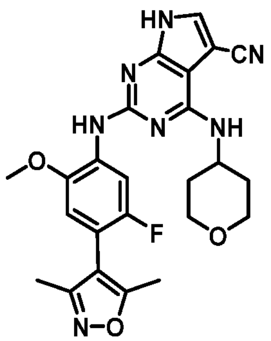 | 2-((4-(3,5-dimethylisoxazol-4-yl)-5-fluoro-2-methoxyphenyl) amin o)-4-((tetrahydro-2*H*-pyran-4-yl)amino)-7*H*-pyrrolo[2,3-*d*] pyrimidine-5-carbonitrile | 1H NMR (400 MHz, DMSO-*d*6) δ 12.34 (s, 1H), 8.55 (d, *J* = 12.8 Hz, 1H), 7.93 (d, *J* = 1.9 Hz, 1H), 7.68 - 7.58 (m, 1H), 6.99 (d, *J* = 6.9 Hz, 1H), 6.13 (d, *J* = 7.5 Hz, 1H), 4.33 - 4.21 (m, 1H), 3.92 (s, 5H), 3.54 - 3.43 (m, 2H), 2.38 - 2.32 (m, 3H), 2.18 (s, 3H), 1.99 (d, *J* = 12.9 Hz, 2H), 1.66 (qd, *J* = 11.9, 4.3 Hz, 2H) ; MS: m/z 478.4 $[M+H]^+$ | 1.70 |
| 103 | 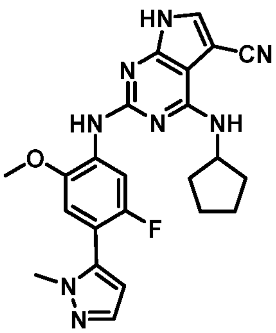 | 4-(cyclopentylamino)-2-((5-fluoro-2-methoxy-4-(1-methyl-1*H*-pyrazol-5-yl)phenyl)amino)-7*H*-pyrrolo[2,3-*d*]pyrimidine-5-carbonitrile | 1H NMR (400 MHz, DMSO-$d_6$) δ 12.36 (s, 1H), 8.66 (d, J = 13.0 Hz, 1H), 7.92 (d, J = 2.4 Hz, 1H), 7.64 (d, J = 1.6 Hz, 1H), 7.50 (d, J = 1.9 Hz, 1H), 7.04 (d, J = 6.9 Hz, 1H), 6.38 (d, J = 1.9 Hz, 1H), 6.05 (d, J = 7.0 Hz, 1H), 4.47 (q, J = 6.8 Hz, 1H), 3.94 (s, 3H), 3.76 (d, J = 1.3 Hz, 3H), 2.08 (td, J = 10.7, 8.9, 5.6 Hz, 2H), 1.74 (s, 2H), 1.61 (tq, J = 13.7, 8.3, 6.8 Hz, 4H) MS: m/z 447.4 $[M+H]^+$ | 1.87 |

(continued)

| 실시예 화합물 | 구조 | 화합물명 | $^1$H NMR, MS | UPLC r.t. (min) |
|---|---|---|---|---|
| 104 | 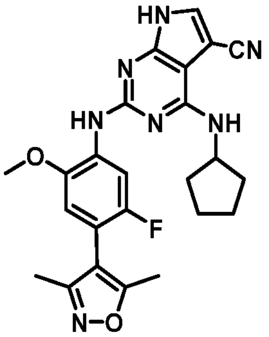 | 4-(cyclopentylamino)-2-((4-(3,5-dimethylisoxazol-4-yl)-5-fluoro-2-methoxyphenyl)amin o)-7*H*-pyrrolo[2,3-*d*]pyrimidine-5-carbonitrile | 1H NMR (400 MHz, DMSO-*d6*) δ 12.39 - 12.24 (m, 1H), 8.61 (d, *J* = 12.9 Hz, 1H), 7.91 (d, *J* = 2.4 Hz, 1H), 7.60 (d, *J* = 1.5 Hz, 1H), 6.99 (d, *J* = 6.9 Hz, 1H), 6.03 (d, *J* = 7.1 Hz, 1H), 4.47 (q, *J* = 6.8 Hz, 1H), 3.92 (s, 3H), 2.35 (s, 3H), 2.18 (s, 3H), 2.07 (dt, *J* = 11.0, 6.6 Hz, 2H), 1.81 - 1.68 (m, 2H), 1.60 (td, *J* = 13.6, 11.7, 6.8 Hz, 4H) ; MS: m/z 462.4 $[M+H]^+$ | 2.00 |
| 105 | 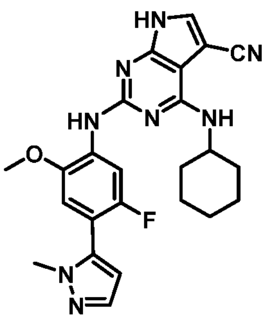 | 4-(cyclohexylamino)-2-((5-fluoro-2-methoxy-4-(1-methyl-1*H*-pyrazol-5-yl)phenyl)amino)-7*H*-pyrrolo[2,3-*d*]pyrimidine-5-carbonitrile | 1H NMR (400 MHz, DMSO-$d_6$) δ 12.34 (s, 1H), 8.61 (d, J = 13.0 Hz, 1H), 7.93 (d, J = 1.9 Hz, 1H), 7.64 (d, J = 1.6 Hz, 1H), 7.50 (d, J = 1.9 Hz, 1H), 7.04 (d, J = 6.9 Hz, 1H), 6.38 (d, J = 1.9 Hz, 1H), 5.90 (d, J = 7.7 Hz, 1H), 4.13 - 4.02 (m, 1H), 3.94 (s, 3H), 3.76 (d, J = 1.2 Hz, 3H), 2.03 (s, 2H), 1.74 (s, 2H), 1.62 (d, J = 12.9 Hz, 1H), 1.44 (q, J = 10.2, 9.6 Hz, 4H), 1.23 (s, 1H) ; MS: m/z 461.4 $[M+H]^+$ | 1.94 |
| 106 | 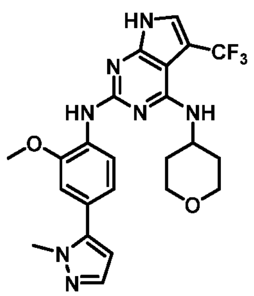 | $N^2$-(2-methoxy-4-(1-methyl-1*H*-pyrazol-5-yl) phenyl)-$N^4$-(tetrahydro-2*H*-pyran-4-yl)-5-(trifluoromethyl)-7*H*-pyrrolo[2,3-*d*]pyrimidine-2,4-diamine | 1H NMR (400 MHz, DMSO-$d_6$) δ 12.06 (d, *J* = 2.7 Hz, 1H), 8.59 (d, *J* = 8.3 Hz, 1H), 7.69 - 7.48 (m, 2H), 7.45 (d, *J* = 1.9 Hz, 1H), 7.20 - 6.98 (m, 2H), 6.39 (d, *J* = 1.9 Hz, 1H), 5.27 - 5.15 (m, 1H), 4.39 - 4.24 (m, 1H), 3.95 (s, 3H), 3.88 (s, 4H), 3.52 (td, *J* = 11.4, 2.2 Hz, 2H), 2.02 (d, *J* = 12.7 Hz, 2H), 1.64 - 1.46 (m, 2H), 1.29 - 1.12 (m, 1H). MS: m/z 488.3 $[M+H]^+$ | 1.69 |

(continued)

| 실시예 화합물 | 구조 | 화합물명 | $^1$H NMR, MS | UPLC r.t. (min) |
|---|---|---|---|---|
| 107 | 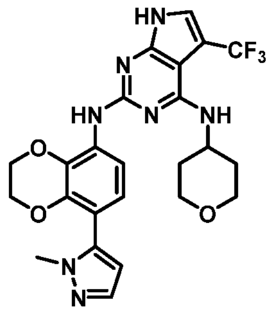 | $N^2$-(8-(1-methyl-1*H*-pyrazol-5-yl)-2,3-dihydrobenzo[b][1,4] dioxin-5-yl)-$N^4$-(tetrahydro-2*H*-pyran-4-yl)-5-(trifluoromethyl)-7*H*-pyrrolo[2,3-*d*]pyrimidine-2,4-diamine | 1H NMR (400 MHz, DMSO-$d_6$) δ 12.07 (d, *J* = 2.8 Hz, 1H), 8.14 (d, *J* = 8.5 Hz, 1H), 7.62 (d, *J* = 2.3 Hz, 1H), 7.52 - 7.39 (m, 2H), 6.82 (d, *J* = 8.5 Hz, 1H), 6.23 (d, *J* = 1.8 Hz, 1H), 5.20 (d, *J* = 7.4 Hz, 1H), 4.36 (ddd, *J* = 36.0, 6.1, 3.0 Hz, 5H), 3.88 (dt, *J* = 11.9, 3.7 Hz, 2H), 3.68 (s, 2H), 3.50 (td, *J* = 11.4, 2.2 Hz, 2H), 2.07 - 1.88 (m, 2H), 1.55 (qd, *J* = 10.9, 4.3 Hz, 2H), 1.30 - 1.11 (m, 1H). MS: m/z 516.3 $[M+H]^+$ | 1.69 |
| 108 | 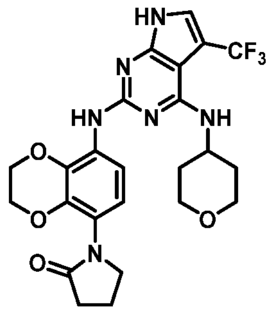 | 1-(8-((4-((tetrahydro-2*H*-pyran-4-yl)amino)-5-(trifluoromethyl)-7*H*-pyrrolo[2,3-*d*]pyrimidin-2-yl) amino)-2,3-dihydrobenzo[b][1,4] dioxin-5-yl)pyrrolidin-2-one | 1H NMR (400 MHz, DMSO-$d_6$) δ 12.03 (d, *J* = 2.7 Hz, 1H), 7.88 (d, *J* = 8.8 Hz, 1H), 7.58 (t, *J* = 2.2 Hz, 1H), 7.43 (s, 1H), 6.75 (d, *J* = 8.7 Hz, 1H), 5.16 (d, *J* = 7.3 Hz, 1H), 4.38 - 4.21 (m, 5H), 3.87 (dt, *J* = 11.8, 3.7 Hz, 2H), 3.64 (t, *J* = 7.0 Hz, 2H), 3.49 (td, *J* = 11.4, 2.3 Hz, 2H), 2.37 (t, *J* = 8.0 Hz, 2H), 2.15 - 1.96 (m, 4H), 1.62 - 1.45 (m, 2H). MS: m/z 519.3 $[M+H]^+$ | 1.53 |
| 109 | 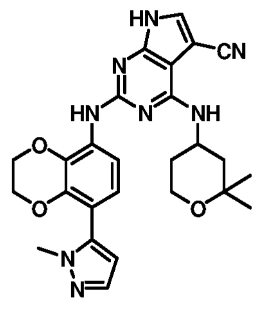 | 4-((2,2-dimethyltetrahydro-2*H*-pyran-4-yl)amino)-2-((8-(1-methyl-1*H*-pyrazol-5-yl)-2,3-dihydrobenzo[b][1,4] dioxin-5-yl) amino)-7*H*-pyrrolo[2,3-*d*] pyrimidine-5-carbonitrile | 1H NMR (400 MHz, DMSO-*d6*) δ 12.29 (s, 1H), 8.09 (d, *J* = 8.5 Hz, 1H), 7.89 (d, *J* = 2.2 Hz, 1H), 7.51 (s, 1H), 7.43 (d, *J* = 1.9 Hz, 1H), 6.77 (d, *J* = 8.5 Hz, 1H), 6.22 (d, *J* = 1.9 Hz, 1H), 5.93 (d, *J* = 7.7 Hz, 1H), 4.48 - 4.27 (m, 5H), 3.78 - 3.61 (m, 5H), 2.01 - 1.91 (m, 2H), 1.58 - 1.35 (m, 2H), 1.26 (s, 3H), 1.18 (s, 3H). MS: m/z 501.3 $[M+H]^+$ | 1.54 |

(continued)

| 실시예 화합물 | 구조 | 화합물명 | $^1$H NMR, MS | UPLC r.t. (min) |
|---|---|---|---|---|
| 110 | 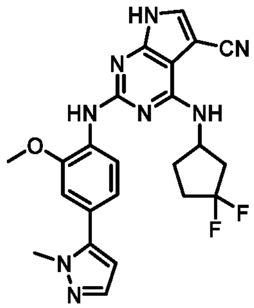 | 4-((3,3-difluorocyclopentyl)amino)-2-((2-methoxy-4-(1-methyl-1*H*-pyrazol-5- yl)phenyl) amino)-7*H*-pyrrolo[2,3-*d*] pyrimidine-5-carbonitrile | 1H NMR (400 MHz, DMSO-$d_6$) δ 12.32 (s, 1H), 8.55 (d, *J* = 8.3 Hz, 1H), 7.90 (s, 1H), 7.62 (s, 1H), 7.45 (d, *J* = 1.9 Hz, 1H), 7.13 (d, *J* = 1.9 Hz, 1H), 7.07 (dd, *J* = 8.3, 1.9 Hz, 1H), 6.47 (d, *J* = 7.4 Hz, 1H), 6.39 (d, *J* = 1.9 Hz, 1H), 4.70 (h, *J* = 7.8 Hz, 1H), 3.95 (s, 3H), 3.88 (s, 3H), 2.64 (p, *J* = 13.3 Hz, 1H), 2.38 - 2.08 (m, 4H), 1.91 (dq, *J* = 12.2, 9.5, 8.4 Hz, 1H). MS: m/z 465.3 $[M+H]^+$ | 1.64 |
| 111 | 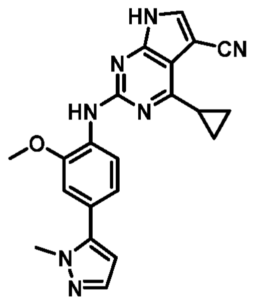 | 4-cyclopropyl-2-((2-methoxy-4-(1-methyl-1*H*-pyrazol-5- yl) phenyl)amino)-7*H*-pyrrolo[2,3-*d*] pyrimidine-5-carbonitrile | 1H NMR (400 MHz, DMSO-$d_6$) δ 12.63 (d, *J* = 2.9 Hz, 1H), 8.45 (d, *J* = 8.3 Hz, 1H), 8.19 (d, *J* = 2.8 Hz, 1H), 7.86 (s, 1H), 7.45 (d, *J* = 1.9 Hz, 1H), 7.15 (d, *J* = 1.9 Hz, 1H), 7.11 (dd, *J* = 8.3, 1.9 Hz, 1H), 6.40 (d, *J* = 1.9 Hz, 1H), 3.95 (s, 3H), 3.89 (s, 3H), 2.65 - 2.56 (m, 1H), 1.24 (dtd, *J* = 15.6, 7.6, 6.2, 3.7 Hz, 4H). MS: m/z 386.2 $[M+H]^+$ | 1.88 |
| 112 | 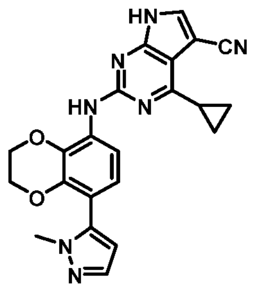 | 4-cyclopropyl-2-((8-(1-methyl-1*H*-pyrazol-5-yl)-2,3-dihydrobenzo [b] [1,4] dioxin-5-yl) amino)-7*H*-pyrrolo[2,3-*d*] pyrimidine-5-carbonitrile | 1H NMR (400 MHz, DMSO-$d_6$) δ 12.63 (s, 1H), 8.18 (d, *J* = 1.8 Hz, 1H), 7.97 (d, *J* = 8.5 Hz, 1H), 7.79 (s, 1H), 7.43 (d, *J* = 1.8 Hz, 1H), 6.83 (d, *J* = 8.5 Hz, 1H), 6.24 (d, *J* = 1.8 Hz, 1H), 4.38 (dt, *J* = 5.8, 3.4 Hz, 2H), 4.31 (q, *J* = 3.1, 2.4 Hz, 2H), 3.68 (s, 3H), 2.58 (td, *J* = 7.9, 7.0, 3.1 Hz, 1H), 1.28 - 1.18 (m, 4H). MS: m/z 414.3 $[M+H]^+$ | 1.65 |

(continued)

| 실시예 화합물 | 구조 | 화합물명 | $^1$H NMR, MS | UPLC r.t. (min) |
|---|---|---|---|---|
| 113 | 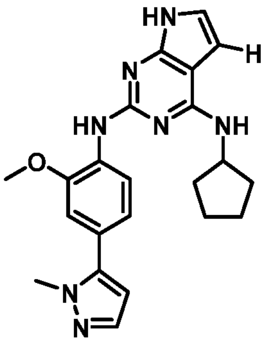 | $N^4$-cyclopentyl-$N^2$-(2-methoxy-4-(1-methyl-1*H*-pyrazol-5-yl) phenyl)-7*H*-pyrrolo[2,3-*d*] pyrimidine-2,4-diamine | 1H NMR (400 MHz, DMSO-$d_6$) δ 12.08 (s, 1H), 9.22 (s, 1H), 9.00 (s, 1H), 8.46 (s, 1 H), 7.48 (d, *J* = 1.9 Hz, 1H), 7.24 (d, *J* = 1.9 Hz, 1H), 7.16 (dd, *J* = 8.4, 1.9 Hz, 1H), 7.06 (s, 1H), 6.77 (d, *J* = 3.0 Hz, 1H), 6.44 (d, *J* = 1.9 Hz, 1H), 3.98 (d, *J* = 8.8 Hz, 4H), 3.90 (s, 3H), 2.13 -2.03 (m, 2H), 1.78 (s, 2H), 1.66 (ddd, *J* = 15.7, 11.9, 7.8 Hz, 4H). MS: m/z 404.3 $[M+H]^+$ | 1.35 |
| 114 | 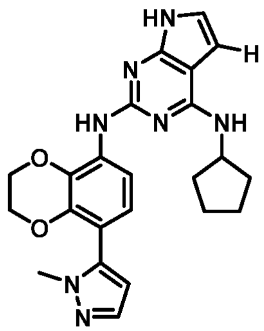 | $N^4$-cyclopentyl-$N^2$-(8-(1-methyl-1*H*-pyrazol-5-yl)-2,3-dihydrobenzo [b] [1,4] dioxin-5-yl)-7*H*-pyrrolo[2,3-*d*]pyrimidine-2,4-diamine | 1H NMR (400 MHz, DMSO-$d_6$) δ 12.07 (s, 1H), 9.20 (s, 1H), 8.98 (s, 1H), 7.99 (s, 1H), 7.45 (d, *J* = 1.8 Hz, 1H), 7.05 (s, 1H), 6.89 (d, *J* = 8.5 Hz, 1H), 6.77 (s, 1H), 6.26 (d, *J* = 1.8 Hz, 1H), 4.44 (dd, *J* = 5.6, 2.5 Hz, 2H), 4.36 (dd, *J* = 5.6, 2.6 Hz, 3H), 3.70 (s, 3H), 2.08 (q, *J* = 6.4, 6.0 Hz, 2H), 1.83 - 1.73 (m, 2H), 1.66 (qd, *J* = 12.5, 9.9, 5.0 Hz, 4H). MS: m/z 432.3 $[M+H]^+$ | 1.33 |
| 115 | 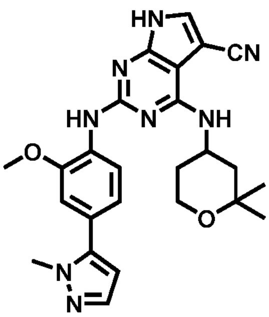 | 4-((2,2-dimethyltetrahydro-2*H*-pyran-4-yl)amino)-2-( (2-methoxy-4-(1-methyl-1H-pyrazol-5-yl)phenyl)amino)-7*H*-pyrrolo[2,3-*d*]pyrimidine-5-carbonitrile | 1H NMR (400 MHz, DMSO-$d_6$) δ 12.32 (s, 1H), 8.51 (dt, *J* = 8.4, 1.7 Hz, 1H), 7.90 (d, *J* = 1.8 Hz, 1H), 7.72 (d, *J* = 14.5 Hz, 1H), 7.46 (d, *J* = 1.9 Hz, 1H), 7.14 (d, *J* = 1.9 Hz, 1H), 7.05 (dd, *J* = 8.3, 1.9 Hz, 1H), 6.38 (d, *J* = 1.9 Hz, 1H), 6.05 (s, 1H), 4.45 (dt, *J* = 8.1, 4.3 Hz, 1H), 3.95 (s, 3H), 3.87 (s, 3H), 3.74 - 3.68 (m, 2H), 2.02 - 1.83 (m, 2H), 1.47 (q, *J* = 11.8 Hz, 2H), 1.27 (s, 3H), 1.18 (d, *J* = 1.9 Hz, 3H). MS: m/z 473.4 $[M+H]^+$ | 1.58 |

(continued)

| 실시예 화합물 | 구조 | 화합물명 | $^1$H NMR, MS | UPLC r.t. (min) |
|---|---|---|---|---|
| 116 | 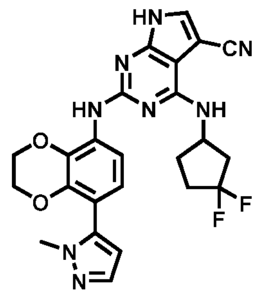 | 4-((3,3-difluorocyclopentyl)amino)-2-((8-(1-methyl-1*H*-pyrazol-5-yl)-2,3-dihydrobenzo [b] [1,4] dioxin-5-yl) amino)-7*H*-pyrrolo[2,3-*d*] pyrimidine-5-carbonitrile | 1H NMR (400 MHz, DMSO-$d_6$) δ 12.37 (s, 1H), 8.08 (d, *J* = 8.5 Hz, 1H), 7.91 (d, *J* = 2.1 Hz, 1H), 7.62 (s, 1H), 7.43 (d, *J* = 1.8 Hz, 1H), 6.80 (d, *J* = 8.6 Hz, 1H), 6.55 (d, *J* = 8.0 Hz, 1H), 6.23 (d, *J* = 1.9 Hz, 1H), 4.68 (h, *J* = 7.9 Hz, 1H), 4.36 (ddd, *J* = 34.6, 6.0, 3.0 Hz, 4H), 3.68 (s, 3H), 2.74 - 2.56 (m, 1H), 2.40 - 2.05 (m, 4H), 1.91 (td, *J* = 8.8, 3.5 Hz, 1H). MS: m/z 493.2 $[M+H]^+$ | 1.64 |
| 117 | 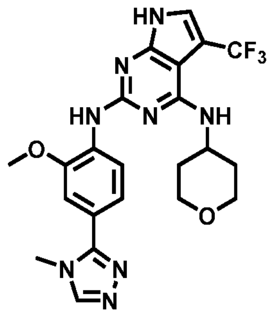 | $N^2$-(2-methoxy-4-(4-methyl-4*H*-1,2,4-triazol-3-yl) phenyl)-$N^4$-(tetrahydro-2*H*-pyran-4-yl)-5-(trifluoromethyl)-7*H*-pyrrolo[2,3-*d*]pyrimidine-2,4-diamine | 1H NMR (400 MHz, DMSO-*d*6) δ 12.10 (d, *J* = 2.7 Hz, 1H), 8.68 (d, *J* = 8.3 Hz, 1H), 8.58 (s, 1H), 7.68 - 7.61 (m, 2H), 7.39 - 7.28 (m, 2H), 5.24 (dd, *J* = 7.5, 2.4 Hz, 1H), 4.35 (ddt, *J* = 14.0, 10.6, 5.3 Hz, 1H), 3.97 (s, 3H), 3.89 (dt, *J* = 11.6, 3.7 Hz, 2H), 3.79 (s, 3H), 3.53 (td, *J* = 11.4, 2.3 Hz, 2H), 2.03 (dd, *J* = 13.1, 3.8 Hz, 2H), 1.57 (dtd, *J* = 12.5, 10.7, 4.3 Hz, 2H). MS: m/z 489.2 $[M+H]^+$ | 1.42 |
| 118 | 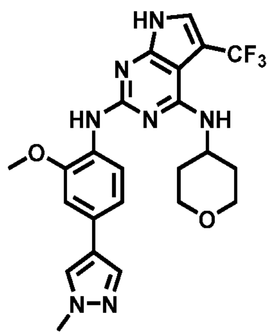 | $N^2$-(2-methoxy-4-(1-methyl-1*H*-pyrazol-4-yl) phenyl)-$N^4$-(tetrahydro-2*H*-pyran-4-yl)-5-(trifluoromethyl)-7*H*-pyrrolo[2,3-*d*]pyrimidine- 2,4-diamine | 1H NMR (400 MHz, DMSO-$d_6$) δ 11.99 (s, 1H), 8.39 (dd, *J* = 8.3, 2.3 Hz, 1H), 8.11 (d, *J* = 2.0 Hz, 1H), 7.86 (d, *J* = 2.1 Hz, 1H), 7.59 (d, *J* = 2.7 Hz, 1H), 7.46 (d, *J* = 2.2 Hz, 1H), 7.24 - 7.08 (m, 2H), 5.17 (d, *J* = 7.3 Hz, 1H), 4.39 - 4.21 (m, 1H), 4.02 - 3.78 (m, 8H), 3.52 (t, *J* = 11.5 Hz, 2H), 2.02 (d, *J* = 12.6 Hz, 2H), 1.62 - 1.46 (m, 2H). MS: m/z 488.3 $[M+H]^+$ | 1.60 |

(continued)

| 실시예 화합물 | 구조 | 화합물명 | $^1$H NMR, MS | UPLC r.t. (min) |
|---|---|---|---|---|
| 119 | 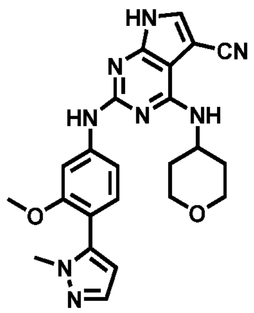 | 2-((3-methoxy-4-(1-methyl-1*H*-pyrazol-5 - yl)phenyl)amino)-4-((tetrahydro-2*H*-pyran-4-yl) amino)-7*H*-pyrrolo[2,3-*d*] pyrimidine-5-carbonitrile | 1H NMR (400 MHz, DMSO-*d*6) δ 12.24 (d, *J* = 2.8 Hz, 1H), 9.21 (s, 1H), 7.89 (d, *J* = 2.8 Hz, 1H), 7.69 (d, *J* = 2.0 Hz, 1H), 7.59 (dd, *J* = 8.3, 1.9 Hz, 1H), 7.40 (d, *J* = 1.8 Hz, 1H), 7.10 (d, *J* = 8.3 Hz, 1H), 6.18 (d, *J* = 1.8 Hz, 1H), 5.93 (d, *J* = 7.6 Hz, 1H), 4.39 - 4.24 (m, 1H), 3.92 (dt, *J* = 11.7, 3.6 Hz, 2H), 3.80 (s, 3H), 3.62 (s, 3H), 3.50 - 3.42 (m, 2H), 2.01 (d, *J* = 13.0 Hz, 2H), 1.65 (qd, *J* = 11.1, 4.4 Hz, 2H) ; MS: m/z 445.3 $[M+H]^+$ | 1.43 |
| 120 | 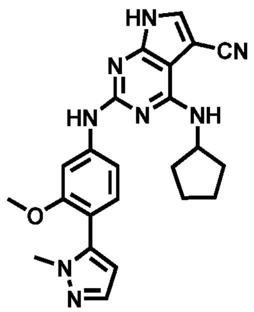 | 4-(cyclopentylamino)-2-((3-methoxy-4-(1-methyl-1*H*-pyrazol-5 - yl)phenyl)amino )-7*H*-pyrrolo[2,3-*d*]pyrimidine-5-carbonitrile | 1H NMR (400 MHz, DMSO-*d*6) δ 12.21 (d, *J* = 2.8 Hz, 1H), 9.22 (s, 1H), 7.88 (d, *J* = 2.8 Hz, 1H), 7.83 (d, *J* = 2.0 Hz, 1H), 7.50 (dd, *J* = 8.4, 1.9 Hz, 1H), 7.40 (d, *J* = 1.8 Hz, 1H), 7.09 (d, *J* = 8.3 Hz, 1H), 6.18 (d, *J* = 1.8 Hz, 1H), 5.87 (d, *J* = 7.3 Hz, 1H), 4.55 (q, *J* = 6.9 Hz, 1H), 3.80 (s, 3H), 3.62 (s, 3H), 2.13 - 2.04 (m, 2H), 1.78 - 1.71 (m, 2H), 1.63 - 1.54 (m, 4H)<br>MS: m/z 429.3 $[M+H]^+$ | 1.69 |
| 121 | 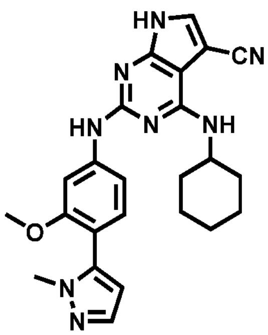 | 4-(cyclohexylamino)-2-((3-methoxy-4-(1-methyl-1*H*-pyrazol-5-yl)phenyl)amino)-7*H*-pyrrolo[2,3-*d*]pyrimidine-5-carbonitrile | 1H NMR (400 MHz, DMSO-*d*6) δ 12.22 (d, *J* = 2.8 Hz, 1H), 9.20 (s, 1H), 7.88 (d, *J* = 2.7 Hz, 1H), 7.71 (d, *J* = 2.0 Hz, 1H), 7.59 (dd, *J* = 8.3, 1.9 Hz, 1H), 7.40 (d, *J* = 1.8 Hz, 1H), 7.09 (d, *J* = 8.3 Hz, 1H), 6.18 (d, *J* = 1.8 Hz, 1H), 5.69 (d, *J* = 7.8 Hz, 1H), 4.16 (s, 1H), 3.80 (s, 3H), 3.62 (s, 3H), 2.05 - 1.99 (m, 2H), 1.73 (d, *J* = 10.5 Hz, 2H), 1.61 (d, *J* = 12.9 Hz, 1H), 1.41 (dt, *J* = 13.4, 8.0 Hz, 4H), 1.27 (d, *J* = 10.5 Hz, 1H)<br>MS: m/z 443.3 $[M+H]^+$ | 1.76 |

(continued)

| 실시예 화합물 | 구조 | 화합물명 | $^{1}$H NMR, MS | UPLC r.t. (min) |
|---|---|---|---|---|
| 122 | 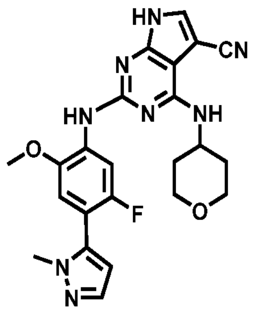 | 2-((5-fluoro-2-methoxy-4-(1-methyl-1*H*-pyrazol-5-yl)phenyl) amino)-4-((tetrahydro-2*H*-pyran-4-yl)amino)-7*H*-pyrrolo[2,3-*d*] pyrimidine-5-carbonitrile | 1H NMR (400 MHz, DMSO-*d*6) δ 12.37 (d, *J* = 2.8 Hz, 1H), 8.60 (d, *J* = 13.0 Hz, 1H), 7.94 (d, *J* = 2.7 Hz, 1H), 7.68 (s, 1H), 7.50 (d, *J* = 1.9 Hz, 1H), 7.04 (d, *J* = 6.9 Hz, 1H), 6.38 (d, *J* = 1.9 Hz, 1H), 6.16 (d, *J* = 7.5 Hz, 1H), 4.27 (ddt, *J* = 15.2, 10.8, 5.6 Hz, 1H), 3.93 (s, 5H), 3.77 (d, *J* = 1.2 Hz, 3H), 3.48 (td, *J* = 11.6, 2.1 Hz, 2H), 2.04 - 1.96 (m, 2H), 1.72 - 1.60 (m, 2H)<br>MS: m/z 463.3 $[M+H]^+$ | 1.55 |
| 123 | 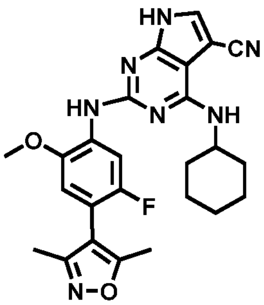 | 4-(cyclohexylamino)-2-((4-(3,5-dimethylisoxazol-4-yl)-5-fluoro-2-methoxyphenyl)amin o)-7*H*-pyrrolo[2,3-*d*]pyrimidine-5-carbonitrile | 1H NMR (400 MHz, DMSO-*d*6) δ 12.32 (s, 1H), 8.56 (d, *J* = 12.8 Hz, 1H), 7.92 (d, *J* = 2.1 Hz, 1H), 7.60 (d, *J* = 1.5 Hz, 1H), 6.99 (d, *J* = 6.9 Hz, 1H), 5.88 (d, *J* = 7.7 Hz, 1H), 4.06 (d, *J* = 14.3 Hz, 1H), 3.92 (s, 3H), 2.35 (s, 3H), 2.18 (s, 3H), 2.02 (s, 2H), 1.74 (s, 2H), 1.62 (d, *J* = 12.7 Hz, 1H), 1.43 (q, *J* = 10.4, 9.6 Hz, 4H), 1.23 (s, 1H)<br>MS: m/z 476.3 $[M+H]^+$ | 2.01 |
| 124 | 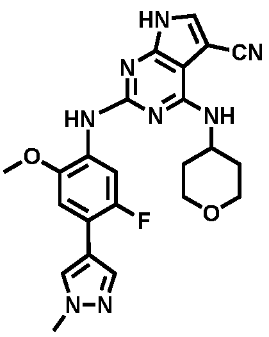 | 2-((5-fluoro-2-methoxy-4-(1-methyl-1*H*-pyrazol-4-yl)phenyl) amino)-4-((tetrahydro-2*H*-pyran-4-yl)amino)-7*H*-pyrrolo[2,3-*d*] pyrimidine-5-carbonitrile | 1H NMR (400 MHz, DMSO-*d*6) δ 12.30 (s, 1H), 8.45 (d, *J* = 13.8 Hz, 1H), 8.09 (d, *J* = 2.1 Hz, 1H), 7.99 - 7.85 (m, 2H), 7.53 (d, *J* = 1.5 Hz, 1H), 7.24 (d, *J* = 7.2 Hz, 1H), 6.10 (d, *J* = 7.5 Hz, 1H), 4.33 - 4.17 (m, 1H), 3.95 (s, 5H), 3.89 (s, 3H), 3.49 (td, *J* = 11.5, 2.1 Hz, 2H), 2.00 (d, *J* = 12.7 Hz, 2H), 1.66 (qd, *J* = 11.5, 4.3 Hz, 2H)<br>MS: m/z 463.3 $[M+H]^+$ | 1.51 |

(continued)

| 실시예 화합물 | 구조 | 화합물명 | $^1$H NMR, MS | UPLC r.t. (min) |
|---|---|---|---|---|
| 125 | 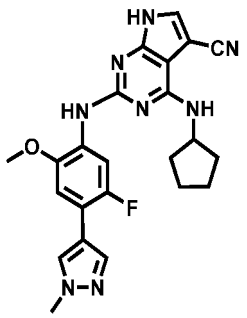 | 4-(cyclopentylamino)-2-((5-fluoro-2-methoxy-4-(1-methyl-1*H*-pyrazol-4-yl)phenyl)amino)-7*H*-pyrrolo[2,3-*d*]pyrimidine-5-carbonitrile | 1H NMR (400 MHz, DMSO-*d*6) δ 12.31 (s, 1H), 8.51 (d, *J* = 13.9 Hz, 1H), 8.09 (s, 1H), 7.89 (s, 2H), 7.51 (s, 1H), 7.24 (d, *J* = 7.3 Hz, 1H), 5.99 (d, *J* = 7.0 Hz, 1H), 4.56 - 4.39 (m, 1H), 3.95 (s, 3H), 3.88 (s, 3H), 2.08 (d, *J* = 10.2 Hz, 2H), 1.75 (s, 2H), 1.62 (s, 4H)<br>MS: m/z 447.3 $[M+H]^+$ | 1.79 |
| 126 | 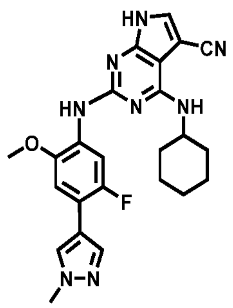 | 4-(cyclohexylamino)-2-((5-fluoro-2-methoxy-4-(1-methyl-1*H*-pyrazol-4-yl)phenyl)amino)-7*H*-pyrrolo[2,3-*d*]pyrimidine-5-carbonitrile | 1H NMR (400 MHz, DMSO-$d_6$) δ 12.28 (s, 1H), 8.46 (d, J = 13.9 Hz, 1H), 8.09 (s, 1H), 7.90 (d, J = 3.0 Hz, 2H), 7.50 (s, 1H), 7.24 (d, J = 7.0 Hz, 1H), 5.85 (d, J = 7.7 Hz, 1H), 4.07 (s, 1H), 3.95 (s, 3H), 3.88 (s, 3H), 2.06 (d, J = 18.2 Hz, 2H), 1.76 (s, 2H), 1.63 (d, J = 12.8 Hz, 1H), 1.43 (d, J = 8.3 Hz, 4H), 1.23 (s, 1H)<br>MS: m/z 461.3 $[M+H]^+$ | 1.86 |
| 127 | 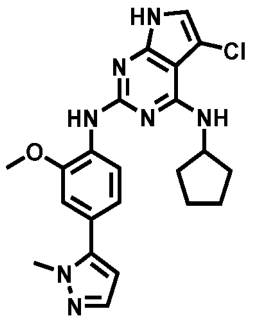 | 5-chloro-$N^4$-cyclopentyl-$N^2$-(2-methoxy-4-(1-methyl-1*H*-pyrazol-5-yl)phenyl)-7*H*-pyrrolo[2,3-*d*]pyrimidine-2,4-diamine | 1H NMR (400 MHz, DMSO-$d_6$) δ 11.89 (s, 1H), 8.52 (d, *J* = 8.3 Hz, 1H), 8.25 (s, 1H), 7.50 - 7.45 (m, 1H), 7.17 (d, *J* = 1.9 Hz, 1H), 7.16 - 7.09 (m, 2H), 6.56 (s, 1H), 6.46 - 6.39 (m, 1H), 4.45 (q, *J* = 6.9 Hz, 1H), 3.97 (s, 3H), 3.89 (s, 3H), 2.12 - 2.01 (m, 2H), 1.74 (tq, *J* = 8.2, 2.8 Hz, 2H), 1.69 - 1.56 (m, 4H).<br>MS: m/z 438.3 [M+H]+ | 1.83 |
| 128 | 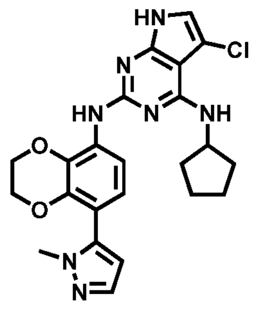 | 5-chloro-$N^4$-cyclopentyl-$N^2$-(8-(1-methyl-1*H*-pyrazol-5-yl)-2,3-dihydrobenzo[b][1,4] dioxin-5-yl)-7*H*-pyrrolo[2,3-*d*] pyrimidine-2,4-diamine | 1H NMR (400 MHz, DMSO-$d_6$) δ 11.89 (s, 1H), 8.18 (s, 1H), 8.06 (d, *J* = 8.5 Hz, 1H), 7.44 (d, *J* = 1.8 Hz, 1H), 7.13 (d, *J* = 2.4 Hz, 1H), 6.84 (d, *J* = 8.5 Hz, 1H), 6.57 (s, 1H), 6.24 (d, *J* = 1.9 Hz, 1H), 4.49 - 4.37 (m, 3H), 4.33 (dd, *J* = 5.4, 2.6 Hz, 2H), 3.69 (s, 3H), 2.12 - 1.99 (m, 2H), 1.74 (dtt, *J* = 8.7, 5.7, 2.6 Hz, 2H), 1.63 (qt, *J* = 10.0, 4.8 Hz, 4H).<br>MS: m/z 466.3 $[M+H]^+$ | 1.81 |

(continued)

| 실시 예 화합 물 | 구조 | 화합물명 | $^1$H NMR, MS | UPLC r.t. (min) |
|---|---|---|---|---|
| 129 | 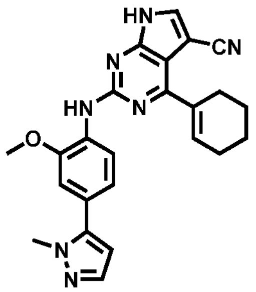 | 4-(cyclohex-1 -en-1 - yl)-2-((2-methoxy-4-(1-methyl-1*H*-pyrazol-5-yl)phenyl)amino)-7*H*-pyrrolo[2,3-*d*]pyrimidine-5-carbonitrile | 1H NMR (400 MHz, DMSO-$d_6$) δ 12.69 (s, 1H), 8.50 (d, *J* = 8.2 Hz, 1H), 8.22 (s, 1H), 8.00 (s, 1H), 7.46 (d, *J* = 1.9 Hz, 1H), 7.16 (d, *J* = 1.9 Hz, 1H), 7.12 (dd, *J* = 8.3, 1.8 Hz, 1H), 6.45 - 6.38 (m, 2H), 3.95 (s, 3H), 3.89 (s, 3H), 2.54 (d, *J* = 3.0 Hz, 2H), 2.28 (d, *J* = 4.5 Hz, 2H), 1.77 (dq, *J* = 8.1, 5.7, 4.6 Hz, 2H), 1.70 (h, *J* = 4.6 Hz, 2H).<br>MS: m/z 426.3 $[M+H]^+$ | 1.82 |
| 130 | 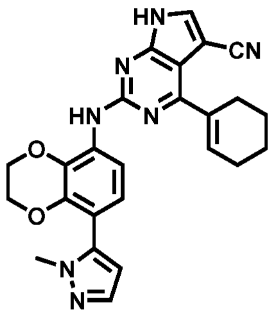 | 4-(cyclohex-1-en-1 - yl)-2-((8-(1-methyl-1*H*-pyrazol-5-yl)-2,3-dihydrobenzo [b] [1,4] dioxin-5-yl) amino)-7*H*-pyrrolo[2,3-*d*] pyrimidine-5-carbonitrile | 1H NMR (400 MHz, DMSO-$d_6$) δ 12.69 (d, *J* = 2.9 Hz, 1H), 8.21 (d, *J* = 2.8 Hz, 1H), 8.00 (d, *J* = 8.5 Hz, 1H), 7.93 (s, 1H), 7.43 (d, *J* = 1.9 Hz, 1H), 6.84 (d, *J* = 8.4 Hz, 1H), 6.42 (td, *J* = 3.9, 1.9 Hz, 1H), 6.24 (d, *J* = 1.8 Hz, 1H), 4.39 (dt, *J* = 4.4, 2.8 Hz, 2H), 4.32 (dt, *J* = 6.3, 2.9 Hz, 2H), 3.69 (s, 3H), 2.58 - 2.52 (m, 2H), 2.28 (h, *J* = 3.1 Hz, 2H), 1.81 - 1.73 (m, 2H), 1.73 - 1.65 (m, 2H).<br>MS: m/z 454.3 $[M+H]^+$ | 1.76 |
| 131 | 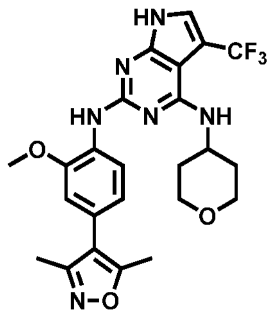 | *N*2-(4-(3,5-dimethylisoxazol-4-yl)-2-methoxyphenyl)-*N*4-(tetrahydro-2*H*-pyran-4-yl)-5-(trifluoromethyl)-7*H*-pyrrolo [2,3-*d*]pyrimidine-2,4-diamine | 1H NMR (400 MHz, DMSO-$d_6$) δ 12.05 (d, *J* = 2.8 Hz, 1H), 8.53 (d, *J* = 8.3 Hz, 1H), 7.59 (d, *J* = 15.7 Hz, 2H), 7.00 (d, *J* = 1.9 Hz, 1H), 6.94 (dt, *J* = 8.3, 1.4 Hz, 1H), 5.26 - 5.19 (m, 1H), 4.37 - 4.25 (m, 1H), 3.93 (s, 3H), 3.91 - 3.82 (m, 2H), 3.54 - 3.45 (m, 2H), 2.43 (s, 3H), 2.26 (s, 3H), 2.05 - 1.98 (m, 2H), 1.56 (qd, *J* = 10.9, 4.3 Hz, 2H). ;<br>MS: m/z 503.3 $[M+H]^+$ | 1.79 |

(continued)

| 실시예 화합물 | 구조 | 화합물명 | $^1$H NMR, MS | UPLC r.t. (min) |
|---|---|---|---|---|
| 132 | 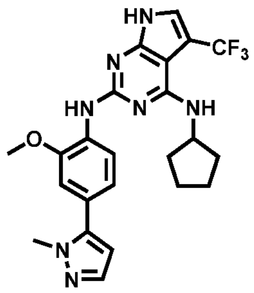 | $N^4$-cyclopentyl-$N^2$-(2-methoxy-4-(1-methyl-1*H*-pyrazol-5 - yl) phenyl)-5-(trifluoromethyl)-7*H*-pyrrolo[2,3-*d*]pyrimidine-2,4-diamine | 1H NMR (400 MHz, DMSO-$d_6$) δ 12.06 (d, *J* = 2.8 Hz, 1H), 8.66 (d, *J* = 8.3 Hz, 1H), 7.61 (q, *J* = 2.1 Hz, 1H), 7.55 (s, 1H), 7.45 (d, *J* = 1.9 Hz, 1H), 7.14 (d, *J* = 1.9 Hz, 1H), 7.09 (dd, *J* = 8.3, 1.9 Hz, 1H), 6.39 (d, *J* = 1.8 Hz, 1H), 5.22 (dd, *J* = 7.0, 2.5 Hz, 1H), 4.52 (q, *J* = 6.5 Hz, 1H), 3.96 (s, 3H), 3.89 (s, 3H), 2.08 (q, *J* = 6.4 Hz, 2H), 1.77 - 1.58 (m, 4H), 1.53 (dt, *J* = 12.7, 6.1 Hz, 2H). ;<br>MS: m/z 472.3 [M+H]$^+$ | 1.99 |
| 133 | 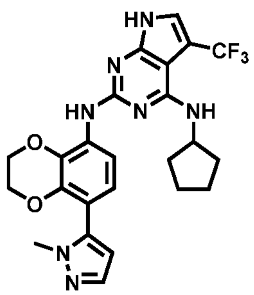 | $N^4$-cyclopentyl-$N^2$-(8-(1-methyl-1*H*-pyrazol-5-yl)-2,3-dihydrobenzo [b] [1,4] dioxin-5-yl)-5-(trifluoromethyl)-7*H*-pyrrolo [2,3-*d*]pyrimidine-2,4-diamine | 1H NMR (400 MHz, DMSO-$d_6$) δ 12.06 (d, *J* = 2.8 Hz, 1H), 8.20 (d, *J* = 8.5 Hz, 1H), 7.60 (d, *J* = 2.3 Hz, 1H), 7.46 - 7.39 (m, 2H), 6.81 (d, *J* = 8.5 Hz, 1H), 6.23 (d, *J* = 1.8 Hz, 1H), 5.24 - 5.18 (m, 1H), 4.50 (d, *J* = 6.5 Hz, 1H), 4.41 (dd, *J* = 5.4, 2.6 Hz, 2H), 4.32 (dd, *J* = 5.5, 2.7 Hz, 2H), 3.68 (s, 3H), 2.12 - 2.01 (m, 2H), 1.75 - 1.60 (m, 4H), 1.57 - 1.45 (m, 2H).<br>MS: m/z 500.3 [M+H]$^+$ | 1.97 |
| 134 | 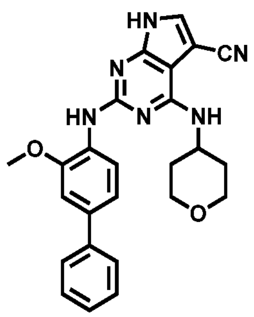 | 2-((3-methoxy-[1,1'-biphenyl]-4-yl)amino)-4-((tetrahydro-2*H*-pyran-4-yl)amino)-7*H*-pyrrolo [2,3-*d*]pyrimidine-5-carbonitrile | 1H NMR (400 MHz, DMSO-$d_6$) δ 12.27 (s, 1H), 8.52 (d, *J* = 8.3 Hz, 1H), 7.88 (s, 1H), 7.70 (d, *J* = 7.6 Hz, 2H), 7.54 (s, 1H), 7.45 (t, *J* = 7.5 Hz, 2H), 7.33 (d, *J* = 7.1 Hz, 1H), 7.29 (d, *J* = 2.0 Hz, 1H), 7.25 (dd, *J* = 8.6, 1.9 Hz, 1H), 6.01 (d, *J* = 7.5 Hz, 1H), 4.35 - 4.20 (m, 1H), 3.98 (s, 3H), 3.91 (q, *J* = 4.3, 3.4 Hz, 2H), 3.51 (td, *J* = 11.5, 2.2 Hz, 2H), 2.05 - 1.93 (m, 2H), 1.64 (tq, *J* = 11.5, 5.3, 4.4 Hz, 2H).;<br>MS: m/z 441.3[M+H]$^+$ | 1.80 |

(continued)

| 실시예 화합물 | 구조 | 화합물명 | $^1H$ NMR, MS | UPLC r.t. (min) |
|---|---|---|---|---|
| 135 | 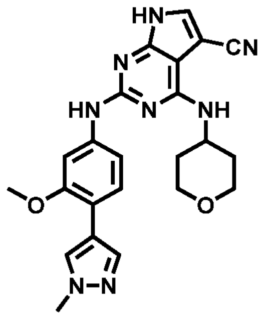 | 2-((3-methoxy-4-(1-methyl-1*H*-pyrazol-4-yl)phenyl)amino)-4-((tetrahydro-2*H*-pyran-4-yl) amino)-7*H*-pyrrolo[2,3-*d*] pyrimidine-5-carbonitrile | 1H NMR (400 MHz, DMSO-*d6*) δ 12.19 (d, *J* = 2.7 Hz, 1H), 9.01 (s, 1H), 7.99 (s, 1H), 7.87 (d, *J* = 2.7 Hz, 1H), 7.81 (s, 1H), 7.58 (s, 1H), 7.50 (dd, *J* = 8.4, 2.0 Hz, 1H), 7.42 (d, *J* = 8.5 Hz, 1H), 5.87 (d, *J* = 7.5 Hz, 1H), 4.30 (s, 1H), 3.92 (dt, *J* = 12.0, 3.4 Hz, 2H), 3.86 (d, *J* = 6.5 Hz, 6H), 3.51 - 3.40 (m, 2H), 2.01 (dd, *J* = 12.6, 3.6 Hz, 2H), 1.69 - 1.59 (m, 2H) ; MS: m/z 445.3 $[M+H]^+$ | 1.83 |
| 136 | 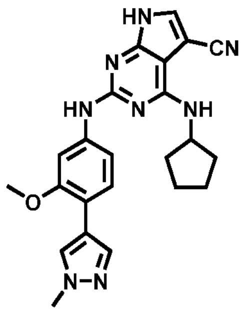 | 4-(cyclopentylamino)-2-((3-methoxy-4-(1-methyl-1*H*-pyrazol-4-yl)phenyl)amino)-7*H*-pyrrolo[2,3-*d*]pyrimidine-5-carbonitrile | 1H NMR (400 MHz, DMSO-*d6*) δ 12.16 (d, *J* = 2.7 Hz, 1H), 9.01 (s, 1H), 7.99 (s, 1H), 7.85 (d, *J* = 2.7 Hz, 1H), 7.80 (s, 1H), 7.70 (s, 1H), 7.41 (d, *J* = 1.1 Hz, 2H), 5.81 (d, *J* = 7.3 Hz, 1H), 4.54 (q, *J* = 6.8 Hz, 1H), 3.86 (d, *J* = 7.4 Hz, 6H), 2.09 (d, *J* = 3.9 Hz, 2H), 1.74 (d, *J* = 5.6 Hz, 2H), 1.66 - 1.52 (m, 4H) ; MS: m/z 429.4 $[M+H]^+$ | 2.05 |
| 137 | 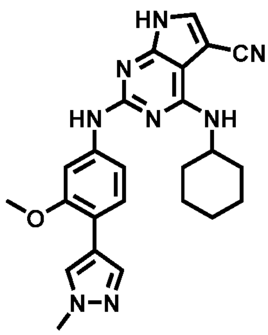 | 4-(cyclohexylamino)-2-((3-methoxy-4-(1-methyl-1*H*-pyrazol-4-yl)phenyl)amino)-7*H*-pyrrolo[2,3-d]pyrimidine-5-carbonitrile | 1H NMR (400 MHz, DMSO-*d6*) δ 12.17 (d, *J* = 2.7 Hz, 1H), 8.98 (s, 1H), 7.99 (s, 1H), 7.85 (d, *J* = 2.6 Hz, 1H), 7.80 (s, 1H), 7.60 (d, *J* = 2.0 Hz, 1H), 7.48 (dd, *J* = 8.5, 2.0 Hz, 1H), 7.40 (d, *J* = 8.4 Hz, 1H), 5.63 (d, *J* = 7.9 Hz, 1H), 4.15 (s, 1H), 3.86 (d, *J* = 6.5 Hz, 6H), 2.05 - 1.95 (m, 2H), 1.74 (s, 2H), 1.61 (d, *J* = 12.9 Hz, 1H), 1.42 (q, *J* = 10.2, 9.5 Hz, 4H), 1.26 (d, *J* = 18.8 Hz, 1H) ; MS: m/z 443.3 $[M+H]^+$ | 2.11 |

(continued)

| 실시예 화합물 | 구조 | 화합물명 | $^1$H NMR, MS | UPLC r.t. (min) |
|---|---|---|---|---|
| 138 | 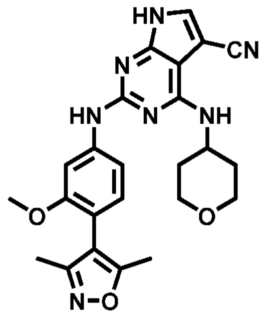 | 2-((4-(3,5-dimethylisoxazol-4-yl)-3-methoxyphenyl)amin o)-4-((tetrahydro-2*H*-pyran-4-yl) amino)-7*H*-pyrrolo[2,3-*d*] pyrimidine-5-carbonitrile | 1H NMR (400 MHz, DMSO-*d*6) δ 12.21 (d, *J* = 2.8 Hz, 1H), 9.13 (s, 1H), 7.88 (d, *J* = 2.7 Hz, 1H), 7.64 (d, *J* = 2.0 Hz, 1H), 7.56 (dd, *J* = 8.4, 2.0 Hz, 1H), 7.05 (d, *J* = 8.3 Hz, 1H), 5.90 (d, *J* = 7.6 Hz, 1H), 4.38 - 4.23 (m, 1H), 3.96 - 3.87 (m, 2H), 3.78 (s, 3H), 3.45 (td, *J* = 11.4, 2.2 Hz, 2H), 2.25 (s, 3H), 2.08 (s, 3H), 2.00 (d, *J* = 12.3 Hz, 2H), 1.72 - 1.57 (m, 2H) MS: m/z 460.3 $[M+H]^+$ | 1.94 |
| 139 | 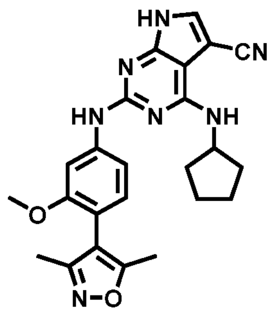 | 4-(cyclopentylamino)-2-((4-(3,5-dimethylisoxazol-4-yl)-3-methoxyphenyl)amin o)-7*H*-pyrrolo[2,3-*d*]pyrimidine-5-carbonitrile | 1H NMR (400 MHz, DMSO-$d_6$) δ 12.19 (s, 1H), 9.14 (s, 1H), 7.87 (d, J = 2.2 Hz, 1H), 7.79 (d, J = 2.1 Hz, 1H), 7.48 (dd, J = 8.3, 2.0 Hz, 1H), 7.04 (d, J = 8.3 Hz, 1H), 5.84 (d, J = 7.3 Hz, 1H), 4.55 (q, J = 7.0 Hz, 1H), 3.78 (s, 3H), 2.25 (s, 3H), 2.08 (d, J = 4.6 Hz, 5H), 1.79 - 1.68 (m, 2H), 1.66 - 1.50 (m, 4H) ; MS: m/z 444.3 $[M+H]^+$ | 2.11 |
| 140 | 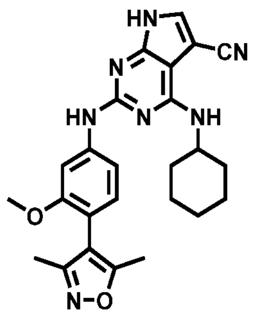 | 4-(cyclohexylamino)-2-((4-(3,5-dimethylisoxazol-4-yl)-3-methoxyphenyl)amin o)-7*H*-pyrrolo[2,3-*d*]pyrimidine-5-carbonitrile | 1H NMR (400 MHz, DMSO-*d*6) δ 12.19 (s, 1H), 9.12 (s, 1H), 7.87 (d, *J* = 2.4 Hz, 1H), 7.67 (d, *J* = 2.0 Hz, 1H), 7.55 (dd, *J* = 8.3, 2.0 Hz, 1H), 7.04 (d, *J* = 8.3 Hz, 1H), 5.67 (d, *J* = 7.9 Hz, 1H), 4.15 (s, 1H), 3.78 (s, 3H), 2.25 (s, 3H), 2.08 (s, 3H), 2.00 (d, *J* = 5.7 Hz, 2H), 1.74 (s, 2H), 1.60 (d, *J* = 12.5 Hz, 1H), 1.40 (s, 4H), 1.26 (s, 1H) ; MS: m/z 458.3 $[M+H]^+$ | 2.15 |

(continued)

| 실시 예 화합 물 | 구조 | 화합물명 | $^1$H NMR, MS | UPLC r.t. (min) |
|---|---|---|---|---|
| 141 | 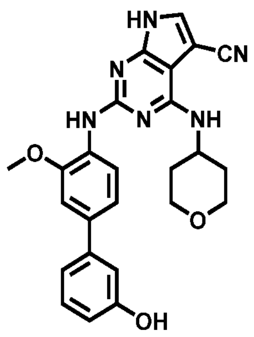 | 2-((3'-hydroxy-3-methoxy-[1,1'-biphenyl]-4-yl)amino)-4-((tetrahydro-2*H*-pyran-4-yl) amino)-7*H*-pyrrolo[2,3-*d*] pyrimidine-5-carbonitrile | 1H NMR (400 MHz, DMSO-$d_6$) δ 12.25 (s, 1H), 9.45 (s, 1H), 8.49 (d, *J* = 8.3 Hz, 1H), 7.88 (s, 1H), 7.53 (s, 1H), 7.26 - 7.15 (m, 3H), 7.10 (d, *J* = 7.7 Hz, 1H), 7.06 (d, *J* = 2.1 Hz, 1H), 6.73 (dd, *J* = 7.9, 2.3 Hz, 1H), 6.01 (d, *J* = 7.5 Hz, 1H), 4.33 - 4.21 (m, 1H), 3.97 (s, 3H), 3.92 (dt, *J* = 11.7, 3.6 Hz, 2H), 3.50 (td, *J* = 11.5, 2.2 Hz, 2H), 2.05 - 1.94 (m, 2H), 1.65 (qd, *J* = 11.6, 4.3 Hz, 2H). ;<br>MS: m/z 457.3[M+H]$^+$ | 1.54 |
| 142 | 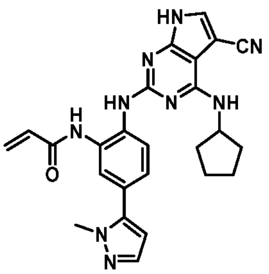 | *N*-(2-((5-cyano-4-(cyclopentylamino)-7*H*-pyrrolo [2,3-*d*]pyrimidin-2-yl)amino)-5-(1-methyl-1*H*-pyrazol-5-yl) phenyl)acrylamide | 1H NMR (400 MHz, DMSO-$d_6$) δ 12.22 (d, *J* = 2.8 Hz, 1H), 9.99 (s, 1H), 8.26 (s, 1H), 8.11 (d, *J* = 8.5 Hz, 1H), 7.84 (d, *J* = 2.6 Hz, 1H), 7.69 (d, *J* = 2.1 Hz, 1H), 7.46 (d, *J* = 1.9 Hz, 1H), 7.34 (dd, *J* = 8.5, 2.1 Hz, 1H), 6.52 (dd, *J* = 17.0, 10.2 Hz, 1H), 6.42 - 6.24 (m, 2H), 5.89 (d, *J* = 7.1 Hz, 1H), 5.80 (dd, *J* = 10.1, 1.9 Hz, 1H), 4.42 (h, *J* = 6.8 Hz, 1H), 3.89 (s, 3H), 2.10 - 2.00 (m, 2H), 1.80 - 1.49 (m, 6H).<br>MS: m/z 468.3 [M+H]$^+$ | 1.55 |
| 143 | 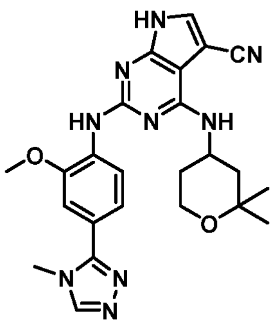 | 4-((2,2-dimethyltetrahydro-2*H*-pyran-4-yl)amino)-2-((2-methoxy-4-(4-methyl-4*H*-1,2,4-triazol-3-yl)phenyl)amino)-7*H*-pyrrolo[2,3-*d*]pyrimidine-5-carbonitrile | 1H NMR (400 MHz, DMSO-$d_6$) δ 12.31 (s, 1H), 8.64 (d, *J* = 8.4 Hz, 1H), 8.57 (s, 1H), 7.91 (d, *J* = 1.9 Hz, 1H), 7.66 (s, 1H), 7.36 (d, *J* = 2.0 Hz, 1H), 7.28 (dd, *J* = 8.4, 1.8 Hz, 1H), 5.94 (d, *J* = 7.7 Hz, 1H), 4.48 (dtt, *J* = 11.7, 7.6, 4.1 Hz, 1H), 3.97 (s, 3H), 3.78 (s, 3H), 3.75 - 3.69 (m, 2H), 2.03 - 1.91 (m, 2H), 1.59 - 1.39 (m, 2H), 1.30 (s, 3H), 1.19 (s, 3H).<br>MS: m/z 474.4 [M+H]$^+$ | 1.28 |

(continued)

| 실시예 화합물 | 구조 | 화합물명 | $^1$H NMR, MS | UPLC r.t. (min) |
|---|---|---|---|---|
| 144 | 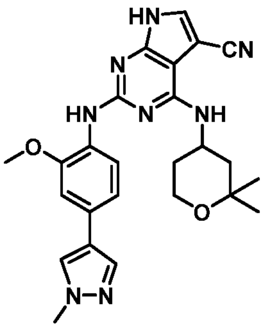 | 4-((2,2-dimethyltetrahydro-2*H*-pyran-4-yl)amino)-2-((2-methoxy-4-(1-methyl-1*H*-pyrazol-$_4$-yl)phenyl)amino)-7*H*-pyrrolo[2,3-d]pyrimidine-5-carbonitrile | 1H NMR (400 MHz, DMSO-*d*6 δ 12.19 (s, 1H), 8.32 (d, *J* = 8.3 Hz, 1H), 8.09 (s, 1H), 7.84 (d, *J* = 7.8 Hz, 2H), 7.47 (s, 1H), 7.19 (d, *J* = 1.8 Hz, 1H), 7.08 (dd, *J* = 8.3, 1.8 Hz, 1H), 5.83 (d, *J* = 7.7 Hz, 1H), 4.51 - 4.36 (m, 1H), 3.92 (s, 3H), 3.86 (s, 3H), 3.76 - 3.63 (m, 2H), 2.00 - 1.89 (m, 2H), 1.56 - 1.35 (m, 2H), 1.28 (s, 3H), 1.19 (s, 3H).<br>MS: m/z 473.3 $[M+H]^+$ | 1.48 |
| 145 | 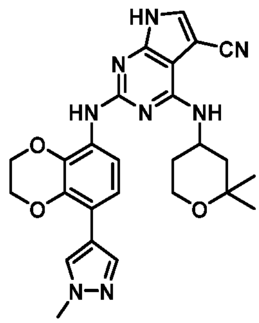 | 4-((2,2-dimethyltetrahydro-2*H*-pyran-4-yl)amino)-2-((8-(1-methyl-1*H*-pyrazol-4-yl)-2,3-dihydrobenzo[b][1,4] dioxin-5-yl) amino)-7*H*-pyrrolo[2,3-d] pyrimidine-5-carbonitrile | 1H NMR (400 MHz, DMSO-$d_6$) δ 12.24 (s, 1H), 8.01 (s, 1H), 7.91 (d, *J* = 8.6 Hz, 1H), 7.86 (s, 1H), 7.80 (s, 1H), 7.43 (s, 1H), 7.06 (d, *J* = 8.6 Hz, 1H), 5.87 (d, *J* = 7.6 Hz, 1H), 4.37 (s, 5H), 3.86 (s, 3H), 3.75 - 3.67 (m, 2H), 1.99 - 1.91 (m, 2H), 1.57 - 1.37 (m, 2H), 1.26 (s, 3H), 1.18 (s, 3H).<br>MS: m/z 501.3 $[M+H]^+$ | 1.46 |
| 146 | 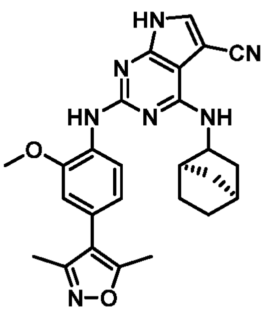 | 4-(((1*R*,4*S*)-bicyclo[2.2. 1] heptan-2-yl)amino)-2-((4-(3,5-dimethylisoxazol-4-yl)-2-methoxyphenyl)amin o)-7*H*-pyrrolo[2,3-*d*]pyrimidine-5-carbonitrile | 1H NMR (400 MHz, DMSO-$d_6$) δ 12.25 (s, 1H), 8.58 (d, *J* = 8.3 Hz, 1H), 7.87 (s, 1H), 7.52 (s, 1H), 6.99 (d, *J* = 1.8 Hz, 1H), 6.91 (dd, *J*= 8.3, 1.9 Hz, 1H), 5.61 (d, *J* = 6.4 Hz, 1H), 3.97 (dd, *J* = 7.7, 3.6 Hz, 1H), 3.93 (s, 3H), 2.43 (s, 3H), 2.41 - 2.30 (m, 2H), 2.25 (s, 3H), 1.88 (ddd, *J* = 12.9, 7.9, 2.2 Hz, 1H), 1.62 - 1.53 (m, 1H), 1.48 (qd, *J* = 9.5, 4.9 Hz, 2H), 1.34 (dt, *J* = 14.1, 7.3 Hz, 2H), 1.20 (d, *J* = 10.3 Hz, 2H).<br>MS: m/z 470.4 $[M+H]^+$ | 2.16 |

(continued)

| 실시예 화합물 | 구조 | 화합물명 | $^1$H NMR, MS | UPLC r.t. (min) |
|---|---|---|---|---|
| 147 | 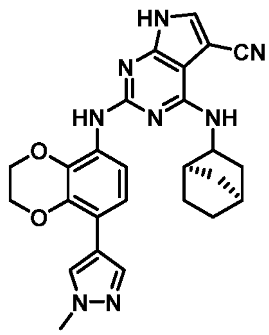 | 4-(((1*R*,4*S*)-bicyclo[2.2.1] heptan-2-yl)amino)-2-((8-(1-methyl-1*H*-pyrazol-4-yl)-2,3-dihydrobenzo[b][1,4] dioxin-5-yl) amino)-7*H*-pyrrolo[2,3-*d*] pyrimidine-5-carbonitrile | 1H NMR (400 MHz, DMSO-$d_6$) δ 12.23 (s, 1H), 8.02 (d, *J* = 8.6 Hz, 2H), 7.83 (d, *J* = 11.6 Hz, 2H), 7.34 (s, 1H), 7.07 (d, *J* = 8.6 Hz, 1H), 5.58 (d, *J* = 6.4 Hz, 1H), 4.38 (s, 4H), 3.95 (td, *J* = 7.4, 3.7 Hz, 1H), 3.86 (s, 3H), 2.41 - 2.28 (m, 2H), 1.92 - 1.83 (m, 1H), 1.57 (dtd, *J* = 12.3, 7.9, 4.4 Hz, 1H), 1.48 (dd, *J* = 12.2, 8.9 Hz, 2H), 1.33 (dt, *J* = 18.9, 6.9 Hz, 2H), 1.25 - 1.17 (m, 2H).<br>MS: m/z 483.4 $[M+H]^+$ | 1.75 |
| 148 | 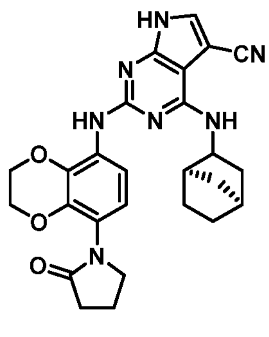 | 4-(((1*R*,4*S*)-bicyclo[2.2. 1] heptan-2-yl)amino)-2-((8-(2-oxopyrrolidin-1-yl)-2,3-dihydrobenzo [b] [1,4] dioxin-5-yl) amino)-7*H*-pyrrolo[2,3-*d*] pyrimidine-5-carbonitrile | 1H NMR (400 MHz, DMSO-$d_6$) δ 12.24 (s, 1H), 7.95 (d, *J* = 8.8 Hz, 1H), 7.84 (s, 1H), 7.40 (s, 1H), 6.73 (d, *J* = 8.7 Hz, 1H), 5.59 (d, *J* = 6.4 Hz, 1H), 4.40 - 4.32 (m, 2H), 4.32 -4.23 (m, 2H), 3.95 (td, *J* = 7.4, 3.6 Hz, 1H), 3.64 (t, *J* = 7.0 Hz, 2H), 2.37 (d, *J* = 4.9 Hz, 2H), 2.36 - 2.28 (m, 2H), 2.07 (p, *J* = 7.5 Hz, 2H), 1.86 (ddd, *J* = 12.9, 7.9, 2.2 Hz, 1H), 1.54 (dt, *J* = 15.5, 4.4 Hz, 1H), 1.48 (dd, *J* = 11.5, 8.6 Hz, 2H), 1.39 - 1.26 (m, 2H), 1.20 (d, *J* = 10.3 Hz, 2H).;<br>MS: m/z 486.4 $[M+H]^+$ | 1.69 |
| 149 | 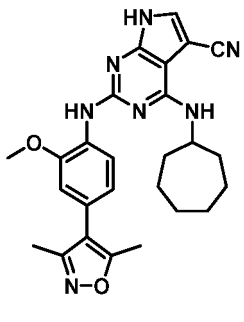 | 4-(cycloheptylamino)-2-((4-(3,5-dimethylisoxazol-4-yl)-2-methoxyphenyl)amin o)-7*H*-pyrrolo[2,3-*d*]pyrimidine-5-carbonitrile | 1H NMR (400 MHz, DMSO-$d_6$) δ 12.24 (s, 1H), 8.53 (d, *J* = 8.3 Hz, 1H), 7.87 (s, 1H), 7.51 (s, 1H), 6.99 (d, *J* = 1.9 Hz, 1H), 6.91 (dd, *J* = 8.3, 1.8 Hz, 1H), 5.76 (d, *J* = 7.7 Hz, 1H), 4.26 (tt, *J* = 8.5, 4.2 Hz, 1H), 3.92 (s, 3H), 2.43 (s, 3H), 2.25 (s, 3H), 2.00 (dq, *J* = 13.0, 4.8, 3.8 Hz, 2H), 1.72 - 1.49 (m, 10H).<br>MS: m/z 472.3$[M+H]^+$ | 2.22 |

(continued)

| 실시예 화합물 | 구조 | 화합물명 | $^1$H NMR, MS | UPLC r.t. (min) |
|---|---|---|---|---|
| 150 | 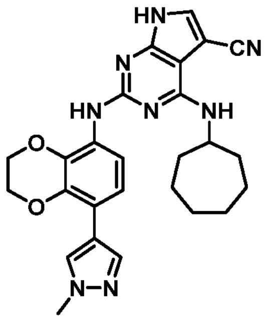 | 4-(cycloheptylamino)-2-((8-(1-methyl-1*H*-pyrazol-4-yl)-2,3-dihydrobenzo[b][1,4]dioxin-5-yl) amino)-7*H*-pyrrolo[2,3-*d*] pyrimidine-5-carbonitrile | 1H NMR (400 MHz, DMSO-$d_6$) δ 12.29 - 12.17 (m, 1H), 8.01 (s, 1H), 7.97 (d, *J* = 8.6 Hz, 1H), 7.89 - 7.78 (m, 2H), 7.34 (s, 1H), 7.08 (d, *J* = 8.6 Hz, 1H), 5.72 (d, *J* = 7.7 Hz, 1H), 4.38 (s, 4H), 4.25 (tt, *J* = 8.5, 4.2 Hz, 1H), 3.86 (s, 3H), 1.99 (ddd, *J* = 12.6, 8.2, 4.3 Hz, 2H), 1.72 - 1.50 (m, 10H).<br>MS: m/z 485.4 [M+H]$^+$ | 2.12 |
| 151 | 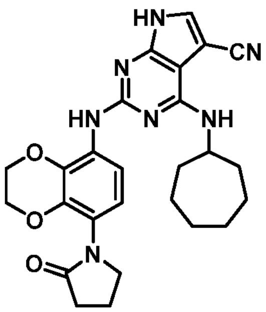 | 4-(cycloheptylamino)-2-((8-(2-oxopyrrolidin-1-yl)-2,3-dihydrobenzo[b][1,4]dioxin-5-yl) amino)-7*H*-pyrrolo[2,3-*d*] pyrimidine-5-carbonitrile | 1H NMR (400 MHz, DMSO-$d_6$) δ 12.24 (s, 1H), 7.88 (d, *J* = 8.8 Hz, 1H), 7.84 (s, 1H), 7.41 (s, 1H), 6.73 (d, *J* = 8.8 Hz, 1H), 5.73 (d, *J* = 7.7 Hz, 1H), 4.34 (dd, *J* = 5.7, 2.7 Hz, 2H), 4.28 (dd, *J* = 5.5, 2.7 Hz, 2H), 4.23 (dq, *J* = 8.5, 4.2 Hz, 1H), 3.64 (t, *J* = 7.0 Hz, 2H), 2.37 (t, *J* = 8.0 Hz, 2H), 2.08 (q, *J* = 7.5 Hz, 2H), 2.04 - 1.95 (m, 2H), 1.72 - 1.49 (m, 10H).<br>MS: m/z 488.4 [M+H]$^+$ | 2.07 |
| 152 | 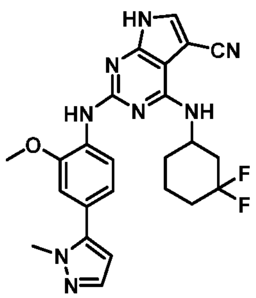 | 4-((3,3-difluorocyclohexyl)a mino)-2-((2-methoxy-4-(1-methyl-1*H*-pyrazol-5 - yl)phenyl) amino)-7*H*-pyrrolo[2,3-*d*] pyrimidine-5-carbonitrile | 1H NMR (400 MHz, DMSO-$d_6$) δ 12.31 (d, *J* = 2.8 Hz, 1H), 8.53 (d, *J* = 8.3 Hz, 1H), 7.91 (d, *J* = 2.7 Hz, 1H), 7.60 (s, 1H), 7.45 (d, *J* = 1.9 Hz, 1H), 7.13 (d, J = 1.9 Hz, 1H), 7.03 (dd, *J* = 8.4, 1.9 Hz, 1H), 6.38 (d, *J* = 1.8 Hz, 1H), 6.17 (d, *J* = 8.1 Hz, 1H), 4.39 (s, 1H), 3.95 (s, 3H), 3.87 (s, 3H), 2.00 (d, *J* = 36.6 Hz, 3H), 1.83 (s, 3H), 1.65 - 1.55 (m, 2H)<br>MS: m/z 479.4 [M+H]$^+$ | 1.67 |

(continued)

| 실시예 화합물 | 구조 | 화합물명 | $^1$H NMR, MS | UPLC r.t. (min) |
|---|---|---|---|---|
| 153 | 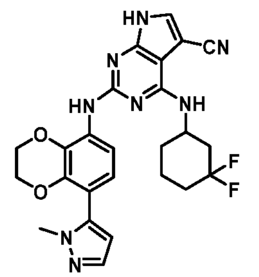 | 4-((3,3-difluorocyclohexyl)amino)-2-((8-(1-methyl-1*H*-pyrazol-5 - yl)-2,3-dihydrobenzo [b] [1,4] dioxin-5-yl) amino)-7*H*-pyrrolo[2,3-*d*] pyrimidine-5-carbonitrile | 1H NMR (400 MHz, DMSO-*d*$_6$) δ 12.31 (d, J = 2.8 Hz, 1H), 8.09 (d, J = 8.5 Hz, 1H), 7.90 (d, J = 2.8 Hz, 1H), 7.48 (s, 1H), 7.42 (d, J = 1.8 Hz, 1H), 6.76 (d, J = 8.5 Hz, 1H), 6.22 (d, J = 1.8 Hz, 1H), 6.16 (d, J = 8.1 Hz, 1H), 4.36 (ddd, J = 35.6, 6.0, 3.1 Hz, 5H), 3.68 (s, 3H), 2.00 (dd, J = 26.4, 11.8 Hz, 3H), 1.83 (s, 3H), 1.58 (q, J = 11.3 Hz, 2H)<br>MS: m/z 507.4 $[M+H]^+$ | 1.64 |
| 154 | 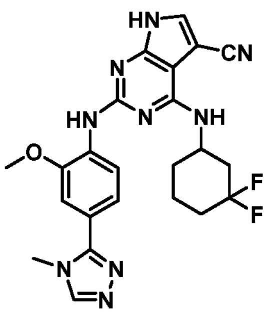 | 4-((3,3-difluorocyclohexyl)amino)-2-((2-methoxy-4-(4-methyl-4*H*-1,2,4-triazol-3-yl) phenyl)amino)-7*H*-pyrrolo[2,3-*d*] pyrimidine-5-carbonitrile | 1H NMR (400 MHz, DMSO-*d6*) δ 12.26 (s, 1H), 8.63 (d, *J* = 8.4 Hz, 1H), 8.54 (s, 1H), 7.91 (s, 1H), 7.63 (s, 1H), 7.35 (d, *J* = 1.9 Hz, 1H), 7.26 (dd, *J* = 8.4, 1.9 Hz, 1H), 6.15 (d, *J* = 8.1 Hz, 1H), 4.40 (s, 1H), 3.97 (s, 3H), 3.76 (s, 3H), 2.01 (dd, *J* = 24.2, 11.0 Hz, 3H), 1.86 (s, 3H), 1.69 - 1.53 (m, 2H)<br>MS: m/z 480.4 $[M+H]^+$ | 1.39 |
| 155 | 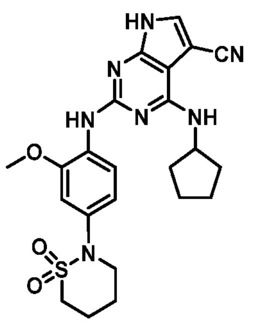 | 4-(cyclopentylamino)-2-((4-(1, 1-dioxido-1,2-thiazinan-2-yl)-2-methoxyphenyl)amin o)-7*H*-pyrrolo[2,3-*d*]pyrimidine-5-carbonitrile | 1H NMR (400 MHz, DMSO-*d6*) δ 12.24 (s, 1H), 8.38 (d, *J* = 9.2 Hz, 1H), 7.85 (d, *J* = 2.3 Hz, 1H), 7.49 (s, 1H), 6.95 - 6.85 (m, 2H), 5.89 (d, *J* = 7.1 Hz, 1H), 4.47 (p, *J* = 6.8 Hz, 1H), 3.88 (s, 3H), 3.62 (t, *J* = 5.6 Hz, 2H), 3.28 (t, *J* = 6.1 Hz, 2H), 2.15 (dd, *J* = 7.7, 4.1 Hz, 2H), 2.04 (dd, *J* = 12.0, 6.8 Hz, 2H), 1.82 (s, 2H), 1.72 (d, *J* = 6.3 Hz, 2H), 1.59 (ddt, *J* = 19.5, 12.5, 6.6 Hz, 4H)<br>MS: m/z 482.3 $[M+H]^+$ | 1.70 |

(continued)

| 실시예 화합물 | 구조 | 화합물명 | $^1$H NMR, MS | UPLC r.t. (min) |
|---|---|---|---|---|
| 156 | 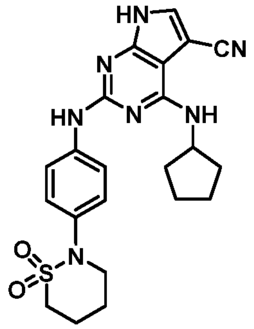 | 4-(cyclopentylamino)-2-((4-(1, 1-dioxido-1,2-thiazinan-2-yl) phenyl)amino)-7*H*-pyrrolo[2,3-*d*] pyrimidine-5-carbonitrile | 1H NMR (400 MHz, DMSO-*d*6) δ 12.17 (s, 1H), 9.11 (s, 1H), 7.88 - 7.73 (m, 3H), 7.23 - 7.12 (m, 2H), 5.82 (d, *J* = 7.2 Hz, 1H), 4.50 (q, *J* = 6.8 Hz, 1H), 3.62 - 3.53 (m, 2H), 3.29 - 3.21 (m, 2H), 2.19 - 2.02 (m, 4H), 1.85 - 1.69 (m, 4H), 1.67 - 1.50 (m, 4H) MS: m/z 452.3 $[M+H]^+$ | 1.63 |
| 157 | 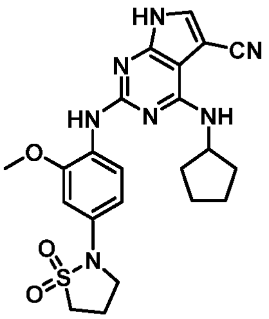 | 4-(cyclopentylamino)-2-((4-(1,1-dioxidoisothiazolidin - 2-yl)-2-methoxyphenyl)amin o)-7*H*-pyrrolo[2,3-*d*]pyrimidine-5-carbonitrile | 1H NMR (400 MHz, DMSO-$d_6$) δ 12.19 (s, 1H), 8.29 (d, J = 8.7 Hz, 1H), 7.83 (d, J = 2.5 Hz, 1H), 7.45 (s, 1H), 6.89 (d, J = 2.4 Hz, 1H), 6.80 (dd, J = 8.6, 2.4 Hz, 1H), 5.85 (d, J = 7.1 Hz, 1H), 4.45 (q, J = 6.8 Hz, 1H), 3.86 (s, 3H), 3.74 (t, J = 6.5 Hz, 2H), 3.48 (t, J = 7.5 Hz, 2H), 2.39 (p, J = 6.9 Hz, 2H), 2.04 (dd, J = 12.1, 6.9 Hz, 2H), 1.72 (d, J = 6.9 Hz, 2H), 1.59 (ddd, J = 25.3, 12.2, 6.4 Hz, 4H) MS: m/z 468.3 $[M+H]^+$ | 1.64 |
| 158 | 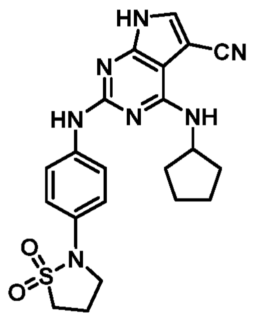 | 4-(cyclopentylamino)-2-((4-(1,1-dioxidoisothiazolidin - 2-yl) phenyl)amino)-7*H*-pyrrolo[2,3-*d*] pyrimidine-5-carbonitrile | 1H NMR (400 MHz, DMSO-$d_6$) δ 12.14 (s, 1H), 9.02 (s, 1H), 7.82 (s, 1H), 7.82 - 7.78 (m, 2H), 7.18 - 7.09 (m, 2H), 5.79 (d, *J* = 7.1 Hz, 1H), 4.48 (q, *J* = 6.8 Hz, 1H), 3.69 (t, *J* = 6.5 Hz, 2H), 3.45 (t, *J* = 7.5 Hz, 2H), 2.38 (p, *J* = 6.9 Hz, 2H), 2.06 (dd, *J* = 11.3, 6.6 Hz, 2H), 1.73 (dtd, *J* = 10.7, 7.6, 4.7 Hz, 2H), 1.60 (tdd, *J* = 19.3, 10.9, 4.0 Hz, 4H). ; MS: m/z 438.3 $[M+H]^+$ | 1.53 |

(continued)

| 실시 예 화합 물 | 구조 | 화합물명 | 1H NMR, MS | UPLC r.t. (min) |
|---|---|---|---|---|
| 159 | 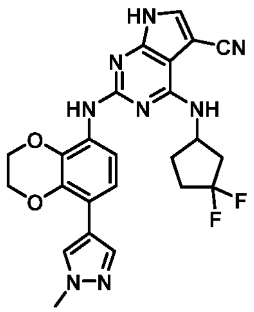 | 4-((3,3-difluorocyclopentyl)amino)-2-((8-(1-methyl-1*H*-pyrazol-4-yl)-2,3-dihydrobenzo [b] [1,4] dioxin-5-yl) amino)-7*H*-pyrrolo[2,3-*d*] pyrimidine-5-carbonitrile | 1H NMR (400 MHz, DMSO-$d_6$) δ 12.27 (s, 1H), 8.01 (s, 1H), 7.93 (d, *J* = 8.6 Hz, 1H), 7.87 (s, 1H), 7.81 (d, *J* = 0.7 Hz, 1H), 7.42 (s, 1H), 7.08 (d, *J* = 8.6 Hz, 1H), 6.41 (d, *J* = 7.3 Hz, 1H), 4.67 (h, *J* = 7.8 Hz, 1H), 4.38 (s, 4H), 3.86 (s, 3H), 2.63 (qd, *J* = 13.5, 8.0 Hz, 1H), 2.38 - 2.02 (m, 4H), 1.90 (dqd, *J* = 12.2, 8.6, 4.8 Hz, 1H).<br>MS: m/z 493.3[M+H]+ | 1.57 |
| 160 | 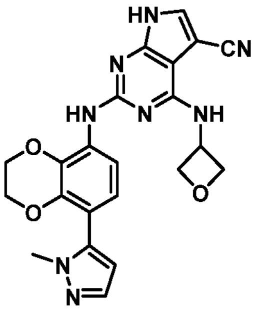 | 2-((8-(1-methyl-1*H*-pyrazol-5-yl)-2,3-dihydrobenzo [b] [1,4] dioxin-5 -yl)amino) -4-(oxetan-3-ylamino)-7*H*-pyrrolo[2,3-*d*] pyrimidine-5-carbonitrile | 1H NMR (400 MHz, DMSO-$d_6$) δ 13.05 (d, *J* = 2.8 Hz, 1H), 10.23 (s, 1H), 9.35 (s, 1H), 8.13 (d, *J* = 2.6 Hz, 1H), 7.47 (d, *J* = 1.8 Hz, 1H), 7.07 (d, *J* = 8.2 Hz, 1H), 6.91 (d, *J* = 8.2 Hz, 1H), 6.32 (d, *J* = 1.8 Hz, 1H), 4.54 (d, *J* = 5.0 Hz, 2H), 3.78 - 3.59 (m, 5H), 1.91 (s, 2H).<br>MS: m/z 445.2 [M+H]+ | 0.92 |
| 161 | 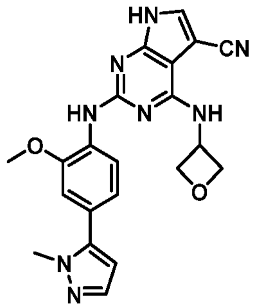 | 2-((2-methoxy-4-(1-methyl-1*H*-pyrazol-5-yl)phenyl)amino)-4-(oxetan-3-ylamino)-7*H*-pyrrolo [2,3-*d*]pyrimidine-5-carbonitrile | 1H NMR (400 MHz, DMSO-$d_6$) δ 11.69 (s, 1H), 8.95 (s, 1H), 7.95 (s, 1H), 7.60 (d, *J* = 8.1 Hz, 1H), 7.49 (d, *J* = 1.9 Hz, 1H), 7.25 (d, *J* = 1.9 Hz, 1H), 7.16 (dd, *J* = 8.0, 1.8 Hz, 1H), 6.48 (d, *J* = 1.9 Hz, 1H), 5.27 (s, 1H), 4.54 - 4.36 (m, 2H), 4.28 (dd, *J* = 9.8, 5.5 Hz, 1H), 3.89 (d, *J* = 15.6 Hz, 6H), 3.65 (t, *J* = 4.1 Hz, 2H).<br>MS: m/z 417.3 [M+H]+ | 0.93 |

(continued)

| 실시예 화합물 | 구조 | 화합물명 | $^1$H NMR, MS | UPLC r.t. (min) |
|---|---|---|---|---|
| 162 | 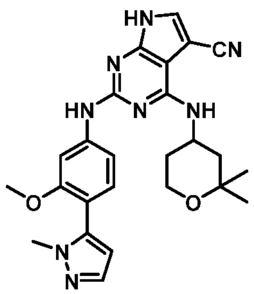 | 4-((2,2-dimethyltetrahydro-2*H*-pyran-4-yl)amino)-2-((3-methoxy-4-(1-methyl-1*H*-pyrazol-5-yl)phenyl)amino)-7*H*-pyrrolo[2,3-*d*]pyrimidine-5-carbonitrile | 1H NMR (400 MHz, DMSO-$d_6$) δ 12.25 (d, *J* = 2.7 Hz, 1H), 9.20 (s, 1H), 7.89 (d, *J* = 2.4 Hz, 1H), 7.69 (dd, *J* = 8.4, 1.9 Hz, 1H), 7.56 (s, 1H), 7.42 (d, *J* = 1.8 Hz, 1H), 7.09 (d, *J* = 8.3 Hz, 1H), 6.18 (d, *J* = 1.8 Hz, 1H), 5.83 (d, *J* = 7.8 Hz, 1H), 4.58 - 4.32 (m, 1H), 3.79 (s, 3H), 3.63 (s, 3H), 3.17 (s, 2H), 2.05 - 1.91 (m, 2H), 1.56 - 1.40 (m, 2H), 1.23 (d, *J* = 29.3 Hz, 6H). MS: m/z 473.3 [M+H]$^+$ | 1.52 |
| 163 | 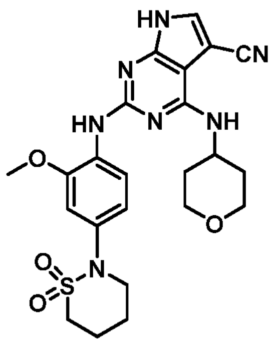 | 2-((4-(1, 1-dioxido-1,2-thiazinan-2-yl)-2-methoxyphenyl)amin o)-4-((tetrahydro-2*H*-pyran-4-yl) amino)-7*H*-pyrrolo[2,3-*d*] pyrimidine-5-carbonitrile | 1H NMR (400 MHz, DMSO-$d_6$) δ 12.24 (d, *J* = 2.7 Hz, 1H), 8.34 - 8.25 (m, 1H), 7.87 (d, *J* = 2.6 Hz, 1H), 7.52 (s, 1H), 6.93 - 6.83 (m, 2H), 5.98 (d, *J* = 7.5 Hz, 1H), 4.29 - 4.19 (m, 1H), 3.94 - 3.88 (m, 2H), 3.87 (s, 3H), 3.62 (t, *J* = 5.6 Hz, 2H), 3.48 (td, *J* = 11.4, 2.1 Hz, 2H), 3.31 - 3.26 (m, 2H), 2.15 (q, *J* = 6.6, 6.0 Hz, 2H), 2.01 - 1.93 (m, 2H), 1.83 (d, *J* = 6.1 Hz, 2H), 1.63 (qd, *J* = 11.2, 4.3 Hz, 2H) MS: m/z 498.3 [M+H]$^+$ | 1.42 |
| 164 | 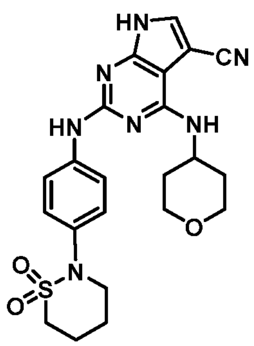 | 2-((4-(1, 1-dioxido-1,2- thiazinan - 2-yl)phenyl)amino)-4-((tetrahydro-2*H*-pyran-4-yl) amino )-7*H*-pyrrolo[2,3-*d*] pyrimidine-5-carbonitrile | 1H NMR (400 MHz, DMSO-$d_6$) δ 12.18 (d, J = 2.9 Hz, 1H), 9.12 (s, 1H), 7.85 (d, J = 2.8 Hz, 1H), 7.83 - 7.72 (m, 2H), 7.23 - 7.11 (m, 2H), 5.92 (d, J = 7.5 Hz, 1H), 4.27 (ddt, J = 15.1, 10.9, 5.6 Hz, 1H), 3.92 (dt, J = 11.5, 3.5 Hz, 2H), 3.59 (t, J = 5.6 Hz, 2H), 3.47 (td, J = 11.5, 2.2 Hz, 2H), 3.29 - 3.23 (m, 2H), 2.21 - 2.10 (m, 2H), 1.99 (t, J = 6.9 Hz, 2H), 1.80 (p, J = 6.4 Hz, 2H), 1.64 (qd, J = 11.4, 4.4 Hz, 2H); MS: m/z 468.3 [M+H]$^+$ | 1.37 |

(continued)

| 실시 예 화합물 | 구조 | 화합물명 | $^1$H NMR, MS | UPLC r.t. (min) |
|---|---|---|---|---|
| 165 | 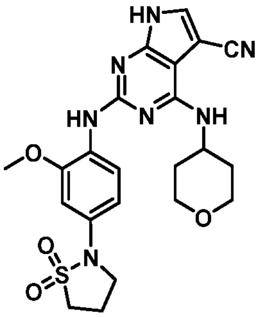 | 2-( (4-(1,1-dioxidoisothiazolidin-2-yl)-2-methoxyphenyl)amin o)-4-((tetrahydro-2*H*-pyran-4-yl) amino)-7*H*-pyrrolo[2,3-*d*] pyrimidine-5-carbonitrile | 1H NMR (400 MHz, DMSO-*d*6) δ 12.20 (s, 1H), 8.22 (d, *J* = 8.7 Hz, 1H), 7.84 (d, *J* = 2.1 Hz, 1H), 7.49 (s, 1H), 6.89 (d, *J* = 2.4 Hz, 1H), 6.81 (dd, *J* = 8.7, 2.4 Hz, 1H), 5.94 (d, *J* = 7.5 Hz, 1H), 4.27 - 4.18 (m, 1H), 3.90 (d, *J* = 10.6 Hz, 2H), 3.85 (s, 3H), 3.74 (t, *J* = 6.5 Hz, 2H), 3.52 - 3.42 (m, 4H), 2.40 (p, *J* = 7.0 Hz, 2H), 1.97 (d, *J* = 12.7 Hz, 2H), 1.67 - 1.57 (m, 2H) ; MS: m/z 484.3 $[M+H]^+$ | 1.37 |
| 166 | 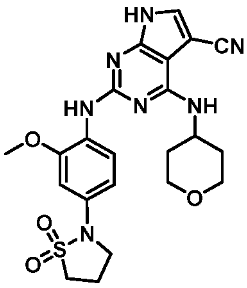 | 2-((4-(1,1-dioxidoisothiazolidin-2-yl)phenyl)amino)-4-((tetrahydro-2*H*-pyran-4-yl) amino)-7*H*-pyrrolo[2,3-*d*] pyrimidine-5-carbonitrile | 1H NMR (400 MHz, DMSO-$d_6$) δ 12.15 (s, 1H), 9.03 (s, 1H), 7.84 (s, 1H), 7.80 - 7.74 (m, 2H), 7.18 - 7.10 (m, 2H), 5.90 (d, *J* = 7.5 Hz, 1H), 4.26 (dtd, *J* = 10.9, 6.9, 3.9 Hz, 1H), 3.92 (dt, *J* = 11.7, 3.5 Hz, 2H), 3.69 (t, *J* = 6.5 Hz, 2H), 3.53 - 3.41 (m, 4H), 2.38 (p, *J* = 6.9 Hz, 2H), 2.05 - 1.95 (m, 2H), 1.71 - 1.58 (m, 2H). MS: m/z 454.3$[M+H]^+$ | 1.29 |
| 167 | 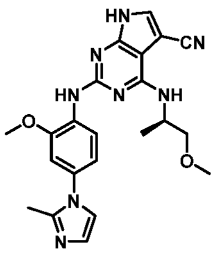 | (*R*)-2-((2-methoxy-4-(2-methyl-1*H*-imidazol-1-yl)phenyl)amino)-4-((1-methoxypropan-2-yl) amino)-7*H*-pyrrolo[2,3-*d*] pyrimidine-5-carbonitrile | 1H NMR (400 MHz, DMSO-$d_6$) δ 12.37 (s, 1H), 8.69 (d, *J* = 8.6 Hz, 1H), 7.90 (dd, *J* = 24.5, 2.1 Hz, 2H), 7.82 - 7.64 (m, 2H), 7.32 (d, *J* = 2.3 Hz, 1H), 7.19 (dd, *J* = 8.7, 2.4 Hz, 1H), 5.98 (d, *J* = 7.9 Hz, 1H), 4.49 (q, *J* = 7.0, 6.4 Hz, 1H), 3.96 (s, 3H), 3.58 - 3.41 (m, 2H), 3.33 (s, 3H), 2.56 (s, 3H), 1.28 (d, *J* = 6.6 Hz, 3H). MS: m/z 433.2 $[M+H]^+$ | 1.20 |

(continued)

| 실시 예 화합 물 | 구조 | 화합물명 | $^1$H NMR, MS | UPLC r.t. (min) |
|---|---|---|---|---|
| 168 | 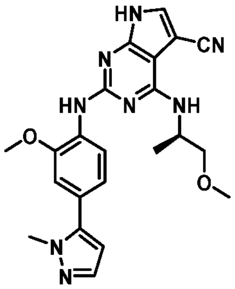 | (*R*)-2-((2-methoxy-4-(1-methyl-1*H*-pyrazol-5-yl)phenyl)amino)-4-((1-methoxypropan-2-yl) amino)-7*H*-pyrrolo[2,3-*d*] pyrimidine-5-carbonitrile | 1H NMR (400 MHz, DMSO-$d_6$) δ 12.32 (d, *J* = 2.8 Hz, 1H), 8.58 (d, *J* = 8.3 Hz, 1H), 7.90 (d, *J* = 2.7 Hz, 1H), 7.58 (s, 1H), 7.45 (d, *J* = 1.8 Hz, 1H), 7.17 - 6.97 (m, 2H), 6.39 (d, *J* = 1.9 Hz, 1H), 5.89 (d, *J* = 7.9 Hz, 1H), 4.49 (tdd, *J* = 9.1, 8.0, 6.5, 4.0 Hz, 1H), 3.92 (d, *J* = 29.4 Hz, 6H), 3.58 - 3.42 (m, 2H), 3.33 (d, *J* = 1.5 Hz, 3H), 1.28 (d, *J* = 6.6 Hz, 3H).<br>MS: m/z 433.2 $[M+H]^+$ | 1.55 |
| 169 | 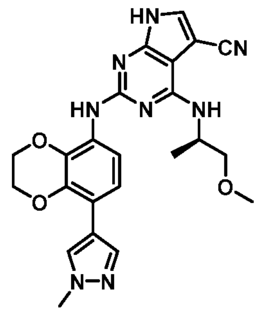 | (*R*)-4-((1-methoxypropan-2-yl) amino)-2-((8-(1-methyl-1*H*-pyrazol-4-yl)-2,3-dihydrobenzo[b][1,4] dioxin-5-yl) amino)-7*H*-pyrrolo[2,3-*d*] pyrimidine-5-carbonitrile | 1H NMR (400 MHz, DMSO-*d6*) δ 12.31 - 12.22 (m, 1H), 8.02 (s, 1H), 7.95 (d, *J* = 8.6 Hz, 1H), 7.90 - 7.79 (m, 2H), 7.38 (s, 1H), 7.09 (d, *J* = 8.6 Hz, 1H), 5.84 (d, *J* = 7.9 Hz, 1H), 4.46 (p, *J* = 5.8 Hz, 1H), 4.38 (s, 4H), 3.86 (s, 3H), 3.56 - 3.42 (m, 2H), 3.34 (s, 3H), 1.27 (d, *J* = 6.6 Hz, 3H).<br>MS: m/z 461.2 $[M+H]^+$ | 1.46 |
| 170 | 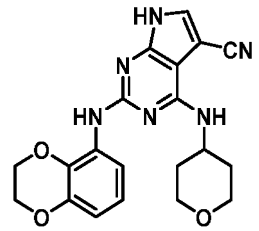 | 2-((2,3-dihydrobenzo [b] [1,4] dioxin-5 -yl)amino) -4-((tetrahydro-2*H*-pyran-4-yl) amino)-7*H*-pyrrolo[2,3-*d*] pyrimidine-5-carbonitrile | 1H NMR (400 MHz, DMSO-$d_6$) δ 12.24 (s, 1H), 7.95 (dd, *J* = 8.2, 1.5 Hz, 1H), 7.86 (s, 1H), 7.36 (s, 1H), 6.77 (t, *J* = 8.2 Hz, 1H), 6.49 (dd, *J* = 8.2, 1.4 Hz, 1H), 5.97 (d, *J* = 7.5 Hz, 1H), 4.39 - 4.14 (m, 5H), 3.90 (dt, *J* = 11.5, 3.4 Hz, 2H), 3.46 (td, *J* = 11.5, 2.1 Hz, 2H), 2.05 - 1.91 (m, 2H), 1.71 - 1.54 (m, 2H).<br>MS: m/z 393.2 $[M+H]^+$ | 1.46 |
| 171 | 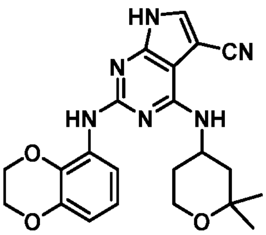 | 2-((2,3-dihydrobenzo [b] [1,4] dioxin-5 -yl)amino) -4-((2,2-dimethyltetrahydro-2*H*-pyran-4-yl)amino)-7*H*-pyrrolo[2,3-*d*] pyrimidine-5-carbonitrile | 1H NMR (400 MHz, DMSO-$d_6$) δ 12.21 (s, 1H), 7.94 (dd, *J* = 8.3, 1.5 Hz, 1H), 7.85 (s, 1H), 7.36 (s, 1H), 6.93 (s, 1H), 6.75 (t, *J* = 8.2 Hz, 1H), 6.50 (dd, *J* = 8.2, 1.5 Hz, 1H), 5.84 (d, *J* = 7.7 Hz, 1H), 4.49 - 4.21 (m, 5H), 3.76 - 3.65 (m, 2H), 2.00 - 1.89 (m, 3H), 1.25 (d, *J* = 3.1 Hz, 3H), 1.18 (s, 3H).<br>MS: m/z 421.2 $[M+H]^+$ | 1.59 |

(continued)

| 실시예 화합물 | 구조 | 화합물명 | $^1$H NMR, MS | UPLC r.t. (min) |
|---|---|---|---|---|
| 172 | 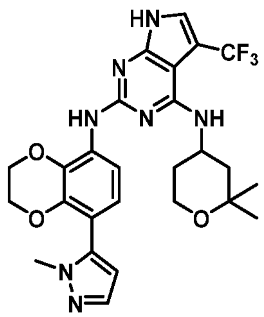 | *N*$^4$-(2,2-dimethyltetrahydro-2*H*-pyran-4-yl)-*N*$^2$-(8-(1-methyl-1*H*-pyrazol-5-yl)-2,3-dihydrobenzo [b] [1,4] dioxin-5-yl)-5-(trifluoromethyl)-7*H*-pyrrolo [2,3-*d*]pyrimidine- 2,4-diamine | 1H NMR (400 MHz, DMSO-$d_6$) δ 12.07 (s, 1H), 8.10 (d, *J* = 8.4 Hz, 1H), 7.62 (q, *J* = 2.1 Hz, 1H), 7.57 - 7.49 (m, 1H), 7.43 (d, *J* = 1.8 Hz, 1H), 6.78 (d, *J* = 8.5 Hz, 1H), 6.22 (d, *J* = 1.8 Hz, 1H), 5.11 (d, *J* = 7.5 Hz, 1H), 4.54 - 4.43 (m, 1H), 4.36 (ddd, *J* = 34.4, 6.1, 3.0 Hz, 4H), 3.77 - 3.62 (m, 5H), 1.98 (d, *J* = 8.2 Hz, 2H), 1.47 - 1.29 (m, 2H), 1.26 (s, 3H), 1.18 (s, 3H).<br>MS: m/z 544.3 [M+H]$^+$ | 1.73 |
| 173 | 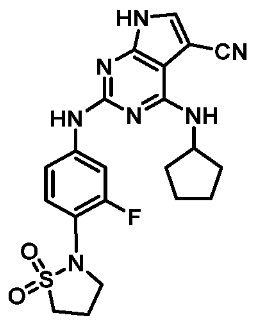 | 4-(cyclopentylamino)-2-((4-(1,1-dioxidoisothiazolidin - 2-yl)-3-fluorophenyl)amino)-7*H*-pyrrolo [2,3-*d*]pyrimidine-5-carbonitrile | 1H NMR (400 MHz, DMSO-$d_6$) δ 12.25 (d, J = 2.6 Hz, 1H), 9.39 (s, 1H), 8.10 (dd, J = 14.5, 2.4 Hz, 1H), 7.88 (d, J = 2.4 Hz, 1H), 7.46 (dd, J = 8.7, 2.4 Hz, 1H), 7.28 (t, J = 8.9 Hz, 1H), 5.93 (d, J = 7.1 Hz, 1H), 4.49 (h, J = 6.8 Hz, 1H), 3.67 (t, *J* = 6.7 Hz, 2H), 3.38 (d, J = 7.4 Hz, 2H), 2.40 (p, J = 7.0 Hz, 2H), 2.09 (s, 2H), 1.74 (d, J = 5.9 Hz, 2H), 1.60 (h, J = 6.8 Hz, 4H) ;<br>MS: m/z 456.3 [M+H]$^+$ | 1.64 |
| 174 | 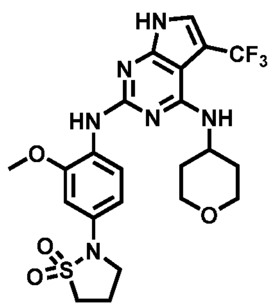 | 2-(3-methoxy-4-((4-((tetrahydro-2*H*-pyran-4-yl)amino)-5 -(trifluoromethyl)-7*H*-pyrrolo[2,3-*d*]pyrimidin-2-yl)amino)phenyl) isot hiazolidine 1,1-dioxide | 1H NMR (400 MHz, DMSO-$d_6$) δ 11.96 (d, J = 2.7 Hz, 1H), 8.25 (d, J = 8.7 Hz, 1H), 7.57 (q, J = 2.2 Hz, 1H), 7.47 (s, 1H), 6.90 (d, J = 2.4 Hz, 1H), 6.81 (dd, J = 8.7, 2.4 Hz, 1H), 5.28 - 5.05 (m, 1H), 4.42 - 4.18 (m, 1H), 3.86 (s, 5H), 3.74 (t, J = 6.6 Hz, 2H), 3.56 - 3.41 (m, 4H), 2.40 (p, J = 6.9 Hz, 2H), 2.00 (dd, J = 13.2, 3.6 Hz, 2H), 1.62 - 1.40 (m, 2H).<br>MS: m/z 527.2 [M+H]$^+$ | 1.51 |

(continued)

| 실시예 화합물 | 구조 | 화합물명 | $^1$H NMR, MS | UPLC r.t. (min) |
|---|---|---|---|---|
| 175 | | 2-(3-methoxy-4-((4-((tetrahydro-2*H*-pyran-4-yl)amino)-5-(trifluoromethyl)-7*H*-pyrrolo [2,3-*d*]pyrimidin-2-yl)amino) phenyl)-1,2-thiazinane 1,1-dioxide | 1H NMR (400 MHz, DMSO-$d_6$) δ 12.48 (s, 1H), 8.79 - 8.29 (m, 1H), 8.19 (d, J = 8.5 Hz, 1H), 7.68 (s, 1H), 6.94 (d, J = 7.6 Hz, 2H), 5.65 (s, 1H), 4.37 - 4.28 (m, 1H), 3.86 (d, J = 3.6 Hz, 3H), 3.64 (t, J = 5.6 Hz, 2H), 3.48 (td, J = 11.4, 2.2 Hz, 2H), 3.34 - 3.25 (m, 2H), 2.16 (ddt, J = 9.0, 6.2, 2.9 Hz, 3H), 2.04 - 1.89 (m, 2H), 1.83 (tt, *J* = 8.1, 4.7 Hz, 3H), 1.62 (qd, J = 11.2, 4.4 Hz, 2H), 1.31 - 1.11 (m, 1H).<br>MS: m/z 541.3 $[M+H]^+$ | 1.61 |
| 176 | 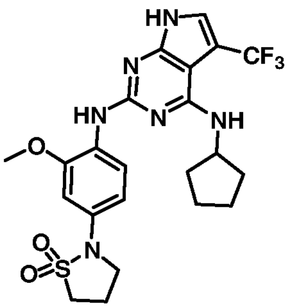 | 2-(4-((4-(cyclopentylamino)-5-(trifluoromethyl)-7*H*-pyrrolo [2,3-*d*]pyrimidin-2-yl)amino)-3-methoxyphenyl)isothi azolidine 1,1-dioxide | 1H NMR (400 MHz, DMSO-$d_6$) δ 11.96 (d, J = 2.8 Hz, 1H), 8.34 (d, J = 8.7 Hz, 1H), 7.55 (t, J = 2.2 Hz, 1H), 7.43 (s, 1H), 6.90 (d, J = 2.4 Hz, 1H), 6.81 (dd, J = 8.7, 2.4 Hz, 1H), 5.16 (dd, J = 7.2, 2.4 Hz, 1H), 4.49 (q, J = 6.5 Hz, 1H), 3.87 (s, 3H), 3.74 (t, J = 6.5 Hz, 2H), 3.48 (t, J = 7.5 Hz, 2H), 2.40 (p, J = 7.0 Hz, 2H), 2.05 (dt, J = 12.2, 6.1 Hz, 2H), 1.66 (ddq, J = 16.2, 7.9, 3.8 Hz, 4H), 1.51 (dt, J = 12.7, 6.2 Hz, 2H).<br>MS: m/z 511.3 $[M+H]^+$ | 1.78 |
| 177 | 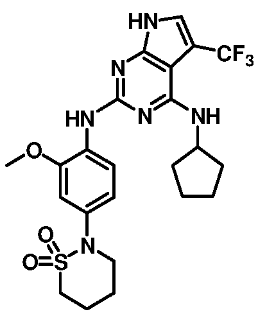 | 2-(4-((4-(cyclopentylamino)-5-(trifluoromethyl)-7*H*-pyrrolo [2,3-*d*]pyrimidin-2-yl)amino)-3-methoxyphenyl)-1,2-thiazinane 1,1-dioxide | 1H NMR (400 MHz, DMSO-$d_6$) δ 12.00 (d, J = 2.8 Hz, 1H), 8.43 (d, J = 9.1 Hz, 1H), 7.58 (s, 1H), 7.48 (s, 1H), 6.91 (dq, J = 5.0, 2.3 Hz, 2H), 5.26 - 5.08 (m, 1H), 4.51 (q, J = 6.5 Hz, 1H), 3.89 (s, 3H), 3.62 (t, J = 5.5 Hz, 2H), 3.29 (t, J = 6.0 Hz, 2H), 2.16 (dd, J = 8.0, 4.4 Hz, 2H), 2.06 (dt, J = 12.3, 6.0 Hz, 2H), 1.82 (p, J = 5.9 Hz, 2H), 1.74 - 1.62 (m, 4H), 1.52 (dt, J = 13.0, 6.3 Hz, 2H).<br>MS: m/z 525.2 $[M+H]^+$ | 1.90 |

**<Experimental Example 1> Evaluation of inhibitory activity of compound according to the present invention against enzymes**

To evaluate an inhibitory activity of the compound according to the present invention against TTK (MPS1), an experiment was performed as follows.

1) TTK enzyme activity

Each of the Example compounds was reacted with a purified human TTK (SignalChem #T20-10G) enzyme to evaluate an ability to inhibit an enzyme using a method as described below. A composition of 40 mM Tris-HCl (pH 7.4), 20 mM $MgCl_2$, 0.5 mg/mL BSA, and 50 μM DTT was used as a reaction buffer, and all reactions of test substances were performed in the reaction buffer. The compound was diluted with 12-fold from a 10 mM DMSO stock using a serial dilution method, and enzyme activity was measured at final compound concentrations of 50, 10, 2, 0.4, 0.08, 0.016, 0.0032, 0.00064, 0.000128, 0.0000256, 0.00000512, and 0.000001024 μM. In the test, purified ATP (10 μM) and an enzyme substrate (0.2 μg) were reacted with a human TTK (25 ng) enzyme at 25°C for 2 hours, and the enzyme activity was determined using an *in vitro* ADP-Glo™ kinase assay (Promega). An enzyme-activity reaction solution, an ADP-Glo reaction solution, and an enzyme-ability detection solution were reacted at a ratio of 2:2:1 to measure a degree of inhibition of the enzyme activity with luminescence. The degree of inhibition of the enzyme activity according to the treatment concentration of each of the compounds was calculated based on the fluorescence of the enzyme activity of the solvent control which was not treated with the compound. In this case, the concentration of each of the compounds inhibiting 50% of the enzyme activity was determined to be an $IC_{50}$ (nM) value, and calculated using Prism (Version 5.01; GraphPad) software. The results are listed in Table 2 below.

In the table below, the following designations are used to evaluate the inhibitory ability against the enzyme.

$$0 - 50\ nM = A;\ 50 - 200\ nM = B;\ 200 - 1{,}000\ nM = C;\ >1{,}000\ nM = D$$

[Table 2]

| Example compound | TTK Activity | Example compound | TTK Activity | Example compound | TTK Activity | Example compound | TTK Activity |
|---|---|---|---|---|---|---|---|
| 1 | A | 50 | B | 97 | A | 138 | A |
| 2 | A | 51 | B | 98 | A | 139 | C |
| 3 | A | 53 | A | 99 | A | 140 | C |
| 7 | A | 54 | B | 100 | B | 141 | A |
| 8 | B | 55 | A | 101 | A | 142 | A |
| 9 | A | 56 | A | 102 | C | 143 | A |
| 10 | A | 57 | B | 103 | C | 144 | A |
| 11 | A | 58 | A | 104 | B | 145 | A |
| 12 | A | 59 | C | 105 | B | 146 | A |
| 13 | A | 60 | B | 106 | B | 147 | A |
| 14 | A | 61 | B | 107 | B | 148 | A |
| 15 | A | 63 | A | 108 | A | 149 | B |
| 16 | A | 64 | A | 109 | A | 150 | B |
| 17 | C | 65 | A | 110 | A | 151 | A |
| 18 | A | 66 | A | 111 | A | 152 | B |
| 19 | B | 67 | A | 112 | A | 153 | B |
| 20 | A | 68 | A | 113 | B | 154 | A |
| 21 | B | 69 | A | 114 | A | 155 | A |

(continued)

| Example compound | TTK Activity | Example compound | TTK Activity | Example compound | TTK Activity | Example compound | TTK Activity |
|---|---|---|---|---|---|---|---|
| 22 | A | 70 | A | 115 | A | 156 | A |
| 23 | B | 71 | A | 116 | A | 157 | A |
| 24 | A | 72 | A | 117 | A | 158 | A |
| 25 | C | 73 | A | 118 | A | 159 | A |
| 26 | B | 75 | A | 119 | A | 162 | A |
| 27 | A | 76 | A | 120 | B | 163 | A |
| 28 | C | 79 | A | 121 | A | 164 | A |
| 29 | A | 82 | A | 122 | A | 165 | A |
| 30 | A | 83 | A | 123 | B | 166 | A |
| 31 | A | 84 | A | 124 | A | 167 | A |
| 32 | A | 85 | A | 125 | A | 168 | A |
| 33 | B | 86 | A | 126 | A | 169 | A |
| 35 | C | 87 | B | 127 | B | 170 | A |
| 38 | A | 88 | B | 128 | B | 171 | A |
| 39 | A | 89 | A | 129 | C | 172 | A |
| 40 | A | 90 | A | 130 | B | 173 | A |
| 41 | B | 91 | A | 131 | B | 174 | A |
| 42 | B | 92 | A | 132 | B | 175 | A |
| 43 | A | 93 | A | 133 | C | 176 | B |
| 44 | B | 94 | A | 134 | A | 177 | B |
| 46 | C | 95 | A | 136 | B | | |
| 49 | A | 96 | A | 137 | B | | |

As shown in Table 2, it was confirmed that the Example compounds of the present invention effectively inhibited TTK kinases. Therefore, the Example compounds of the present invention may be effectively used in the pharmaceutical composition for preventing or treating diseases associated with the TTK kinases.

**<Experimental Example 2> Evaluation of inhibitory activity against proliferation of triple-negative breast cell (TNBC) and cancer cells**

To evaluate a therapeutic effect of the compound represented by Formula 1 according to the present invention on triple-negative breast cancer, an experiment was performed as follows. The results are listed in Table 3.

Specifically, the following test was performed in order to evaluate the inhibitory activity of the compound according to the present invention against the cell proliferation in triple-negative breast cancer cells (TNBCs).

To check an ability to inhibit the growth of (MDA-MB-231) cells in two triple-negative breast cancer cell lines, a test was performed, as follows. Cells were seeded in 180 μL of a culture broth at a density of 2,000 to 3,000 cells/well in a white clear bottom 96-well plate (Corning) according to the growth ability of each cell, and then cultured for 24 hours under conditions of 5% $CO_2$ and 37°C. Thereafter, the compound was prepared by diluting a 10 mM stock solution threefold with 100% DMSO at a total of 12 concentrations so that a peak concentration of the compound was in a range of 10 mM to 0.05 μM, and the diluted compound was then diluted in each cell culture medium so that a final concentration of the compound was in a range of 100 μM to 0.5 nM. 20 μL of a diluted solution diluted with a medium was added to the cell solution in which cells were seeded in the 96-well plate on the previous day so that a final concentration of the diluted solution was in a range of 10 μM to 0.05 nM. Each of the cells was cultured for 5 days. To check cell viability, a mixture provided in a CellTiter-Glo® Luminescent Cell Viability Assay Kit (Promega) was added to each of the cultured

cell media, and cultured for another 10 minutes under a condition of 37°C. Then, a degree of luminescence (fluorescence) was measured. A degree of inhibition of the cell growth according to the treatment concentration of each of the compounds was calculated based on the fluorescence of the solvent control cells which were not treated with the compound. In this case, the concentration of each of the compounds inhibiting 50% of the cell growth was determined to be a $GI_{50}$ (nM) value, and calculated using Prism (Version 7.01; GraphPad) software.

In the table below, the following designations are used to evaluate cell proliferation inhibitory activity.

$$0 - 100 \text{ nM} = \text{A}; 100 - 1{,}000 \text{ nM} = \text{B}; >1{,}000 \text{ nM} = \text{C};$$

[Table 3]

| Example compound | MDA-MB-231 Activity | Example compound | MDA-MB-231 Activity | Example compound | MDA-MB-231 Activity | Example compound | MDA-MB-231 Activity |
|---|---|---|---|---|---|---|---|
| 1 | A | 46 | C | 92 | A | 134 | A |
| 2 | A | 49 | A | 93 | A | 136 | A |
| 3 | B | 50 | A | 94 | A | 137 | A |
| 4 | B | 51 | C | 95 | B | 138 | A |
| 5 | C | 53 | B | 96 | A | 139 | B |
| 6 | C | 54 | B | 97 | A | 140 | A |
| 7 | A | 55 | A | 98 | A | 141 | A |
| 9 | C | 56 | A | 99 | A | 142 | B |
| 10 | A | 57 | B | 100 | A | 143 | A |
| 11 | A | 58 | A | 101 | A | 144 | A |
| 12 | C | 59 | A | 102 | B | 145 | A |
| 14 | B | 60 | A | 103 | B | 146 | A |
| 16 | A | 61 | A | 104 | B | 147 | A |
| 17 | A | 63 | B | 105 | B | 148 | A |
| 18 | A | 64 | A | 106 | A | 149 | A |
| 19 | A | 65 | A | 107 | A | 150 | A |
| 20 | A | 66 | A | 108 | B | 151 | A |
| 21 | B | 67 | A | 109 | A | 152 | A |
| 22 | B | 68 | A | 110 | A | 153 | A |
| 23 | A | 69 | C | 111 | A | 154 | A |
| 24 | A | 70 | C | 112 | B | 155 | A |
| 25 | A | 71 | B | 113 | B | 156 | A |
| 26 | A | 72 | B | 114 | B | 157 | A |
| 27 | A | 73 | A | 115 | A | 158 | A |
| 28 | C | 74 | C | 116 | A | 159 | A |
| 29 | B | 75 | A | 117 | A | 160 | C |
| 30 | A | 76 | A | 118 | A | 161 | C |
| 31 | A | 77 | B | 119 | A | 162 | A |
| 32 | A | 78 | B | 120 | B | 163 | A |
| 33 | A | 79 | A | 121 | A | 164 | A |

(continued)

| Example compound | MDA-MB-231 Activity | Example compound | MDA-MB-231 Activity | Example compound | MDA-MB-231 Activity | Example compound | MDA-MB-231 Activity |
|---|---|---|---|---|---|---|---|
| 34 | A | 80 | A | 122 | A | 165 | A |
| 35 | B | 81 | A | 123 | C | 166 | A |
| 36 | A | 82 | A | 124 | A | 167 | A |
| 37 | A | 83 | B | 125 | A | 168 | A |
| 38 | A | 84 | C | 126 | C | 169 | A |
| 39 | A | 85 | B | 127 | A | 170 | A |
| 40 | A | 86 | A | 128 | B | 171 | A |
| 41 | B | 87 | A | 129 | B | 172 | A |
| 42 | A | 88 | A | 130 | B | 173 | B |
| 43 | A | 89 | A | 131 | A | 174 | A |
| 44 | A | 90 | A | 132 | B | 175 | A |
| 45 | A | 91 | A | 133 | B | 176 | B |
| | | | | | | 177 | B |

As shown in Table 3, it can be seen that the Example compounds according to the present invention inhibited the proliferation of the triple-negative breast cancer cells.

Next, the activities of the Example compounds against various types of carcinomas other than the TNBC were evaluated in a similar manner as described above. The results are listed in Table 4 below. In the following table, the following active value range ($GI_{50}$ (nM)) was used to evaluate cell proliferation inhibitory activity.

$$0 - 100\ nM = A;\ 100 - 1{,}000\ nM = B;\ >1{,}000\ nM = C;$$

[Table 4]

| Example compound | HCT116 | BxPC3 | DU145 | A549 |
|---|---|---|---|---|
| 47 | A | A | A | A |
| 48 | A | A | A | A |
| 52 | B | B | B | B |
| 53 | B | B | B | B |
| 54 | B | B | B | B |
| 55 | A | A | A | A |
| 56 | A | A | A | A |
| 62 | B | B | B | B |
| 63 | A | A | A | A |
| 64 | A | A | A | A |

As shown in Table 4, it was confirmed that the Example compounds according to the present invention had activity against various solid cancers other than TNBC as previously described above, and since TTK is involved in the cell division cycle in cells, the Example compounds according to the present invention can be expected to inhibit the proliferation of various cells in blood cancer, brain cancer, and the like.

**<Experimental Example 3> Evaluation of inhibitory activity against proliferation of small cell lung cancer cells (SCLCs) and cancer cells**

To evaluate a therapeutic effect of the compound represented by Formula 1 according to the present invention on SCLCs, an experiment was performed as follows. The results are listed in Table 5.

Specifically, the following test was performed to evaluate the inhibitory activity of the compound according to the present invention against the cell proliferation in the SCLC cells.

To check an inhibitory ability of cell growth in one SCLC cell line, a test was performed as follows. Cells were seeded in 180 μL of a culture broth at a density of 2,000 to 3,000 cells/well in a white clear bottom 96-well plate (Corning) according to the growth ability of each cell, and then cultured for 24 hours under conditions of 5% $CO_2$ and 37°C. Thereafter, the compound was prepared by diluting a 10 mM stock solution three-fold with 100% DMSO at a total of 12 concentrations so that a peak concentration of the compound was in a range of 10 mM to 0.05 μM, and the diluted compound was then diluted in each cell culture medium so that a final concentration of the compound was in a range of 100 μM to 0.5 nM. 20 μL of a diluted solution diluted with a medium was added to the cell solution in which cells were seeded in the 96-well plate on the previous day so that a final concentration of the diluted solution was in a range of 10 μM to 0.05 nM. Each of the cells was cultured for 5 days. To check cell viability, a mixture provided in aCellTiter-Glo® Luminescent Cell Viability Assay Kit (Promega) was added to each of the cultured cell media, and cultured for another 10 minutes under a condition of 37°C. Then, a degree of luminescence (fluorescence) was measured. A degree of inhibition of the cell growth according to the treatment concentration of each of the compounds was calculated based on the fluorescence of the solvent control cells which were not treated with the compound. In this case, the concentration of each of the compounds inhibiting 50% of the cell growth was determined to be a $GI_{50}$ (nM) value, and calculated using Prism (Version 7.01; GraphPad) software.

In the table below, the following designations are used to evaluate cell proliferation inhibitory activity.

$$0 - 100\ nM = A;\ 100 - 1{,}000\ nM = B;\ >1000\ nM = C;$$

[Table 5]

| Example compound | SHP-77 Activity | Example compound | SHP-77 Activity | Example compound | SHP-77 Activity | Example compound | SHP-77 Activity |
|---|---|---|---|---|---|---|---|
| 16 | A | 99 | A | 126 | C | 154 | A |
| 29 | B | 100 | A | 127 | A | 155 | A |
| 38 | A | 101 | A | 128 | A | 156 | A |
| 40 | A | 102 | B | 129 | B | 157 | A |
| 49 | A | 103 | B | 130 | B | 158 | A |
| 50 | A | 104 | B | 131 | A | 159 | A |
| 56 | A | 105 | B | 132 | B | 160 | C |
| 57 | A | 106 | A | 133 | B | 161 | C |
| 58 | A | 107 | A | 134 | A | 162 | A |
| 59 | A | 108 | B | 136 | A | 163 | A |
| 60 | A | 109 | A | 137 | A | 164 | A |
| 61 | A | 110 | A | 138 | A | 165 | A |
| 83 | B | 111 | A | 139 | A | 166 | A |
| 84 | C | 112 | A | 140 | A | 167 | A |
| 85 | A | 113 | B | 141 | A | 168 | A |
| 87 | A | 114 | B | 142 | B | 169 | A |
| 88 | A | 115 | A | 143 | A | 170 | A |
| 89 | A | 116 | A | 144 | A | 171 | A |

(continued)

| Example compound | SHP-77 Activity | Example compound | SHP-77 Activity | Example compound | SHP-77 Activity | Example compound | SHP-77 Activity |
|---|---|---|---|---|---|---|---|
| 90 | A | 117 | A | 145 | A | 172 | A |
| 91 | A | 118 | A | 146 | A | 173 | A |
| 92 | A | 119 | A | 147 | A | 174 | A |
| 93 | A | 120 | B | 148 | A | 175 | A |
| 94 | A | 121 | A | 149 | A | 176 | B |
| 95 | A | 122 | A | 150 | A | 177 | B |
| 96 | A | 123 | B | 151 | A | | |
| 97 | A | 124 | A | 152 | A | | |
| 98 | A | 125 | B | 153 | A | | |

As shown in Table 5, it can be seen that the Example compounds according to the present invention inhibited the proliferation of the SCLC cells.

**<Experimental Example 4> Evaluation of inhibitory activity of compound according to the present invention against various kinases**

To evaluate the inhibitory activity of the compound according to the present invention against more enzymes, an experiment was performed as follows.

Specifically, the enzyme (kinase) selectivity of the compound of Example 7 selected from the Example compounds of the present invention was measured by commissioning DiscoverX Corp., and an experiment was performed using a panel for ScanMAX™ Kinase analysis.

In this case, the concentration of a drug treated with the enzyme was set at 1 μM in DMSO, and percent control (% control) was defined in the same manner as in the following Expression 1. The results are listed in Table 6 below.

[Expression 1]

(Example compound - Positive control)/(Negative control - Positive control) X 100

Wherein the positive control represents a compound having a percent control of 0%, and the negative control represents a compound having a percent control of 100% in DMSO. For the enzyme selectivity of the present invention, the compound was also judged to have activity against the corresponding enzyme when the percent control for each of the enzymes was < 30% (i.e., less than 30%).

[Table 6]

| Kinase | Example 7 compound (% Cont @ 1 μM) |
|---|---|
| TTK | 0.25 |
| JNK1 | 2 |
| LRRK2(G2019S) | 2.7 |
| JNK3 | 4.3 |
| LRRK2 | 4.8 |
| FLT3(D835V) | 5.6 |
| JNK2 | 13 |
| RIPK5 | 17 |
| MEK3 | 18 |

(continued)

| Kinase | Example 7 compound (% Cont @ 1 μM) |
|---|---|
| ABL1(F317L)-nonphosphorylated | 28 |
| MAPKAPK2 | 28 |
| GRK4 | 29 |
| SRPK3 | 30 |

Further, to evaluate the inhibitory activity of the compound according to the present invention against more enzymes, an experiment was performed as follows. Specifically, the enzyme (kinase) selectivity of the compound of Example 64 selected from the Example compounds of the present invention was measured by commissioning DiscoverX Corp., and an experiment was performed using a panel for ScanMAX™ Kinase analysis. In this case, the concentration of a drug treated with the enzyme was set at 1 μM in DMSO, and the percent control (% control) were defined in the same manner as in the following Expression 1. The results are listed in Table 7 below.

[Expression 1]

$$(\text{Example compound - Positive control})/(\text{Negative control - Positive control}) \times 100$$

Wherein the positive control represents a compound having a percent control of 0%, and the negative control represents a compound having a percent control of 100% in DMSO. For the enzyme selectivity of the present invention, the compound was also judged to have activity against the corresponding enzyme when the percent control for each of the enzymes was < 35% (i.e., less than 35%).

[Table 7]

| Kinase | Example 64 compound (% Cont @ 1 μM) | Kinase | Example 64 compound (% Cont @ 1 μM) |
|---|---|---|---|
| JAK2(JH1domain-catalytic) | 0 | SBK1 | 5.7 |
| SNARK | 0 | TYK2(JH2domain-pseudokinase) | 7.2 |
| TTK | 0.25 | CAMK2D | 12 |
| YSK4 | 0.3 | MAP3K2 | 13 |
| JNK1 | 0.9 | KIT(V559D,V654A) | 14 |
| FLT3(ITD,D835V) | 1.2 | LRRK2 | 14 |
| PRKCE | 1.4 | FLT3(ITD) | 17 |
| CAMKK1 | 1.6 | LRRK2(G2019S) | 17 |
| JNK3 | 1.7 | CSNK1D | 19 |
| TYK2(JH1domain-catalytic) | 1.7 | CSNK1E | 24 |
| RSK2(Kin.Dom.1-N-terminal) | 1.9 | ABL1 (F317L)-nonphosphorylated | 26 |
| CAMKK2 | 2.2 | GRK4 | 26 |
| ULK3 | 2.6 | MEK4 | 27 |
| ULK1 | 3.1 | RIOK1 | 27 |
| RSK4(Kin.Dom.1-N-terminal) | 3.7 | DYRK1B | 28 |

(continued)

| Kinase | Example 64 compound (% Cont @ 1 μM) | Kinase | Example 64 compound (% Cont @ 1 μM) |
|---|---|---|---|
| TRKB | 3.9 | MAPKAPK2 | 28 |
| JNK2 | 4.1 | PKN2 | 28 |
| AAK1 | 4.4 | SRPK3 | 28 |
| GAK | 4.4 | RIPK5 | 30 |

**<Experimental Example 5> Measurement of activities of some kinases based on Experimental Example 3**

To evaluate the inhibitory activity of the compound according to the present invention against LRRK2 and JNKs, an experiment was performed as follows.

1) LRRK2 enzyme activity

The compound of Example 7 was reacted with a purified human LRRK2 (Invitrogen #PR8604B) enzyme to evaluate an ability of the compound of Example 7 to inhibit the enzyme using a method as described below. A composition of 40 mM Tris-HCl (pH 7.4), 20 mM $MgCl_2$, 0.5 mg/mL BSA, and 50 μM DTT was used as a reaction buffer, and all reactions of test substances were performed in the reaction buffer. The compound was diluted 12-fold from a 10 mM DMSO stock using a serial dilution method, and enzyme activity was measured at final compound concentrations of 50, 10, 2, 0.4, 0.08, 0.016, 0.0032, 0.00064, 0.000128, 0.0000256, 0.00000512, and 0.000001024 μM. In the test, purified ATP (10 μM) and an enzyme substrate (0.2 μg) were reacted with a human LRRK2 (25 ng) enzyme at 25°C for 2 hours, and the enzyme activity was then determined using an *in vitro* ADP-Glo™ kinase assay (Promega). An enzyme-activity reaction solution, an ADP-Glo reaction solution, and an enzyme-ability detection solution were reacted at a ratio of 2:2:1 to measure a degree of inhibition of the enzyme activity with luminescence. The degree of inhibition of the enzyme activity according to the treatment concentration of each of the compounds was calculated based on the fluorescence of the enzyme activity of the solvent control which was not treated with the compound. In this case, the concentration of each of the compounds inhibiting 50% of the enzyme activity was determined to be an $IC_{50}$ (nM) value, and calculated using Prism (Version 5.01; GraphPad) software. The results are listed in Table 8 below.

2) JNK1, 2, and 3 enzyme activities

$IC_{50}$ values of the compound of Example 7 for the enzymes were measured using the Kinase HotSpot service (Reaction Biology Corporation), and a test was performed under the same conditions at an ATP concentration of 10 μM. The concentration of the compound was measured at a 3-fold concentration gradient from the peak concentration of 10 mM.

All the experimental methods were performed as provided in the Kinase HotSpot Customer Protocol (http://www.reactionbiology.com//Kinase_Assay_Protocol). The results of experiments are listed in Table 8 below.

[Table 8]

| Enzyme | Activity |
|---|---|
| LRRK2 | < 1,000 nM |
| JNK1 | <100 nM |
| JNK2 | <100 nM |
| JNK3 | <100 nM |

While the present invention has been described in detail with reference to preferred preparation examples, examples, and experimental examples, the scope of the present disclosure is not limited to specific examples and should be interpreted by the appended claims. Also, it will be understood by those of ordinary skill in the art that changes and modifications can be made in various ways without departing from the scope of the present invention.

**Claims**

1. A compound represented by the following Formula 1, or an isomer thereof, a solvate thereof, a hydrate thereof, or a pharmaceutically acceptable salt thereof:

[Formula 1]

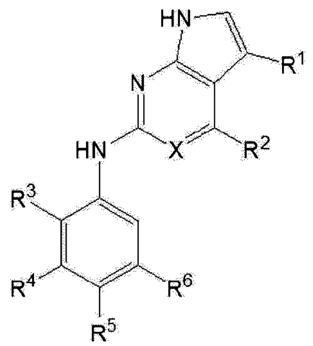

(wherein,

X is CH or N;
$R^1$ is -H, a halogen, a cyano, or a haloalkyl;
$R^2$ is a $C_{3-10}$ cycloalkyl, a $C_{3-10}$ cycloalkenyl, $-NHA^1$, or $-OA^2$;
wherein $A^1$ is a $C_{1-10}$ linear or branched alkyl, a $C_{3-10}$ cycloalkyl, or a 3- to 9-membered heterocycloalkyl including one or more heteroatoms selected from the group consisting of N, O, and S, wherein the alkyl, the cycloalkyl, and the heterocycloalkyl are each independently unsubstituted or substituted with one or more non-hydrogen substituents selected from the group consisting of a halogen, a $C_{1-5}$ linear or branched alkyl, a $C_{3-10}$ cycloalkyl, a $C_{1-4}$ linear or branched alkylsulfonyl, a $C_{1-4}$ alkylaminosulfonyl, and a $C_{1-5}$ linear or branched alkoxy;
$A^2$ is a $C_{3-10}$ cycloalkyl, wherein the cycloalkyl is unsubstituted or substituted with one or more non-hydrogen substituents selected from a $C_{1-3}$ linear or branched alkyl and hydroxy;
$R^3$ is -H, a $C_{1-6}$ linear or branched alkoxy, or acrylamide, wherein the $C_{1-6}$ linear or branched alkoxy is unsubstituted or substituted with a haloalkyl, and the $R^4$ is -H or a $C_{1-6}$ linear or branched alkoxy, or
$R^3$ and $R^4$, together with a benzene ring containing carbon atoms to which they are bonded, form a 9- to 10-membered bicyclic ring comprising one or more heteroatoms selected from the group consisting of N, O, and S;
$R^5$ is -H, a $C_{1-6}$ alkylaminocarbonyl, unsubstituted or substituted phenyl, oxooxazolidinonyl, dioxidothiazolidinyl, oxopyrrolidinyl, dioxidothiazinanyl, oxomorpholinyl, or a heteroaryl selected from the group consisting of pyrazolyl, triazolyl, thiazolyl, oxazolyl, pyridinyl, and imidazolyl, wherein the heteroaryl may be unsubstituted or substituted with one or more non-hydrogen substituents selected from the group consisting of a $C_{1-5}$ alkyl, a halogen, and a 3- to 7-membered heterocycloalkyl comprising one or more heteroatoms selected from the group consisting of N, O, and S, or may be fused with a $C_{3-10}$ cycloalkyl to form a bicyclic ring, and the substituted phenyl is substituted with hydroxy, or sequentially substituted with a $C_{1-5}$ alkyl, a 3- to 7-membered heterocycloalkyl comprising one or more heteroatoms selected from the group consisting of N, O, and S, and a $C_{3-10}$ cycloalkyl or alkylcarbonyl; and
$R^6$ is -H, a halogen, or a $C_{1-10}$ linear or branched alkyl).

2. The compound of Formula 1, or the isomer thereof, the solvate thereof, the hydrate thereof, or the pharmaceutically acceptable salt thereof of claim 1, wherein $R^2$ is a $C_{3-8}$ cycloalkyl, a $C_{3-6}$ cycloalkenyl, $-NHA^1$, or $-OA^2$,

$A^1$ is a $C_{1-6}$ linear or branched alkyl, a $C_{3-7}$ cycloalkyl, or a 3- to 6-membered heterocycloalkyl comprising one or more O atoms,
wherein, when $A^1$ is a $C_{1-6}$ linear or branched alkyl, the alkyl is unsubstituted or substituted with one or more non-hydrogen substituents selected from the group consisting of a $C_{3-6}$ cycloalkyl, a $C_{1-3}$ linear or branched alkylsulfonyl, a $C_{1-3}$ alkylaminosulfonyl, and a $C_{1-3}$ linear or branched alkoxy,
when $A^1$ is a $C_{3-7}$ cycloalkyl, the cycloalkyl is unsubstituted or substituted with one or more fluoro moieties, and when $A^1$ is a 3- to 6-membered heterocycloalkyl comprising one or more O atoms, the heterocycloalkyl is unsubstituted or substituted with one or more $C_{1-3}$ linear alkyl moieties, and

$A^2$ is a $C_{3-6}$ cycloalkyl, wherein the cycloalkyl is unsubstituted or substituted with one or more non-hydrogen substituents selected from a $C_{1-3}$ linear or branched alkyl and a hydroxyl group.

**3.** The compound of Formula 1, or the isomer thereof, the solvate thereof, the hydrate thereof, or the pharmaceutically acceptable salt thereof of claim 1, wherein $R^2$ is

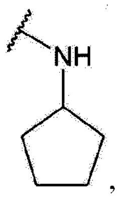,

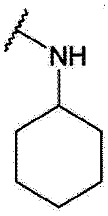, 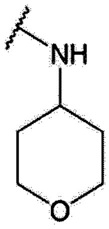, 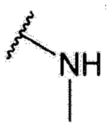, 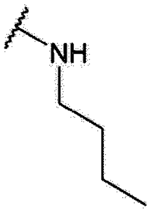, 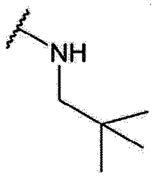, 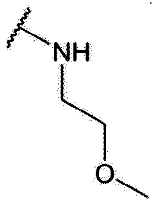, 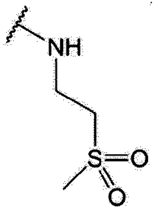,

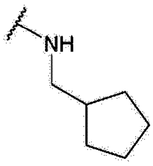, 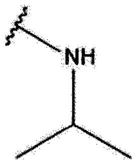, 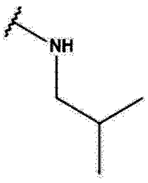, 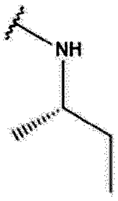, 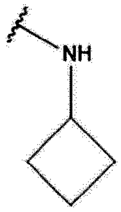, 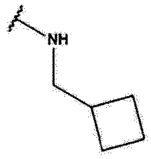, 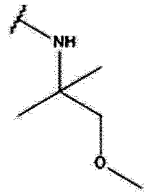,

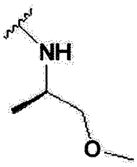, 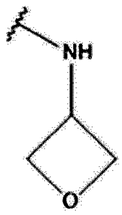, 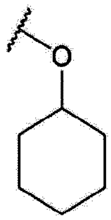, 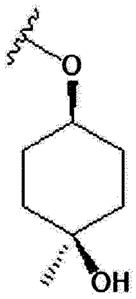, 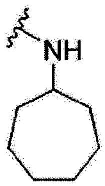, 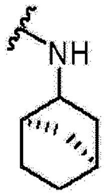, 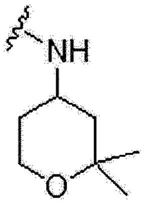,

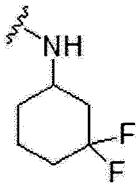, 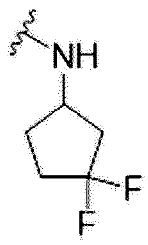, 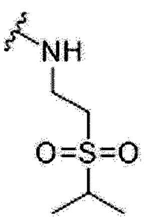, 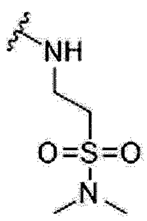, 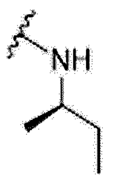, 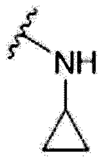, 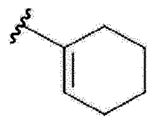,

or

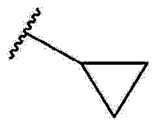.

4. The compound of Formula 1, or the isomer thereof, the solvate thereof, the hydrate thereof, or the pharmaceutically acceptable salt thereof of claim 1, wherein $R^3$ is -H, a $C_{1-4}$ linear or branched alkoxy, or acrylamide, wherein the $C_{1-4}$ linear alkoxy is unsubstituted or substituted with trifluoromethyl, and $R^4$ is -H or a $C_{1-3}$ linear alkoxy, or $R^3$ and $R^4$, together with a benzene ring containing carbon atoms to which they are bonded, form a 9- and 10-membered bicyclic ring comprising one or more O atoms.

5. The compound of Formula 1, or the isomer thereof, the solvate thereof, the hydrate thereof, or the pharmaceutically acceptable salt thereof of claim 4, wherein $R^3$ and $R^4$, together with a benzene ring containing carbon atoms to which they are bonded, form a 9- and 10-membered bicyclic ring comprising one or more O atoms, and the 9- to 10-membered bicyclic ring is dihydrobenzodioxin or dihydrobenzofuran.

6. The compound of Formula 1, or the isomer thereof, the solvate thereof, the hydrate thereof, or the pharmaceutically acceptable salt thereof of claim 1, wherein $R^5$ is -H, a $C_{1-3}$ alkylaminocarbonyl, unsubstituted or substituted phenyl, oxooxazolidinonyl, dioxidothiazolidinyl, oxopyrrolidinyl, dioxidothiazinanyl, oxomorpholinyl, or a heteroaryl selected from the group consisting of pyrazolyl, triazolyl, thiazolyl, oxazolyl, pyridinyl, and imidazolyl, wherein the heteroaryl may be unsubstituted or substituted with one or more non-hydrogen substituents selected from the group consisting of a $C_{1-3}$ alkyl, fluoro, and a 4- to 6-membered heterocycloalkyl comprising one or more O atoms, or may be fused with a $C_{3-5}$ cycloalkyl to form a bicyclic ring, and the substituted phenyl is substituted with hydroxy or a $C_{1-3}$ alkyl, wherein the $C_{1-3}$ alkyl is substituted with acetylpiperazine or a $C_{3-6}$ cycloalkylpiperazine.

7. The compound of Formula 1, or the isomer thereof, the solvate thereof, the hydrate thereof, or the pharmaceutically acceptable salt thereof of claim 1, wherein $R^5$ is -H,

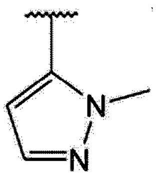, 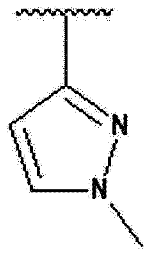, 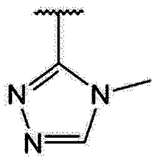, 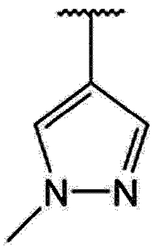, 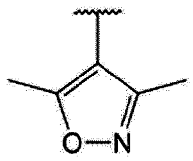, 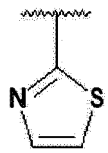,

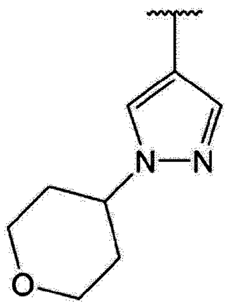, 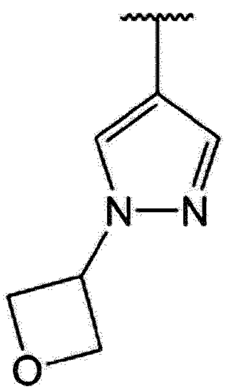, 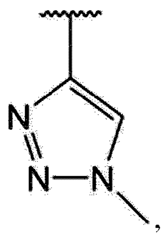, 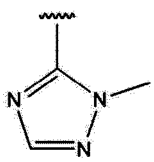, 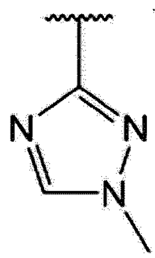, 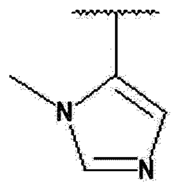,

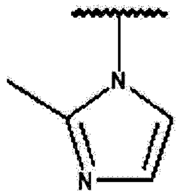, 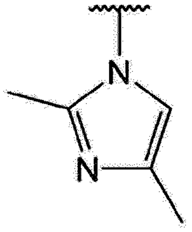, 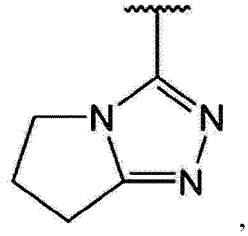, 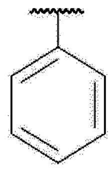, 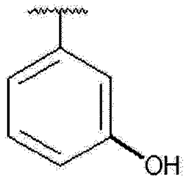, 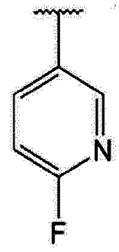,

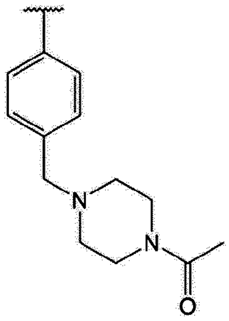, 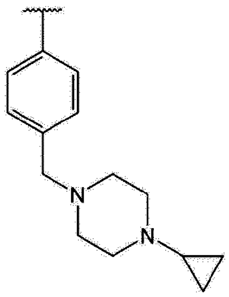, 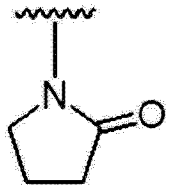, 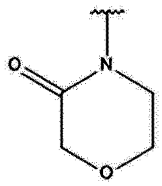, 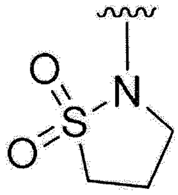,

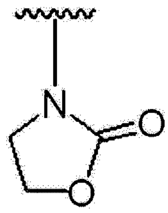, 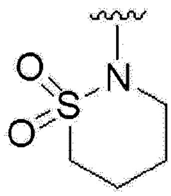, or 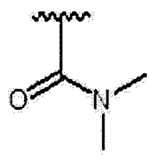.

**8.** The compound of Formula 1, or the isomer thereof, the solvate thereof, the hydrate thereof, or the pharmaceutically acceptable salt thereof of claim 1, wherein, when X is CH,

$R^1$ is cyano or trifluoromethyl;
$R^2$ is -NH$A^1$, wherein $A^1$ is a $C_{3-7}$ cycloalkyl;
$R^3$ is a $C_{1-6}$ linear or branched alkoxy, and $R^4$ is -H, or $R^3$ and $R^4$, together with a benzene ring containing carbon atoms to which they are bonded, form a 9- and 10-membered bicyclic ring comprising one or more O atoms;
$R^5$ is oxopyrrolidinyl; and
$R^6$ is -H.

**9.** The compound of Formula 1, or the isomer thereof, the solvate thereof, the hydrate thereof, or the pharmaceutically acceptable salt thereof of claim 1, wherein, when X is N,

$R^1$ is -H, chloro, fluoro, bromo, iodo, cyano, or trifluoromethyl,
$R^2$ is a $C_{3-7}$ cycloalkyl, cyclohexenyl, -NH$A^1$, or -O$A^2$,
$A^1$ is a $C_{1-6}$ linear or branched alkyl, a $C_{3-7}$ cycloalkyl, or a 3- to 6-membered heterocycloalkyl comprising one or more O atoms,
wherein, when $A^1$ is a $C_{1-6}$ linear or branched alkyl, the alkyl is unsubstituted or substituted with one or more non-hydrogen substituents selected from the group consisting of a $C_{3-6}$ cycloalkyl, a $C_{1-3}$ linear or branched alkylsulfonyl, a $C_{1-3}$ alkylaminosulfonyl, and a $C_{1-3}$ linear or branched alkoxy,
when $A^1$ is a $C_{3-7}$ cycloalkyl, the cycloalkyl is unsubstituted or substituted with one or more fluoro moieties, and when $A^1$ is a 3- to 6-membered heterocycloalkyl comprising one or more O atoms, the heterocycloalkyl is unsubstituted or substituted with one or more $C_{1-3}$ linear alkyl moieties,
$A^2$ is a $C_{3-6}$ cycloalkyl, wherein the cycloalkyl is unsubstituted or substituted with one or more non-hydrogen substituents selected from a $C_{1-3}$ linear or branched alkyl and a hydroxyl group,
$R^3$ is -H, a $C_{1-4}$ linear or branched alkoxy, or acrylamide, wherein the $C_{1-4}$ linear alkoxy is unsubstituted or

substituted with trifluoromethyl, and $R^4$ is -H or a $C_{1-3}$ linear alkoxy, or
$R^3$ and $R^4$, together with a benzene ring containing carbon atoms to which they are bonded, form a 9- to 10-membered bicyclic ring comprising one or more O atoms,
$R^5$ is -H, a $C_{1-3}$ alkylaminocarbonyl, unsubstituted or substituted phenyl, oxooxazolidinonyl, dioxidothiazolidinyl, oxopyrrolidinyl, dioxidothiazinanyl, oxomorpholinyl, or a heteroaryl selected from the group consisting of pyrazolyl, triazolyl, thiazolyl, oxazolyl, pyridinyl, and imidazolyl, wherein the heteroaryl may be unsubstituted or substituted with one or more non-hydrogen substituents selected from the group consisting of a $C_{1-3}$ alkyl, fluoro, and a 4- to 6-membered heterocycloalkyl comprising one or more O atoms, or may be fused with a $C_{3-5}$ cycloalkyl to form a bicyclic ring, and the substituted phenyl is substituted with hydroxy or a $C_{1-3}$ alkyl, wherein the $C_{1-3}$ alkyl is substituted with acetylpiperazine or a $C_{3-6}$ cycloalkylpiperazine, and
$R^6$ is -H, a halogen, or a $C_{1-3}$ linear alkyl.

**10.** The compound of Formula 1, or the isomer thereof, the solvate thereof, the hydrate thereof, or the pharmaceutically acceptable salt thereof of claim 1, wherein, when X is N,
$R^1$ is -H, chloro, cyano, or trifluoromethyl,
$R^2$ is

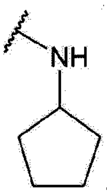, 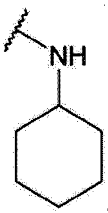, 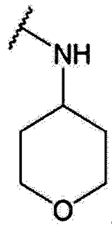, 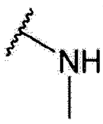, 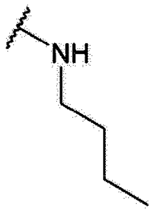, 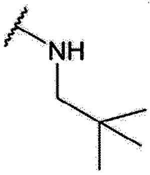, 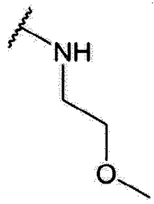,

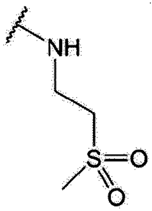, 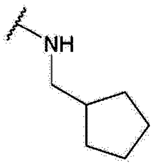, 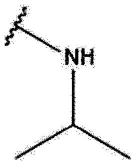, 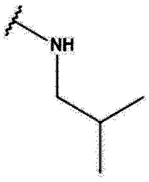, 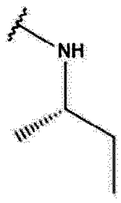, 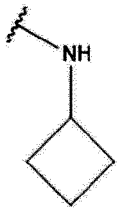, 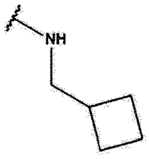,

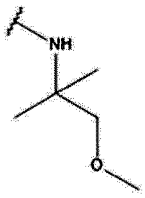, 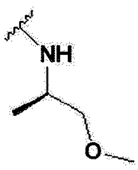, 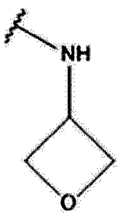, 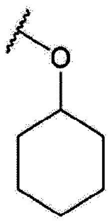, 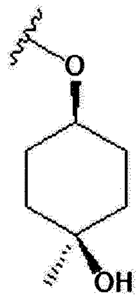, 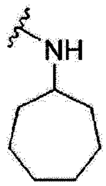, 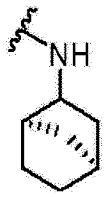,

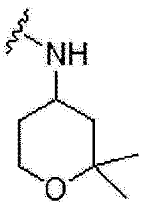, 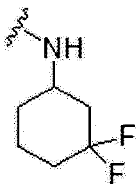, 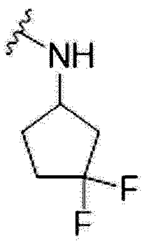, 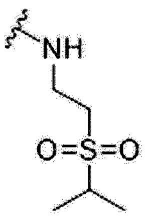, 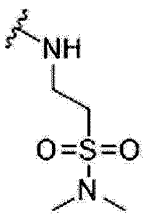, 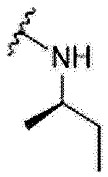, 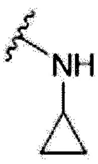,

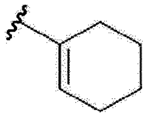, or 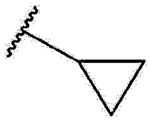,

$R^3$ is -H, a $C_{1-4}$ linear or branched alkoxy, or acrylamide, wherein the $C_{1-4}$ linear alkoxy is unsubstituted or substituted with trifluoromethyl,
$R^4$ is -H or a $C_{1-3}$ linear alkoxy, or
$R^3$ and $R^4$, together with a benzene ring containing carbon atoms to which they are bonded, form a 9- to 10-membered bicyclic ring comprising one or more O atoms, and the 9-to 10-membered bicyclic ring is dihydrobenzodioxin or dihydrobenzofuran,
$R^5$ is -H,

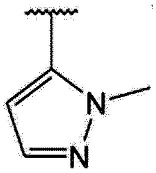, 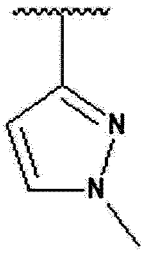, 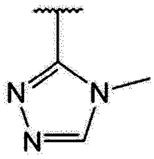, 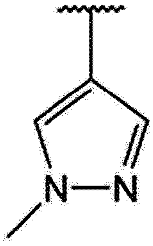, 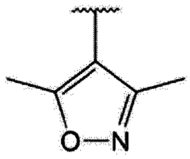,

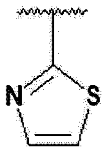, 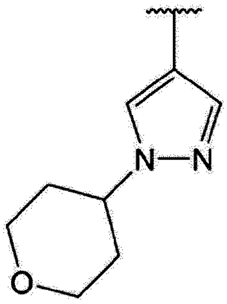, 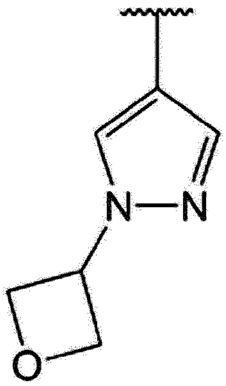, 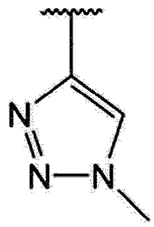, 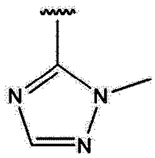, 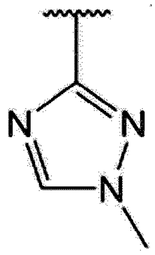,

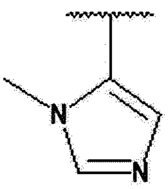, 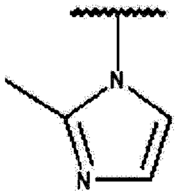, 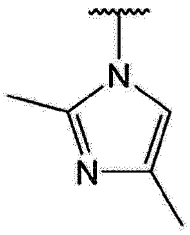, 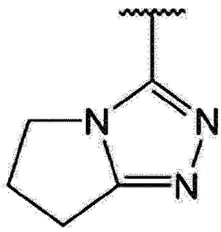, 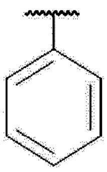, 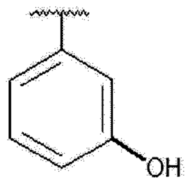,

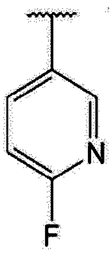, 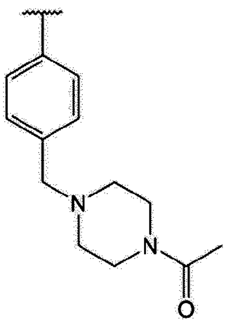, 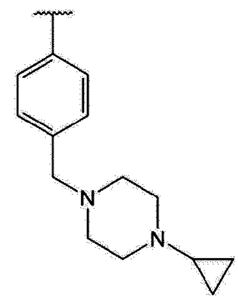, 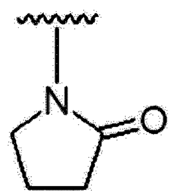, 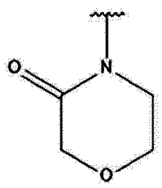,

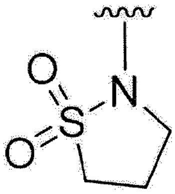, 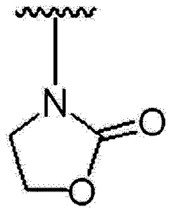, 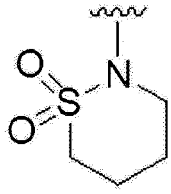, or 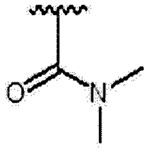,

and
$R^6$ is -H, a halogen, or a $C_{1-3}$ linear alkyl.

**11.** The compound of Formula 1, or the isomer thereof, the solvate thereof, the hydrate thereof, or the pharmaceutically acceptable salt thereof of claim 10, wherein, when X is N, and $R^3$ and $R^4$, together with a benzene ring containing carbon atoms to which they are bonded, form a bicyclic ring of dihydrobenzodioxin or dihydrobenzofuran,

$R^1$ is -H, chloro, cyano, or trifluoromethyl,
$R^2$ is

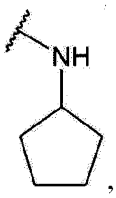, 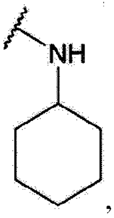, 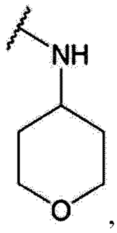, 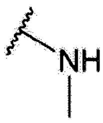, 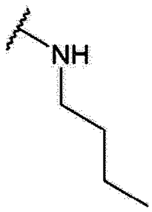, 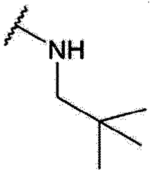, 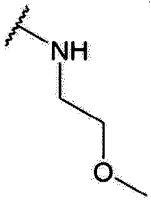,

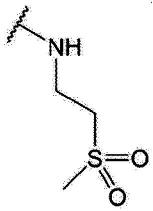, 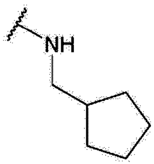, 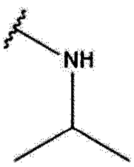, 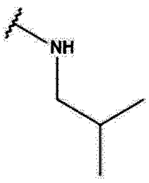, 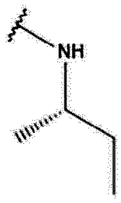, 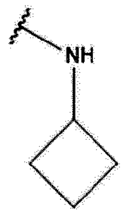, 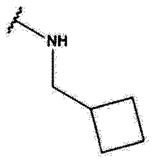,

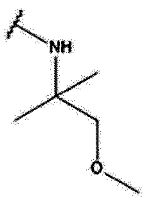, 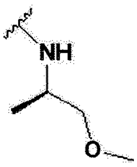, 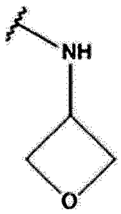, 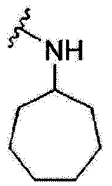, 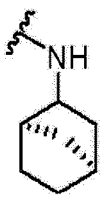, 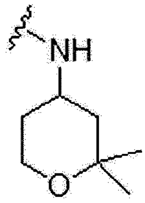, 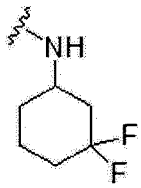,

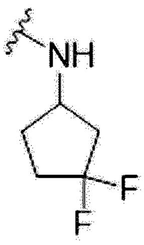, 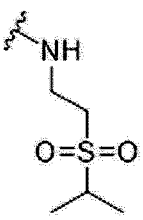, 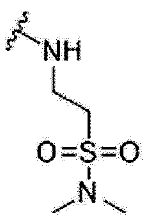, 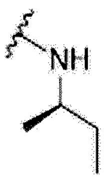, 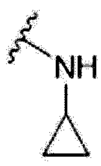, 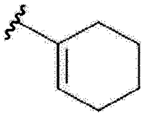, or 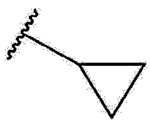,

$R^5$ is -H,

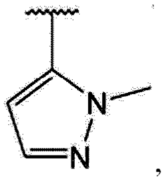, 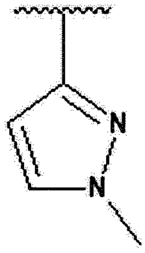, 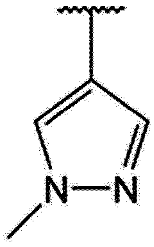, or 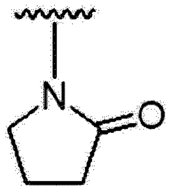

, and
$R^6$ is -H.

**12.** The compound of Formula 1, or the isomer thereof, the solvate thereof, the hydrate thereof, or the pharmaceutically acceptable salt thereof of claim 10, wherein, when X is N, $R^3$ is -H, a $C_{1-4}$ linear or branched alkoxy, or acrylamide, wherein the $C_{1-4}$ linear alkoxy is unsubstituted or substituted with trifluoromethyl, and $R^4$ is -H or a $C_{1-3}$ linear alkoxy,

$R^1$ is -H, chloro, cyano, or trifluoromethyl,
$R^2$ is

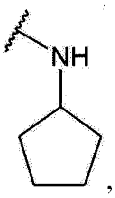, 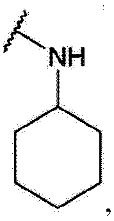, 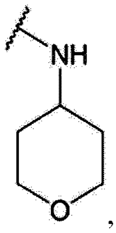, 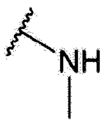, 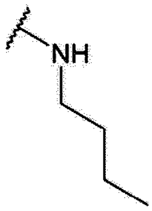, 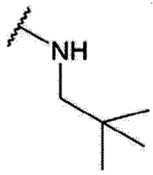, 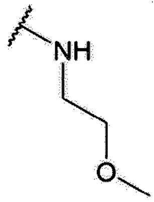,

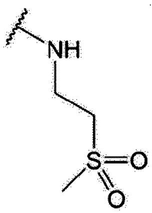, 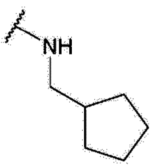, 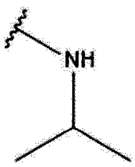, 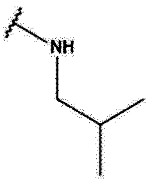, 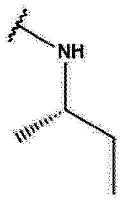, 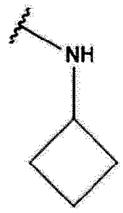, 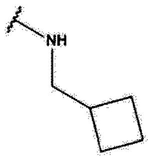,

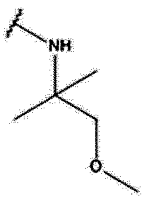, 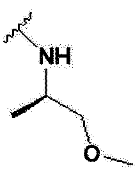, 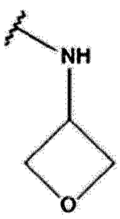, 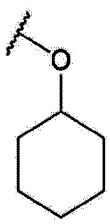, 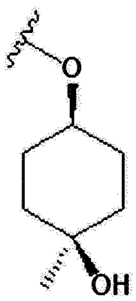, 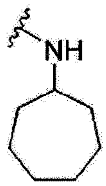, 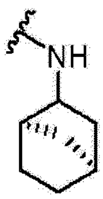,

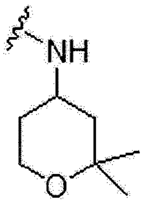, 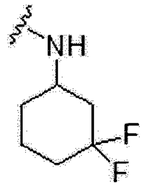, 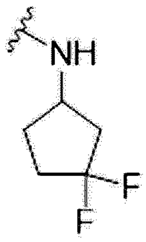, 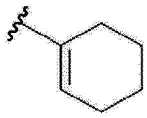, or 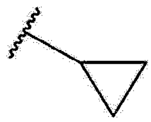,

$R^5$ is -H,

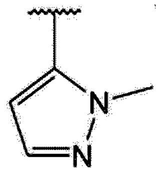, 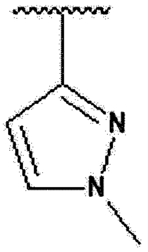, 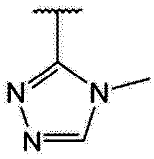, 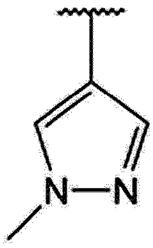, 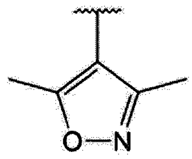,

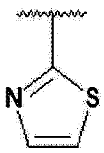, 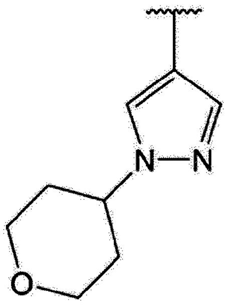, 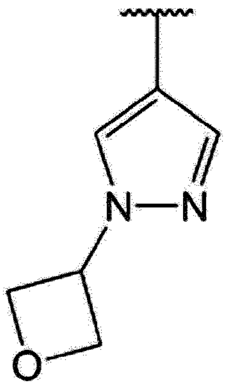, 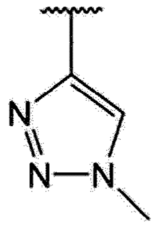, 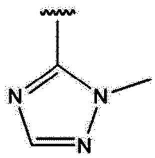, 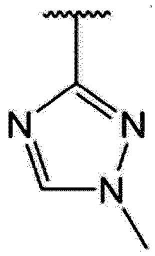,

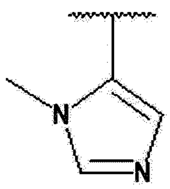, 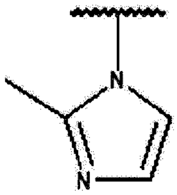, 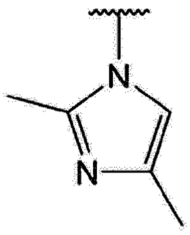, 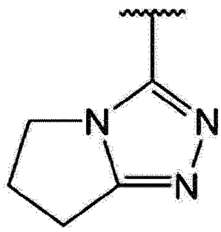, 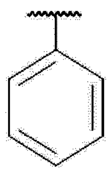, 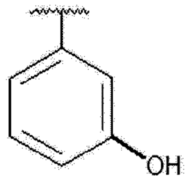,

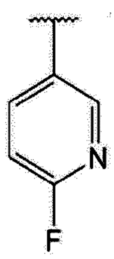, 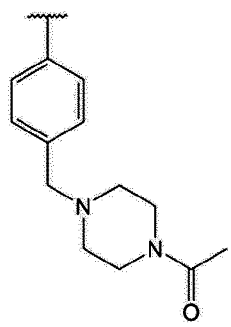, 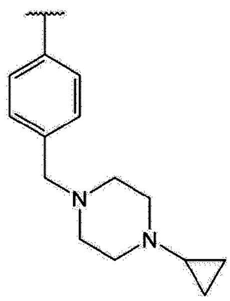, 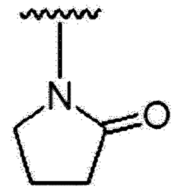, 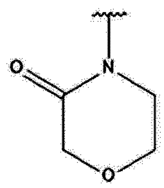,

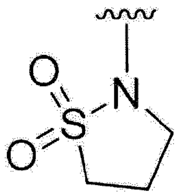, 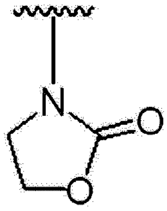, 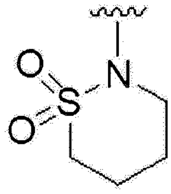, or 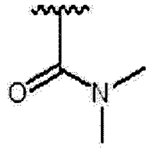,

and

$R^6$ is -H, a halogen, or a $C_{1-3}$ linear alkyl.

**13.** The compound of Formula 1, or the isomer thereof, the solvate thereof, the hydrate thereof, or the pharmaceutically acceptable salt thereof of claim 1, wherein the compound represented by Formula 1 comprises any one selected from the group consisting of the following compounds:

(1)(*S*)-4-(*sec*-butylamino)-2-((2-methoxy-4-(1-methyl-1*H*-pyrazol-5-yl)phenyl)amino)-7*H*-pyrrolo[2,3-d]pyrimidine-5-carbonitrile;
(2)4-(cyclopentylamino)-2-((2-methoxy-4-(1-methyl-1H-pyrazol-3-yl)phenyl)amino)-7*H*-pyrrolo[2,3-d]pyrimidine-5-carbonitrile;
(3)4-(cyclohexylamino)-2-((2-methoxy-4-(2-oxooxazolidin-3-yl)phenyl)amino)-7*H*-pyrrolo[2,3-d]pyrimidine-5-carbonitrile;
(4)$N^4$-cyclohexyl-$N^2$-(2-methoxy-4-(1-methyl-1*H*-pyrazol-5-yl)phenyl)-7*H*-pyrrolo[2,3-*d*]pyrimidine-2,4-diamine;
(5)5-chloro-$N^4$-cyclohexyl-$N^2$-(2-methoxy-4-(1-methyl-1*H*-pyrazol-5-yl)phenyl)-7*H*-pyrrolo[2,3-*d*]pyrimidine-2,4-diamine;
(6)$N^4$-cyclohexyl-$N^2$-(2-methoxy-4-(1-methyl-1*H*-pyrazol-5-yl)phenyl)-5-(trifluoromethyl)-7*H*-pyrrolo[2,3-*d*]pyrimidine-2,4-diamine;
(7)4-(cyclohexylamino)-2-((2-methoxy-4-(1-methyl-1*H*-pyrazol-5-yl)phenyl)amino)-7*H*-pyrrolo[2,3-*d*]pyrimidine-5-carbonitrile;
(8)4-((cyclopentylmethyl)amino)-2-((2-methoxy-4-(1-methyl-1*H*-pyrazol-5-yl)phenyl)amino)-7*H*-pyrrolo[2,3-d]pyrimidine-5-carbonitrile;
(9)2-((2-methoxy-4-(1-methyl-1*H*-pyrazol-5-yl)phenyl)amino)-4-(methylamino)-7*H*-pyrrolo[2,3-d]pyrimidine-5-carbonitrile;
(10)(*R*)-4-(*sec*-butylamino)-2-((2-methoxy-4-(1-methyl-1H-pyrazol-5-yl)phenyl)amino)-7*H*-pyrrolo[2,3-d]pyrimidine-5-carbonitrile;
(11)4-(butylamino)-2-((2-ethoxy-4-(4-methyl-4*H*-1,2,4-triazol-3-yl)phenyl)amino)-7*H*-pyrrolo[2,3-*d*]pyrimidine-5-carbonitrile;
(12)2-((2-methoxy-4-(1-methyl-1*H*-pyrazol-5-yl)phenyl)amino)-4-(neopentylamino)-7*H*-pyrrolo[2,3-*d*]pyrimidine-5-carbonitrile;
(13)2-((2-methoxy-4-(1-methyl-1*H*-pyrazol-5-yl)phenyl)amino)-4-((2-methoxyethyl)amino)-7*H*-pyrrolo[2,3-*d*]pyrimidine-5-carbonitrile;
(14)2-((2-ethoxy-4-(4-methyl-4*H*-1,2,4-triazol-3-yl)phenyl)amino)-4-((2-(methylsulfonyl)ethyl)amino)-7*H*-pyrrolo[2,3-*d*]pyrimidine-5-carbonitrile;
(15)2-((2-methoxy-4-(1-methyl-1*H*-pyrazol-5-yl)phenyl)amino)-4-((tetrahydro-2*H*-pyran-4-yl)amino)-7*H*-pyrrolo[2,3-*d*]pyrimidine-5-carbonitrile;
(16)4-(cyclopentylamino)-2-((2-methoxy-4-(1-methyl-1*H*-pyrazol-5-yl)phenyl)amino)-7*H*-pyrrolo[2,3-*d*]pyrimidine-5-carbonitrile;
(17)4-(cyclopentylamino)-2-((2-methoxy-4-(thiazol-2-yl)phenyl)amino)-7*H*-pyrrolo[2,3-d]pyrimidine-5-carbonitrile;
(18)4-(cyclohexylamino)-2-((4-(3,5-dimethylisoxazol-4-yl)-2-methoxyphenyl)amino)-7*H*-pyrrolo[2,3-*d*]pyrimidine-5-carbonitrile;
(19)4-(cyclohexylamino)-2-((2-methoxy-4-(1-(tetrahydro-2*H*-pyran-4-yl)-1*H*-pyrazol-4-yl)phenyl)amino)-7*H*-pyrrolo[2,3-*d*]pyrimidine-5-carbonitrile;
(20)4-(cyclohexylamino)-2-((2-methoxy-4-(1-(oxetan-3-yl)-1*H*-pyrazol-4-yl)phenyl)amino)-7*H*-pyrrolo[2,3-*d*]pyrimidine-5-carbonitrile;
(21)2-((4'-((4-acetylpiperazin-1-yl)methyl)-[1,1'-biphenyl]-4-yl)amino)-4-(cyclohexylamino)-7*H*-pyrrolo[2,3-d]pyrimidine-5-carbonitrile;

(22)4-(cyclohexylamino)-2-((4'-((4-cyclopropylpiperazin-1-yl)methyl)-[1,1'-biphenyl]-4-yl)amino)-7*H*-pyrrolo[2,3-*d*]pyrimidine-5-carbonitrile;
(23)2-((4'-((4-acetylpiperazin-1-yl)methyl)-3-methoxy-[1,1'-biphenyl]-4-yl)amino)-4-(cyclohexylamino)-7*H*-pyrrolo[2,3-*d*]pyrimidine-5-carbonitrile;
(24)4-(cyclohexylamino)-2-((4-(6-fluoropyridin-3-yl)-2-methoxyphenyl)amino)-7*H*-pyrrolo[2,3-*d*]pyrimidine-5-carbonitrile;
(25)4-(cyclohexylamino)-2-((2-methoxy-4-(1-methyl-1*H*-1,2,3-triazol-4-yl)phenyl)amino)-7*H*-pyrrolo[2,3-*d*]pyrimidine-5-carbonitrile;
(26)4-(cyclohexylamino)-2-((2-methoxy-4-(1-methyl-1*H*-1,2,4-triazol-5-yl)phenyl)amino)-7*H*-pyrrolo[2,3-*d*]pyrimidine-5-carbonitrile;
(27)4-(cyclohexylamino)-2-((2-methoxy-4-(1-methyl-1*H*-1,2,4-triazol-3-yl)phenyl)amino)-7*H*-pyrrolo[2,3-*d*]pyrimidine-5-carbonitrile;
(28)4-(cyclopentylamino)-2-((2-isobutoxy-4-(2-oxopyrrolidin-1-yl)phenyl)amino)-7*H*-pyrrolo[2,3-*d*]pyrimidine-5-carbonitrile;
(29)2-((4-(1,1-dioxidoisothiazolidin-2-yl)-3-fluorophenyl)amino)-4-((tetrahydro-2H-pyran-4-yl)amino)-7*H*-pyrrolo[2,3-*d*]pyrimidine-5-carbonitrile;
(30)4-(cyclopentylamino)-2-((2-methoxy-4-(1-methyl-1*H*-imidazol-5-yl)phenyl)amino)-7*H*-pyrrolo[2,3-*d*]pyrimidine-5-carbonitrile;
(31)4-(cyclohexylamino)-2-((4-(6,7-dihydro-5*H*-pyrrolo[2,1-*c*][1,2,4]triazol-3-yl)-2-methoxyphenyl)amino)-7*H*-pyrrolo[2,3-*d*]pyrimidine-5-carbonitrile;
(32)4-(((1s,4s)-4-hydroxy-4-methylcyclohexyl)oxy)-2-((2-methoxy-4-(2-oxopyrrolidin-1-yl)phenyl)amino)-7*H*-pyrrolo[2,3-*d*]pyrimidine-5-carbonitrile;
(33)4-(cyclopentylamino)-2-((4-(1-methyl-1*H*-pyrazol-5-yl)-2,3-dihydrobenzofuran-7-yl)amino)-7*H*-pyrrolo[2,3-*d*]pyrimidine-5-carbonitrile;
(34)4-(cyclohexylamino)-2-((4-(2,4-dimethyl-1*H*-imidazol-1-yl)-2-methoxyphenyl)amino)-7*H*-pyrrolo[2,3-*d*]pyrimidine-5-carbonitrile;
(35)4-(cyclopentylamino)-2-((2-methoxy-5-methyl-4-(1-methyl-1*H*-pyrazol-5-yl)phenyl)amino)-7*H*-pyrrolo[2,3-*d*]pyrimidine-5-carbonitrile;
(36)4-(cyclohexylamino)-2-((2-ethoxy-4-(1-methyl-1*H*-pyrazol-5-yl)phenyl)amino)-7*H*-pyrrolo[2,3-*d*]pyrimidine-5-carbonitrile;
(37)4-(cyclohexylamino)-2-((4-(1-methyl-1*H*-pyrazol-5-yl)phenyl)amino)-7*H*-pyrrolo[2,3-d]pyrimidine-5-carbonitrile;
(38)4-(cyclopentylamino)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(39)4-(cyclohexylamino)-2-((8-(1-methyl-1H-pyrazol-5-yl)-2,3-dihydrobenzo[b][1,4]dioxin-5-yl)amino)-7*H*-pyrrolo[2,3-*d*]pyrimidine-5-carbonitrile;
(40)2-((8-(1-methyl-1*H*-pyrazol-5-yl)-2,3-dihydrobenzo[b][1,4]dioxin-5-yl)amino)-4-((tetrahydro-2*H*-pyran-4-yl)amino)-7*H*-pyrrolo[2,3-*d*]pyrimidine-5-carbonitrile;
(41)2-((4'-((4-acetylpiperazin-1-yl)methyl)-3-methoxy-[1,1'-biphenyl]-4-yl)amino)-4-(cyclopentylamino)-7*H*-pyrrolo[2,3-*d*]pyrimidine-5-carbonitrile;
(42)4-(cyclopentylamino)-2-((4-(6-fluoropyridin-3-yl)-2-methoxyphenyl)amino)-7*H*-pyrrolo[2,3-d]pyrimidine-5-carbonitrile;
(43)4-(cyclopentylamino)-2-((2-methoxy-4-(1-methyl-1*H*-pyrazol-4-yl)phenyl)amino)-7*H*-pyrrolo[2,3-*d*]pyrimidine-5-carbonitrile;
(44)4-(cyclopentylamino)-2-((4-(3,5-dimethylisoxazol-4-yl)-2-methoxyphenyl)amino)-7*H*-pyrrolo[2,3-*d*]pyrimidine-5-carbonitrile;
(45)4-(cyclohexyloxy)-2-((2-methoxy-4-(1-methyl-1*H*-pyrazol-5-yl)phenyl)amino)-7*H*-pyrrolo[2,3-*d*]pyrimidine-5-carbonitrile;
(46)4-cyclopropyl-2-((2-ethoxy-4-(4-methyl-4*H*-1,2,4-triazol-3-yl)phenyl)amino)-7*H*-pyrrolo[2,3-*d*]pyrimidine-5-carbonitrile;
(47)1-(4-((4-(cyclopentylamino)-7*H*-pyrrolo[2,3-*d*]pyrimidin-2-yl)amino)-3-methoxyphenyl)pyrrolidin-2-one;
(48)1-(4-((4-(cyclohexylamino)-7*H*-pyrrolo[2,3-*d*]pyrimidin-2-yl)amino)-3-methoxyphenyl)pyrrolidin-2-one;
(49)4-((2-methoxyethyl)amino)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(50)4-((cyclopentylmethyl)amino)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(51)1-(3-methoxy-4-((4-(methylamino)-5-(trifluoromethyl)-7*H*-pyrrolo[2,3-*d*]pyrimidin-2-yl)amino)phenyl)pyrrolidin-2-one;

(52)1-(4-((4-(cyclopentylamino)-5-(trifluoromethyl)-7*H*-pyrrolo[2,3-*d*]pyrimidin-2-yl)amino)-3-methoxyphenyl)pyrrolidin-2-one;
(53)1-(4-((4-(cyclohexylamino)-5-(trifluoromethyl)-7*H*-pyrrolo[2,3-*d*]pyrimidin-2-yl)amino)-3-methoxyphenyl)pyrrolidin-2-one;
(54)2-((2-methoxy-4-(2-oxopyrrolidin-1-yl)phenyl)amino)-4-(methylamino)-7*H*-pyrrolo[2,3-d]pyrimidine-5-carbonitrile;
(55)4-(cyclopentylamino)-2-((2-methoxy-4-(2-oxopyrrolidin-1-yl)phenyl)amino)-7*H*-pyrrolo[2,3-*d*]pyrimidine-5-carbonitrile;
(56)4-(cyclohexylamino)-2-((2-methoxy-4-(2-oxopyrrolidin-1-yl)phenyl)amino)-7*H*-pyrrolo[2,3-d]pyrimidine-5-carbonitrile;
(57)4-(cyclopentylamino)-2-((4-(1,1-dioxido-1,2-thiazinan-2-yl)-3-fluorophenyl)amino)-7*H*-pyrrolo[2,3-*d*]pyrimidine-5-carbonitrile;
(58)2-((4-(1,1-dioxido-1,2-thiazinan-2-yl)-3-fluorophenyl)amino)-4-((tetrahydro-2*H*-pyran-4-yl)amino)-7*H*-pyrrolo[2,3-*d*]pyrimidine-5-carbonitrile;
(59)4-(cycloheptylamino)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(60)2-((8-(1-methyl-1*H*-pyrazol-5-yl)-2,3-dihydrobenzo[b][1,4]dioxin-5-yl)amino)-4-(neopentylamino)-7*H*-pyrrolo[2,3-*d*]pyrimidine-5-carbonitrile;
(61)4-(((1R,4S)-bicyclo[2.2.1]heptan-2-yl)amino)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(62)1-(4-((4-(cyclohexylamino)-3-(trifluoromethyl)-1*H*-pyrrolo[2,3-*b*]pyridin-6-yl)amino)-3-methoxyphenyl)pyrrolidin-2-one;
(63)4-(cyclopentylamino)-6-((2-methoxy-4-(2-oxopyrrolidin-1-yl)phenyl)amino)-1*H*-pyrrolo[2,3-*b*]pyridine-3-carbonitrile;
(64)4-(cyclohexylamino)-6-((2-methoxy-4-(2-oxopyrrolidin-1-yl)phenyl)amino)-1*H*-pyrrolo[2,3-*b*]pyridine-3-carbonitrile;
(65)4-(isopropylamino)-2-((2-methoxy-4-(2-oxopyrrolidin-1-yl)phenyl)amino)-7*H*-pyrrolo[2,3-*d*]pyrimidine-5-carbonitrile;
(66)4-(isobutylamino)-2-((2-methoxy-4-(2-oxopyrrolidin-1-yl)phenyl)amino)-7*H*-pyrrolo[2,3-*d*]pyrimidine-5-carbonitrile;
(67)4-(butylamino)-2-((2-methoxy-4-(2-oxopyrrolidin-1-yl)phenyl)amino)-7*H*-pyrrolo[2,3-*d*]pyrimidine-5-carbonitrile;
(68)(*S*)-4-(*sec*-butylamino)-2-((2-methoxy-4-(2-oxopyrrolidin-1-yl)phenyl)amino)-7*H*-pyrrolo[2,3-*d*]pyrimidine-5-carbonitrile;
(69)4-(cyclobutylamino)-2-((2-methoxy-4-(2-oxopyrrolidin-1-yl)phenyl)amino)-7*H*-pyrrolo[2,3-*d*]pyrimidine-5-carbonitrile;
(70)4-((cyclobutylmethyl)amino)-2-((2-methoxy-4-(2-oxopyrrolidin-1-yl)phenyl)amino)-7*H*-pyrrolo[2,3-*d*]pyrimidine-5-carbonitrile;
(71)4-((cyclopentylmethyl)amino)-2-((2-methoxy-4-(2-oxopyrrolidin-1-yl)phenyl)amino)-7*H*-pyrrolo[2,3-*d*]pyrimidine-5-carbonitrile;
(72)4-((1-methoxy-2-methylpropan-2-yl)amino)-2-((2-methoxy-4-(2-oxopyrrolidin-1-yl)phenyl)amino)-7*H*-pyrrolo[2,3-*d*]pyrimidine-5-carbonitrile;
(73)2-((2-methoxy-4-(2-oxopyrrolidin-1-yl)phenyl)amino)-4-(oxetan-3-ylamino)-7*H*-pyrrolo[2,3-*d*]pyrimidine-5-carbonitrile;
(74)2-((2-methoxy-4-(2-oxopyrrolidin-1-yl)phenyl)amino)-4-((tetrahydro-2*H*-pyran-4-yl)amino)-7*H*-pyrrolo[2,3-*d*]pyrimidine-5-carbonitrile;
(75)2-((8-(1-methyl-1*H*-pyrazol-5-yl)-2,3-dihydrobenzo[b][1,4]dioxin-5-yl)amino)-4-(methylamino)-7*H*-pyrrolo[2,3-d]pyrimidine-5-carbonitrile;
(76)4-(isopropylamino)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(77)2-((2-methoxy-4-(2-oxopyrrolidin-1-yl)phenyl)amino)-4-(neopentylamino)-7*H*-pyrrolo[2,3-*d*]pyrimidine-5-carbonitrile;
(78)4-(cyclohexylamino)-2-((2-ethoxy-4-(2-oxopyrrolidin-1-yl)phenyl)amino)-7*H*-pyrrolo[2,3-*d*]pyrimidine-5-carbonitrile;
(79)4-(cyclohexylamino)-2-((8-(2-oxopyrrolidin-1-yl)-2,3-dihydrobenzo[b][1,4]dioxin-5-yl)amino)-7*H*-pyrrolo[2,3-*d*]pyrimidine-5-carbonitrile;
(80)4-(cyclohexylamino)-2-((4-(2-oxopyrrolidin-1-yl)phenyl)amino)-7*H*-pyrrolo[2,3-*d*]pyrimidine-5-carbonitrile;
(81)4-(cyclohexylamino)-2-((2-methoxy-4-(3-oxomorpholino)phenyl)amino)-7*H*-pyrrolo[2,3-*d*]pyrimidine-5-carbonitrile;
(82)4-(cyclobutylamino)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(83)4-((2-(isopropylsulfonyl)ethyl)amino)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(84)2-((8-(1-methyl-1*H*-pyrazol-5-yl)-2,3-dihydrobenzo[b][1,4]dioxin-5-yl)amino)-4-((2-(methylsulfonyl)ethyl)amino)-7*H*-pyrrolo[2,3-*d*]pyrimidine-5-carbonitrile;
(85)2-((5-cyano-2-((8-(1-methyl-1*H*-pyrazol-5-yl)-2,3-dihydrobenzo[b][1,4]dioxin-5-yl)amino)-7*H*-pyrrolo[2,3-*d*]pyrimidin-4-yl)amino)-N,N-dimethylethane-1-sulfonamide;
(86)4-((5-chloro-4-(((1s,4*S*)-4-hydroxy-4-methylcyclohexyl)oxy)-7*H*-pyrrolo[2,3-d]pyrimidin-2-yl)amino)-*N*,*N*-dimethyl-3-(((*R*)-1,1,1-trifluoropropan-2-yl)oxy)benzamide;
(87)4-(cyclopentylamino)-6-((2-methoxy-4-(1-methyl-1*H*-pyrazol-5-yl)phenyl)amino)-1*H*-pyrrolo[2,3-*b*]pyridine-3-carbonitrile;
(88)4-(cyclopentylamino)-6-((8-(1-methyl-1*H*-pyrazol-5-yl)-2,3-dihydrobenzo[b][1,4]dioxin-5-yl)amino)-1*H*-pyrrolo[2,3-*b*]pyridine-3-carbonitrile;
(89)2-((8-(1-methyl-1*H*-pyrazol-4-yl)-2,3-dihydrobenzo[b][1,4]dioxin-5-yl)amino)-4-((tetrahydro-2*H*-pyran-4-yl)amino)-7*H*-pyrrolo[2,3-*d*]pyrimidine-5-carbonitrile;
(90)2-((2-methoxy-4-(4-methyl-4*H*-1,2,4-triazol-3-yl)phenyl)amino)-4-((tetrahydro-2*H*-pyran-4-yl)amino)-7*H*-pyrrolo[2,3-*d*]pyrimidine-5-carbonitrile;
(91)2-((4-(3,5-dimethylisoxazol-4-yl)-2-methoxyphenyl)amino)-4-((tetrahydro-2*H*-pyran-4-yl)amino)-7*H*-pyrrolo[2,3-*d*]pyrimidine-5-carbonitrile;
(92)2-((2-methoxy-4-(1-methyl-1*H*-pyrazol-4-yl)phenyl)amino)-4-((tetrahydro-2*H*-pyran-4-yl)amino)-7*H*-pyrrolo[2,3-*d*]pyrimidine-5-carbonitrile;
(93)2-((2-methoxy-4-(2-methyl-1*H*-imidazol-1-yl)phenyl)amino)-4-((tetrahydro-2*H*-pyran-4-yl)amino)-7*H*-pyrrolo[2,3-*d*]pyrimidine-5-carbonitrile;
(94)4-((2-methoxyethyl)amino)-2-((8-(1-methyl-1*H*-pyrazol-4-yl)-2,3-dihydrobenzo[b][1,4]dioxin-5-yl)amino)-7*H*-pyrrolo[2,3-*d*]pyrimidine-5-carbonitrile;
(95)2-((2-methoxy-4-(4-methyl-4*H*-1,2,4-triazol-3-yl)phenyl)amino)-4-((2-methoxyethyl)amino)-7*H*-pyrrolo[2,3-*d*]pyrimidine-5-carbonitrile;
(96)2-((4-(3,5-dimethylisoxazol-4-yl)-2-methoxyphenyl)amino)-4-((2-methoxyethyl)amino)-7*H*-pyrrolo[2,3-*d*]pyrimidine-5-carbonitrile;
(97)2-((2-methoxy-4-(1-methyl-1*H*-pyrazol-4-yl)phenyl)amino)-4-((2-methoxyethyl)amino)-7*H*-pyrrolo[2,3-*d*]pyrimidine-5-carbonitrile;
(98)2-((2-methoxy-4-(2-methyl-1*H*-imidazol-1-yl)phenyl)amino)-4-((2-methoxyethyl)amino) -7*H*-pyrrolo[2,3-*d*]pyrimidine-5-carbonitrile;
(99)4-(cyclopropylamino)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(100)4-((cyclobutylmethyl)amino)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(101)(*R*)-4-((1-methoxypropan-2-yl)amino)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(102)2-((4-(3,5-dimethylisoxazol-4-yl)-5-fluoro-2-methoxyphenyl)amino)-4-((tetrahydro-2*H*-pyran-4-yl)amino)-7*H*-pyrrolo[2,3-*d*]pyrimidine-5-carbonitrile;
(103)4-(cyclopentylamino)-2-((5-fluoro-2-methoxy-4-(1-methyl-1*H*-pyrazol-5-yl)phenyl)amino)-7*H*-pyrrolo[2,3-*d*]pyrimidine-5-carbonitrile;
(104)4-(cyclopentylamino)-2-((4-(3,5-dimethylisoxazol-4-yl)-5-fluoro-2-methoxyphenyl)amino)-7*H*-pyrrolo[2,3-*d*]pyrimidine-5-carbonitrile;
(105)4-(cyclohexylamino)-2-((5-fluoro-2-methoxy-4-(1-methyl-1*H*-pyrazol-5-yl)phenyl)amino)-7*H*-pyrrolo[2,3-*d*]pyrimidine-5-carbonitrile;
(106)$N^2$-(2-methoxy-4-(1-methyl-1*H*-pyrazol-5-yl)phenyl)-$N^4$-(tetrahydro-2*H*-pyran-4-yl)-5-(trifluoromethyl)-7*H*-pyrrolo[2,3-*d*]pyrimidine-2,4-diamine;
(107)$N^2$-(8-(1-methyl-1*H*-pyrazol-5-yl)-2,3-dihydrobenzo[b][1,4]dioxin-5-yl)-$N^4$-(tetrahydro-2*H*-pyran-4-yl)-5-(trifluoromethyl)-7*H*-pyrrolo[2,3-*d*]pyrimidine-2,4-diamine;
(108)1-(8-((4-((tetrahydro-2*H*-pyran-4-yl)amino)-5-(trifluoromethyl)-7*H*-pyrrolo[2,3-*d*]pyrimidin-2-yl)amino)-2,3-dihydrobenzo[b][1,4]dioxin-5-yl)pyrrolidin-2-one;
(109)4-((2,2-dimethyltetrahydro-2*H*-pyran-4-yl)amino)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(110)4-((3,3-difluorocyclopentyl)amino)-2-((2-methoxy-4-(1-methyl-1*H*-pyrazol-5-yl)phenyl)amino)-7*H*-pyrrolo[2,3-*d*]pyrimidine-5-carbonitrile;
(111)4-cyclopropyl-2-((2-methoxy-4-(1-methyl-1*H*-pyrazol-5-yl)phenyl)amino)-7*H*-pyrrolo[2,3-*d*]pyrimidine-5-carbonitrile;
(112)4-cyclopropyl-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(113)*N*4-cyclopentyl-*N*2-(2-methoxy-4-(1-methyl-1*H*-pyrazol-5-yl)phenyl)-7*H*-pyrrolo[2,3-*d*]pyrimidine-2,4-diamine;
(114)*N*4-cyclopentyl-*N*2-(8-(1-methyl-1*H*-pyrazol-5-yl)-2,3-dihydrobenzo[b][1,4]dioxin-5-yl)-7*H*-pyrrolo[2,3-*d*]pyrimidine-2,4-diamine;
(115)4-((2,2-dimethyltetrahydro-2*H*-pyran-4-yl)amino)-2-((2-methoxy-4-(1-methyl-1*H*-pyrazol-5-yl)phenyl)amino)-7*H*-pyrrolo[2,3-*d*]pyrimidine-5-carbonitrile;
(116)4-((3,3-difluorocyclopentyl)amino)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(117)*N*2-(2-methoxy-4-(4-methyl-4*H*-1,2,4-triazol-3-yl)phenyl)-*N*4-(tetrahydro-2*H*-pyran-4-yl)-5-(trifluoromethyl)-7*H*-pyrrolo[2,3-*d*]pyrimidine-2,4-diamine;
(118)*N*2-(2-methoxy-4-(1-methyl-1*H*-pyrazol-4-yl)phenyl)-*N*4-(tetrahydro-2*H*-pyran-4-yl)-5-(trifluoromethyl)-7*H*-pyrrolo[2,3-*d*]pyrimidine-2,4-diamine;
(119)2-((3-methoxy-4-(1-methyl-1*H*-pyrazol-5-yl)phenyl)amino)-4-((tetrahydro-2*H*-pyran-4-yl)amino)-7*H*-pyrrolo[2,3-*d*]pyrimidine-5-carbonitrile;
(120)4-(cyclopentylamino)-2-((3-methoxy-4-(1-methyl-1*H*-pyrazol-5-yl)phenyl)amino)-7*H*-pyrrolo[2,3-*d*]pyrimidine-5-carbonitrile;
(121)4-(cyclohexylamino)-2-((3-methoxy-4-(1-methyl-1*H*-pyrazol-5-yl)phenyl)amino)-7*H*-pyrrolo[2,3-*d*]pyrimidine-5-carbonitrile;
(122)2-((5-fluoro-2-methoxy-4-(1-methyl-1*H*-pyrazol-5-yl)phenyl)amino)-4-((tetrahydro-2*H*-pyran-4-yl)amino)-7*H*-pyrrolo[2,3-*d*]pyrimidine-5-carbonitrile;
(123)4-(cyclohexylamino)-2-((4-(3,5-dimethylisoxazol-4-yl)-5-fluoro-2-methoxyphenyl)amino)-7*H*-pyrrolo[2,3-*d*]pyrimidine-5-carbonitrile;
(124)2-((5-fluoro-2-methoxy-4-(1-methyl-1*H*-pyrazol-4-yl)phenyl)amino)-4-((tetrahydro-2*H*-pyran-4-yl)amino)-7*H*-pyrrolo[2,3-*d*]pyrimidine-5-carbonitrile;
(125)4-(cyclopentylamino)-2-((5-fluoro-2-methoxy-4-(1-methyl-1*H*-pyrazol-4-yl)phenyl)amino)-7*H*-pyrrolo[2,3-*d*]pyrimidine-5-carbonitrile;
(126)4-(cyclohexylamino)-2-((5-fluoro-2-methoxy-4-(1-methyl-1*H*-pyrazol-4-yl)phenyl)amino)-7*H*-pyrrolo[2,3-*d*]pyrimidine-5-carbonitrile;
(127)5-chloro-*N*4-cyclopentyl-*N*2-(2-methoxy-4-(1-methyl-1*H*-pyrazol-5-yl)phenyl)-7*H*-pyrrolo[2,3-*d*]pyrimidine-2,4-diamine;
(128)5-chloro-*N*4-cyclopentyl-*N*2-(8-(1-methyl-1*H*-pyrazol-5-yl)-2,3-dihydrobenzo[b][1,4]dioxin-5-yl)-7*H*-pyrrolo[2,3-*d*]pyrimidine-2,4-diamine;
(129)4-(cyclohex-1-en-1-yl)-2-((2-methoxy-4-(1-methyl-1*H*-pyrazol-5-yl)phenyl)amino)-7*H*-pyrrolo[2,3-*d*]pyrimidine-5-carbonitrile;
(130)4-(cyclohex-1-en-1-yl)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(131)*N*2-(4-(3,5-dimethylisoxazol-4-yl)-2-methoxyphenyl)-*N*4-(tetrahydro-2*H*-pyran-4-yl)-5-(trifluoromethyl)-7*H*-pyrrolo[2,3-*d*]pyrimidine-2,4-diamine;
(132)*N*4-cyclopentyl-*N*2-(2-methoxy-4-(1-methyl-1*H*-pyrazol-5-yl)phenyl)-5-(trifluoromethyl)-7*H*-pyrrolo[2,3-*d*]pyrimidine-2,4-diamine;
(133)*N*4-cyclopentyl-*N*2-(8-(1-methyl-1*H*-pyrazol-5-yl)-2,3-dihydrobenzo[b][1,4]dioxin-5-yl)-5-(trifluoromethyl)-7*H*-pyrrolo[2,3-*d*]pyrimidine-2,4-diamine;
(134)2-((3-methoxy-[1,1'-biphenyl]-4-yl)amino)-4-((tetrahydro-2*H*-pyran-4-yl)amino)-7*H*-pyrrolo[2,3-d]pyrimidine-5-carbonitrile;
(135)2-((3-methoxy-4-(1-methyl-1*H*-pyrazol-4-yl)phenyl)amino)-4-((tetrahydro-2*H*-pyran-4-yl)amino)-7*H*-pyrrolo[2,3-*d*]pyrimidine-5-carbonitrile;
(136)4-(cyclopentylamino)-2-((3-methoxy-4-(1-methyl-1*H*-pyrazol-4-yl)phenyl)amino)-7*H*-pyrrolo[2,3-*d*]pyrimidine-5-carbonitrile;
(137)4-(cyclohexylamino)-2-((3-methoxy-4-(1-methyl-1*H*-pyrazol-4-yl)phenyl)amino)-7*H*-pyrrolo[2,3-d]pyrimidine-5-carbonitrile;
(138)2-((4-(3,5-dimethylisoxazol-4-yl)-3-methoxyphenyl)amino)-4-((tetrahydro-2*H*-pyran-4-yl)amino)-7*H*-pyrrolo[2,3-*d*]pyrimidine-5-carbonitrile;
(139)4-(cyclopentylamino)-2-((4-(3,5-dimethylisoxazol-4-yl)-3-methoxyphenyl)amino)-7*H*-pyrrolo[2,3-*d*]pyrimidine-5-carbonitrile;
(140)4-(cyclohexylamino)-2-((4-(3,5-dimethylisoxazol-4-yl)-3-methoxyphenyl)amino)-7*H*-pyrrolo[2,3-*d*]pyrimidine-5-carbonitrile;
(141)2-((3'-hydroxy-3-methoxy-[1,1'-biphenyl]-4-yl)amino)-4-((tetrahydro-2*H*-pyran-4-yl)amino)-7*H*-pyrrolo[2,3-*d*]pyrimidine-5-carbonitrile;
(142)*N*-(2-((5-cyano-4-(cyclopentylamino)-7*H*-pyrrolo[2,3-*d*]pyrimidin-2-yl)amino)-5-(1-methyl-1*H*-pyrazol-5-yl)phenyl)acrylamide;
(143)4-((2,2-dimethyltetrahydro-2*H*-pyran-4-yl)amino)-2-((2-methoxy-4-(4-methyl-4*H*-1,2,4-triazol-3-yl)phenyl)amino)-7*H*-pyrrolo[2,3-*d*]pyrimidine-5-carbonitrile;
(144)4-((2,2-dimethyltetrahydro-2*H*-pyran-4-yl)amino)-2-((2-methoxy-4-(1-methyl-1*H*-pyrazol-4-yl)phenyl)amino)-7*H*-pyrrolo[2,3-*d*]pyrimidine-5-carbonitrile;
(145)4-((2,2-dimethyltetrahydro-2*H*-pyran-4-yl)amino)-2-((8-(1-methyl-1*H*-pyrazol-4-yl)-2,3-dihydrobenzo[b][1,4]dioxin-5-yl)amino)-7*H*-pyrrolo[2,3-*d*]pyrimidine-5-carbonitrile;
(146)4-(((1*R*,4*S*)-bicyclo[2.2.1]heptan-2-yl)amino)-2-((4-(3,5-dimethylisoxazol-4-yl)-2-methoxyphenyl)amino)-7*H*-pyrrolo[2,3-*d*]pyrimidine-5-carbonitrile;
(147)4-(((1*R*,4*S*)-bicyclo[2.2.1]heptan-2-yl)amino)-2-((8-(1-methyl-1*H*-pyrazol-4-yl)-2,3-dihydrobenzo[b][1,4]dioxin-5-yl)amino)-7*H*-pyrrolo[2,3-*d*]pyrimidine-5-carbonitrile;
(148)4-(((1*R*,4*S*)-bicyclo[2.2.1]heptan-2-yl)amino)-2-((8-(2-oxopyrrolidin-1-yl)-2,3-dihydrobenzo[b][1,4]dioxin-5-yl)amino)-7*H*-pyrrolo[2,3-*d*]pyrimidine-5-carbonitrile;
(149)4-(cycloheptylamino)-2-((4-(3,5-dimethylisoxazol-4-yl)-2-methoxyphenyl)amino)-7*H*-pyrrolo[2,3-*d*]pyrimidine-5-carbonitrile;
(150)4-(cycloheptylamino)-2-((8-(1-methyl-1*H*-pyrazol-4-yl)-2,3-dihydrobenzo[b][1,4]dioxin-5-yl)amino)-7*H*-pyrrolo[2,3-*d*]pyrimidine-5-carbonitrile;
(151)4-(cycloheptylamino)-2-((8-(2-oxopyrrolidin-1-yl)-2,3-dihydrobenzo[b][1,4]dioxin-5-yl)amino)-7*H*-pyrrolo[2,3-*d*]pyrimidine-5-carbonitrile;
(152)4-((3,3-difluorocyclohexyl)amino)-2-((2-methoxy-4-(1-methyl-1*H*-pyrazol-5-yl)phenyl)amino)-7*H*-pyrrolo[2,3-*d*]pyrimidine-5-carbonitrile;
(153)4-((3,3-difluorocyclohexyl)amino)-2-((8-(1-methyl-1*H*-pyrazol-5-yl)-2,3-dihydrobenzo[b][1,4]dioxin-5-yl)amino)-7*H*-pyrrolo[2,3-*d*]pyrimidine-5-carbonitrile;
(154)4-((3,3-difluorocyclohexyl)amino)-2-((2-methoxy-4-(4-methyl-4*H*-1,2,4-triazol-3-yl)phenyl)amino)-7*H*-pyrrolo[2,3-*d*]pyrimidine-5-carbonitrile;
(155)4-(cyclopentylamino)-2-((4-(1,1-dioxido-1,2-thiazinan-2-yl)-2-methoxyphenyl)amino)-7*H*-pyrrolo[2,3-*d*]pyrimidine-5-carbonitrile;
(156)4-(cyclopentylamino)-2-((4-(1,1-dioxido-1,2-thiazinan-2-yl)phenyl)amino)-7*H*-pyrrolo[2,3-d]pyrimidine-5-carbonitrile;
(157)4-(cyclopentylamino)-2-((4-(1,1-dioxidoisothiazolidin-2-yl)-2-methoxyphenyl)amino)-7*H*-pyrrolo[2,3-*d*]pyrimidine-5-carbonitrile;
(158)4-(cyclopentylamino)-2-((4-(1,1-dioxidoisothiazolidin-2-yl)phenyl)amino)-7*H*-pyrrolo[2,3-d]pyrimidine-5-carbonitrile;
(159)4-((3,3-difluorocyclopentyl)amino)-2-((8-(1-methyl-1*H*-pyrazol-4-yl)-2,3-dihydrobenzo[b][1,4]dioxin-5-yl)amino)-7*H*-pyrrolo[2,3-*d*]pyrimidine-5-carbonitrile;
(160)2-((8-(1-methyl-1*H*-pyrazol-5-yl)-2,3-dihydrobenzo[b][1,4]dioxin-5-yl)amino)-4-(oxetan-3-ylamino)-7*H*-pyrrolo[2,3-d]pyrimidine-5-carbonitrile;
(161)2-((2-methoxy-4-(1-methyl-1*H*-pyrazol-5-yl)phenyl)amino)-4-(oxetan-3-ylamino)-7*H*-pyrrolo[2,3-*d*]pyrimidine-5-carbonitrile;
(162)4-((2,2-dimethyltetrahydro-2*H*-pyran-4-yl)amino)-2-((3-methoxy-4-(1-methyl-1*H*-pyrazol-5-yl)phenyl)amino)-7*H*-pyrrolo[2,3-*d*]pyrimidine-5-carbonitrile;
(163)2-((4-(1,1-dioxido-1,2-thiazinan-2-yl)-2-methoxyphenyl)amino)-4-((tetrahydro-2*H*-pyran-4-yl)amino)-7*H*-pyrrolo[2,3-*d*]pyrimidine-5-carbonitrile;
(164)2-((4-(1,1-dioxido-1,2-thiazinan-2-yl)phenyl)amino)-4-((tetrahydro-2*H*-pyran-4-yl)amino)-7*H*-pyrrolo[2,3-*d*]pyrimidine-5-carbonitrile;
(165)2-((4-(1,1-dioxidoisothiazolidin-2-yl)-2-methoxyphenyl)amino)-4-((tetrahydro-2*H*-pyran-4-yl)amino)-7*H*-pyrrolo[2,3-*d*]pyrimidine-5-carbonitrile;
(166)2-((4-(1,1-dioxidoisothiazolidin-2-yl)phenyl)amino)-4-((tetrahydro-2*H*-pyran-4-yl)amino)-7*H*-pyrrolo[2,3-*d*]pyrimidine-5-carbonitrile;
(167)(*R*)-2-((2-methoxy-4-(2-methyl-1*H*-imidazol-1-yl)phenyl)amino)-4-((1-methoxypropan-2-yl)amino)-7H-pyrrolo[2,3-*d*]pyrimidine-5-carbonitrile ;
(168)(*R*)-2-((2-methoxy-4-(1-methyl-1*H*-pyrazol-5-yl)phenyl)amino)-4-((1-methoxypropan-2-yl)amino)-7*H*-pyrrolo[2,3-*d*]pyrimidine-5-carbonitrile;
(169)(*R*)-4-((1-methoxypropan-2-yl)amino)-2-((8-(1-methyl-1*H*-pyrazol-4-yl)-2,3-dihydrobenzo[b][1,4]dioxin-5-yl)amino)-7*H*-pyrrolo[2,3-*d*]pyrimidine-5-carbonitrile;
(170)2-((2,3-dihydrobenzo[b][1,4]dioxin-5-yl)amino)-4-((tetrahydro-2*H*-pyran-4-yl)amino)-7*H*-pyrrolo[2,3-*d*]pyrimidine-5-carbonitrile;
(171)2-((2,3-dihydrobenzo[b][1,4]dioxin-5-yl)amino)-4-((2,2-dimethyltetrahydro-2*H*-pyran-4-yl)amino)-7*H*-pyrrolo[2,3-*d*]pyrimidine-5-carbonitrile;
(172)$N^4$-(2,2-dimethyltetrahydro-2*H*-pyran-4-yl)-$N^2$-(8-(1-methyl-1*H*-pyrazol-5-yl)-2,3-dihydrobenzo[b][1,4]dioxin-5-yl)-5-(trifluoromethyl)-7*H*-pyrrolo[2,3-*d*]pyrimidine-2,4-diamine;
(173)4-(cyclopentylamino)-2-((4-(1,1-dioxidoisothiazolidin-2-yl)-3-fluorophenyl)amino)-7*H*-pyrrolo[2,3-*d*]pyrimidine-5-carbonitrile;
(174)2-(3-methoxy-4-((4-((tetrahydro-2*H*-pyran-4-yl)amino)-5-(trifluoromethyl)-7*H*-pyrrolo[2,3-d]pyrimidin-2-yl)amino)phenyl)isothiazolidine 1,1-dioxide;
(175)2-(3-methoxy-4-((4-((tetrahydro-2*H*-pyran-4-yl)amino)-5-(trifluoromethyl)-7*H*-pyrrolo[2,3-d]pyrimidin-2-yl)amino)phenyl)-1,2-thiazinane 1,1-dioxide;
(176)2-(4-((4-(cyclopentylamino)-5-(trifluoromethyl)-7*H*-pyrrolo[2,3-*d*]pyrimidin-2-yl)amino)-3-methoxyphenyl)isothiazolidine 1,1-dioxide;
(177)2-(4-((4-(cyclopentylamino)-5-(trifluoromethyl)-7*H*-pyrrolo[2,3-*d*]pyrimidin-2-yl)amino)-3-methoxyphenyl)-1,2-thiazinane1,1-dioxide.

**14.** A pharmaceutical composition for use in preventing or treating cancer, comprising, as an active ingredient, the compound represented by Formula 1, or the stereoisomer, the hydrate thereof, or the pharmaceutically acceptable salt thereof defined in claim 1.

**15.** The pharmaceutical composition of claim 14, wherein the compound exhibits an inhibitory activity against one or more protein kinases selected from the group consisting of JAK2, SNARK, TTK, YSK4, JNK1, FLT3, PRKCE, CAMKK1, JNK3, TYK2, RSK2, CAMKK2, ULK3, ULK1, RSK4, TRKB, LRRK2, JNK3, AAK1, GAK, SBK1, TYK2, CAMK2D, MAP3K2, KIT, CSNK1D, CSNK1E, MEK4, RIOK1, DYRK1B, PKN2, FLT3, JNK2, RIPK5, MEK3, ABL1, MAPKAPK2, GRK4, and SRPK3.

**16.** The pharmaceutical composition of claim 14, wherein the compound exhibits inhibitory activity against a TTK kinase.

**17.** The pharmaceutical composition of claim 14, wherein the cancer comprises one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphocytic leukemia, basal cell carcinoma, ovarian epithelial cancer, ovarian germ cell cancer, male breast cancer, brain cancer, pituitary adenomas, multiple myeloma, gallbladder cancer, biliary tract cancer, colorectal cancer, chronic myelogenous leukemia, chronic lymphocytic leukemia, retinoblastoma, choroidal melanoma, ampulla of vater cancer, bladder cancer, peritoneal cancer, parathyroid cancer, adrenal cancer, nasal and paranasal sinus cancer, non-small cell lung cancer, tongue cancer, astrocytoma, small cell lung cancer, childhood brain cancer, pediatric lymphoma, pediatric leukemia, small intestine cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureter cancer, urethral cancer, cancer of unknown primary site, gastric lymphoma, gastric cancer, gastric carcinoid tumors, gastrointestinal stromal tumors, Wilms cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid tumors, vaginal cancer, spinal carcinoma, acoustic neuroma, pancreatic cancer, salivary gland cancer, Kaposi’s sarcoma, Paget’s disease, tonsillar cancer, squamous cell carcinoma, lung adenocarcinoma, lung cancer, lung squamous cell carcinoma, skin cancer, anal cancer, rhabdomyosarcoma, laryngeal cancer, pleural cancer, blood cancer, and thymic carcinoma.

**INTERNATIONAL SEARCH REPORT**

International application No.
**PCT/KR2020/003558**

A. CLASSIFICATION OF SUBJECT MATTER
***C07D 471/04(2006.01)i, A61K 31/437(2006.01)i, A61K 31/519(2006.01)i, C07D 487/04(2006.01)i, A61P 35/00(2006.01)i, A23L 29/00(2016.01)i, A23L 33/10(2016.01)i***
According to International Patent Classification (IPC) or to both national classification and IPC

B. FIELDS SEARCHED

Minimum documentation searched (classification system followed by classification symbols)
C07D 471/04; A23L 33/10; A61K 31/416; A61K 31/437; C07D 231/56; C07D 519/00; A61K 31/519; C07D 487/04; A61P 35/00; A23L 29/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal), STN (Registry, Caplus), Google & Keywords: pyrrolo[2,3-d]pyrimidine, TTK kinase, cancer

C. DOCUMENTS CONSIDERED TO BE RELEVANT

| Category* | Citation of document, with indication, where appropriate, of the relevant passages | Relevant to claim No. |
|---|---|---|
| X | KR 10-1896568 B1 (DAEGU-GYEONGBUK MEDICAL INNOVATION FOUNDATION et al.) 10 September 2018<br>See claims 1, 5, 7-9; paragraphs [0284], [0413]. | 1-17 |
| A | WO 2012-123745 A1 (CANCER RESEARCH TECHNOLOGY LIMITED) 20 September 2012<br>See abstract; claims 1, 12-14, 17, 20. | 1-17 |
| A | WO 2016-010886 A1 (SIGNAL PHARMACEUTICALS, LLC.) 21 January 2016<br>See claims 1, 17, 25. | 1-17 |
| A | WO 2011-123937 A1 (UNIVERSITY HEALTH NETWORK) 13 October 2011<br>See claims 1, 44. | 1-17 |
| A | WO 2015-128676 A1 (CANCER RESEARCH TECHNOLOGY LIMITED) 03 September 2015<br>See the entire document. | 1-17 |
| PX | LEE, Y. et al. Abstract 2211: Discovery of orally available and potent MPS1(TTK) kinase inhibitors for anti-cancer drugs. In: AACR Annual Meeting 2019. American Association for Cancer Research. July 2019, vol. 79, no. 13, supplement, page 2211<br>See the entire document.<br>This document is a known document declaring exceptions to lack of novelty by the applicant. | 1-17 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 JUNE 2020 (30.06.2020) | **30 JUNE 2020 (30.06.2020)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |

Form PCT/ISA/210 (second sheet) (January 2015)

**INTERNATIONAL SEARCH REPORT**

International application No.
**PCT/KR2020/003558**

C (Continuation). DOCUMENTS CONSIDERED TO BE RELEVANT

| Category* | Citation of document, with indication, where appropriate, of the relevant passages | Relevant to claim No. |
|---|---|---|
| PX | CHOI, H. G. et al. Novel Mps1/TTK kinase inhibitors for Triple-negative breast cancer (TNBC). In: 2019 Fall International Convention of The Pharmaceutical Society of Korea. Intergrating Vison and Mission for Pharmaceutical Science Communities. Yeosu Expo Convention Center, Korea. 14 October 2019, page 87<br>See the entire document.<br>This document is a known document declaring exceptions to lack of novelty by the applicant. | 1-17 |
| PX | LEE, Y. et al. Novel MPS1/TTK Kinase Inhibitors for Breast Cancer. 한양대 ERICA BK21 플러스 바이오나노융합인력사업단(Hanyang University ERICA BK21 Plus Graduate Program for Bionano Fusion Technology). 16 October 2019<br>See the entire document.<br>This document is a known document declaring exceptions to lack of novelty by the applicant. | 1-17 |
| PX | LEE, Y. et al. Novel Mps1/TTK kinase inhibitors for triple-negative breast cancer (TNBC). In: 2020 제5회 하이원 신약개발 심포지아(2020 5th HIGH1 the Development of New Pharmaceuticals Symposium). 08 January 2020<br>See the entire document.<br>This document is a known document declaring exceptions to lack of novelty by the applicant. | 1-17 |

Form PCT/ISA/210 (continuation of second sheet) (January 2015)

INTERNATIONAL SEARCH REPORT
Information on patent family members

International application No.
PCT/KR2020/003558

| Patent document cited in search report | Publication date | Patent family member | Publication date |
|---|---|---|---|
| KR 10-1896568 B1 | 10/09/2018 | AU 2018-239798 A1 | 31/10/2019 |
| | | BR 112019019872 A2 | 22/04/2020 |
| | | CA 3057591 A1 | 27/09/2018 |
| | | CN 110662745 A | 07/01/2020 |
| | | EP 3604304 A1 | 05/02/2020 |
| | | WO 2018-174650 A1 | 27/09/2018 |
| | | WO 2018-174650 A9 | 27/09/2018 |
| WO 2012-123745 A1 | 20/09/2012 | AU 2012-228090 A1 | 03/10/2013 |
| | | AU 2012-228090 B2 | 30/03/2017 |
| | | CA 2830143 A1 | 20/09/2012 |
| | | CA 2830143 C | 01/10/2019 |
| | | CN 103517903 A | 15/01/2014 |
| | | CN 103517903 B | 01/03/2017 |
| | | EP 2686318 A1 | 22/01/2014 |
| | | EP 2686318 B1 | 09/03/2016 |
| | | ES 2573778 T3 | 10/06/2016 |
| | | JP 2014-508185 A | 03/04/2014 |
| | | JP 6027989 B2 | 16/11/2016 |
| | | US 2013-0345181 A1 | 26/12/2013 |
| | | US 9371319 B2 | 21/06/2016 |
| WO 2016-010886 A1 | 21/01/2016 | AU 2015-289929 A1 | 02/03/2017 |
| | | CA 2955009 A1 | 21/01/2016 |
| | | CN 106715427 A | 24/05/2017 |
| | | EA 201790189 A1 | 30/11/2017 |
| | | EP 3169686 A1 | 24/05/2017 |
| | | EP 3169686 A4 | 24/01/2018 |
| | | JP 2017-520603 A | 27/07/2017 |
| | | KR 10-2017-0024120 A | 06/03/2017 |
| | | US 2016-0008365 A1 | 14/01/2016 |
| | | US 2017-0173024 A1 | 22/06/2017 |
| | | US 9623028 B2 | 18/04/2017 |
| WO 2011-123937 A1 | 13/10/2011 | None | |
| WO 2015-128676 A1 | 03/09/2015 | AU 2015-221956 A1 | 07/07/2016 |
| | | AU 2015-221956 B2 | 09/05/2019 |
| | | CA 2939058 A1 | 03/09/2015 |
| | | CN 106459035 A | 22/02/2017 |
| | | CN 106459035 B | 06/04/2018 |
| | | EP 3110816 A1 | 04/01/2017 |
| | | EP 3110816 B1 | 19/06/2019 |
| | | EP 3575299 A1 | 04/12/2019 |
| | | ES 2739148 T3 | 29/01/2020 |
| | | JP 2017-506661 A | 09/03/2017 |
| | | JP 6440728 B2 | 19/12/2018 |
| | | RU 2016137789 A | 03/04/2018 |
| | | RU 2016137789 A3 | 23/10/2018 |

Form PCT/ISA/210 (patent family annex) (January 2015)

**INTERNATIONAL SEARCH REPORT**
Information on patent family members

International application No.
**PCT/KR2020/003558**

| Patent document cited in search report | Publication date | Patent family member | Publication date |
|---|---|---|---|
| | | RU 2693460 C2 | 03/07/2019 |
| | | US 10399974 B2 | 03/09/2019 |
| | | US 2016-0362409 A1 | 15/12/2016 |
| | | US 2018-0194761 A1 | 12/07/2018 |
| | | US 9902721 B2 | 27/02/2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ABRIEU A et al.** *Cell,* 2001, vol. 106, 83-93